# EUROPEAN PATENT APPLICATION

(11) **EP 1 792 988 A2**
(43) Date of publication of application: **06.06.2007**
(21) Application number: 06023711.2
(22) Date of filing: 17.03.1999
(51) Int. Cl.: C12N 15/12, A61K 38/17, C07K 14/47, C07K 16/18, A61K 35/14

(54) **Compounds and methods for therapy and diagnosis of lung cancer**

(30) Priority: 18.03.1998 US 40802; 18.03.1998 US 40984; 27.07.1998 US 123912; 27.07.1998 US 123933
(62) Divisional of application: 99912607.1
(71) Applicant: CORIXA CORPORATION, Seattle, WA 98104 (US)
(72) Inventor: Reed, Steven G., Bellevue, WA 98805 (US); Wang, Tongtong, Medina, WA 98039 (US)
(74) Representative: Walker, Ross Thomson

(57) **Abstract**

Compounds and methods for the treatment and diagnosis of lung cancer are provided. The inventive compounds include polypeptides containing at least a portion of a lung tumour protein. Vaccines and pharmaceutical compositions for immunotherapy of lung cancer comprising such peptides, or DNA molecules encoding such polypeptides, are also provided, together with DNA molecules for preparing the inventive polypeptides.

## Description

### TECHNICAL FIELD

The present invention relates generally to compositions and methods for the treatment and diagnosis of lung cancer. The invention is more specifically related to nucleotide sequences that are preferentially expressed in lung tumor tissue, together with polypeptides encoded by such nucleotide sequences. The inventive nucleotide sequences and polypeptides may be used in vaccines and pharmaceutical compositions for the treatment and diagnosis of lung cancer.

### BACKGROUND OF THE INVENTION

Lung cancer is the primary cause of cancer death among both men and women in the U.S., with an estimated 172,000 new cases being reported in 1994. The five-year survival rate among all lung cancer patients, regardless of the stage of disease at diagnosis, is only 13%. This contrasts with a five-year survival rate of 46% among cases detected while the disease is still localized. However, only 16% of lung cancers are discovered before the disease has spread.

Early detection is difficult since clinical symptoms are often not seen until the disease has reached an advanced stage. Currently, diagnosis is aided by the use of chest x-rays, analysis of the type of cells contained in sputum and fiberoptic examination of the bronchial passages. Treatment regimens are determined by the type and stage of the cancer, and include surgery, radiation therapy and/or chemotherapy. In spite of considerable research into therapies for the disease, lung cancer remains difficult to treat.

Accordingly, there remains a need in the art for improved vaccines, treatment methods and diagnostic techniques for lung cancer.

### SUMMARY OF THE INVENTION

Briefly stated, the present invention provides compounds and methods for the therapy of lung cancer. In a first aspect, isolated polynucleotide molecules encoding lung tumor polypeptides are provided, such polynucleotide molecules comprising a nucleotide sequence selected from the group consisting of: (a) sequences provided in SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168 and 171; (b) sequences complementary to a sequence provided in SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168 and 171; and (b) sequences that hybridize to a sequence of (a) or (b) under moderately stringent conditions.

In a second aspect, isolated polypeptides are provided that comprise at least an immunogenic portion of a lung tumor protein or a variant thereof. In specific embodiments, such polypeptides comprise an amino acid sequence encoded by a polynucleotide molecule comprising a nucleotide sequence selected from the group consisting of (a) sequences recited in SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168 and 171; (b) sequences complementary to a sequence provided in SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168 and 171; and (c) sequences that hybridize to a sequence of (a) or (b) under moderately stringent conditions.

In related aspects, expression vectors comprising the inventive polynucleotide molecules, together with host cells transformed or transfected with such expression vectors are provided. In preferred embodiments, the host cells are selected from the group consisting of *E. coli,* yeast and mammalian cells.

In another aspect, fusion proteins comprising a first and a second inventive polypeptide or, alternatively, an inventive polypeptide and a known lung tumor antigen, are provided.

The present invention further provides pharmaceutical compositions comprising one or more of the above polypeptides, fusion proteins or polynucleotide molecules and a physiologically acceptable carrier, together with vaccines comprising one or more such polypeptides, fusion proteins or polynucleotide molecules in combination with an immune response enhancer.

In related aspects, the present invention provides methods for inhibiting the development of lung cancer in a patient, comprising administering to a patient an effective amount of at least one of the above pharmaceutical compositions and/or vaccines.

Additionally, the present invention provides methods for immunodiagnosis of lung cancer, together with kits for use in such methods. Polypeptides are disclosed which comprise at least an immunogenic portion of a lung tumor protein or a variant of said protein that differs only in conservative substitutions and/or modifications, wherein the lung tumor protein comprises an amino acid sequence encoded by a polynucleotide molecule having a sequence selected from the group consisting of nucleotide sequences recited in SEQ ID NO: 1-109, 111, 113, 115-151, 153, 154, 157, 158, 160, 162-164, 167, 168 and 171, and variants thereof. Such polypeptides may be usefully employed in the diagnosis and monitoring of lung cancer.

In one specific aspect of the present invention, methods are provided for detecting lung cancer in a patient, comprising: (a) contacting a biological sample obtained from a patient with a binding agent that is capable of binding to one of the above polypeptides; and (b) detecting in the sample a protein or polypeptide that binds to the binding agent. In preferred embodiments, the binding agent is an antibody, most preferably a monoclonal antibody.

In related aspects, methods are provided for monitoring the progression of lung cancer in a patient, comprising: (a) contacting a biological sample obtained from a patient with a binding agent that is capable of binding to one of the above polypeptides; (b) determining in the sample an amount of a protein or polypeptide that binds to the binding agent; (c) repeating steps (a) and (b); and comparing the amounts of polypeptide detected in steps (b) and (c).

Within related aspects, the present invention provides antibodies, preferably monoclonal antibodies, that bind to the inventive polypeptides, as well as diagnostic kits comprising such antibodies, and methods of using such antibodies to inhibit the development of lung cancer.

The present invention further provides methods for detecting lung cancer comprising: (a) obtaining a biological sample from a patient; (b) contacting the sample with a first and a second oligonucleotide primer in a polymerase chain reaction, at least one of the oligonucleotide primers being specific for a polynucleotide molecule that encodes one of the above polypeptides; and (c) detecting in the sample a polynucleotide sequence that amplifies in the presence of the first and second oligonucleotide primers. In a preferred embodiment, at least one of the oligonucleotide primers comprises at least about 10 contiguous nucleotides of a polynucleotide molecule including a sequence selected from the group consisting of SEQ ID NO: 1-109, 111, 113, 115-151, 153, 154, 157, 158, 160, 162-164, 167, 168 and 171.

In a further aspect, the present invention provides a method for detecting lung cancer in a patient comprising: (a) obtaining a biological sample from the patient; (b) contacting the sample with an oligonucleotide probe specific for a polynucleotide molecule that encodes one of the above polypeptides; and (c) detecting in the sample a polynucleotide sequence that hybridizes to the oligonucleotide probe. Preferably, the oligonucleotide probe comprises at least about 15 contiguous nucleotides of a polynucleotide molecule having a partial sequence selected from the group consisting of SEQ ID NO: 1-109, 111, 113, 115-151, 153, 154,157, 158, 160,162-164, 167, 168 and 171.

In related aspects, diagnostic kits comprising the above oligonucleotide probes or primers are provided.

In yet a further aspect, methods for the treatment of lung cancer in a patient are provided, the methods comprising obtaining PBMC from the patient, incubating the PBMC with a polypeptide of the present invention (or a polynucleotide that encodes such a polypeptide) to provide incubated T cells and administering the incubated T cells to the patient. The present invention additionally provides methods for the treatment of lung cancer that comprise incubating antigen presenting cells with a polypeptide of the present invention (or a polynucleotide that encodes such a polypeptide) to provide incubated antigen presenting cells and administering the incubated antigen presenting cells to the patient. In certain embodiments, the antigen presenting cells are selected from the group consisting of dendritic cells and macrophages. Compositions for the treatment of lung cancer comprising T cells or antigen presenting cells that have been incubated with a polypeptide or polynucleotide of the present invention are also provided. These and other aspects of the present invention will become apparent upon reference to the following detailed description. All references disclosed herein are hereby incorporated by reference in their entirety as if each was incorporated individually.

### DETAILED DESCRIPTION OF THE INVENTION

As noted above, the present invention is generally directed to compositions and methods for the therapy and diagnosis of lung cancer. The compositions described herein include polypeptides, fusion proteins and polynucleotide molecules. Also included within the present invention are molecules (such as an antibody or fragment thereof) that bind to the inventive polypeptides. Such molecules are referred to herein as "binding agents."

In one aspect, the subject invention discloses polypeptides comprising an immunogenic portion of a human lung tumor protein, wherein the lung tumor protein includes an amino acid sequence encoded by a polynucleotide molecule including a sequence selected from the group consisting of (a) nucleotide sequences recited in SEQ ID NO: 1-109,, 111, 113 115-151, 153, 154,157, 158, 160, 162-164, 167, 168 and 171, (b) the complements of said nucleotide sequences, and (c) variants of such sequences. As used herein, the term "polypeptide" encompasses amino acid chains of any length, including full length proteins, wherein the amino acid residues are linked by covalent peptide bonds. Thus, a polypeptide comprising a portion of one of the above lung tumor proteins may consist entirely of the portion, or the portion may be present within a larger polypeptide that contains additional sequences. The additional sequences may be derived from the native protein or may be heterologous, and such sequences may (but need not) be immunoreactive and/or antigenic. As detailed below, such polypeptides may be isolated from lung tumor tissue or prepared by synthetic or recombinant means.

As used herein, an "immunogenic portion" of a lung tumor protein is a portion that is capable of eliciting an immune response in a patient inflicted with lung cancer and as such binds to antibodies present within sera from a lung cancer patient. Such immunogenic portions generally comprise at least about 5 amino acid residues, more preferably at least about 10, and most preferably at least about 20 amino acid residues. Immunogenic portions of the proteins described herein may be identified in antibody binding assays. Such assays may generally be performed using any of a variety of means known to those of ordinary skill in the art, as described, for example, in Harlow and Lane, Antibodies: A Laboratory Manual, Cold Spring Harbor Laboratory, Cold Spring Harbor, NY, 1988. For example, a polypeptide may be immobilized on a solid support (as described below) and contacted with patient sera to allow binding of antibodies within the sera to the immobilized polypeptide. Unbound sera may then be removed and bound antibodies detected using, for example, ¹²⁵I-labeled Protein A. Alternatively, a polypeptide may be used to generate monoclonal and polyclonal antibodies for use in detection of the polypeptide in blood or other fluids of lung cancer patients. Methods for preparing and identifying immunogenic portions of antigens of known sequence are well known in the art and include those summarized in Paul. Fundamental Immunology, 3rd ed., Raven Press, 1993, pp. 243-247.

The term "polynucleotide(s)," as used herein, means a single or double-stranded polymer of deoxyribonucleotide or ribonucleotide bases and includes DNA and corresponding RNA molecules, including HnRNA and mRNA molecules, both sense and anti-sense strands, and comprehends cDNA, genomic DNA and recombinant DNA, as well as wholly or partially synthesized polynucleotides. An HnRNA molecule contains introns and corresponds to a DNA molecule in a generally one-to-one manner. An mRNA molecule corresponds to an HnRNA and DNA molecule from which the introns have been excised. A polynucleotide may consist of an entire gene, or any portion thereof. Operable anti-sense polynucleotides may comprise a fragment of the corresponding polynucleotide, and the definition of "polynucleotide" therefore includes all such operable anti-sense fragments.

The compositions and methods of the present invention also encompass variants of the above polypeptides and polynucleotides. A polypeptide "variant," as used herein, is a polypeptide that differs from the recited polypeptide only in conservative substitutions and/or modifications, such that the therapeutic, antigenic and/or immunogenic properties of the polypeptide are retained. In a preferred embodiment, variant polypeptides differ from an identified sequence by substitution, deletion or addition of five amino acids or fewer. Such variants may generally be identified by modifying one of the above polypeptide sequences, and evaluating the antigenic properties of the modified polypeptide using, for example, the representative procedures described herein. Polypeptide variants preferably exhibit at least about 70%, more preferably at least about 90% and most preferably at least about 95% identity (determined as describe below) to the identified polypeptides.

As used herein, a "conservative substitution" is one in which an amino acid is substituted for another amino acid that has similar properties, such that one skilled in the art of peptide chemistry would expect the secondary structure and hydropathic nature of the polypeptide to be substantially unchanged. In general, the following groups of amino acids represent conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

Variants may also, or alternatively, contain other modifications, including the deletion or addition of amino acids that have minimal influence on the antigenic properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification or identification of the polypeptide (e.g., poly-His), or to enhance binding of the polypeptide to a solid support. For example, a polypeptide may be conjugated to an immunoglobulin Fc region.

A nucleotide "variant" is a sequence that differs from the recited nucleotide sequence in having one or more nucleotide deletions, substitutions or additions. Such modifications may be readily introduced using standard mutagenesis techniques, such as oligonucleotide-directed site-specific mutagenesis as taught, for example, by Adelman et al. (DNA, 2:183, 1983). Nucleotide variants may be naturally occurring allelic variants, or non-naturally occurring variants. Variant nucleotide sequences preferably exhibit at least about 70%, more preferably at least about 80% and most preferably at least about 90% identity (determined as described below) to the recited sequence.

The antigens provided by the present invention include variants that are encoded by polynucleotide sequences which are substantially homologous to one or more of the polynucleotide sequences specifically recited herein. "Substantial homology," as used herein, refers to polynucleotide sequences that are capable of hybridizing under moderately stringent conditions. Suitable moderately stringent conditions include prewashing in a solution of 5X SSC, 0.5% SDS, 1.0 mM EDTA (pH 8.0); hybridizing at 50°C-65°C, 5X SSC, overnight or, in the event of cross-species homology, at 45°C with 0.5X SSC; followed by washing twice at 65°C for 20 minutes with each of 2X, 0.5X and 0.2X SSC containing 0.1% SDS. Such hybridizing polynucleotide sequences are also within the scope of this invention, as are nucleotide sequences that, due to code degeneracy, encode an immunogenic polypeptide that is encoded by a hybridizing polynucleotide sequence.

Two nucleotide or polypeptide sequences are said to be "identical" if the sequence of nucleotides or amino acid residues in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) Fast and sensitive multiple sequence alignments on a microcomputer CABIOS 5:151-153; Myers, E.W. and Muller W. (1988) Optimal alignments in linear space CABIOS 4:11-17; Robinson, E.D. (1971) Comb. Theor 11:105; Santou, N. Nes, M. (1987) The neighbor joining method. A new method for reconstructing phylogenetic trees Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Rapid similarity searches of nucleic acid and protein data banks Proc. Natl. Acad., Sci. USA 80:726-730.

Preferably, the "percentage of sequence identity" is determined by comparing two optimally aligned sequences over a window of comparison of at least 20 positions, wherein the portion of the polynucleotide sequence in the comparison window may comprise additions or deletions (i.e. gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e. the window size) and multiplying the results by 100 to yield the percentage of sequence identity.

Also included in the scope of the present invention are alleles of the genes encoding the nucleotide sequences recited in herein. As used herein, an "allele" or "allellic sequence" is an alternative form of the gene which may result from at least one mutation in the nucleic acid sequence. Alleles may result in altered mRNAs or polypeptides whose structure or function may or may not be altered. Any given gene may have none, one, or many allelic forms. Common mutational changes which give rise to alleles are generally ascribed to natural deletions, additions, or substitutions of nucleotides. Each of these types of changes may occur alone or in combination with the others, one or more times in a given sequence.

For lung tumor polypeptides with immunoreactive properties, variants may, alternatively, be identified by modifying the amino acid sequence of one of the above polypeptides, and evaluating the immunoreactivity of the modified polypeptide. For lung tumor polypeptides useful for the generation of diagnostic binding agents, a variant may be identified by evaluating a modified polypeptide for the ability to generate antibodies that detect the presence or absence of lung cancer. Such modified sequences may be prepared and tested using, for example, the representative procedures described herein.

The lung tumor polypeptides of the present invention, and polynucleotide molecules encoding such polypeptides, may be isolated from lung tumor tissue using any of a variety of methods well known in the art. Polynucleotide sequences corresponding to a gene (or a portion thereof) encoding one of the inventive lung tumor proteins may be isolated from a lung tumor cDNA library using a subtraction technique as described in detail below. Examples of such polynucleotide sequences are provided in SEQ ID NO: 1-109,111,113 115-151, 153, 154, 157, 158, 160, 162-164, 167, 168 and 171. Partial polynucleotide sequences thus obtained may be used to design oligonucleotide primers for the amplification of full-length polynucleotide sequences from a human genomic DNA library or from a lung tumor cDNA library in a polymerase chain reaction (PCR), using techniques well known in the art (see, for example, Mullis et al., Cold Spring Harbor Symp. Quant. Biol. 51:263, 1987; Erlich ed., PCR Technology, Stockton Press, NY, 1989). For this approach, sequence-specific primers may be designed based on the nucleotide sequences provided herein and may be purchased or synthesized.

An amplified portion may be used to isolate a full length gene from a suitable library (*e.g.,* a lung tumor cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (*e.g.,* by nick-translation or end-labeling with ³²P) using well known techniques. A bacterial or bacteriophage library is then screened by hybridizing filters containing denatured bacterial colonies (or lawns containing phage plaques) with the labeled probe (*see* Sambrook et al., Molecular Cloning: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences are then assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using techniques well known in the art (*see,* for example, Mullis et al., Cold Spring Harbor Symp. Quant. Biol. 51:263, 1987; Erlich ed., PCR Technology, Stockton Press, NY, 1989), and software well known in the art may also be employed. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR (*see* Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Additional techniques include capture PCR (Lagerstrom et al., PCR Methods Applic. 1:111-19, 1991) and walking PCR (Parker et al., Nucl. Acids. Res. 19:3055-60, 1991). Transcription-Mediated Amplification, or TMA is another method that may be utilized for the amplification of DNA, rRNA, or mRNA, as described in Patent No. PCT/US91/03184. This autocatalytic and isothermic non-PCR based method utilizes two primers and two enzymes: RNA polymerase and reverse transcriptase. One primer contains a promoter sequence for RNA polymerase. In the first amplification, the promoter-primer hybridizes to the target rRNA at a defined site. Reverse transcriptase creates a DNA copy of the target rRNA by extension from the 3'end of the promoter-primer. The RNA in the resulting complex is degraded and a second primer binds to the DNA copy. A new strand of DNA is synthesized from the end of the primer by reverse transcriptase creating double stranded DNA. RNA polymerase recognizes the promoter sequence in the DNA template and initiates transcription. Each of the newly synthesized RNA amplicons re-enters the TMA process and serves as a template for a new round of replication leading to the expotential expansion of the RNA amplicon. Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (e.g., NCBI BLAST searches), and such ESTs may be.used to generate a contiguous full length sequence.

Once a polynucleotide sequence encoding a polypeptide is obtained, the polypeptide may be produced recombinantly by inserting the polynucleotide sequence into an expression vector and expressing the polypeptide in an appropriate host. Any of a variety of expression vectors known to those of ordinary skill in the art may be employed to express recombinant polypeptides of this invention. Expression may be achieved in any appropriate host cell that has been transformed or transfected with an expression vector containing a polynucleotide molecule that encodes the recombinant polypeptide. Suitable host cells include prokaryotes, yeast, insect and higher eukaryotic cells. Preferably, the host cells employed are *E. coli,* yeast or a mammalian cell line, such as COS or CHO cells. The polynucleotide sequences expressed in this manner may encode naturally occurring polypeptides, portions of naturally occurring polypeptides, or other variants thereof. Supernatants from suitable host/vector systems which secrete the recombinant polypeptide may first be concentrated using a commercially available filter. The concentrate may then be applied to a suitable purification matrix, such as an affinity matrix or ion exchange resin. Finally, one or more reverse phase HPLC steps can be employed to further purify the recombinant polypeptide.

The lung tumor polypeptides disclosed herein may also be generated by synthetic means. In particular, synthetic polypeptides having fewer than about 100 amino acids, and generally fewer than about 50 amino acids, may be generated using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain (see, for example, Merrifield, J. Am. Chem. Soc. 85:2149-2146, 1963). Equipment for automated synthesis of polypeptides is commercially available from suppliers such as Perkin Elmer/Applied BioSystems Division (Foster City, CA), and may be operated according to the manufacturer's instructions.

In addition, lung tumor antigens may be identified by T cell expression cloning. One source of tumor specific T cells is from surgically excised tumors from human patients. In one method for isolating and characterizing tumor specific T cells, the excised tumor is minced and enzymatically digested for several hours to release tumor cells and infiltrating lymphocytes (tumor infiltrating T cells, or TILs). The cells are washed in HBSS buffer and passed over a Ficoll (100%/75%/HBSS) discontinuous gradient to separate tumor cells and lymphocytes from non-viable cells. Two bands are harvested from the interfaces; the upper band at the 75%/HBSS interface contains predominantly tumor cells, while the lower band at the 100%/75%/HBSS interface contains a majority of lymphocytes. The TILs are expanded in culture by techniques well known in the art, but preferably in culture media supplemented with 10 ng/ml IL-7 and 100 U/ml IL-2, or alternatively, cultured and expanded in tissue culture plates that have been pre-adsorbed with anti-CD3 monoclonal antibody (OKT3). The resulting TIL cultures are analyzed by FACS to confirm that the vast majority are CD8+ T cells (>90% of gated population).

In addition, the tumor cells are also expanded in culture using standard techniques well known in the art to establish a tumor cell line, which is later confirmed to be lung carcinoma cells by immunohistochemical analysis. The tumor cell line is transduced with a retroviral vector to express human CD80. The tumor cell line is further characterized by FACS analysis to confirm the strong expression levels of CD80, class I and II MHC molecules.

The specificity of the TIL lines to lung tumor is confirmed by INF-γ and/or TNF-α cytokine release assays. For example, TIL cells from day 21 cultures are co-cultured with either autologous or allogeneic tumor cells, EBV-immortalized LCL, or control cell lines Daudi and K562 and the culture supernatant monitored by ELISA for the presence of cytokines. The expression of these specific cytokines in the presence of tumor or negative control cells indicates whether the TIL lines are tumor specific and potentially recognizing tumor antigen presented by the autologous MHC molecules.

The characterized tumor-specific TIL lines can be expanded and cloned by methods well known in the art. For example, the TIL lines may be expanded to suitable numbers for T cell expression cloning by using soluble anti-CD3 antibody in culture with irradiated EBV transformed LCLs and PBL feeder cells in the presence of 20 U/ml IL-2. Clones from the expanded TIL lines can be generated by standard limiting dilution techniques. In particular, TIL cells are seeded at 0.5 cells/well in a 96-well U bottom plate and stimulated with CD-80-transduced autologous tumor cells, EBV transformed LCL, and PBL feeder cells in the presence of 50 U/ml IL-2. These clones may be further analyzed for tumor specificity by ⁵¹Cr microcytotoxicity and IFN-γ bioassays. Additionally, the MHC restriction element recognized by the TIL clones may be determined by antibody blocking studies well known in the art.

The CTL lines or clones described above may be employed to identify tumor specific antigens. For example, autologous fibroblasts or LCL from a patient may be transfected or transduced with polynucleotide fragments derived from a lung tumor cDNA library to generate target cells expressing tumor polypeptides. The target cells expressing tumor polypeptides in the context of MHC will be recognized by the CTL line or clone resulting in T-cell activation, which can be monitored by cytokine detection assays. The tumor gene being expressed by the target cell and recognized by the tumor-specific CTL is then isolated by techniques described above. In general, regardless of the method of preparation, the polypeptides disclosed herein are prepared in an isolated, substantially pure form (*i.e.,* the polypeptides are homogenous as determined by amino acid composition and primary sequence analysis). Preferably, the polypeptides are at least about 90% pure, more preferably at least about 95% pure and most preferably at least about 99% pure. In certain preferred embodiments, described in more detail below, the substantially pure polypeptides are incorporated into pharmaceutical compositions or vaccines for use in one or more of the methods disclosed herein.

In a related aspect, the present invention provides fusion proteins comprising a first and a second inventive polypeptide or, alternatively, a polypeptide of the present invention and a known lung tumor antigen, together with variants of such fusion proteins. The fusion proteins of the present invention may (but need not) include a linker peptide between the first and second polypeptides.

A polynucleotide sequence encoding a fusion protein of the present invention is constructed using known recombinant DNA techniques to assemble separate polynucleotide sequences encoding the first and second polypeptides into an appropriate expression vector. The 3' end of a DNA sequence encoding the first polypeptide is ligated, with or without a peptide linker, to the 5' end of a DNA sequence encoding the second polypeptide so that the reading frames of the sequences are in phase to permit mRNA translation of the two DNA sequences into a single fusion protein that retains the biological activity of both the first and the second polypeptides.

A peptide linker sequence may be employed to separate the first and the second polypeptides by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may be from 1 to about 50 amino acids in length. Peptide sequences are not required when the first and second polypeptides have non-essential N-terminal amino acid regions that can be used to separate the functional domains and prevent steric interference.

The ligated polynucleotide sequences are operably linked to suitable transcriptional or translational regulatory elements. The regulatory elements responsible for expression of polynucleotide are located only 5' to the DNA sequence encoding the first polypeptides. Similarly, stop codons require to end translation and transcription termination signals are only present 3' to the DNA sequence encoding the second polypeptide.

Fusion proteins are also provided that comprise a polypeptide of the present invention together with an unrelated immunogenic protein. Preferably the immunogenic protein is capable of eliciting a recall response. Examples of such proteins include tetanus, tuberculosis and hepatitis proteins (see, for example, Stoute et al. New Engl. J Med., 336:86-91 (1997)).

Polypeptides of the present invention that comprise an immunogenic portion of a lung tumor protein may generally be used for therapy of lung cancer, wherein the polypeptide stimulates the patient's own immune response to lung tumor cells. The present invention thus provides methods for using one or more of the compounds described herein (which may be polypeptides, polynucleotide molecules or fusion proteins) for immunotherapy of lung cancer in a patient. As used herein, a "patient" refers to any warm-blooded animal, preferably a human. A patient may be afflicted with disease, or may be free of detectable disease. Accordingly, the compounds disclosed herein may be used to treat lung cancer or to inhibit the development of lung cancer. The compounds are preferably administered either prior to or following surgical removal of primary tumors and/or treatment by administration of radiotherapy and conventional chemotherapeutic drugs.

In these aspects, the inventive polypeptide is generally present within a pharmaceutical composition or a vaccine. Pharmaceutical compositions may comprise one or more polypeptides, each of which may contain one or more of the above sequences (or variants thereof), and a physiologically acceptable carrier. The vaccines may comprise one or more such polypeptides and a non-specific immune-response enhancer, wherein the non-specific immune response enhancer is capable of eliciting or enhancing an immune response to an exogenous antigen. Examples of non-specific-immune response enhancers include adjuvants, biodegradable microspheres (e.g., polylactic galactide) and liposomes (into which the polypeptide is incorporated). Pharmaceutical compositions and vaccines may also contain other epitopes of lung tumor antigens, either incorporated into a fusion protein as described above *(i.e.,* a single polypeptide that contains multiple epitopes) or present within a separate polypeptide.

Alternatively, a pharmaceutical composition or vaccine may contain polynucleotide encoding one or more of the above polypeptides and/or fusion proteins, such that the polypeptide is generated *in situ.* In such pharmaceutical compositions and vaccines, the polynucleotide may be present within any of a variety of delivery systems known to those of ordinary skill in the art, including nucleic acid expression systems, bacteria and viral expression systems. Appropriate nucleic acid expression systems contain the necessary polynucleotide sequences for expression in the patient (such as a suitable promoter). Bacterial delivery systems involve the administration of a bacterium (such as *Bacillus-Calmette-Guerrin*) that expresses an epitope of a lung cell antigen on its cell surface. In a preferred embodiment, the polynucleotides may be introduced using a viral expression system (e.g., vaccinia or other pox virus, retrovirus, or adenovirus), which may involve the use of a non-pathogenic (defective), replication competent virus. Suitable systems are disclosed, for example, in Fisher-Hoch et al., PNAS 86:317-321, 1989; Flexner et al., Ann. N.Y. Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., PNAS 91:215-219, 1994; Kass-Eisler et al., PNAS 90:11498-11502, 1993; Guzman et al., Circulation 88:2838-2848, 1993; and Guzman et al., Cir. Res. 73:1202-1207, 1993. Techniques for incorporating polynucleotide into such expression systems are well known to those of ordinary skill in the art. The polynucleotides may also be "naked," as described, for example, in published PCT application WO 90/11092, and Ulmer et al., Science 259:1745-1749, 1993, reviewed by Cohen, Science 259:1691-1692, 1993. The uptake of naked polynucleotides may be increased by coating the polynucleotides onto biodegradable beads, which are efficiently transported into the cells.

Routes and frequency of administration, as well as dosage, will vary from individual to individual and may parallel those currently being used in immunotherapy of other diseases. In general, the pharmaceutical compositions and vaccines may be administered by injection (e.g., intracutaneous, intramuscular, intravenous or subcutaneous), intranasally (*e.g.,* by aspiration) or orally. Between 1 and 10 doses may be administered over a 3-24 week period. Preferably, 4 doses are administered, at an interval of 3 months, and booster administrations may be given periodically thereafter. Alternate protocols may be appropriate for individual patients. A suitable dose is an amount of polypeptide or polynucleotide that is effective to raise an immune response (cellular and/or humoral) against lung tumor cells in a treated patient. A suitable immune response is at least 10-50% above the basal (*i.e.,* untreated) level. In general, the amount of polypeptide present in a dose (or produced *in situ* by the polynucleotide molecule(s) in a dose) ranges from about 1 pg to about 100 mg per kg of host, typically from about 10 pg to about 1 mg, and preferably from about 100 pg to about 1 µg. Suitable dose sizes will vary with the size of the patient, but will typically range from about 0.01 mL to about 5 mL.

While any suitable carrier known to those of ordinary skill in the art may be employed in the pharmaceutical compositions of this invention, the type of carrier will vary depending on the mode of administration. For parenteral administration, such as subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres *(e.g.,* polylactic glycolide) may also be employed as carriers for the pharmaceutical compositions of this invention. Suitable biodegradable microspheres are disclosed, for example, in U.S. Patent Nos. 4,897,268 and 5,075,109.

Any of a variety of immune-response enhancers may be employed in the vaccines of this invention. For example, an adjuvant may be included. Most adjuvants contain a substance designed to protect the antigen from rapid catabolism, such as aluminum hydroxide or mineral oil, and a nonspecific stimulator of immune response, such as lipid A, *Bordella pertussis* or *Mycobacterium tuberculosis.* Such adjuvants are commercially available as, for example, Freund's Incomplete Adjuvant and Complete Adjuvant (Difco Laboratories, Detroit, MI) and Merck Adjuvant 65 (Merck and Company, Inc., Rahway, NJ).Polypeptides and polynucleotides disclosed herein may also be employed in adoptive immunotherapy for the treatment of cancer. Adoptive immunotherapy may be broadly classified into either active or passive immunotherapy. In active immunotherapy, treatment relies on the *in vivo* stimulation of the endogenous host immune system to react against tumors with the administration of immune response-modifying agents (for example, tumor vaccines, bacterial adjuvants, and/or cytokines).

In passive immunotherapy, treatment involves the delivery of biologic reagents with established tumor-immune reactivity (such as effector cells or antibodies) that can directly or indirectly mediate antitumor effects and does not necessarily depend on an intact host immune system. Examples of effector cells include T lymphocytes (for example, CD8+ cytotoxic T-lymphocyte, CD4+ T-helper, gamma/delta T lymphocytes, tumor-infiltrating lymphocytes), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Patent No. 4,918,164), for passive immunotherapy.

The predominant method of procuring adequate numbers of T-cells for adoptive immunotherapy is to grow immune T-cells *in vitro.* Culture conditions for expanding single antigen-specific T-cells to several billion in number with retention of antigen recognition *in vivo* are well known in the art. These *in vitro* culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T cell cultures in order to generate sufficient number of cells for immunotherapy. In particular, antigen-presenting cells, such as dendritic, macrophage, monocyte, fibroblast, or B-cells, may be pulsed with immunoreactive polypeptides, or polynucleotide sequence(s) may be introduced into antigen presenting cells, using a variety of standard techniques well known in the art. For example, antigen presenting cells may be transfected or transduced with a polynucleotide sequence, wherein said sequence contains a promoter region appropriate for increasing expression, and can be expressed as part of a recombinant virus or other expression system. Several viral vectors may be used to transduce an antigen presenting cell, including pox virus, vaccinia virus, and adenovirus; also, antigen presenting cells may be transfected with polynucleotide sequences disclosed herein by a variety of means, including gene-gun technology, lipid-mediated delivery, electroporation, osmotic shock, and particlate delivery mechanisms, resulting in efficient and acceptable expression levels as determined by one of ordinary skill in the art. For cultured T-cells to be effective in therapy, the cultured T-cells must be able to grow and distribute widely and to survive long term *in vivo.* Studies have demonstrated that cultured T-cells can be induced to grow *in vivo* and to survive long term in substantial numbers by repeated stimulation with antigen supplemented with IL-2 (see, for example, Cheever, M., et al, "Therapy With Cultured T Cells: Principles Revisited, " Immunological Reviews, 157:177, 1997).

The polypeptides disclosed herein may also be employed to generate and/or isolate tumor-reactive T-cells, which can then be administered to the patient. In one technique, antigen-specific T-cell lines may be generated by *in vivo* immunization with short peptides corresponding to immunogenic portions of the disclosed polypeptides. The resulting antigen specific CD8+ CTL clones may be isolated from the patient, expanded using standard tissue culture techniques, and returned to the patient.

Alternatively, peptides corresponding to immunogenic portions of the polypeptides may be employed to generate tumor reactive T cell subsets by selective *in vitro* stimulation and expansion of autologous T cells to provide antigen-specific T cells which may be subsequently transferred to the patient as described, for example, by Chang et al, (Crit. Rev. Oncol. Hematol., 22(3), 213, 1996). Cells of the immune system, such as T cells, may be isolated from the peripheral blood of a patient, using a commercially available cell separation system, such as CellPro Incorporated's (Bothell, WA) CEPRATE^{™} system (see U.S. Patent No. 5,240,856; U.S. Patent No. 5,215,926; WO 89/06280; WO 91/16116 and WO 92/07243). The separated cells are stimulated with one or more of the immunoreactive polypeptides contained within a delivery vehicle, such as a microsphere, to provide antigen-specific T cells. The population of tumor antigen-specific T cells is then expanded using standard techniques and the cells are administered back to the patient.

In other embodiments, T-cell and/or antibody receptors specific for the polypeptides disclosed herein can be cloned, expanded, and transferred into other vectors or effector cells for use in adoptive immunotherapy. In particular, T cells may be transfected with the appropriate genes to express the variable domains from tumor specific monoclonal antibodies as the extracellular recognition elements and joined to the T cell receptor signaling chains, resulting in T cell activation, specific lysis, and cytokine release. This enables the T cell to redirect its specificity in an MHC-independent manner. See for example, Eshhar, Z., Cancer Immunol Immunother, 45(3-4):131-6, 1997 and Hwu, P., et al, Cancer Res, 55(15):3369-73, 1995. Another embodiment may include the transfection of tumor antigen specific alpha and beta T cell receptor chains into alternate T cells, as in Cole, DJ, et al, Cancer Res, 55(4):748-52, 1995.

In a further embodiment, syngeneic or autologous dendritic cells may be pulsed with peptides corresponding to at least an immunogenic portion of a polypeptide disclosed herein. The resulting antigen-specific dendritic cells may either be transferred into a patient, or employed to stimulate T cells to provide antigen-specific T cells which may, in turn, be administered to a patient. The use of peptide-pulsed dendritic cells to generate antigen-specific T cells and the subsequent use of such antigen-specific T cells to eradicate tumors in a murine model has been demonstrated by Cheever et al, Immunological Reviews, 157: 177, 1997).

Furthermore, vectors expressing the disclosed polynucleotides may be introduced into stem cells taken from the patient and clonally propagated *in vitro* for autologous transplant back into the same patient.

Additionally, vectors expressing the disclosed polynucleotides may be introduced into stem cells taken from the patient and clonally propagated *in vitro* for autologous transplant back into the same patient. Polypeptides and fusion proteins of the present invention may also, or alternatively, be used to generate binding agents, such as antibodies or fragments thereof, that are capable of detecting metastatic human lung tumors. Binding agents of the present invention may generally be prepared using methods known to those of ordinary skill in the art, including the representative procedures described herein. Binding agents are capable of differentiating between patients with and without lung cancer, using the representative assays described herein. In other words, antibodies or other binding agents raised against a lung tumor protein, or a suitable portion thereof, will generate a signal indicating the presence of primary or metastatic lung cancer in at least about 20% of patients afflicted with the disease, and will generate a negative signal indicating the absence of the disease in at least about 90% of individuals without primary or metastatic lung cancer. Suitable portions of such lung tumor proteins are portions that are able to generate a binding agent that indicates the presence of primary or metastatic lung cancer in substantially all *(i.e.,* at least about 80%, and preferably at least about 90%) of the patients for which lung cancer would be indicated using the full length protein, and that indicate the absence of lung cancer in substantially all of those samples that would be negative when tested with full length protein. The representative assays described below, such as the two-antibody sandwich assay, may generally be employed for evaluating the ability of a binding agent to detect metastatic human lung tumors.

The ability of a polypeptide prepared as described herein to generate antibodies capable of detecting primary or metastatic human lung tumors may generally be evaluated by raising one or more antibodies against the polypeptide (using, for example, a representative method described herein) and determining the ability of such antibodies to detect such tumors in patients. This determination may be made by assaying biological samples from patients with and without primary or metastatic lung cancer for the presence of a polypeptide that binds to the generated antibodies. Such test assays may be performed, for example, using a representative procedure described below. Polypeptides that generate antibodies capable of detecting at least 20% of primary or metastatic lung tumors by such procedures are considered to be useful in assays for detecting primary or metastatic human lung tumors. Polypeptide specific antibodies may be used alone or in combination to improve sensitivity.

Polypeptides capable of detecting primary or metastatic human lung tumors may be used as markers for diagnosing lung cancer or for monitoring disease progression in patients. In one embodiment, lung cancer in a patient may be diagnosed by evaluating a biological sample obtained from the patient for the level of one or more of the above polypeptides, relative to a predetermined cut-off value. As used herein, suitable "biological samples" include blood, sera, urine and/or lung secretions.

The level of one or more of the above polypeptides may be evaluated using any binding agent specific for the polypeptide(s). A "binding agent," in the context of this invention, is any agent (such as a compound or a cell) that binds to a polypeptide as described above. As used herein, "binding" refers to a noncovalent association between two separate molecules (each of which may be free *(i.e.,* in solution) or present on the surface of a cell or a solid support), such that a "complex" is formed. Such a complex may be free or immobilized (either covalently or noncovalently) on a support material. The ability to bind may generally be evaluated by determining a binding constant for the formation of the complex. The binding constant is the value obtained when the concentration of the complex is divided by the product of the component concentrations. In general, two compounds are said to "bind" in the context of the present invention when the binding constant for complex formation exceeds about 10³ L/mol. The binding constant may be determined using methods well known to those of ordinary skill in the art.

Any agent that satisfies the above requirements may be a binding agent. For example, a binding agent may be a ribosome with or without a peptide component, an RNA molecule or a peptide. In a preferred embodiment, the binding partner is an antibody, or a fragment thereof. Such antibodies may be polyclonal, or monoclonal. In addition, the antibodies may be single chain, chimeric, CDR-grafted or humanized. Antibodies may be prepared by the methods described herein and by other methods well known to those of skill in the art.

There are a variety of assay formats known to those of ordinary skill in the art for using a binding partner to detect polypeptide markers in a sample. *See, e.g.,* Harlow and Lane, Antibodies: A Laboratory Manual, Cold Spring Harbor Laboratory, 1988. In a preferred embodiment, the assay involves the use of binding partner immobilized on a solid support to bind to and remove the polypeptide from the remainder of the sample. The bound polypeptide may then be detected using a second binding partner that contains a reporter group. Suitable second binding partners include antibodies that bind to the binding partner/polypeptide complex. Alternatively, a competitive assay may be utilized, in which a polypeptide is labeled with a reporter group and allowed to bind to the immobilized binding partner after incubation of the binding partner with the sample. The extent to which components of the sample inhibit the binding of the labeled polypeptide to the binding partner is indicative of the reactivity of the sample with the immobilized binding partner.

The solid support may be any material known to those of ordinary skill in the art to which the antigen may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the antigen and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (*see, e.g.,* Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a second antibody (containing a reporter group) capable of binding to a different site on the polypeptide is added. The amount of second antibody that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20™ (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i.e.,* incubation time) is that period of time that is sufficient to detect the presence of polypeptide within a sample obtained from an individual with lung cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1 % Tween 20™. The second antibody, which contains a reporter group, may then be added to the solid support. Preferred reporter groups include enzymes (such as horseradish peroxidase), substrates, cofactors, inhibitors, dyes, radionuclides, luminescent groups, fluorescent groups and biotin. The conjugation of antibody to reporter group may be achieved using standard methods known to those of ordinary skill in the art.

The second antibody is then incubated with the immobilized antibody-polypeptide complex for an amount of time sufficient to detect the bound polypeptide. An appropriate amount of time may generally be determined by assaying the level of binding that occurs over a period of time. Unbound second antibody is then removed and bound second antibody is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of lung cancer, the signal detected from the reporter group that remains bound to the solid support is generally compared to a signal that corresponds to a predetermined cut-off value. In one preferred embodiment, the cut-off value is the average mean signal obtained when the immobilized antibody is incubated with samples from patients without lung cancer. In general, a sample generating a signal that is three standard deviations above the predetermined cut-off value is considered positive for lung cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-7. Briefly, in this embodiment, the cut-off value may be determined from a plot of pairs of true positive rates *(i.e.,* sensitivity) and false positive rates (100%-specificity) that correspond to each possible cut-off value for the diagnostic test result. The cut-off value on the plot that is the closest to the upper left-hand corner (*i.e.*, the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for lung cancer.

In a related embodiment, the assay is performed in a flow-through or strip test format, wherein the antibody is immobilized on a membrane, such as nitrocellulose. In the flow-through test, polypeptides within the sample bind to the immobilized antibody as the sample passes through the membrane. A second, labeled antibody then binds to the antibody-polypeptide complex as a solution containing the second antibody flows through the membrane. The detection of bound second antibody may then be performed as described above. In the strip test format, one end of the membrane to which antibody is bound is immersed in a solution containing the sample. The sample migrates along the membrane through a region containing second antibody and to the area of immobilized antibody. Concentration of second antibody at the area of immobilized antibody indicates the presence of lung cancer. Typically, the concentration of second antibody at that site generates a pattern, such as a line, that can be read visually. The absence of such a pattern indicates a negative result. In general, the amount of antibody immobilized on the membrane is selected to generate a visually discernible pattern when the biological sample contains a level of polypeptide that would be sufficient to generate a positive signal in the two-antibody sandwich assay, in the format discussed above. Preferably, the amount of antibody immobilized on the membrane ranges from about 25 ng to about 1 µg, and more preferably from about 50 ng to about 500 ng. Such tests can typically be performed with a very small amount of biological sample.

Of course, numerous other assay protocols exist that are suitable for use with the antigens or antibodies of the present invention. The above descriptions are intended to be exemplary only.

In another embodiment, the above polypeptides may be used as markers for the progression of lung cancer. In this embodiment, assays as described above for the diagnosis of lung cancer may be performed over time, and the change in the level of reactive polypeptide(s) evaluated. For example, the assays may be performed every 24-72 hours for a period of 6 months to 1 year, and thereafter performed as needed. In general, lung cancer is progressing in those patients in whom the level of polypeptide detected by the binding agent increases over time. In contrast, lung cancer is not progressing when the level of reactive polypeptide either remains constant or decreases with time.

Antibodies for use in the above methods may be prepared by any of a variety of techniques known to those of ordinary skill in the art. *See, e.g.,* Harlow and Lane, Antibodies: A Laboratory Manual, Cold Spring Harbor Laboratory, 1988. In one such technique, an immunogen comprising the antigenic polypeptide is initially injected into any of a wide variety of mammals (e.g., mice, rats, rabbits, sheep and goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for the antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i.e.,* reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Monoclonal antibodies of the present invention may also be used as therapeutic reagents, to diminish or eliminate lung tumors. The antibodies may be used on their own (for instance, to inhibit metastases) or coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin. Shigella toxin, and pokeweed antiviral protein.

A therapeutic agent may be coupled (e.g., covalently bonded) to a suitable monoclonal antibody either directly or indirectly (e.g., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (e.g., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g.*, U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (e.g., U.S. Patent No. 4,489,710, to Spitler), by irradiation of a photolabile bond (e.g., U.S. Patent No. 4,625,014, to Senter et al.), by hydrolysis of derivatized amino acid side chains (*e.g.,* U.S. Patent No. 4,638,045, to Kohn et al.), by serum complement-mediated hydrolysis (e.g., U.S. Patent No. 4,671,958, to Rodwell et al.), and acid-catalyzed hydrolysis (e.g., U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins (e.g., U.S. Patent No. 4,507,234, to Kato et al.), peptides and polysaccharides such as aminodextran (e.g., U.S. Patent No. 4,699,784, to Shih et al.). A carrier may also bear an agent by noncovalent bonding or by encapsulation, such as within a liposome vesicle (e.g., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

Diagnostic reagents of the present invention may also comprise polynucleotide sequences encoding one or more of the above polypeptides, or one or more portions thereof. For example, at least two oligonucleotide primers may be employed in a polymerase chain reaction (PCR) based assay to amplify lung tumor-specific cDNA derived from a biological sample, wherein at least one of the oligonucleotide primers is specific for a polynucleotide molecule encoding a lung tumor protein of the present invention. The presence of the amplified cDNA is then detected using techniques well known in the art, such as gel electrophoresis. Similarly, oligonucleotide probes specific for a polynucleotide molecule encoding a lung tumor protein of the present invention may be used in a hybridization assay to detect the presence of an inventive polypeptide in a biological sample.

As used herein, the term "oligonucleotide primer/probe specific for a polynucleotide molecule" means an oligonucleotide sequence that has at least about 60%, preferably at least about 75% and more preferably at least about 90%, identity to the polynucleotide molecule in question. Oligonucleotide primers and/or probes which may be usefully employed in the inventive diagnostic methods preferably have at least about 10-40 nucleotides. In a preferred embodiment, the oligonucleotide primers comprise at least about 10 contiguous nucleotides of a polynucleotide molecule comprising sequence selected from SEQ ID NO: 1-109, 111, 113 115-151, 153, 154, 157, 158, 160, 162-164, 167, 168 and 171. Preferably, oligonucleotide probes for use in the inventive diagnostic methods comprise at least about 15 contiguous oligonucleotides of a polynucleotide molecule comprising a sequence provided in SEQ ID NO: 1-109,111, 113 115-151, 153, 154, 157, 158, 160, 162-164, 167, 168 and 171. Techniques for both PCR based assays and hybridization assays are well known in the art (see, for example, Mullis *et al. Ibid;* Ehrlich, *Ibid*). Primers or probes may thus be used to detect lung tumor-specific sequences in biological samples, including blood, semen, lung tissue and/or lung tumor tissue.

The following Examples are offered by way of illustration and not by way of limitation.

### EXAMPLES

### Example 1

### ISOLATION AND CHARACTERIZATION OF cDNA SEQUENCES ENCODING LUNG TUMOR POLYPEPTIDES

This example illustrates the isolation of cDNA molecules encoding lung tumor-specific polypeptides from lung tumor cDNA libraries.

### A. Isolation of cDNA Sequences from a Lung Squamous Cell Carcinoma Library

A human lung squamous cell carcinoma cDNA expression library was constructed from poly A⁺ RNA from a pool of two patient tissues using a Superscript Plasmid System for cDNA Synthesis and Plasmid Cloning kit (BRL Life Technologies, Gaithersburg, MD) following the manufacturer's protocol. Specifically, lung carcinoma tissues were homogenized with polytron (Kinematica, Switzerland) and total RNA was extracted using Trizol reagent (BRL Life Technologies) as directed by the manufacturer. The poly A⁺ RNA was then purified using an oligo dT cellulose column as described in Sambrook et al., Molecular Cloning: A Laboratory Manual, Cold Spring Harbor Laboratories, Cold Spring Harbor, NY, 1989. First-strand cDNA was synthesized using the NotI/Oligo-dT18 primer. Double-stranded cDNA was synthesized, ligated with BstXI/EcoRI adaptors (Invitrogen, San Diego, CA) and digested with NotI. Following size fractionation with cDNA size fractionation columns (BRL Life Technologies), the cDNA was ligated into the BstXI/NotI site of pcDNA3.1 (Invitrogen) and transformed into ElectroMax *E. coli* DH10B cells (BRL Life Technologies) by electroporation.

Using the same procedure, a normal human lung cDNA expression library was prepared from a pool of four tissue specimens. The cDNA libraries were characterized by determining the number of independent colonies, the percentage of clones that carried insert, the average insert size and by sequence analysis. The lung squamous cell carcinoma library contained 2.7 x 10⁶ independent colonies, with 100% of clones having an insert and the average insert size being 2100 base pairs. The normal lung cDNA library contained 1.4 x 10⁶ independent colonies, with 90% of clones having inserts and the average insert size being 1800 base pairs. For both libraries, sequence analysis showed that the majority of clones had a full length cDNA sequence and were synthesized from mRNA

cDNA library subtraction was performed using the above lung squamous cell carcinoma and normal lung cDNA libraries, as described by Hara et al. (Blood, 84:189-199, 1994) with some modifications: Specifically, a lung squamous cell carcinoma-specific subtracted cDNA library was generated as follows. Normal tissue cDNA library (80 µg) was digested with BamHI and XhoI, followed by a filling-in reaction with DNA polymerase Klenow fragment. After phenol-chloroform extraction and ethanol precipitation, the DNA was dissolved in 133 µl of H₂O, heat-denatured and mixed with 133 µl (133 µg) of Photoprobe biotin (Vector Laboratories, Burlingame, CA). As recommended by the manufacturer, the resulting mixture was irradiated with a 270 W sunlamp on ice for 20 minutes. Additional Photoprobe biotin (67 µl) was added and the biotinylation reaction was repeated. After extraction with butanol five times, the DNA was ethanol-precipitated and dissolved in 23 µl H₂O to form the driver DNA.

To form the tracer DNA, 10 µg lung squamous cell carcinoma cDNA library was digested with NotI and SpeI, phenol chloroform extracted and passed through Chroma spin-400 columns (Clontech, Palo Alto, CA). Typically, 5 µg of cDNA was recovered after the sizing column. Following ethanol precipitation, the tracer DNA was dissolved in 5 µl H₂O. Tracer DNA was mixed with 15 µl driver DNA and 20 µl of 2 x hybridization buffer (1.5 M NaCl/10 mM EDTA/50 mM HEPES pH 7.5/0.2% sodium dodecyl sulfate), overlaid with mineral oil, and heat-denatured completely. The sample was immediately transferred into a 68 °C water bath and incubated for 20 hours (long hybridization [LH]). The reaction mixture was then subjected to a streptavidin treatment followed by phenol/chloroform extraction. This process was repeated three more times. Subtracted DNA was precipitated, dissolved in 12 µl H₂O, mixed with 8 µl driver DNA and 20 µl of 2 x hybridization buffer, and subjected to a hybridization at 68 °C for 2 hours (short hybridization [SH]). After removal of biotinylated double-stranded DNA, subtracted cDNA was ligated into NotI/SpeI site of chloramphenicol resistant pBCSK⁺ (Stratagene, La Jolla, CA) and transformed into ElectroMax *E. coli* DH10B cells by electroporation to generate a lung squamous cell carcinoma specific subtracted cDNA library (herein after referred to as "lung subtraction I").

A second lung squamous cell carcinoma specific subtracted cDNA library (referred to as "lung subtraction II") was generated in a similar way to the lung subtraction library I, except that eight frequently recovered genes from lung subtraction I were included in the driver DNA, and 24,000 independent clones were recovered.

To analyze the subtracted cDNA libraries, plasmid DNA was prepared from 320 independent clones, randomly picked from the subtracted lung squamous cell carcinoma specific libraries. Representative cDNA clones were further characterized by DNA sequencing with a Perkin Elmer/Applied Biosystems Division Automated Sequencer Model 373A and/or Model 377 (Foster City, CA). The cDNA sequences for sixty isolated clones are provided in SEQ ID NO: 1-60. These sequences were compared to known sequences in the gene bank using the EMBL and GenBank databases (release 96). No significant homologies were found to the sequences provided in SEQ ID NO: 2, 3, 19, 38 and 46. The sequences of SEQ ID NO: 1, 6-8, 10-13, 15, 17, 18, 20-27, 29, 30, 32, 34-37, 39-45, 47-49, 51, 52, 54, 55 and 57-59 were found to show some homology to previously identified expressed sequence tags (ESTs). The sequences of SEQ ID NO: 9, 28, 31 and 33 were found to show some homology to previously identified non-human gene sequences and the sequences of SEQ ID NO: 4, 5, 14, 50, 53, 56 and 60 were found to show some homology to gene sequences previously identified in humans.

The subtraction procedure described above was repeated using the above lung squamous cell carcinoma cDNA library as the tracer DNA, and the above normal lung tissue cDNA library and a cDNA library from normal liver and heart (constructed from a pool of one sample of each tissue as described above), plus twenty other cDNA clones that were frequently recovered in lung subtractions I and II, as the driver DNA (lung subtraction III). The normal liver and heart cDNA library contained 1.76 x 10⁶ independent colonies, with 100% of clones having inserts and the average insert size being 1600 base pairs. Ten additional clones were isolated (SEQ ID NO: 61-70). Comparison of these cDNA sequences with those in the gene bank as described above, revealed no significant homologies to the sequences provided in SEQ ID NO: 62 and 67. The sequences of SEQ ID NO: 61, 63-66, 68 and 69 were found to show some homology to previously isolated ESTs and the sequence provided in SEQ ID NO: 70 was found to show some homology to a previously identified rat gene.

### B. Isolation of cDNA Sequences from a Lung Adenocarcinoma Library

A human lung adenocarcinoma cDNA expression library was constructed as described above. The library contained 3.2 x 10⁶ independent colonies, with 100% of clones having an insert and the average insert size being 1500 base pairs. Library subtraction was performed as described above using the normal lung and normal liver and heart cDNA expression libraries described above as the driver DNA. Twenty-six hundred independent clones were recovered.

Initial cDNA sequence analysis from 100 independent clones revealed many ribosomal protein genes. The cDNA sequences for fifteen clones isolated in this subtraction are provided in SEQ ID NO: 71-86. Comparison of these sequences with those in the gene bank as described above revealed no significant homologies to the sequence provided in SEQ ID NO: 84. The sequences of SEQ ID NO: 71, 73, 74, 77, 78 and 80-82 were found to show some homology to previously isolated ESTs, and the sequences of SEQ ID NO: 72, 75, 76, 79, 83 and 85 were found to show some homology to previously identified human genes.

### Example 2

### DETERMINATION OF TISSUE SPECIFICITY OF LUNG TUMOR POLYPEPTIDES

Using gene specific primers, mRNA expression levels for seven representative lung tumor polypeptides described in Example I were examined in a variety of normal and tumor tissues using RT-PCR.

Briefly, total RNA was extracted from a variety of normal and tumor tissues using Trizol reagent as described above. First strand synthesis was carried out using 2 µg of total RNA with SuperScript II reverse transcriptase (BRL Life Technologies) at 42 °C for one hour. The cDNA was then amplified by PCR with gene-specific primers. To ensure the semi-quantitative nature of the RT-PCR, β-actin was used as an internal control for each of the tissues examined. 1 µl of 1:30 dilution of cDNA was employed to enable the linear range amplification of the β-actin template and was sensitive enough to reflect the differences in the initial copy numbers. Using these conditions, the β-actin levels were determined for each reverse transcription reaction from each tissue. DNA contamination was minimized by DNase treatment and by assuring a negative PCR result when using first strand cDNA that was prepared without adding reverse transcriptase.

mRNA Expression levels were examined in five different types of tumor tissue (lung squamous cell carcinoma from 3 patients, lung adenocarcinoma, colon tumor from 2 patients, breast tumor and prostate tumor), and thirteen different normal tissues (lung from 4 donors, prostate, brain, kidney, liver, ovary, skeletal muscle, skin, small intestine, stomach, myocardium, retina and testes). Using a 10-fold amount of cDNA, the antigen LST-S1-90 (SEQ ID NO: 3) was found to be expressed at high levels in lung squamous cell carcinoma and in breast tumor, and at low to undetectable levels in the other tissues examined.

The antigen LST-S2-68 (SEQ ID NO: 15) appears to be specific to lung and breast tumor, however, expression was also detected in normal kidney. Antigens LST-S1-169 (SEQ ID NO: 6) and LST-S1-133 (SEQ ID NO: 5) appear to be very abundant in lung tissues (both normal and tumor), with the expression of these two genes being decreased in most of the normal tissues tested. Both LST-S1-169 and LST-S1-133 were also expressed in breast and colon tumors. Antigens LST-S1-6 (SEQ ID NO: 7) and LST-S2-I2-5F (SEQ ID NO: 47) did not show tumor or tissue specific expression, with the expression of LST-S1-28 being rare and only detectable in a few tissues. The antigen LST-S3-7 (SEQ ID NO: 63) showed lung and breast tumor specific expression, with its message only being detected in normal testes when the PCR was performed for 30 cycles. Lower level expression was detected in some normal tissues when the cycle number was increased to 35. Antigen LST-S3-13 (SEQ ID NO: 66) was found to be expressed in 3 out of 4 lung tumors, one breast tumor and both colon tumor samples. Its expression in normal tissues was lower compared to tumors, and was only detected in 1 out of 4 normal lung tissues and in normal tissues from kidney, ovary and retina. Expression of antigens LST-S3-4 (SEQ ID NO: 62) and LST-S3-14 (SEQ ID NO: 67) was rare and did not show any tissue or tumor specificity. Consistent with Northern blot analyses, the RT-PCT results on antigen LAT-S1-A-10A (SEQ ID NO: 78) suggested that its expression is high in lung, colon, stomach and small intestine tissues, including lung and colon tumors, whereas its expression was low or undetectable in other tissues.

A total of 2002 cDNA fragments isolated in lung subtractions I, II and III, described above, were colony PCR amplified and their mRNA expression levels in lung tumor, normal lung, and various other normal and tumor tissues were determined using microarray technology (Synteni, Palo Alto, CA). Briefly, the PCR amplification products were dotted onto slides in an array format, with each product occupying a unique location in the array. mRNA was extracted from the tissue sample to be tested, reverse transcribed, and fluorescent-labeled cDNA probes were generated. The microarrays were probed with the labeled cDNA probes, the slides scanned and fluorescence intensity was measured. This intensity correlates with the hybridization intensity. Seventeen non-redundant cDNA clones showed over-expression in lung squamous tumors, with expression in normal tissues tested (lung, skin, lymph node, colon, liver, pancreas, breast, heart, bone marrow, large intestine, kidney, stomach, brain, small intestine, bladder and salivary gland) being either undetectable, or 10-fold less compared to lung squamous tumors. The determined partial cDNA sequences for the clone L513S are provided in SEQ ID NO: 87 and 88; those for L514S are provided in SEQ ID NO: 89 and 90; those for L516S in SEQ ID NO: 91 and 92; that for L517S in SEQ ID NO: 93; that for L519S in SEQ ID NO: 94; those for L520S in SEQ ID NO: 95 and 96; those for L521 S in SEQ ID NO: 97 and 98; that for L522S in SEQ ID NO: 99; that for L523S in SEQ ID NO: 100; that for L524S in SEQ ID NO: 101; that for L525S in SEQ ID NO: 102; that for L526S in SEQ ID NO: 103; that for L527S in SEQ ID NO: 104; that for L528S in SEQ ID NO: 105; that for L529S in SEQ ID NO: 106; and those for L530S in SEQ ID NO: 107 and 108. Additionally, the full-length cDNA sequences for L503S and L514S (variants 1 and 2), are provided in SEQ ID NO: 151, 153 and 154, respectively, with the corresponding predicted amino acid sequence being provided in SEQ ID NO: 152, 155 and 156. Due to polymorphisms, the clone L531S appears to have two forms. A first determined full-length cDNA sequence for L531S is provided in SEQ ID NO: 109, with the corresponding predicted amino acid sequence being provided in SEQ ID NO: 110. A second determined full-length cDNA sequence for L531S is provided in SEQ ID NO: 111, with the corresponding predicted amino acid sequence being provided in SEQ ID NO: 112. The sequence of SEQ ID NO: 111 is identical to that of SEQ ID NO: 109, except that it contains a 27 bp insertion. Similarly, L514S also has two alternatively spliced forms; the first variant cDNA is listed as SEQ ID NO: 153, with the corresponding amino acid sequence as SEQ ID NO: 155. The second variant form of L514S full-length cDNA is referred to as SEQ ID NO: 154, with its corresponding amino acid sequence as SEQ ID NO: 156.

Full length cloning for L524S (SEQ ID NO: 101) yielded two variants (SEQ ID NO: 163 and 164) with the corresponding predicted amino acid sequences (SEQ ID NO: 165 and 166), respectively. Both variants have been shown to encode parathyroid hormone-related peptide.

Comparison of the sequences of L514S and L531S (SEQ ID NO: 87 and 88, 89 and 90, and 109, respectively) with those in the gene bank, as described above, revealed no significant homologies to known sequences. The sequences of L513S, L516S, L517S, L519S, L520S and L530S (SEQ ID NO: 87 and 88, 91 and 92, 93, 94, 95 and 96, 107 and 108, respectively) were found to show some homology to previously identified ESTs. The sequences of L521S, L522S, L523S, L524S, L525S, L526S, L527S, L528S and L529S (SEQ ID NO: 97 and 98, 99, 99, 101, 102, 103, 104, 105, and 106, respectively) were found to represent known genes. The determined full-length cDNA sequences for L520S is provided in SEQ ID NO: 113, with the corresponding predicted amino acid sequence being provided in SEQ ID NO: 114. Subsequent microarray analysis has shown L520S to be overexpressed in breast tumors in addition to lung squamous tumors.

Further analysis has demonstrated L529S (SEQ ID NO: 106 and 115), L525S (SEQ ID NO: 102 and 120) and L527S (SEQ ID NO: 104) are cytosleletal components and potentially squamous cell specific proteins. L529S is connexin 26, a gap junction protein. It is highly expressed in lung squamous tumor 9688T, and moderately over-expressed in two others. However, lower level expression of connexin 26 is also detectable in normal skin, colon, liver and stomach. The over-expression of connexin 26 in some breast tumors has been reported and a mutated form of L529S may result in over-expression in lung tumors. L525S is plakophilin 1, a desmosomal protein found in plaque-bearing adhering junctions of the skin. Expression levels for L525S mRNA is highly elevated in three out of four lung squamous tumors tested, and in normal skin. L527S has been identified as keratin 6 isoform, type II 58 Kd keratin, and cytokeratin 13 and shows over-expression in squamous tumors and low expression in normal skin, breast and colon tissues. Notably, keratin and keratin-related genes have been extensively documented as potential markers for lung cancer including CYFRA2.1 (Pastor, A., et al, Eur. Respir. J., 10:603-609, 1997). L513S (SEQ ID NO: 87 and 88) shows moderate over-expression in several tumor tissues tested, and encodes a protein that was first isolated as a pemphigus vulgaris antigen.

L520S (SEQ ID NO: 95 and 96) and L521S (SEQ ID NO: 97 and 98) are highly expressed in lung squamous tumors, and L520S is up-regulated in normal salivary gland and L521 S is over-expressed in normal skin. Both belong to a family of small proline rich proteins and represent markers for fully differentiated squamous cells. L521S has been described as a specific marker for lung squamous tumor (Hu, R., et al, Lung Cancer, 20:25-30, 1998). L515S (SEQ ID NO: 162) encodes IGF-β2 and L516S is an aldose reductase homologue and both are moderately expressed in lung squamous tumors and in normal colon. Notably, L516S (SEQ ID NO: 91 and 92) is up-regulated in metastatic tumors but not primary lung adenocarcinoma., an indication of its potential role in metatasis and a potential prognostic marker. L522S (SEQ ID NO: 99) is moderately over-expressed in lung squamous tumors with minimum expression in normal tissues. L522S has been shown to belong to a class IV alcohol dehydrogenase, ADH7, and its expression profile suggests it is a squamous cell specific antigen. L523S (SEQ ID NO: 100) is moderately over-expressed in lung squamous tumor, human pancreatic cancer cell lines and pancreatic cancer tissues, suggesting this gene may be a shared antigen between pancreatic and lung squamous cell cancer.

L524S (SEQ ID NO: 101) is over-expressed in the majority of squamous tumors tested and is homolgous with parathyroid hormone-related peptide (PTHrP), which is best known to cause humoral hypercalcaemia associated with malignant tumors such as leukemia, prostate and breast cancer. It is also believed that PTHrP is most commonly associated with squamous carcinoma of lung and rarely with lung adenocarcinoma (Davidson, L.A., et al, J. Pathol., 178: 398-401, 1996). L528S (SEQ ID NO: 105) is highly over-expressed in two lung squamous tumors with moderate expression in two other squamous tumors, one lung adenocarcinoma and some normal tissues, including skin, lymph nodes, heart, stomach and lung. It encodes the NMB gene that is similar to the precursor of melanocyte specific gene Pme117, wfhich is reported to be preferentially expressed in low-metastatic potential melanoma cell lines. This suggests that L528S may be a shared antigen in both melanoma and lung aquamous cell carcinoma. L526S (SEQ ID NO: 103) is overexpressed in all lung squamous cell tumor tissues tested and has been shown to share homology with a gene (ATM) in which a mutation causes ataxia telangiectasia, a genetic disorder in humans causing a predisposition to cancer, among other symptoms. ATM encodes a protein that activates p53 mediated cell-cycle checkpoint through direct binding and phosphorylation of the p53 molecule. Approximately 40% of lung cancer is associated with p53 mutations, and it is speculated that over-expression of ATM is a result of compensation for loss of p53 function, but it is unknown whether over-expression is the cause of result of lung squamous cell carcinoma. Additionally, expression of L526S (ATM) is also detected in a metastatic but not lung adenocarcinoma, suggesting a role in metastasis.

### Example 3

### ISOLATION AND CHARACTERIZATION OF LUNG TUMOR POLYPEPTIDES BY PCR-BASED SUBTRACTION

Eight hundred and fifty seven clones from a cDNA subtraction library, containing cDNA from a pool of two human lung squamous tumors subtracted against eight normal human tissue cDNAs including lung, PBMC, brain, heart, kidney, liver, pancreas, and skin, (Clontech, Palo Alto, CA) were derived and submitted to a first round of PCR amplification. This library was subjected to a second round of PCR amplification, following the manufacturer's protocol. The resulting cDNA fragments were subcloned into the vector P7- Adv vector (Clontech, Palo Alto, CA) and transformed into DH5α *E. coli* (Gibco, BRL). DNA was isolated from independent clones and sequenced using a Perkin Elmer/Applied Biosystems Division Automated Sequencer Model 373A.

One hundred and sixty two positive clones were sequenced. Comparison of the DNA sequences of these clones with those in the gene bank using the EMBL and GenBank databases, as described above, revealed no significant homologies to 13 of these clones, hereinafter referred to as Contig 13, 16, 17, 19, 22, 24, 29, 47, 49, 56-59 . The determined cDNA sequences for these clones are provided in SEQ ID NO: 125, 127-129, 131-133, 142, 144, 148-150, and 157, respectively. Contigs 1, 3-5, 7-10, 12, 11, 15, 20, 31, 33, 38, 39, 41, 43, 44, 45, 48, 50, 53, 54 (SEQ ID NO: 115-124, 126, 130, 134-141, 143, 145-147, respectively) were found to show some degree of homology to previously identified DNA sequences. Contig 57 (SEQ ID NO: 149) was found to represent the clone L519S (SEQ ID NO: 94) disclosed in US. Patent Application No. 09/123,912, filed July 27, 1998. To the best of the inventors' knowledge, none of these sequences have been previously shown to be differentially over-expressed in lung tumors. mRNA expression levels for representative clones in lung tumor tissues, normal lung tissues (n=4), resting PBMC, salivary gland, heart, stomach, lymph nodes, skeletal muscle, soft palate, small intestine, large intestine, bronchial, bladder, tonsil, kidney, esophagus, bone marrow, colon, adrenal gland, pancreas, and skin, (all derived from human) were determined by RT-PCR as described above. Expression levels using microarray technology, as described above, were examined in one sample of each tissue type unless otherwise indicated.

Contig 3 (SEQ ID NO: 116) was found to be highly expressed in all head and neck squamous cell tumors tested (17/17), and expressed in the majority (8/12) of lung squamous tumors, (high expression in 7/12, moderate in 2/12, and low in 2/12), while showing negative expression for 2/4 normal lung tissues and low expression in the remaining two samples. Contig 3 showed moderate expression in skin and soft palate, and lowered expression levels in resting PBMC, large intestine, salivary gland, tonsil, pancreas, esophagus, and colon. Contig 11 (SEQ ID NO: 124) was found to be expressed in all head and neck squamous cell tumors tested (17/17): highly expressed in 14/17, and moderately expressed in 3/17. Additionally, expression in lung squamous tumors showed high expression in 3/12 and moderate in 4/12. Contig 11 was negative for 3/4 normal lung samples, with the remaining sample having only low expression. Contig 11 showed low to moderate reactivity to salivary gland, soft palate, bladder, tonsil, skin, esophagus, and large intestine. Contig 13 (SEQ ID NO: 125) was found to be expressed in all head and neck squamous cell tumors tested (17/17): highly expressed in 12/17, and moderately expressed in 5/17. Contig 13 was expressed in 7/12 lung squamous tumors, with high expression in 4/12 and moderate expression in three samples. Analysis of normal lung samples showed negative expression for 2/4 and low to moderate expression in the remaining two samples. Contig 13 did show low to moderate reactivity to resting PBMC, salivary gland, bladder, pancreas, tonsil, skin, esophagus, and large intestine, as well as high expression in soft palate. Contig 16 (SEQ ID NO: 127) was found to be moderately expressed in some head and neck squamous cell tumors (6/17) and one lung squamous tumor; while showing no expression in any normal lung samples tested. Contig 16 did show low reactivity to resting PBMC, large intestine, skin, salivary gland, and soft palate. Contig 17 (SEQ ID NO: 128) was shown to be expressed in all head and neck squamous cell tumors tested (17/17): highly expressed in 5/17, and moderately expressed in 12/17. Expression levels in lung squamous tumors showed one tumor sample with high expression and 3/12 with moderate levels. Contig 17 was negative for 2/4 normal lung samples, with the remaining samples having only low expression. Additionally, low level expression was found in esophagus and soft palate. Contig 19 (SEQ ID NO: 129) was found to be expressed in most head and neck squamous cell tumors tested (11/17): with two samples having high levels, 6/17 showing moderate expression, and low expression being found in 3/17. Testing in lung squamous tumors revealed only moderate expression in 3/12 samples. Expression levels in 2/4 of normal lung samples were negative, the two other samples having only low expression. Contig 19 did show low expression levels in esophagus, resting PBMC, salivary gland, bladder, soft palate, and pancreas.

Contig 22, (SEQ ID NO: 131) was shown to be expressed in most head and neck squamous cell tumors tested (13/17) with high expression in four of these samples, moderate expression in 6/17, and low expression in 3/17. Expression levels in lung squamous tumors were found to be moderate to high for 3/12 tissues tested, with negative expression in two normal lung samples and low expression in two other samples (n=4). Contig 22 did show low expression in skin, salivary gland and soft palate. Similarly, Contig 24 (SEQ ID NO: 132) was found to be expressed in most head and neck squamous cell tumors tested (13/17) with high expression in three of these samples, moderate expression in 6/17, and low expression in 4/17. Expression levels in lung squamous tumors were found to be moderate to high for 3/12 tissues tested, with negative expression for three normal lung samples and low expression in one sample (n=4). Contig 24 did show low expression in skin, salivary gland and soft palate. Contig 29 (SEQ ID NO: 133) was expressed in nearly all head and neck squamous cell tumors tested (16/17): highly expressed in 4/17, moderately expressed in 11/17, with low expression in one sample. Also, it was moderately expressed in 3/12 lung squamous tumors, while being negative for 2/4 normal lung samples. Contig 29 showed low to moderate expression in large intestine, skin, salivary gland, pancreas, tonsil, heart and soft palate. Contig 47 (SEQ ID NO: 142) was expressed in most head and neck squamous cell tumors tested (12/17): moderate expression in 10/17, and low expression in two samples. In lung squamous tumors, it was highly expressed in one sample and moderately expressed in two others (n=13). Contig 47 was negative for 2/4 normal lung samples, with the remaining two samples having moderate expression. Also, Contig 47 showed moderate expression in large intestine, and pancreas, and low expression in skin, salivary gland, soft palate, stomach, bladder, resting PBMC, and tonsil.

Contig 48 (SEQ ID NO: 143) was expressed in all head and neck squamous cell tumors tested (17/17): highly expressed in 8/17 and moderately expressed in 7/17, with low expression in two samples. Expression levels in lung squamous tumors were high to moderate in three samples (n=13). Contig 48 was negative for one out of four normal lung samples, the remaining showing low or moderate expression. Contig 48 showed moderate expression in soft palate, large intestine, pancreas, and bladder, and low expression in esophagus, salivary gland, resting PBMC, and heart. Contig 49 (SEQ ID NO: 144) was expressed at low to moderate levels in 6/17 head and neck squamous cell tumors tested. Expression levels in lung squamous tumors were moderate in three samples (n=13). Contig 49 was negative for 2/4 normal lung samples, the remaining samples showing low expression. Moderate expression levels in skin, salivary gland, large intestine, pancreas, bladder and resting PBMC were shown, as well as low expression in soft palate, lymph nodes, and tonsil. Contig 56 (SEQ ID NO: 148) was expressed in low to moderate levels in 3/17 head and neck squamous cell tumors tested, and in lung squamous tumors, showing low to moderate levels in three out of thirteen samples. Notably, low expression levels were detected in one adenocarcinoma lung tumor sample (n=2). Contig 56 was negative for 3/4 normal lung samples, and showed moderate expression levels in only large intestine, and low expression in salivary gland, soft palate, pancreas, bladder, and resting PBMC. Contig 58, also known as L769P, (SEQ ID NO: 150) was expressed at moderate levels in 11/17 head and neck squamous cell tumors tested and low expression in one additional sample. Expression in lung squamous tumors showed low to moderate levels in three out of thirteen samples. Contig 58 was negative for 3/4 normal lung samples, with one sample having low expression. Moderate expression levels in skin, large intestine, and resting PBMC were demonstrated, as well as low expression in salivary gland, soft palate, pancreas, and bladder. Contig 59 (SEQ ID NO: 157) was expressed in some head, neck, and lung squamous tumors. Low level expression of Contig 59 was also detected in salivary gland and large intestine.

Additionally, the full-length cDNA sequence for Contigs 22, referred to as L763P, is provided in SEQ ID NO: 158, with the corresponding predicted amino acid sequence being provided in SEQ ID NO: 159. Also, the full-length cDNA sequence incorporating Contigs 17, 19, and 24, referred to as L762P, is provided in SEQ ID NO: 160, with the corresponding predicted amino acid sequence being provided in SEQ ID NO: 161. Further analysis of L762P has determined it to be a type I membrane protein and two additional variants have been sequenced. Variant 1 (SEQ ID NO: 167 and the corresponding amino acid sequence in SEQ ID NO: 169) is an alternatively spliced form of SEQ ID NO: 160 resulting in deletion of 503 nucleotides, as well as deletion of a short segment of the expressed protein. Variant 2 (SEQ ID NO: 168 and the corresponding amino acid sequence in SEQ ID NO: 170) has a two nucleotide deletion at the 3' coding region in comparison to SEQ ID NO: 160, resulting in a secreted form of the expressed protein.

The full-length cDNA sequence for contig 56 (SEQ ID NO: 148), referred to as L773P, is provided in SEQ ID NO: 171, with the predicted amino acid sequence in SEQ ID NO: 172. Subsequent Northern blot analysis of L773P demonstrates this transcript is differentially over-expressed in squamous tumors and detected at approximately 1.6 Kb in primary lung tumor tissue and approximately 1.3 Kb in primary head and neck tumor tissue.

Subsequent microarray analysis has shown Contig 58, also referred to as L769S (SEQ ID NO: 150), to be overexpressed in breast tumors in addition to lung squamous tumors.

### Example 4

### SYNTHESIS OF POLYPEPTIDES

Polypeptides may be synthesized on a Perkin Elmer/Applied Biosystems Division 430A peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation, binding to an immobilized surface, or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C18 reverse phase HPLC. A gradient of 0%-60% acetonitrile (containing 0.1% TFA) in water (containing 0.1% TFA) may be used to elute the peptides. Following lyophilization of the pure fractions, the peptides may be characterized using electrospray or other types of mass spectrometry and by amino acid analysis.

From the foregoing, it will be appreciated that, although specific embodiments of the invention have been described herein for the purposes of illustration, various modifications may be made without deviating from the spirit and scope of the invention.

The present invention will now be described by way of reference to the following clauses:
1. An isolated polynucleotide molecule comprising a nucleotide sequence selected from the group consisting of:
   (a) sequences provided in SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168 and 171;
   (b) the complements of sequences provided in SEQ ID NO: 1-3, 6-8, 10-13, 15-27, 29, 30, 32, 34-49, 51, 52, 54, 55, 57-59, 61-69. 71, 73, 74, 77, 78, 80-82, 84, 86-96, 107-109, 111, 113, 125, 127, 128, 129, 131-133, 142, 144, 148-151, 153, 154, 157, 158, 160, 167, 168 and 171; and
   (c) sequences that hybridize to a sequence of (a) or (b) under moderately stringent conditions.
2. An isolated polypeptide comprising an immunogenic portion of a lung tumor protein or a variant thereof, wherein said protein comprises an amino acid sequence encoded by a polynucleotide molecule of clause 1.
3. An isolated polynucleotide molecule comprising a nucleotide sequence encoding the polypeptide of clause 2.
4. An expression vector comprising an isolated polynucleotide molecule of clauses 1 or 3.
5. A host cell transformed with the expression vector of clause 4.
6. The host cell of clause 5 wherein the host cell is selected from the group consisting of *E. coli,* yeast and mammalian cell lines.
7. A pharmaceutical composition comprising the polypeptide of clause 2 and a physiologically acceptable carrier.
8. A vaccine comprising the polypeptide of clause 2 and a non-specific immune response enhancer.
9. The vaccine of clause 8 wherein the non-specific immune response enhancer is an adjuvant.
10. A vaccine comprising an isolated polynucleotide molecule of clause 1 or 3 and a non-specific immune response enhancer.
11. The vaccine of clause 10 wherein the non-specific immune response enhancer is an adjuvant.
12. A pharmaceutical composition for the treatment of lung cancer comprising a polypeptide and a physiologically acceptable carrier, the polypeptide comprising an immunogenic portion of a lung protein or a variant thereof, wherein said protein comprises an amino acid sequence encoded by a polynucleotide molecule comprising a sequence selected from the group consisting of:
   (a) sequences recited in SEQ ID NO: 4, 5, 9, 14, 28, 31, 33, 50, 53, 56, 60, 70, 72, 75, 76, 79, 83, 85, 97-106, 115-124, 126, 130, 134-141, 143, 145-147 and 162-164;
   (b) sequences complementary to the sequences of SEQ ID NO: 4, 5, 9, 14, 28, 31, 33, 50, 53, 56, 60, 70, 72, 75, 76, 79, 83, 85, 97-106, 115-124, 126, 130, 134-141, 143, 145-147 and 162-164 ; and
   (c) sequences that hybridize to a sequence of (a) or (b) under moderately stringent conditions.
13. A vaccine for the treatment of lung cancer comprising a polypeptide and a non-specific immune response enhancer, said polypeptide comprising an immunogenic portion of a lung protein or a variant thereof, wherein said protein comprises an amino acid sequence encoded by a polynucleotide molecule comprising a sequence selected from the group consisting of:
   (a) sequences recited in SEQ ID NO: 4, 5, 9, 14, 28, 31, 33, 50, 53, 56, 60, 70, 72, 75, 76, 79, 83, 85, 97-106, 115-124, 126, 130, 134-141, 143, 145-147 and 162-164;
   (b) sequences complementary to the sequences of SEQ ID NO: 4, 5, 9, 14, 28, 31, 33, 50, 53, 56, 60, 70, 72, 75, 76, 79, 83, 85, 97-106, 115-124, 126, 130, 134-141, 143, 145-147 and 162-164; and
   (c) sequences that hybridize to a sequence of (a) or (b) under moderately stringent conditions.
14. A vaccine for the treatment of lung cancer comprising a DNA molecule and a non-specific immune response enhancer, the polynucleotide molecule comprising a sequence selected from the group consisting of:
   (a) sequences recited in SEQ ID NO: 4, 5, 9, 14, 28, 31, 33, 50, 53, 56, 60, 70, 72, 75, 76, 79, 83, 85, 97-106, 115-124, 126, 130, 134-141, 143, 145-147 and 162-164;
   (b) sequences complementary to the sequences of SEQ ID NO: 4, 5, 9, 14, 28, 31, 33, 50, 53, 56, 60, 70, 72, 75, 76, 79, 83, 85, 97-106, 115-124, 126, 130, 134-141, 143, 145-147 and 162-164; and
   (c) sequences that hybridize to a sequence of (a) or (b) under moderately stringent conditions.
15. A method for inhibiting the development of lung cancer in a patient, comprising administering to the patient an effective amount of the pharmaceutical composition of clause 7 or 12.
16. A method for inhibiting the development of lung cancer in a patient, comprising administering to the patient an effective amount of the vaccine of any one of clauses 8, 10, 13 or 14.
17. A fusion protein comprising at least one polypeptide according to clause 2.
18. A fusion protein comprising a polypeptide according to clause 2 and a known lung tumor antigen.
19. A pharmaceutical composition comprising a fusion protein according to any one of clause 17-18 and a physiologically acceptable carrier.
20. A vaccine comprising a fusion protein according to any one of clause; 17-18 and a non-specific immune response enhancer.
21. The vaccine of clause 20 wherein the non-specific immune response enhancer is an adjuvant.
22. A method for inhibiting the development of lung cancer in a patient, comprising administering to the patient an effective amount of the pharmaceutical composition of clause 19.
23. A method for inhibiting the development of lung cancer in a patient, comprising administering to the patient an effective amount of the vaccine of clause 20.
24. A method for detecting lung cancer in a patient, comprising:
   (a) contacting a biological sample obtained from the patient with a binding agent which is capable of binding to a polypeptide, the polypeptide comprising an immunogenic portion of a lung protein or a variant thereof, wherein said protein comprises an amino acid sequence encoded by a polynucleotide molecule comprising a sequence selected from the group consisting of nucleotide sequences recited in SEQ ID NO: 1-109, 111, 113, 115-151, 153, 154, 157, 158, 160, 162-164, 167, 168 and 171 the complements of said nucleotide sequences and sequences that hybridize to a sequence of SEQ ID NO: 1-109, 111, 113, 115-151, 153, 154, 157, 158, 160, 162-164, 167, 168 and 171 under moderately stringent conditions; and
   (b) detecting in the sample a protein or polypeptide that binds to the binding agent, thereby detecting lung cancer in the patient.
25. The method of clause 24 wherein the binding agent is a monoclonal antibody.
26. The method of clause 25 wherein the binding agent is a polyclonal antibody.
27. A method for monitoring the progression of lung cancer in a patient, comprising:
   (a) contacting a biological sample obtained from the patient with a binding agent that is capable of binding to a polypeptide, said polypeptide comprising an immunogenic portion of a lung protein or a variant thereof, wherein said protein comprises an amino acid sequence encoded by a polynucleotide molecule comprising a sequence selected from the group consisting of nucleotide sequences recited in SEQ ID NO: 1-109, 111, 113, 115-151, 153, 154, 157, 158, 160, 162-164, 167, 168 and 171 the complements of said nucleotide sequences and sequences that hybridize to a nucleotide sequence of SEQ ID NO: 1-109, 111, 113, 115-151, 153, 154, 157, 158, 160, 162-164, 167, 168 and 171 under moderately stringent conditions;
   (b) determining in the sample an amount of a protein or polypeptide that binds to the binding agent;
   (c) repeating steps (a) and (b); and
   (d) comparing the amount of polypeptide detected in steps (b) and (c) to monitor the progression of lung cancer in the patient.
28. A monoclonal antibody that binds to a polypeptide comprising an immunogenic portion of a lung protein or a variant thereof, wherein said protein comprises an amino acid sequence encoded by a polynucleotide molecule comprising a sequence selected from the group consisting of: nucleotide sequences recited in SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168 and 171; the complements of said nucleotide sequences; and sequences that hybridize to a nucleotide sequence of SEQ ID NO: 1-3, 6-8, 10-13, 15-27, 29, 30. 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168 or 171 under moderately stringent conditions.
29. A method for inhibiting the development of lung cancer in a patient, comprising administering to the patient a therapeutically effective amount of a monoclonal antibody according to clause 28.
30. The method of clause 29 wherein the monoclonal antibody is conjugated to a therapeutic agent.
31. A method for detecting lung cancer in a patient comprising:
   (a) obtaining a biological sample from the patient;
   (b) contacting the sample with at least two oligonucleotide primers in a polymerase chain reaction, wherein at least one of the oligonucleotides is specific for a polynucleotide molecule encoding a polypeptide comprising an immunogenic portion of a lung protein or of a variant thereof, said protein comprising an amino acid sequence encoded by a polynucleotide molecule comprising a sequence selected from the group consisting of nucleotide sequences recited in SEQ ID NO: 1-109, 111, 113, 115-151, 153, 154, 157, 158, 160, 162-164, 167, 168 and 171 the complements of said nucleotide sequences, and sequences that hybridize to a sequence of SEQ ID NO: 1-109, 111, 113, 115-151. 153, 154, 157, 158, 160, 162-164, 167, 168 or 171 under moderately stringent conditions; and
   (c) detecting in the sample a polynucleotide sequence that amplifies in the presence of the oligonucleotide primers, thereby detecting lung cancer.
32. The method of clause 31, wherein at least one of the oligonucleotide primers comprises at least about 10 contiguous nucleotides of a polynucleotide molecule comprising a sequence selected from SEQ ID NO: 1-109, 111, 113, 115-151, 153, 154, 157, 158, 160, 162-164, 167, 168 and 171.
33. A diagnostic kit comprising:
   (a) one or more monoclonal antibodies of clause28; and
   (b) a detection reagent.
34. A diagnostic kit comprising:
   (a) one or more monoclonal antibodies that bind to a polypeptide encoded by a polynucleotide molecule comprising a nucleotide sequence selected from the group consisting of SEQ ID NO: 4, 5, 9, 14, 28, 31, 33, 50, 53, 56, 60, 70, 72, 75, 76, 79, 83, 85, 97-106, 115-124, 126, 130, 134-141, 143, 145-147 and 162-164 the complements of said sequences, and sequences that hybridize to a sequence of SEQ ID NO: 4, 5, 9, 14, 28, 31, 33, 50, 53, 56, 60, 70, 72, 75, 76, 79, 83, 85, 97-106, 115-124, 126, 130, 134-141, 143, 145-147 or 162-164 under moderately stringent conditions; and
   (b) a detection reagent.
35. The kit of clauses 33 or 34 wherein the monoclonal antibodies are immobilized on a solid support.
36. The kit of clause 35 wherein the solid support comprises nitrocellulose, latex or a plastic material.
37. The kit of clauses 33 or 34 wherein the detection reagent comprises a reporter group conjugated to a binding agent.
38. The kit of clause 37 wherein the binding agent is selected from the group consisting of anti-immunoglobulins, Protein G, Protein A and lectins.
39. The kit of clause 37 wherein the reporter group is selected from the group consisting of radioisotopes, fluorescent groups, luminescent groups, enzymes, biotin and dye particles.
40. A diagnostic kit comprising at least two oligonucleotide primers, at least one of the oligonucleotide primers being specific for a polynucleotide molecule encoding a polypeptide comprising an immunogenic portion of a lung protein or a variant thereof, said protein comprising an amino acid sequence encoded by a polynucleotide molecule comprising a sequence selected from the group consisting of nucleotide sequences recited in SEQ ID NO: 1-109, 111, 113, 115-151, 153, 154, 157, 158, 160, 162-164, 167, 168 and 171 the complements of said nucleotide sequences and sequences that hybridize to a sequence of SEQ ID NO: 1-109, 111, 113, 115-151, 153, 154, 157, 158, 160, 162-164. 167, 168 or 171 under moderately stringent conditions.
41. A diagnostic kit of clause40 wherein at least one of the oligonucleotide primers comprises at least about 10 contiguous nucleotides of a polynucleotide molecule comprising a sequence selected from SEQ ID NO: 1-109, 111, 113, 115-151, 153, 154, 157, 158, 160, 162-164, 167, 168 and 171.
42. A method for detecting lung cancer in a patient, comprising:
   (a) obtaining a biological sample from the patient;
   (b) contacting the biological sample with an oligonucleotide probe specific for a polynucleotide molecule encoding a polypeptide comprising an immunogenic portion of a lung protein or a variant thereof, said protein comprising an amino acid sequence encoded by a polynucleotide molecule comprising a sequence selected from the group consisting of nucleotide sequences recited in SEQ ID NO: 1-109, 111, 113, 115-151, 153, 154, 157, 158, 160, 162-164, 167, 168 and 171 the complements of said nucleotide sequences, and sequences that hybridize to a sequence of SEQ ID NO: 1-109, 111, 113, 115-151, 153, 154, 157, 158, 160, 162-164, 167, 168 or 171 under moderately stringent conditions; and
   (c) detecting in the sample a polynucleotide sequence that hybridizes to the oligonucleotide probe, thereby detecting lung cancer in the patient.
43. The method of clause 42 wherein the oligonucleotide probe comprises at least about 15 contiguous nucleotides of a polynucleotide molecule comprising a sequence selected from the group consisting of SEQ ID NO: 1-109, 111, 113, 115-151, 153, 154, 157, 158, 160, 162-164, 167, 168 and 171.
44. A diagnostic kit comprising an oligonucleotide probe specific for a polynucleotide molecule encoding a polypeptide comprising an immunogenic portion of a lung protein or a variant thereof, said protein comprising an amino acid sequence encoded by a polynucleotide molecule comprising a sequence selected from the group consisting of: nucleotide sequences recited in SEQ ID NO: 1-109, 111, 113, 115-151, 153, 154, 157, 158, 160, 162-164, 167, 168 and 171; the complements of said nucleotide sequences; and sequences that hybridize to a sequence of SEQ ID NO: 1-109, 111, 113, 115-151, 153, 154, 157, 158, 160, 162-164, 167, 168 or 171 under moderately stringent conditions.
45. The diagnostic kit of clause 44, wherein the oligonucleotide probe comprises at least about 15 contiguous nucleotides of a polynucleotide molecule comprising a sequence selected from the group consisting of SEQ ID NO: 1-109, 111, 113, 115-151, 153, 154, 157, 158, 160, 162-164, 167, 168 and 171.
46. A method for treating lung cancer in a patient, comprising the steps of:
   (a) obtaining peripheral blood cells from the patient;
   (b) incubating the cells in the presence of at least one polypeptide of claim 2, such that T cells proliferate; and
   (c) administering to the patient the proliferated T cells.
47. A method for treating lung cancer in a patient, comprising the steps of:
   (a) obtaining peripheral blood cells from the patient;
   (b) incubating the cells in the presence of at least one polynucleotide of claim 1, such that T cells proliferate; and
   (c) administering to the patient the proliferated T cells.
48. The method of any one of clauses 46 and 47 wherein the step of incubating the T cells is repeated one or more times.
49. The method of any one of clauses 46 and 47 wherein step (a) further comprises separating T cells from the peripheral blood cells, and the cells incubated in step (b) are the T cells.
50. The method of any one of clauses 46 and 47 wherein step (a) further comprises separating CD4+ cells or CD8+ cells from the peripheral blood cells, and the cells proliferated in step (b) are CD4+ or CD8+T cells.
51. The method of any one of clauses 46 and 47 wherein step (b) further comprises cloning one or more T cells that proliferated in the presence of the polypeptide.
52. A composition for the treatment of lung cancer in a patient, comprising T cells proliferated in the presence of a polypeptide of clause 2, in combination with a pharmaceutically acceptable carrier.
53. A composition for the treatment of lung cancer in a patient, comprising T cells proliferated in the presence of a polynucleotide of clause 1, in combination with a pharmaceutically acceptable carrier.
54. A method for treating lung cancer in a patient, comprising the steps of:
   (a) incubating antigen presenting cells in the presence of at least one polypeptide of clause 2;
   (b) administering to the patient the incubated antigen presenting cells.
55. A method for treating lung cancer in a patient, comprising the steps of:
   (a) incubating antigen presenting cells in the presence of at least one polynucleotide of clause 1;
   (b) administering to the patient the incubated antigen presenting cells.
56. The method of clauses 54 or 55 wherein the antigen presenting cells are selected from the group consisting of dendritic cells and macrophage cells.
57. A composition for the treatment of lung cancer in a patient, comprising antigen presenting cells incubated in the presence of a polypeptide of clause 2, in combination with a pharmaceutically acceptable carrier.
58. A composition for the treatment if lung cancer in a patient, comprising antigen presenting cells incubated in the presence of a polynucleotide of clause 1, in combination with a pharmaceutically acceptable carrier.

## Claims

1. An isolated polynucleotide molecule comprising a polynucleotide sequence selected from the group consisting of:
a. the nucleotide sequence provided in any one of SEQ ID NOs:167, 168, 128, 129, 132, 1-109, 111, 113, 115-127, 131, 133-151, 153, 154, 157, 158, 162-164, and 171, or the complement thereof; and
b. nucleotide sequences that hybridize to a sequence of (a) under moderately stringent conditions.

2. An isolated polypeptide comprising an amino acid sequence encoded by the polynucleotide molecule of claim 1.

3. An isolated polypeptide comprising the amino acid sequence provided in SEQ ID NO:169, 170, 110, 112, 114, 152, 155, 156, 159, 165, 166, and 172.

4. An expression vector comprising the isolated polynucleotide molecule of claim 1.

5. A host cell transformed with the expression vector of claim 4.

6. The host cell of claim 5 wherein the host cell is selected from the group consisting of *E. coli,* yeast and mammalian cell lines.

7. A pharmaceutical composition comprising the polypeptide of claim 2 or claim 3 and a physiologically acceptable carrier.

8. A vaccine comprising an isolated polynucleotide molecule of claim 1 or the polypeptide of claims 2 or 3 and a non-specific immune response enhancer.

9. A pharmaceutical composition for the treatment of lung cancer comprising a polypeptide according to claim 2 or claim 3 and a physiologically acceptable carrier.

10. A vaccine for the treatment of lung cancer comprising a polypeptide according to claim 2 or claim 3 and a non-specific immune response enhancer.

11. A vaccine for the treatment of lung cancer comprising an isolated polynucleotide molecule according to claim 1 and a non-specific immune response enhancer..

12. A fusion protein comprising at least one polypeptide according to claim 2, preferably further comprising a known lung tumor antigen.

13. A pharmaceutical composition comprising a fusion protein according to claim 12 and a physiologically acceptable carrier.

14. A vaccine comprising a fusion protein according to claim 12 and a non-specific immune response enhancer.

15. The vaccine of claim 8 or 14 wherein the non-specific immune response enhancer is an adjuvant.

16. The pharmaceutical composition of any one of claims 7, 9 or 13 for use in a method for inhibiting the development of lung cancer in a patient.

17. The vaccine of any one of claims 8, 10, 11, 14 or 15 for use in a method for inhibiting the development of lung cancer in a patient.

18. A method for detecting lung cancer in a patient, comprising:
(a) contacting a biological sample obtained from the patient with a binding agent which is capable of binding to a polypeptide, the polypeptide comprising at least an immunogenic portion of a lung protein or a variant thereof, wherein said protein comprises an amino acid sequence encoded by a polynucleotide molecule comprising the polynucleotide provided in any one of SEQ ID NOs:167, 168, 128, 129, 132, 1-109, 111, 113, 115-127, 131, 133-151, 153, 154, 157, 158, 162-164, and 171; and
(b) detecting in the sample a protein or polypeptide that binds to the binding agent, thereby detecting lung cancer in the patient.

19. The method of claim 18 wherein the binding agent is a monoclonal antibody, or a polyclonal antibody.

20. A method for monitoring the progression of lung cancer in a patient, comprising:
(a) contacting a biological sample obtained from the patient with a binding agent that is capable of binding to a polypeptide, said polypeptide comprising at least an immunogenic portion of a lung protein or a variant thereof, wherein said protein comprises an amino acid sequence encoded by the polynucleotide provided in any one of SEQ ID NOs:167, 168, 128, 129, 132, 1-109, 111, 113, 115-127, 131, 133-151, 153, 154, 157, 158, 162-164, and 171; and;
(b) determining in the sample an amount of a protein or polypeptide that binds to the binding agent;
(c) repeating steps (a) and (b); and
(d) comparing the amount of polypeptide detected in steps (b) and (c) to monitor the progression of lung cancer in the patient.

21. A monoclonal antibody that binds to a polypeptide comprising an amino acid sequence encoded by the polynucleotide provided in any one of SEQ ID NOs:167, 168, 128, 129, 132, 1-109, 111, 113, 115-127, 131, 133-151, 153, 154, 157, 158, 162-164, and 171.

22. A monoclonal antibody according to claim 21 for use in a method for inhibiting the development of lung cancer in a patient.

23. The antibody of claim 22 wherein the monoclonal antibody is conjugated to a therapeutic agent.

24. A method for detecting lung cancer in a patient comprising:
(a) contacting a biological sample obtained from the patient with at least two oligonucleotide primers in a polymerase chain reaction, wherein at least one of the oligonucleotides is specific for the polynucleotide provided in any one of SEQ ID NOs:167, 168, 128, 129, 132, 1-109, 111, 113, 115-127, 131, 133-151, 153, 154, 157, 158, 162-164, and 171, or the complement thereof; and
(b) detecting in the sample a polynucleotide sequence that amplifies in the presence of the oligonucleotide primers, thereby detecting lung cancer.

25. A diagnostic kit comprising:
(a) one or more monoclonal antibodies of claim 21; and
(b) a detection reagent.

26. The kit of claims 25 wherein the monoclonal antibodies are immobilized on a solid support, preferably comprising nitrocellulose, latex or a plastic materiel

27. The kit of claims 25 or 26 wherein the detection reagent comprises a reporter group, preferably selected from the group consisting of radioisotopes, fluorescent groups, luminescent groups, enzymes, biotin and dye particles, conjugated to a binding agent, preferably selected from the group consisting of anti-immunoglobulins, Protein G, Protein A and lectins.

28. A diagnostic kit comprising at least two oligonucleotide primers, at least one of the oligonucleotide primers being specific for the polynucleotide molecule provided in any one of SEQ ID NOs:167, 168, 128, 129, 132, 1-109, 111, 113, 115-127, 131, 133-151, 153, 154, 157, 158, 162-164, and 171, or the complement thereof.

29. A method according to claim 24 or a diagnostic kit of claim 28 wherein at least one of the oligonucleotide primers comprises at least 10 contiguous nucleotides of a polynucleotide molecule comprising the sequence provided in any one of SEQ ID NOs:167, 168, 128, 129, 132, 1-109, 111, 113, 115-127, 131, 133-151, 153, 154, 157, 158, 162-164, and 171.

30. A method for detecting lung cancer in a patient, comprising:
(b) contacting a biological sample obtained from the patient with an oligonucleotide probe specific for the polynucleotide molecule provided in any one of SEQ ID NOs:167, 168, 128, 129, 132, 1-109, 111, 113, 115-127, 131, 133-151, 153, 154, 157, 158, 162-164, and 171, or the complement thereof; and
(c) detecting in the sample a polynucleotide sequence that hybridizes to the oligonucleotide probe, thereby detecting lung cancer in the patient.

31. A diagnostic kit comprising an oligonucleotide probe specific for the polynucleotide molecule provided in any one of SEQ ID NOs:167, 168, 128, 129, 132, 1-109, 111, 113, 115-127, 131, 133-151, 153, 154, 157, 158, 162-164, and 171, or the complement thereof.

32. The method of claim 30 or the diagnostic kit of claim 31, wherein the oligonucleotide probe comprises at least 15 contiguous nucleotides of the polynucleotide molecule provided in any one of SEQ ID NOs:167, 168, 128, 129, 132, 1-109, 111, 113, 115-127, 131, 133-151, 153, 154, 157, 158, 162-164, and 171, or the complement thereof.

33. A method for preparing a blood product, comprising the step of: incubating peripheral blood cells, obtained from a patient, in the presence of at least one polypeptide of claim 2, or at least one polynucleotide of claim 1, such that T cells proliferate.

34. The method of claim 33 wherein the step of incubating the T cells is repeated one or more times.

35. The method of claim 33 or 34, further comprising the step of separating T cells from the peripheral blood cells, such that the cells incubated are the T cells.

36. The method of claim 33 or 34 further comprising separating CD4+ cells or CD8+ cells from the peripheral blood cells such that the cells proliferated are CD4+ or CD8+ T cells.

37. The method of any one of claims 33 to 36 further comprising cloning one or more T cells that proliferated in the presence of the polypeptide or polynucleotide.

38. A blood product prepared according to any one of claims 33 to 37 for use in a method for treating lung cancer.

39. A composition for the treatment of lung cancer in a patient, comprising T cells proliferated in the presence of a polypeptide of claim 2 or a polynucleotide of claim 1, in combination with a pharmaceutically acceptable carrier.

40. Antigen presenting cells incubated in the presence of at least one polypeptide of claim 2 or at least one polynucleotide of claim 1 for use in a method of treating lung cancer in a patient.

41. The antigen presenting cells of claim 40 wherein the antigen presenting cells are selected from the group consisting of dendritic cells and macrophage cells.

42. A composition for the treatment of lung cancer in a patient, comprising antigen presenting cells incubated in the presence of a polypeptide of claim 2 or a polynucleotide of claim 1 in combination with a pharmaceutically acceptable carrier.


SEQUENCE LISTING

<110> Wang, Tongtong

<120> COMPOUNDS AND METHODS FOR THERAPY OF
LUNG CANCER

<130> 210121.455PC

<140> PCT
<141> 1999-03-16

<160> 172

<170> FastSEQ for Windows Version 3.0

<210> 1
<211> 315
<212> DNA
<213> Homo sapien

<220>
<221> misc_feature
<222> (1)...(315)
<223> n = A,T,C or G

<400> 1
gcagagacag actggtggtt gaacctggag gtgccaaaaa agccagctgc gggcccagga 60
cagctgccgt gagactcccg atgtcacagg cagtctgtgt ggttacagcg cccctcagtg 120
ttcatctcca gcagagacaa cggaggaggc tcccaccagg acggttctca ttatttatat 180
gttaatatgt ttgtaaactc atgtacagtt ttttttgggg gggaagcaat gggaanggta 240
naaattacaa atagaatcat ttgctgtaat ccttaaatgg caaacggtca ggccacgtga 300
aaaaaaaaaa aaaaa 315

<210> 2
<211> 380
<212> DNA
<213> Homo sapien

<400> 2
   atttaggctt aagattttgt ttacccttgt tactaaggag caaattagta ttaaagtata 60
   atatatataa acaaatacaa aaagttttga gtggttcagc tttttatttt tttttaatgg 120
   cataactttt aacaacactg ctctgtaatg ggttgaactg tggtactcag actgagataa 180
   ctgaaatgag tggatgtata gtgttattgc ataattatcc cactatgaag caaagggact 240
   ggataaattc ccagtctaga ttattagcct ttgttaacca tcaagcacct agaagaagaa 300
   ttattggaaa ttttgtcctc tgtaactggc actttggggt gtgacttatc ttttgccttt 360
   gtaaaaaaaa aaaaaaaaaa 380

<210> 3
<211> 346
<212> DNA
<213> Homo sapien

<220>
<221> misc_feature
<222> (1)...(346)
<223> n = A,T,C or G

<400> 3
ttgtaagtat acaattttag aaaggattaa atgttattga tcattttact gaatactgca 60
catcctcacc atacaccatc cactttccaa taacatttaa tcctttctaa aattgtaagt 120
atacaattgt actttctttg gattttcata acaaatatac catagactgt taattttatt 180
gaagtttcct taatggaatg agtcattttt gtcttgtgct tttgaggtta cctttgcttt 240
gacttccaac aatttgatca tatagtgttg agctgtggaa atctttaagt ttattctata 300
gcaataattt ctattnnnag annccnggnn naaaannann annaaa 346

<210> 4
<211> 372
<212> DNA
<213> Homo sapien

<220>
<221> misc_feature
<222> (1)...(372)
<223> n = A,T,C or G

<400> 4
actagtctca ttactccaga attatgctct tgtacctgtg tggctgggtt tcttagtcgt 60
tggtttggtt tggttttttg aactggtatg tagggtggtt cacagttcta atgtaagcac 120
tctcttctcc aagttgtgct ttgtggggac aatcattctt tgaacattag agaggaaggc 180
agttcaagct gttgaaaaga ctattgctta tttttgtttt taaagaccta cttgacgtca 240
tgtggacagt gcacgtgcct tacgctacat cttgttttct aggaagaagg ggatgcnggg 300
aaggantggg tgctttgtga tggataaaac gnctaaataa cacaccttta cattttgaaa 360
aaaacaaaac aa 372

<210> 5
<211> 698
<212> DNA
<213> Homo sapien

<220>
<221> misc_feature
<222> (1)...(698)
<223> n = A,T,C or G

<400> 5
actagtanga tagaaacact gtgtcccgag agtaaggaga gaagctacta ttgattagag 60
cctaacccag gttaactgca agaagaggcg ggatactttc agctttccat gtaactgtat 120
gcataaagcc aatgtagtcc agtttctaag atcatgttcc aagctaactg aatcccactt 180
caatacacac tcatgaactc ctgatggaac aataacaggc ccaagcctgt ggtatgatgt 240
gcacacttgc tagactcaga aaaaatacta ctctcataaa tgggtgggag tattttgggt 300
gacaacctac tttgcttggc tgagtgaagg aatgatattc atatnttcat ttattccatg 360
gacatttagt tagtgctttt tatataccag gcatgatgct gagtgacact cttgtgtata 420
tntccaaatn ttngtncngt cgctgcacat atctgaaatc ctatattaag antttcccaa 480
natgangtcc ctggtttttc cacgccactt gatcngtcaa ngatctcacc tctgtntgtc 540
ctaaaaccnt ctnctnnang gttagacngg acctctcttc tcccttcccg aanaatnaag 600
tgtgngaaga nanccncncn cccccctncn tncnncctng ccngctnnnc cncntgtngg 660

gggngccgcc cccgcggggg gacccccccn ttttcccc 698

<210> 6
<211> 740
<212> DNA
<213> Homo sapien

<220>
<221> misc_feature
<222> (1)...(740)
<223> n = A,T,C or G

<400> 6
actagtcaaa aatgctaaaa taatttggga gaaaatattt tttaagtagt gttatagttt 60
catgtttatc ttttattatg tnttgtgaag ttgtgtcttt tcactaatta cctatactat 120
gccaatattt ccttatatct atccataaca tttatactac atttgtaaga gaatatgcac 180
gtgaaactta acactttata aggtaaaaat gaggtttcca agatttaata atctgatcaa 240
gttcttgtta tttccaaata gaatggactt ggtctgttaa ggggctaagg gagaagaaga 300
agataaggtt aaaagttgtt aatgaccaaa cattctaaaa gaaatgcaaa aaaaaattta 360
ttttcaagcc ttcgaactat ttaaggaaag caaaatcatt tcctanatgc atatcatttg 420
tgagantttc tcantaatat cctgaatcat tcatttcagc tnaggcttca tgttgactcg 480
atatgtcatc tagggaaagt ctatttcatg gtccaaacct gttgccatag ttggtnaggc 540
tttcctttaa ntgtgaanta ttnacangaa atttctcttt tnanagttct tnatagggtt 600
aggggtgtgg gaaaagcttc taacaatctg tagtgttncg tgttatctgt ncagaaccan 660
aatnacggat cgnangaagg actgggtcta tttacangaa cgaatnatct ngttnnntgt 720
gtnnncaact ccngggagcc 740

<210> 7
<211> 670
<212> DNA
<213> Homo sapien

<220>
<221> misc_feature
<222> (1)...(670)
<223> n = A,T,C or G

<400> 7
gctggggagc tcggcatggc ggtccccgct gcagccatgg ggccctcggc gttgggccag 60
agcggccccg gctcgatggc cccgtggtgc tcagtgagca gcggcccgtc gcgctacgtg 120
cttgggatgc aggagctgtt ccggggccac agcaagaccg cgagttcctg gcgcacagcg 180
ccaaggtgca ctcggtggcc tggagttgcg acgggcgtcg cctacctcgg ggtcttcgac 240
aagacgccac gtcttcttgc tgganaanga ccgttggtca aagaaaacaa ttatcgggga 300
catggggata gtgtggacca ctttgttggc atccaagtaa tcctgaccta tttgttacgg 360
cgtctggaga taaaaccatt cgcatctggg atgtgaggac tacaaaatgc attgccactg 420
tgaacactaa aggggagaac attaatatct gctggantcc tgatgggcan accattgctg 480
tagcnacaag gatgatgtgg tgactttatt gatgccaaga aaccccgttc caaagcaaaa 540
aaacanttcc aanttcgaag tcaccnaaat ctcctggaac aatgaacatn aatatntcct 600
tcctgacaat ggnccttggg tgtntcacat cctcagctnc cccaaaactg aancctgtnc 660
natccacccc 670

<210> 8
<211> 689
<212> DNA
<213> Homo sapien

<220>
<221> misc_feature
<222> (1)...(689)
<223> n = A,T,C or G

<400> 8
actagtatct aggaatgaac agtaaaagag gagcagttgg ctacttgatt acaacagagt 60
aaatgaagta ctggatttgg gaaaacctgg ttttattaga acatatggaa tgaaagccta 120
cacctagcat tgcctactta gccccctgaa ttaacagagc ccaattgaga caaacccctg 180
gcaacaggaa attcaaggga gaaaaagtaa gcaacttggg ctaggatgag ctgactccct 240
tagagcaaag ganagacagc ccccattacc aaataccatt tttgcctggg gcttgtgcag 300
ctggcagtgt tcctgcccca gcatggcacc ttatngtttt gatagcaact tcgttgaatt 360
ttcaccaact tattacttga aattataata tagcctgtcc gtttgctgtn tccaggctgt 420
gatatatntt cctagtggtt tgactttnaa aataaatnag gtttantttt ctccccccnn 480
cnntnctncc nntcnctcnn cnntcccccc cnctcngtcc tccnnnnttn gggggggccn 540
cccccncggn ggacccccct ttggtccctt agtggaggtt natggcccct ggnnttatcc 600
nggccntann tttccccgtn nnaaatgntt ccccctccca ntcccnccac ctcaanccgg 660
aagcctaagt ttntaccctg ggggtcccc 689

<210> 9
<211> 674
<212> DNA
<213> Homo sapien

<220>
<221> misc_feature
<222> (1)...(674)
<223> n = A,T,C or G

<400> 9
gtccactctc ctttgagtgt actgtcttac tgtgcactct gtttttcaac tttctagata 60
taaaaaatgc ttgttctata gtggagtaag agctcacaca cccaaggcag caagataact 120
gaaaaaagcg aggctttttt gccaccttgg taaaggccag ttcactgcta tagaactgct 180
ataagcctga agggaagtag ctatgagact ttccattttt cttagttctc ccaataggct 240
ccttcatgga aaaaggcttc ctgtaataat tttcacctaa tgaattagca gtgtgattat 300
ttctgaaata agagacaaat tgggccgcag agtcttcctg tgatttaaaa taaacaaccc 360
aaagttttgt ttggtcttca ccaaaggaca tactctaggg ggtatgttgt tgaagacatt 420
caaaaacatt agctgttctg tctttcaatt tcaagttatt ttggagactg cctccatgtg 480
agttaattac tttgctctgg aactagcatt attgtcatta tcatcacatt ctgtcatcat 540
catctgaata atattgtgga tttccccctc tgcttgcatc ttcttttgac tcctctggga 600
anaaatgtca aaaaaaaagg tcgatctact cngcaaggnc catctaatca ctgcgctgga 660
aggacccnct gccc 674

<210> 10
<211> 346
<212> DNA
<213> Homo sapien

<220>
<221> misc_feature
<222> (1)...(346)
<223> n = A,T,C or G

<400> 10

<221> misc_feature
<222> (1)...(694)
<223> n = A,T,C or G

<400> 13
cactagtcac tcattagcgt tttcaatagg gctcttaagt ccagtagatt acgggtagtc 60
agttgacgaa gatctggttt acaagaacta attaaatgtt tcattgcatt tttgtaagaa 120
cagaataatt ttataaaatg tttgtagttt ataattgccg aaaataattt aaagacactt 180
tttctctgtg tgtgcaaatg tgtgtttgtg atccattttt tttttttttt taggacacct 240
gtttactagc tagctttaca atatgccaaa aaaggatttc tccctgaccc catccgtggt 300
tcaccctctt ttccccccat gctttttgcc ctagtttata acaaaggaat gatgatgatt 360
taaaaagtag ttctgtatct tcagtatctt ggtcttccag aaccctctgg ttgggaaggg 420
gatcattttt tactggtcat ttccctttgg agtgtactac tttaacagat ggaaagaact 480
cattggccat ggaaacagcc gangtgttgg gagccagcag tgcatggcac cgtccggcat 540
ctggcntgat tggtctggct gccgtcattg tcagcacagt gccatgggac atggggaana 600
ctgactgcac ngccaatggt tttcatgaag aatacngcat ncncngtgat cacgtnancc 660
angacgctat gggggncana gggccanttg cttc 694

<210> 14
<211> 679
<212> DNA
<213> Homo sapien

<220>
<221> misc_feature
<222> (1)...(679)
<223> n = A,T,C or G

<400> 14
   cagccgcctg catctgtatc cagcgccang tcccgccagt cccagctgcg cgcgcccccc 60
   agtcccgnac ccgttcggcc cangctnagt tagncctcac catnccggtc aaaggangca 120
   ccaagtgcat caaatacctg cngtncggat ntaaattcat cttctggctt gccgggattg 180
   ctgtccntgc cattggacta nggctccgat ncgactctca gaccanganc atcttcganc 240
   naganactaa tnatnattnt tccagcttct acacaggagt ctatattctg atcggatccg 300
   gcnccctcnt gatgctggtg ggcttcctga gctgctgcgg ggctgtgcaa gagtcccant 360
   gcatgctggg actgttcttc ggcttcntct tggtgatatn cgccattgaa atacctgcgg 420
   ccatctgggg atattccact ncgatnatgt gattaaggaa ntccacggag ttttacaagg 480
   acacgtacaa cnacctgaaa accnnggatg anccccaccg ggaancnctg aangccatcc 540
   actatgcgtt gaactgcaat ggtttggctg gggnccttga acaatttaat cncatacatc 600
   tggccccann aaaggacntn ctcganncct tcnccgtgna attcngttct gatnccatca 660
   cagaagtctc gaacaatcc 679

<210> 15
<211> 695
<212> DNA
<213> Homo sapien

<220>
<221> misc_feature
<222> (1)...(695)
<223> n = A,T,C or G

<400> 15
actagtggat aaaggccagg gatgctgctc aacctcctac catgtacagg gacgtctccc 60
cattacaact acccaatccg aagtgtcaac tgtgtcagga ctaanaaacc ctggttttga 120

actagtctgc tgatagaaag cactatacat cctattgttt ctttctttcc aaaatcagcc 60
ttctgtctgt aacaaaaatg tactttatag agatggagga aaaggtctaa tactacatag 120
ccttaagtgt ttctgtcatt gttcaagtgt attttctgta acagaaacat atttggaatg 180
tttttctttt ccccttataa attgtaattc ctgaaatact gctgctttaa aaagtcccac 240
tgtcagatta tattatctaa caattgaata ttgtaaatat acttgtctta cctctcaata 300
aaagggtact tttctattan nnagnngnnn gnnnnataaa anaaaa 346

<210> 11
<211> 602
<212> DNA
<213> Homo sapien

<400> 11
actagtaaaa agcagcattg ccaaataatc cctaattttc cactaaaaat ataatgaaat 60
gatgttaagc tttttgaaaa gtttaggtta aacctactgt tgttagatta atgtatttgt 120
tgcttccctt tatctggaat gtggcattag cttttttatt ttaaccctct ttaattctta 180
ttcaattcca tgacttaagg ttggagagct aaacactggg atttttggat aacagactga 240
cagttttgca taattataat cggcattgta catagaaagg atatggctac cttttgttaa 300
atctgcactt tctaaatatc aaaaaaggga aatgaagtta taaatcaatt tttgtataat 360
ctgtttgaaa catgagtttt atttgcttaa tattagggct ttgccccttt tctgtaagtc 420
tcttgggatc ctgtgtagaa ctgttctcat taaacaccaa acagttaagt ccattctctg 480
gtactagcta caaattcggt ttcatattct acttaacaat ttaaataaac tgaaatattt 540
ctagatggtc tacttctgtt catataaaaa caaaacttga tttccaaaaa aaaaaaaaaa 600
aa 602

<210> 12
<211> 685
<212> DNA
<213> Homo sapien

<220>
<221> misc_feature
<222> (1)...(685)
<223> n = A,T,C or G

<400> 12
actagtcctg tgaaagtaca actgaaggca gaaagtgtta ggattttgca tctaatgttc 60
attatcatgg tattgatgga cctaagaaaa taaaaattag actaagcccc caaataagct 120
gcatgcattt gtaacatgat tagtagattt gaatatatag atgtagtatn ttgggtatct 180
aggtgtttta tcattatgta aaggaattaa agtaaaggac tttgtagttg tttttattaa 240
atatgcatat agtagagtgc aaaaatatag caaaaatana aactaaaggt agaaaagcat 300
tttagatatg ccttaatnta nnaactgtgc caggtggccc tcggaataga tgccaggcag 360
agaccagtgc ctgggtggtg cctccccttg tctgcccccc tgaagaactt ccctcacgtg 420
angtagtgcc ctcgtaggtg tcacgtggan tantggganc aggccgnncn gtnanaagaa 480
ancanngtga nagtttcncc gtngangcng aactgtccct gngccnnnac gctcccanaa 540
cntntccaat ngacaatcga gtttccnnnc tccngnaacc tngccgnnnn cnngcccnnc 600
cantntgnta accccgcgcc cggatcgctc tcnnntcgtt ctcncncnaa ngggntttcn 660
cnnccgccgt cncnnccccg cnncc 685

<210> 13
<211> 694
<212> DNA
<213> Homo sapien

<220>

ttaaaaaagg gcctgaaaaa aggggagcca caaatctgtc tgcttcctca cnttantcnt 180
tggcaaatna gcattctgtc tcnttggctg cngcctcanc ncaaaaaanc ngaactcnat 240
cnggcccagg aatacatctc ncaatnaacn aaattganca aggcnntggg aaatgccnga 300
tgggattatc ntccgcttgt tganctteta agtttcnttc ccttcattcn accctgccag 360
ccnagttctg ttagaaaaat gccngaattc naacnccggt tttcntactc ngaatttaga 420
tctncanaaa cttcctggcc acnattcnaa ttnanggnca cgnacanatn ccttccatna 480
ancncacccc acntttgana gccangacaa tgactgcntn aantgaaggc ntgaaggaan 540
aactttgaaa ggaaaaaaaa ctttgtttcc ggccccttcc aacncttctg tgttnancac 600
tgccttctng naaccctgga agcccngnga cagtgttaca tgttgttcta nnaaacngac 660
ncttnaatnt cnatcttccc nanaacgatt ncncc 695

<210> 16
<211> 669
<212> DNA
<213> Homo sapien

<220>
<221> misc_feature
<222> (1)...(669)
<223> n = A,T,C or G

<400> 16
   cgccgaagca gcagcgcagg ttgtccccgt ttcccctccc ccttcccttc tccggttgcc 60
   ttcccgggcc ccttacactc cacagtcccg gtcccgccat gtcccagaaa caagaagaag 120
   agaaccctgc ggaggagacc ggcgaggaga agcaggacac gcaggagaaa gaaggtattc 180
   tgcctgagag agctgaagag gcaaagctaa aggccaaata cccaagccta ggacaaaagc 240
   ctggaggctc cgacttcctc atgaagagac tccagaaagg gcaaaagtac tttgactcng 300
   gagactacaa catggccaaa gccaacatga agaataagca gctgccaagt gcangaccag 360
   acaagaacct ggtgactggt gatcacatcc ccaccccaca ggatctgccc agagaaagtc 420
   ctcgctcgtc accagcaagc ttgcgggtgg ccaagttgaa tgatgctgcc ggggctctgc 480
   canatctgag acgcttccct ccctgcccca cccgggtcct gtgctggctc ctgcccttcc 540
   tgcttttgca gccangggtc aggaagtggc ncnggtngtg gctggaaagc aaaacccttt 600
   cctgttggtg tcccacccat ggagcccctg gggcgagccc angaacttga ncctttttgt 660
   tntcttncc 669

<210> 17
<211> 697
<212> DNA
<213> Homo sapien

<220>
<221> misc_feature
<222> (1)...(697)
<223> n = A,T,C or G

<400> 17
gcaagatatg gacaactaag tgagaaggta atnctctact gctctagntn ctccnggcnn 60
gacgcgctga ggagannnac gctggcccan ctgccggcca cacacgggga tcntggtnat 120
gcctgcccan gggancccca ncnctcggan cccatntcac acccgnnccn tncgcccacn 180
ncctggctcn cncngcccng nccagctcnc gnccccctcc gccnnnctcn ttnncntctc 240
cncncccctcc ncnacnacct cctacccncg gctccctccc cagcccccccc ccgcaancct 300
ccacnacncc ntcnncncga ancnccnctc gcnctcngcc ccngccccct gcccccgcc 360
cncnacnncg cgntcccccg cgcncgcngc ctcnccccct cccacnacag ncncacccgc 420
agncacgcnc tccgcccnct gacgccccnn cccgccgcgc tcaccttcat ggnccnacng 480
ccccgctcnc nccnctgcnc gccgncnngg cgccccgccc cnnccgngtn ccncncgnng 540

ccccngcngn angcngtgcg cnncangncc gngccgnncn ncaccctccg nccnccgccc 600
cgcccgctgg gggctcccgc cncgcggntc antccccncc cntncgccca ctntccgntc 660
cnncnctcnc gctcngcgcn cgcccnccnc ccccccc 697

<210> 18
<211> 670
<212> DNA
<213> Homo sapien

<220>
<221> misc_feature
<222> (1)...(670)
<223> n = A,T,C or G

<400> 18
   ctcgtgtgaa gggtgcagta cctaagccgg agcggggtag aggcgggccg gcaccccctt 60
   ctgacctcca gtgccgccgg cctcaagatc agacatggcc cagaacttga acgacttggc 120
   gggacggctg cccgccgggc cccggggcat gggcacggcc ctgaagctgt tgctgggggc 180
   cggcgccgtg gcctacggtg tgcgcgaatc tgtgttcacc gtggaaggcg ggcncagagc 240
   catcttcttc aatcggatcg gtggagtgca caggacacta tcctgggccg anggccttca 300
   cttcaggatc cttggttcca gtaccccanc atctatgaca ttcgggccag acctcgaaaa 360
   aatctcctcc ctacaggctc caaagaccta cagatggtga atatctccct gcgagtgttg 420
   tctcgaccaa tgctcangaa cttcctaaca tgttccancg cctaagggct ggactacnaa 480
   gaacgantgt tgccgtccat tgtcacgaag tgctcaagaa tttnggtggc caagttcaat 540
   gncctcacnn ctgatcnccc agcggggcca agttanccct ggttgatccc cgggganctg 600
   acnnaaaagg gccaaggact tcccctcatc ctggataatg tggccntcac aaagctcaac 660
   tttanccacc 670

<210> 19
<211> 606
<212> DNA
<213> Homo sapien

<220>
<221> misc_feature
<222> (1)...(606)
<223> n = A,T,C or G

<400> 19
   actagtgcca acctcagctc ccaggccagt tctctgaatg tcgaggagtt ccaggatctc 60
   tggcctcagt tgtccttggt tattgatggg ggacaaattg gggatggcca gagccccgag 120
   tgtcgccttg gctcaactgt ggttgatttg tctgtgcccg gaaagtttgg catcattcgt 180
   ccaggctgtg ccctggaaag tactacagcc atcctccaac agaagtacgg actgctcccc 240
   tcacatgcgt cctacctgtg aaactctggg aagcaggaag gcccaagacc tggtgctgga 300
   tactatgtgt ctgtccactg acgactgtca aggcctcatt tgcagaggcc accggagcta 360
   gggcactagc ctgactttta aggcagtgtg tctttctgag cactgtagac caagcccttg 420
   gagctgctgg tttagccttg cacctgggga aaggatgtat ttatttgtat tttcatatat 480
   cagccaaaag ctgaatggaa aagttnagaa cattcctagg tggccttatt ctaataagtt 540
   tcttctgtct gttttgtttt tcaattgaaa agttattaaa taacagattt agaatctagt 600
   gagacc 606

<210> 20
<211> 449
<212> DNA
<213> Homo sapien

<400> 20
actagtaaac aacagcagca gaaacatcag tatcagcagc gtcgccagca ggagaatatg 60
cagcgccaga gccgaggaga acccccgctc cctgaggagg acctgtccaa actcttcaaa 120
ccaccacagc cgcctgccag gatggactcg ctgctcattg caggccagat aaacacttac 180
tgccagaaca tcaaggagtt cactgcccaa aacttaggca agctcttcat ggcccaggct 240
cttcaagaat acaacaacta agaaaaggaa gtttccagaa aagaagttaa catgaactct 300
tgaagtcaca ccagggcaac tcttggaaga aatatatttg catattgaaa agcacagagg 360
atttctttag tgtcattgcc gattttggct ataacagtgt ctttctagcc ataataaaat 420
aaaacaaaat cttgactgct tgctcaaaa 449

<210> 21
<211> 409
<212> DNA
<213> Homo sapien

<400> 21
tatcaatcaa ctggtgaata attaaacaat gtgtggtgtg atcatacaaa gggtaccact 60
caatgataaa aggaacaagc tgcctatatg tggaacaaca tggatgcatt tcagaaactt 120
tatgttgagt gaaagaacaa acacggagaa catactatgt ggttctcttt atgtaacatt 180
acagaaataa aaacagaggc aaccaccttt gaggcagtat ggagtgagat agactggaaa 240
aaggaaggaa ggaaactcta cgctgatgga aatgtctgtg tcttcattgg gtggtagtta 300
tgtggggata tacatttgtc aaaatttatt gaactatata ctaaagaact ctgcatttta 360
ttgggatgta aataatacct caattaaaaa gacaaaaaaa aaaaaaaaa 409

<210> 22
<211> 649
<212> DNA
<213> Homo sapien

<220>
<221> misc_feature
<222> (1)...(649)
<223> n = A,T,C or G

<400> 22
acaattttca ttatcttaag cacattgtac atttctacag aacctgtgat tattctcgca 60
tgataaggat ggtacttgca tatggtgaat tactactgtt gacagtttcc gcagaaatcc 120
tatttcagtg gaccaacatt gtggcatggc agcaaatgcc aacattttgt ggaatagcag 180
caaatctaca agagaccctg gttggttttt cgttttgttt tctttgtttt ttcccccttc 240
tcctgaatca gcagggatgg aangagggta gggaagttat gaattactcc ttccagtagt 300
agctctgaag tgtcacattt aatatcagtt ttttttaaac atgattctag ttnaatgtag 360
aagagagaag aaagaggaag tgttcacttt tttaatacac tgatttagaa atttgatgtc 420
ttatatcagt agttctgagg tattgatagc ttgctttatt tctgccttta cgttgacagt 480
gttgaagcag ggtgaataac taggggcata tatatttttt tttttgtaa gctgtttcat 540
gatgttttct ttggaatttc cggataagtt caggaaaaca tctgcatgtt gttatctagt 600
ctgaagttcn tatccatctc attacaacaa aaacncccag aacggnttg 649

<210> 23
<211> 669
<212> DNA
<213> Homo sapien

<220>
<221> misc_feature

<222> (1)...(669)
<223> n = A,T,C or G

<400> 23
actagtgccg tactggctga aatccctgca ggaccaggaa gagaaccagt tcagactttg 60
tactctcagt caccagctct ggaattagat aaattccttg aagatgtcag gaatgggatc 120
tatcctctga cagcctttgg gctgcctcgg ccccagcagc cacagcagga ggaggtgaca 180
tcacctgtcg tgccccccctc tgtcaagact ccgacacctg aaccagctga ggtggagact 240
cgcaaggtgg tgctgatgca gtgcaacatt gagtcggtgg aggagggagt caaacaccac 300
ctgacacttc tgctgaagtt ggaggacaaa ctgaaccggc acctgagctg tgacctgatg 360
ccaaatgaga atatccccga gttggcggct gagctggtgc agctgggctt cattagtgag 420
gctgaccaga gccggttgac ttctctgcta gaagagactt gaacaagttc aatttgcca 480
ggaacagtac cctcaactca gccgctgtca ccgtctcctc ttagagctca ctcgggccag 540
gccctgatct gcgctgtggc tgtcctggac gtgctgcacc ctctgtcctt ccccccagtc 600
agtattacct gtgaagccct tccctccttt attattcagg anggctgggg gggctccttg 660
nttctaacc 669

<210> 24
<211> 442
<212> DNA
<213> Homo sapien

<400> 24
actagtacca tcttgacaga ggatacatgc tcccaaaacg tttgttacca cacttaaaaa 60
tcactgccat cattaagcat cagtttcaaa attatagcca ttcatgattt actttttcca 120
gatgactatc attattctag tcctttgaat ttgtaagggg aaaaaaaaca aaaacaaaaa 180
cttacgatgc acttttctcc agcacatcag atttcaaatt gaaaattaaa gacatgctat 240
ggtaatgcac ttgctagtac tacacacttt ggtacaacaa aaaacagagg caagaaacaa 300
cggaaagaga aaagccttcc tttgttggcc cttaaactga gtcaagatct gaaatgtaga 360
gatgatctct gacgatacct gtatgttctt attgtgtaaa taaaattgct ggtatgaaat 420
gacctaaaaa aaaaaaaaga aa 442

<210> 25
<211> 656
<212> DNA
<213> Homo sapien

<220>
<221> misc_feature
<222> (1)...(656)
<223> n = A,T,C or G

<400> 25
tgcaagtacc acacactgtt tgaattttgc acaaaaagtg actgtaggat caggtgatag 60
ccccggaatg tacagtgtct tggtgcacca agatgccttc taaaggctga cataccttgg 120
accctaatgg ggcagagagt atagccctag cccagtggtg acatgaccac tccctttggg 180
aggcctgagg tagaggggag tggtatgtgt tttctcagtg gaagcagcac atgagtgggt 240
gacaggatgt tagataaagg ctctagttag ggtgtcattg tcatttgaga gactgacaca 300
ctcctagcag ctggtaaagg ggtgctggan gccatggagg anctctagaa acattagcat 360
gggctgatct gattacttcc tggcatcccg ctcactttta tgggaagtct tattagangg 420
atgggacagt tttccatatc cttgctgtgg agctctggaa cactctctaa atttccctct 480
attaaaaatc actgccctaa ctacacttcc tccttgaagg aatagaaatg gaactttctc 540
tgacatantt cttggcatgg ggagccagcc acaaatgana atctgaacgt gtccaggttt 600
ctcctganac tcatctacat agaattggtt aaaccctccc ttggaataag gaaaaa 656

<210> 26
<211> 434
<212> DNA
<213> Homo sapien

<220>
<221> misc_feature
<222> (1)...(434)
<223> n = A,T,C or G

<400> 26
actagttcag actgccacgc caaccccaga aaatacccca catgccagaa aagtgaagtc 60
ctaggtgttt ccatctatgt ttcaatctgt ccatctacca ggcctcgcga taaaaacaaa 120
acaaaaaaac gctgccaggt tttagaagca gttctggtct caaaaccatc aggatcctgc 180
caccagggtt cttttgaaat agtaccacat gtaaaaggga atttggcttt cacttcatct 240
aataactgaa ttgtcaggct ttgattgata attgtagaaa taagtagcct tctgttgtgg 300
gaataagtta taatcagtat tcatctcttt gtttttgtc actcttttct ctctaattgt 360
gtcatttgta ctgtttgaaa aatatttctt ctatnaaatt aaactaacct gccttaaaaa 420
aaaaaaaaaa aaaa 434

<210> 27
<211> 654
<212> DNA
<213> Homo sapien

<220>
<221> misc_feature
<222> (1)...(654)
<223> n = A,T,C or G

<400> 27
actagtccaa cacagtcaga aacattgttt tgaatcctct gtaaaccaag gcattaatct 60
taataaacca ggatccattt aggtaccact tgatataaaa aggatatcca taatgaatat 120
tttatactgc atcctttaca ttagccacta aatacgttat tgcttgatga agacctttca 180
cagaatccta tggattgcag catttcactt ggctacttca tacccatgcc ttaaagaggg 240
gcagtttctc aaaagcagaa acatgccgcc agttctcaag ttttcctcct aactccattt 300
gaatgtaagg gcagctggcc cccaatgtgg ggaggtccga acattttctg aattcccatt 360
ttcttgttcg cggctaaatg acagtttctg tcattactta gattccgatc tttcccaaag 420
gtgttgattt acaaagaggc cagctaatag cagaaatcat gaccctgaaa gagagatgaa 480
attcaagctg tgagccaggc agganctcag tatggcaaag gtcttgagaa tcngccattt 540
ggtacaaaaa aaattttaaa gcntttatgt tataccatgg aaccatagaa anggcaaggg 600
aattgttaag aanaatttta agtgtccaga cccanaanga aaaaaaaaaa aaaa 654

<210> 28
<211> 670
<212> DNA
<213> Homo sapien

<220>
<221> misc_feature
<222> (1)...(670)
<223> n = A,T,C or G

<400> 28
cgtgtgcaca tactgggagg atttccacag ctgcacggtc acagccctta cggattgcca 60

ggaaggggcg aaagatatgt gggataaact gagaaaagaa nccaaaaacc tcaacatcca 120
aggcagctta ttcgaactct gcggcagcgg caacggggcg gcggggtccc tgctcccggc 180
gttcccggtg ctcctggtgt ctctctcggc agctttagcg acctgncttt ccttctgagc 240
gtggggccag ctccccccgc ggcgcccacc cacnctcact ccatgctccc ggaaatcgag 300
aggaagatca ttagttcttt ggggacgttn gtgattctct gtgatgctga aaaacactca 360
tatagggaat gtgggaaatc ctganctctt tnttatntcg tntgatttct tgtgttttat 420
ttgccaaaat gttaccaatc agtgaccaac cnagcacagc caaaaatcgg acntcngctt 480
tagtccgtct tcacacacag aataagaaaa cggcaaaccc accccacttt tnantttnat 540
tattactaan tttttctgt tgggcaaaag aatctcagga acngccctgg ggccnccgta 600
ctanagttaa ccnagctagt tncatgaaaa atgatgggct ccncctcaat gggaaagcca 660
agaaaaagnc 670

<210> 29
<211> 551
<212> DNA
<213> Homo sapien

<220>
<221> misc_feature
<222> (1)...(551)
<223> n = A,T,C or G

<400> 29
actagtcctc cacagcctgt gaatccccct agacctttca agcatagtga gcggagaaga 60
agatctcagc gtttagccac cttacccatg cctgatgatt ctgtagaaaa ggtttcttct 120
ccctctccag ccactgatgg gaaagtattc tccatcagtt ctcaaaatca gcaagaatct 180
tcagtaccag aggtgcctga tgttgcacat ttgccacttg agaagctggg accctgtctc 240
cctcttgact taagtcgtgg ttcagaagtt acagcaccgg tagcctcaga ttcctcttac 300
cgtaatgaat gtcccagggc agaaaaagag gatacncaga tgcttccaaa tccttcttcc 360
aaagcaatag ctgatgggaa gaggagctcc agcagcagca ggaatatcga aaacagaaaa 420
aaaagtgaaa ttgggaagac aaaagctcaa cagcatttgg taaggagaaa aganaagatg 480
aggaaggaag agagaagaga gacnaagatc nctacggacc gnnncggaag aagaagaagn 540
aaaaaanaaa a 551

<210> 30
<211> 684
<212> DNA
<213> Homo sapien

<220>
<221> misc_feature
<222> (1)...(684)
<223> n = A,T,C or G

<400> 30
actagttcta tctggaaaaa gcccgggttg gaagaagctg tggagagtgc gtgtgcaatg 60
cgagactcat ttcttggaag catccctggc aaaaatgcag ctgagtacaa ggttatcact 120
gtgatagaac ctggactgct tttgagata atagagatgc tgcagtctga agagacttcc 180
agcacctctc agttgaatga attaatgatg gcttctgagt caactttact ggctcaggaa 240
ccacgagaga tgactgcaga tgtaatcgag cttaaaggga aattcctcat caacttagaa 300
ggtggtgata ttcgtgaaga gtcttcctat aaagtaattg tcatgccgac tacgaaagaa 360
aaatgccccc gttgttggaa gtatacagcg ggagtcttca gatacactgt gtcctcgatg 420
tgcagaagtt gtcagtggga aaatagtatt aacagctcac tcgagcaaga accctcctga 480
cagtactggg ctagaagttt ggatggatta tttacaatat aggaaagaaa gccaagaatt 540
aggtnatgag tggatgagta aatggtggan gatggggaat tcaaatcaga attatggaag 600

aagttnttcc tgttactata gaaaggaatt atgtttattt acatgcagaa aatatanatg 660
tgtggtgtgt accgtggatg gaan 684

<210> 31
<211> 654
<212> DNA
<213> Homo sapien

<220>
<221> misc_feature
<222> (1)...(654)
<223> n = A,T,C or G

<400> 31
gcgcagaaaa ggaaccaata tttcagaaac aagcttaata ggaacagctg cctgtacatc 60
aacatcttct cagaatgacc cagaagttat catcgtggga gctggcgtgc ttggctctgc 120
tttggcagct gtgctttcca gagatggaag aaaggtgaca gtcattgaga gagacttaaa 180
agagcctgac agaatagttg gagaattcct gcagccgggt ggttatcatg ttctcaaaga 240
ccttggtctt ggagatacag tggaaggtct tgatgcccag gttgtaaatg gttacatgat 300
tcatgatcag ggaaagcaaa tcagangttc agattcctta ccccctgtca gaaaacaatc 360
aagtgcagag tggaagagct ttccatcacg gaagattcat catgagtctc cggaaagcag 420
ctatggcaga gcccaatgca aagtttattg aaggtgttgt gttacagtta ttagaggaag 480
atgatgttgt gatgggagtt cagtacaagg ataaagagac tgggagatat caaggaactc 540
catgctccac tgactgttgt tgcagatggg ctttctccca anttcaggaa aagcctggtc 600
tcaataaagt ttctgtatca ctcatttggt tggcttctta tgaagaatgc nccc 654

<210> 32
<211> 673
<212> DNA
<213> Homo sapien

<220>
<221> misc_feature
<222> (1)...(673)
<223> n = A,T,C or G

<400> 32
actagtgaag aaaaagaaat tctgatacgg gacaaaaatg ctcttcaaaa catcattctt 60
tatcacctga caccaggagt tttcattgga aaaggatttg aacctggtgt tactaacatt 120
ttaaagacca cacaaggaag caaaatcttt ctgaaagaag taaatgatac acttctggtg 180
aatgaattga aatcaaaaga atctgacatc atgacaacaa atggtgtaat tcatgttgta 240
gataaactcc tctatccagc agacacacct gttggaaatg atcaactgct ggaaatactt 300
aataaattaa tcaaatacat ccaaattaag tttgttcgtg gtagcacctt caaagaaatc 360
cccgtgactg tctatnagcc aattattaaa aaatacacca aaatcattga tgggagtgcc 420
tgtgggaaat aactgaaaaa gagaccgaga agaacgaatc attacaggtc ctgaaataaa 480
atacctagga tttctactgg aggtggagaa acagaagaac tctgaagaaa ttgttacaag 540
aagangtccc aaggtcacca aattcattga aggtggtgat ggtctttatt tgaagatgaa 600
gaaattaaaa gacgcttcag ggagacnccc catgaaggaa ttgccagcca caaaaaaatt 660
cagggattag aaa 673

<210> 33
<211> 673
<212> DNA
<213> Homo sapien

<220>
<221> misc_feature
<222> (1)...(673)
<223> n = A,T,C or G

<400> 33
actagttatt tactttcctc cgcttcagaa ggtttttcag actgagagcc taagcatact 60
ggatctgttg tttcttttgg gtctcacctc atcagtgtgc atagtggcag aaattataaa 120
gaaggttgaa aggagcaggg aaaagatcca gaagcatgtt agttcgacat catcatcttt 180
tcttgaagta tgatgcatat tgcattattt tatttgcaaa ctaggaattg cagtctgagg 240
atcatttaga agggcaagtt caagaggata tgaagatttg agaacttttt aactattcat 300
tgactaaaaa tgaacattaa tgttnaagac ttaagacttt aacctgctgg cagtcccaaa 360
tgaaattatg caactttgat atcatattcc ttgatttaaa ttgggctttt gtgattgant 420
gaaactttat aaagcatatg gtcagttatt tnattaaaaa ggcaaaacct gaaccacctt 480
ctgcacttaa agaagtctaa cagtacaaat acctatctat cttagatgga tntatttntt 540
tntattttta aatattgtac tatttatggt nggtggggct ttcttactaa tacacaaatn 600
aatttatcat ttcaanggca ttctatttgg gtttagaagt tgattccaag nantgcatat 660
ttcgctactg tnt 673

<210> 34
<211> 684
<212> DNA
<213> Homo sapien

<220>
<221> misc_feature
<222> (1)...(684)
<223> n = A,T,C or G

<400> 34
actagtttat tcaagaaaag aacttactga ttcctctgtt cctaaagcaa gagtggcagg 60
tgatcagggc tggtgtagca tccggttcct ttagtgcagc taactgcatt tgtcactgat 120
gaccaaggag gaaatcacta agacatttga gaagcagtgg tatgaacgtt cttggacaag 180
ccacagttct gagccttaac cctgtagttt gcacacaaga acgagctcca cctccccttc 240
ttcaggagga atctgtgcgg atagattggc tggacttttc aatggttctg ggttgcaagt 300
gggcactgtt atggctgggt atggagcgga cagccccagg aatcagagcc tcagcccggc 360
tgcctggttg gaaggtacag gtgttcagca ccttcggaaa aagggcataa agtngtgggg 420
gacaattctc agtccaagaa gaatgcattg accattgctg gctatttgct tncctagtan 480
gaattggatn catttttgac cangatnntt ctnctatgct ttnttgcaat gaaatcaaat 540
cccgcattat ctacaagtgg tatgaagtcc tgcnnccccc agagaggctg ttcaggcnat 600
gtcttccaag ggcagggtgg gttacaccat tttacctccc ctctcccccc agattatgna 660
cncagaagga atttntttcc tccc 684

<210> 35
<211> 614
<212> DNA
<213> Homo sapien

<220>
<221> misc_feature
<222> (1)...(614)
<223> n = A,T,C or G

<400> 35
   actagtccaa cgcgttngcn aatattcccc tggtagccta ctttcttacc cccgaatatt 60
   ggtaagatcg agcaatggct tcaggacatg ggttctcttc tcctgtgatc attcaagtgc 120
   tcactgcatg aagactggct tgtctcagtg tntcaacctc accagggctg tctcttggtc 180
   cacacctcgc tccctgttag tgccgtatga cagcccccat canatgacct tggccaagtc 240
   acggtttctc tgtggtcaat gttggtnggc tgattggtgg aaagtanggt ggaccaaagg 300
   aagncncgtg agcagncanc nccagttctg caccagcagc gcctccgtcc tactngggtg 360
   ttccngtttc tcctggccct gngtgggcta nggcctgatt cgggaanatg cctttgcang 420
   gaagggamga taantgggat ctaccaattg attctggcaa aacnatntct aagattnttn 480
   tgctttatgt ggganacana tctanctctc atttnntgct gnanatnaca ccctactcgt 540
   gntcgancnc gtcttcgatt ttcgganaca cnccantnaa tactggcgtt ctgttgttaa 600
   aaaaaaaaaa aaaa 614

<210> 36
<211> 686
<212> DNA
<213> Homo sapien

<220>
<221> misc_feature
<222> (1)...(686)
<223> n = A,T,C or G

<400> 36
gtggctggcc cggttctccg cttctcccca tcccctactt tcctccctcc ctccctttcc 60
ctccctcgtc gactgttgct tgctggtcgc agactccctg acccctccct cacccctccc 120
taacctcggt gccaccggat tgcccttctt ttcctgttgc ccagcccagc cctagtgtca 180
gggcgggggc ctggagcagc ccgaggcact gcagcagaag ananaaaaga cacgacnaac 240
ctcagctcgc cagtccggtc gctngcttcc cgccgcatgg caatnagaca gacgccgctc 300
acctgctctg ggcacacgcg acccgtggtt gatttggcct tcagtggcat cacccttatg 360
ggtatttctt aatcagcgct tgcaaagatg gttaacctat gctacgccag ggagatacag 420
gagactggat tggaacattt ttggggtcta aaggtctgtt tggggtgcaa cactgaataa 480
ggatgccacc aaagcagcta cagcagctgc agatttcaca gcccaagtgt gggatgctgt 540
ctcagganat naattgataa cctggctcat aacacattgt caagaatgtg gatttcccca 600
ggatattatt atttgtttac cggggganag gataactgtt tcncntattt taattgaaca 660
aactnaaaca aaanctaagg aaatcc 686

<210> 37
<211> 681
<212> DNA
<213> Homo sapien

<220>
<221> misc_feature
<222> (1)...(681)
<223> n = A,T,C or G

<400> 37
   gagacanacn naacgtcang agaanaaaag angcatggaa cacaanccag gcncgatggc 60
   caccttccca ccagcancca gcgcccccca gcngccccca ngnccggang accangactc 120
   cancctgnat caatctganc tctattcctg gcccatnccc acctcggagg tggangccgn 180
   aaaggtcgca cnnncagaga agctgctgcc ancaccancc gccccnnccc tgncgggctn 240
   nataggaaac tggtgaccnn gctgcanaat tcatacagga gcacgcgang ggcacnnnct 300
   cacactgagt tnnngatgan gcctnaccan ggacctnccc cagcnnattg annacnggac 360
   tgcggaggaa ggaagacccc gnacnggatc ctggccggcn tgccaccccc ccacccctag 420
   gattatnccc cttgactgag tctctgaggg gctacccgaa cccgcctcca ttccctacca 480
   natnntgctc natcgggact gacangctgg ggatnggagg ggctatcccc cancatcccc 540
   tnanaccaac agcnacngan natnggggct ccccngggtc ggngcaacnc tcctncaccc 600
   cggcgcnggc cttcggtgnt gtcctccntc aacnaattcc naaanggcgg gccccccngt 660
   ggactcctcn ttgttccctc c 681

<210> 38
<211> 687
<212> DNA
<213> Homo sapien

<220>
<221> misc_feature
<222> (1)...(687)
<223> n = A,T,C or G

<400> 38
canaaaaaaa aaaacatggc cgaaaccagn aagctgcgcg atggcgccac ggcccctctt 60
ctcccggcct gtgtccggaa ggtttccctc cgaggcgccc cggctcccgc aagcggagga 120
gagggcggga cntgccgggg ccggagctca naggccctgg ggccgctctg ctctcccgcc 180
atcgcaaggg cggcgctaac ctnaggcctc cccgcaaagg tccccnangc ggnggcggcg 240
gggggctgtg anaaccgcaa aaanaacgct gggcgcgcng cgaacccgtc cacccccgcg 300
aaggananac ttccacagan gcagcgtttc cacagcccan agccacnttt ctagggtgat 360
gcaccccagt aagttcctgn cggggaagct caccgctgtc aaaaaanctc ttcgctccac 420
cggcgcacna aggggangan ggcangangc tgccgcccgc acaggtcatc tgatcacgtc 480
gcccgcccta ntctgctttt gtgaatctcc actttgttca accccacccg ccgttctctc 540
ctccttgcgc cttcctctna ccttaanaac cagcttcctc tacccnatng tanttnctct 600
gcncnngtng aaattaattc ggtccnccgg aacctcttnc ctgtggcaac tgctnaaaga 660
aactgctgtt ctgnttactg cngtccc 687

<210> 39
<211> 695
<212> DNA
<213> Homo sapien

<220>
<221> misc_feature
<222> (1)...(695)
<223> n = A,T,C or G

<400> 39
   actagtctgg cctacaatag tgtgattcat gtaggacttc tttcatcaat tcaaaacccc 60
   tagaaaaacg tatacagatt atataagtag ggataagatt tctaacattt ctgggctctc 120
   tgacccctgc gctagactgt ggaaagggag tattattata gtatacaaca ctgctgttgc 180
   cttattagtt ataacatgat aggtgctgaa ttgtgattca caatttaaaa acactgtaat 240
   ccaaactttt ttttttaact gtagatcatg catgtgaatg ttaatgttaa tttgttcaan 300
   gttgttatgg gtagaaaaaa ccacatgcct taaaatttta aaaagcaggg cccaaactta 360
   ttagtttaaa attaggggta tgtttccagt ttgttattaa ntggttatag ctctgtttag 420
   aanaaatcna ngaacangat ttngaaantt aagntgacat tatttnccag tgacttgtta 480
   atttgaaatc anacacggca ccttccgttt tggtnctatt ggnntttgaa tccaancngg 540
   ntccaaatct tnttggaaac ngtccnttta acttttttac nanatcttat ttttttattt 600
   tggaatggcc ctatttaang ttaaaagggg ggggnnccac naccattcnt gaataaaact 660
   naatatatat ccttggtccc ccaaaattta aggng 695

<210> 40
<211> 674
<212> DNA

<213> Homo sapien

<220>
<221> misc_feature
<222> (1)...(674)
<223> n = A,T,C or G

<400> 40
actagtagtc agttgggagt ggttgctata ccttgacttc atttatatga atttccactt 60
tattaaataa tagaaaagaa aatcccggtg cttgcagtag agttatagga cattctatgc 120
ttacagaaaa tatagccatg attgaaatca aatagtaaag gctgttctgg ctttttatct 180
tcttagctca tcttaaataa gtagtacact tgggatgcag tgcgtctgaa gtgctaatca 240
gttgtaacaa tagcacaaat cgaacttagg atgtgtttct tctcttctgt gtttcgattt 300
tgatcaattc tttaattttg ggaacctata atacagtttt cctattcttg gagataaaaa 360
ttaaatggat cactgatatt taagtcattc tgcttctcat ctnaatattc catattctgt 420
attagganaa antacctccc agcacagccc cctctcaaac cccacccaaa accaagcatt 480
tggaatgagt ctcctttatt tccgaantgt ggatggtata acccatatcn ctccaatttc 540
tgnttgggtt gggtattaat ttgaactgtg catgaaaagn ggnaatcttt nctttgggtc 600
aaantttncc ggttaatttg nctngncaaa tccaatttnc tttaagggtg tctttataaa 660
atttgctatt cngg 674

<210> 41
<211> 657
<212> DNA
<213> Homo sapien

<220>
<221> misc_feature
<222> (1)...(657)
<223> n = A,T,C or G

<400> 41
gaaacatgca agtaccacac actgtttgaa ttttgcacaa aaagtgactg tagggatcag 60
gtgatagccc cggaatgtac agtgtcttgg tgcaccaaga tgccttctaa aggctgacat 120
accttgggac cctaatgggg cagagagtat agccctagcc cagtggtgac atgaccactc 180
cctttgggag gctgaagtta aagggaatgg tatgtgtttt ctcatggaag cagcacatga 240
atnggtnaca ngatgttaaa ntaaggntct antttgggtg tcttgtcatt tgaaaaantg 300
acacactcct ancanctggt aaaggggtgc tggaagccat ggaagaactc taaaaacatt 360
agcatgggct gatctgatta cttcctggca tcccgctcac ttttatggga agtcttatta 420
naaggatggg anantttccc atatccttgc tgttggaact ctggaacact ctctaaattt 480
ccctctatta aaaatcactg nccttactac acttcctcct tganggaata gaaatggacc 540
tttctctgac ttagttcttg gcatgggang cagcccaaat taaaatctga cttntccggt 600
ttctccngaa ctcacctact tgaattggta aaacctcctt tggaattagn aaaaacc 657

<210> 42
<211> 389
<212> DNA
<213> Homo sapien

<220>
<221> misc_feature
<222> (1)...(389)
<223> n = A,T,C or G

<400> 42

actagtgctg aggaatgtaa acaagtttgc tgggccttgc gagacttcac caggttgttt 60
cgatagctca cactcctgca ctgtgcctgt cacccaggaa tgtctttttt aattagaaga 120
caggaagaaa acaaaaacca gactgtgtcc cacaatcaga aacctccgtt gtggcagang 180
ggccttcacc gccaccaggg tgtcccgcca gacagggaga gactccagcc ttctgaggcc 240
atcctgaaga attcctgttt gggggttgtg aaggaaaatc acccggattt aaaaagatgc 300
tgttgcctgc ccgcgtngtn gggaagggac tggtttcctg gtgaatttct taaaagaaaa 360
atattttaag ttaagaaaaa aaaaaaaaa 389

<210> 43
<211> 279
<212> DNA
<213> Homo sapien

<400> 43
actagtgaca agctcctggt cttgagatgt cttctcgtta aggagatggg ccttttggag 60
gtaaaggata aaatgaatga gttctgtcat gattcactat tctagaactt gcatgacctt 120
tactgtgtta gctctttgaa tgttcttgaa atttagactt ttctttgtaa acaaataata 180
tgtccttatc attgtataaa agctgttatg tgcaacagtg tggagatcct tgtctgattt 240
aataaaatac ttaaacactg aaaaaaaaaa aaaaaaaaa 279

<210> 44
<211> 449
<212> DNA
<213> Homo sapien

<220>
<221> misc_feature
<222> (1)...(449)
<223> n = A,T,C or G

<400> 44
   actagtagca tcttttctac aacgttaaaa ttgcagaagt agcttatcat taaaaaacaa 60
   caacaacaac aataacaata aatcctaagt gtaaatcagt tattctaccc cctaccaagg 120
   atatcagcct gttttttccc ttttttctcc tgggaataat tgtgggcttc ttcccaaatt 180
   tctacagcct ctttcctctt ctcatgcttg agcttccctg tttgcacgca tgcgttgtgc 240
   aagantgggc tgtttngctt ggantncggt ccnagtggaa ncatgctttc ccttgttact 300
   gttggaagaa actcaaacct tcnancccta ggtgttncca ttttgtcaag tcatcactgt 360
   atttttgtac tggcattaac aaaaaaagaa atnaaatatt gttccattaa actttaataa 420
   aactttaaaa gggaaaaaaa aaaaaaaaa 449

<210> 45
<211> 559
<212> DNA
<213> Homo sapien

<220>
<221> misc_feature
<222> (1)...(559)
<223> n = A,T,C or G

<400> 45
actagtgtgg gggaatcacg gacacttaaa gtcaatctgc gaaataattc ttttattaca 60
cactcactga agtttttgag tcccagagag ccattctatg tcaaacattc caagtacttt 120
ttgagagccc agcattacat caacatgccc gtgcagttca aaccgaagtc cgcaggcaaa 180
tttgaagctt tgcttgtcat tcaaacagat gaaggcaaga gtattgctat tcgactaatt 240

ggtgaagctc ttggaaaaaa ttnactagaa tactttttgt gttaagttaa ttacataagt 300
tgtattttgt taactttatc tttctacact acaattatgc ttttgtatat atattttgta 360
tgatggatat ctataattgt agattttgtt tttacaagct aatactgaag actcgactga 420
aatattatgt atctagccca tagtattgta cttaactttt acagggtgaa aaaaaaattc 480
tgtgtttgca ttgattatga tattctgaat aaatatggga atatatttta atgtgggtaa 540
aaaaaaaaaa aaaaaggaa 559

<210> 46
<211> 731
<212> DNA
<213> Homo sapien

<220>
<221> misc_feature
<222> (1)...(731)
<223> n = A,T,C or G

<400> 46
actagttcta gtaccatggc tgtcatagat gcaaccatta tattccattt agtttctttc 60
tcaggttccc taacaattgt ttgaaactga atatatatgt ttatgtatgt gtgtgtgttc 120
actgtcatgt atatggtgta tatgggatgt gtgcagtttt cagttatata tatattcata 180
tatacatatg catatatatg tataatatac atatatacat gcatacactt gtataatata 240
catatatata cacatatatg cacacatatn atcactgagt tccaaagtga gtctttattt 300
ggggcaattg tattctctcc ctctgtctgc tcactgggcc tttgcaagac atagcaattg 360
cttgatttcc tttggataag agtcttatct tcggcactct tgactctagc cttaacttta 420
gatttctatt ccagaatacc tctcatatct atcttaaaac ctaaganggg taaagangtc 480
ataagattgt agtatgaaag antttgctta gttaaattat atctcaggaa actcattcat 540
ctacaaatta aattgtaaaa tgatggtttg ttgtatctga aaaaatgttt agaacaagaa 600
atgtaactgg gtacctgtta tatcaaagaa cctcnattta ttaagtctcc tcatagccan 660
atccttatat ngccctctct gacctgantt aatananact tgaataatga atagttaatt 720
taggnttggg c 731

<210> 47
<211> 640
<212> DNA
<213> Homo sapien

<220>
<221> misc_feature
<222> (1)...(640)
<223> n = A,T,C or G

<400> 47
tgcgngccgg tttggccctt ctttgtanga cactttcatc cgccctgaaa tcttcccgat 60
cgttaataac tcctcaggtc cctgcctgca cagggttttt tcttantttg ttgcctaaca 120
gtacaccaaa tgtgacatcc tttcaccaat atngattnct tcataccaca tcntcnatgg 180
anacgactnc aacaattttt tgatnacccn aaanactggg ggctnnaana agtacantct 240
ggagcagcat ggacctgtcn gcnactaang gaacaanagt nntgaacatt tacacaacct 300
ttggtatgtc ttactgaaag anagaaacat gcttctnncc ctagaccacg aggncaaccg 360
caganattgc caatgccaag tccgagcggt tagatcaggt aatacattcc atggatgcat 420
tacatacntt gtccccgaaa nanaagatgc cctaanggct tcttcanact ggtccngaaa 480
acanctacac ctggtgcttg ganaacanac tctttggaag atcatctggc acaagttccc 540
cccagtgggt tttnccttgg cacctanctt accanatcna ttcggaancc attctttgcc 600
ntggcnttnt nttgggacca ntcttctcac aactgnaccc 640

<210> 48
<211> 257
<212> DNA
<213> Homo sapien

<400> 48
actagtatat gaaaatgtaa atatcacttg tgtactcaaa caaaagttgg tcttaagctt 60
ccaccttgag cagccttgga aacctaacct gcctctttta gcataatcac attttctaaa 120
tgattttctt tgttcctgaa aaagtgattt gtattagttt tacatttgtt ttttggaaga 180
ttatatttgt atatgtatca tcataaaata tttaaataaa aagtatcttt agagtgaaaa 240
aaaaaaaaaa aaaaaaa 257

<210> 49
<211> 652
<212> DNA
<213> Homo sapien

<220>
<221> misc_feature
<222> (1)...(652)
<223> n = A,T,C or G

<400> 49
actagttcag atgagtggct gctgaagggg ccccccttgtc attttcatta taacccaatt 60
tccacttatt tgaactctta agtcataaat gtataatgac ttatgaatta gcacagttaa 120
gttgacacta gaaactgccc atttctgtat tacactatca aataggaaac attggaaaga 180
tggggaaaaa aatcttattt taaaatggct tagaaagttt tcagattact ttgaaaattc 240
taaacttctt tctgtttcca aaacttgaaa atatgtagat ggactcatgc attaagactg 300
ttttcaaagc tttcctcaca tttttaaagt gtgattttcc ttttaatata catatttatt 360
ttctttaaag cagctatatc ccaacccatg actttggaga tatacctatn aaaccaatat 420
aacagcangg ttattgaagc agctttctca aatgttgctt cagatgtgca agttgcaaat 480
tttattgtat ttgtanaata caatttttgt tttaaactgt atttcaatct atttctccaa 540
gatgcttttc atatagagtg aaatatccca ngataactgc ttctgtgtcg tcgcatttga 600
cgcataactg cacaaatgaa cagtgtatac ctcttggttg tgcattnacc cc 652

<210> 50
<211> 650
<212> DNA
<213> Homo sapien

<220>
<221> misc_feature
<222> (1)...(650)
<223> n = A,T,C or G

<400> 50
ttgcgctttg atttttttag ggcttgtgcc ctgtttcact tatagggtct agaatgcttg 60
tgttgagtaa aaaggagatg cccaatattc aaagctgcta aatgttctct ttgccataaa 120
gactccgtgt aactgtgtga acacttggga tttttctcct ctgtcccgag gtcgtcgtct 180
gctttctttt ttgggttctt tctagaagat tgagaaatgc atatgacagg ctgagancac 240
ctccccaaac acacaagctc tcagccacan gcagcttctc cacagcccca gcttcgcaca 300
ggctcctgga nggctgcctg ggggaggcag acatgggagt gccaaggtgg ccagatggtt 360
ccaggactac aatgtcttta tttttaactg tttgccactg ctgccctcac ccctgcccgg 420
ctctggagta ccgtctgccc canacaagtg ggantgaaat gggggtgggg gggaacactg 480
attcccantt agggggtgcc taactgaaca gtagggatan aaggtgtgaa cctgngaant 540

gcttttataa attatnttcc ttgttanatt tattttttaa tttaatctct gttnaactgc 600
ccngggaaaa ggggaaaaaa aaaaaaaaat tctntttaaa cacatgaaca 650

<210> 51
<211> 545
<212> DNA
<213> Homo sapien

<220>
<221> misc_feature
<222> (1)...(545)
<223> n = A,T,C or G

<400> 51
tggcgtgcaa ccagggtagc tgaagtttgg gtctgggact ggagattggc cattaggcct 60
cctganattc cagctccctt ccaccaagcc cagtcttgct acgtggcaca gggcaaacct 120
gactcccttt gggcctcagt ttcccctccc cttcatgana tgaaaagaat actacttttt 180
cttgttggtc taacnttgct ggacncaaag tgtngtcatt attgttgtat tgggtgatgt 240
gtncaaaact gcagaagctc actgcctatg agaggaanta agagagatag tggatganag 300
ggacanaagg agtcattatt tggtatagat ccacccntcc caacctttct ctcctcagtc 360
cctgcncctc atgtntctgg tntggtgagt cctttgtgcc accanccatc atgctttgca 420
ttgctgccat cctgggaagg gggtgnatcg tctcacaact tgttgtcatc gtttganatg 480
catgctttct tnatnaaaca aanaaannaa tgtttgacag ngtttaaaat aaaaaanaaa 540
caaaa 545

<210> 52
<211> 678
<212> DNA
<213> Homo sapien

<220>
<221> misc_feature
<222> (1)...(678)
<223> n = A,T,C or G

<400> 52
actagtagaa gaactttgcc gcttttgtgc ctctcacagg cgcctaaagt cattgccatg 60
ggaggaagac gatttggggg gggagggggg gggggcangg tccgtggggc tttccctant 120
ntatctccat ntccantgnn cnntgtcgcc tcttccctcg tcncattnga anttantccc 180
tggnccccnn nccctctccn ncctncncct cccccctccg ncncctccnn ctttttntan 240
ncttccccat ctccntcccc cctnanngtc ccaacnccgn cagcaatnnc ncacttnctc 300
nctccncncc tccnnccgtt cttctnttct cnacntntnc ncnnntnccn tgccnntnaa 360
annctctccc cnctgcaanc gattctctcc ctccncnnan ctntccactc cntncttctc 420
ncncgctcct nttcntcnnc ccacctctcn ccttcgnccc cantacnctc nccncccttn 480
cgnntcnttn nnntcctcnn accncccncc tcccttcncc cctcttctcc ccggtntntc 540
tctctcccnc nncncnncct cnncccntcc nngcgnccnt ttccgccccn cnccnccntt 600
ccttcntcnc cantccatcn cntntnccat nctncctncc nctcacnccc gctncccccn 660
ntctctttca cacngtcc 678

<210> 53
<211> 502
<212> DNA
<213> Homo sapien

<220>

<221> misc_feature
<222> (1)...(502)
<223> n = A,T,C or G

<400> 53
tgaagatcct ggtgtcgcca tgggccgccg ccccgcccgt tgttaccggt attgtaagaa 60
caagccgtac ccaaagtctc gcttctgccg aggtgtccct gatgccaaaa ttcgcatttt 120
tgacctgggg cggaaaaang caaaantgga tgagtctccg ctttgtggcc acatggtgtc 180
agatcaatat gagcagctgt cctctgaagc cctgnangct gcccgaattt gtgccaataa 240
gtacatggta aaaagtngtg gcnaagatgc ttccatatcc gggtgcggnt ccaccccttc 300
cacgtcatcc gcatcaacaa gatgttgtcc tgtgctgggg ctgacaggct cccaacaggc 360
atgcgaagtg cctttggaaa acccanggca ctgtggccag ggttcacatt gggccaattn 420
atcatgttca tccgcaccaa ctgcagaaca angaacntgt naattnaagc cctgcccagg 480
gncaanttca aatttcccgg cc 502

<210> 54
<211> 494
<212> DNA
<213> Homo sapien

<220>
<221> misc_feature
<222> (1)...(494)
<223> n = A,T,C or G

<400> 54
actagtccaa gaaaaatatg cttaatgtat attacaaagg ctttgtatat gttaacctgt 60
tttaatgcca aaagtttgct ttgtccacaa tttccttaag acctcttcag aaagggattt 120
gtttgcctta atgaatactg ttgggaaaaa acacagtata atgagtgaaa agggcagaag 180
caagaaattt ctacatctta gcgactccaa gaagaatgag tatccacatt tagatggcac 240
attatgagga ctttaatctt tccttaaaca caataatgtt ttcttttttc ttttattcac 300
atgatttcta agtatatttt tcatgcagga cagtttttca accttgatgt acagtgactg 360
tgttaaattt ttctttcagt ggcaacctct ataatcttta aaatatggtg agcatcttgt 420
ctgttttgaa ngggatatga cnatnaatct atcagatggg aaatcctgtt tccaagttag 480
aaaaaaaaaa aaaa 494

<210> 55
<211> 606
<212> DNA
<213> Homo sapien

<220>
<221> misc_feature
<222> (1)...(606)
<223> n = A,T,C or G

<400> 55
actagtaaaa agcagcattg ccaaataatc cctaattttc cactaaaaat ataatgaaat 60
gatgttaagc tttttgaaaa gtttaggtta aacctactgt tgttagatta atgtatttgt 120
tgcttccctt tatctggaat gtggcattag cttttttatt ttaaccctct ttaattctta 180
ttcaattcca tgacttaagg ttggagagct aaacactggg atttttggat aacagactga 240
cagttttgca taattataat cggcattgta catagaaagg atatggctac cttttgttaa 300
atctgcactt tctaaatatc aaaaaaggga aatgaagtat aaatcaattt ttgtataatc 360
tgtttgaaac atgantttta tttgcttaat attanggctt tgcccttttc tgttagtctc 420
ttgggatcct gtgtaaaact gttctcatta aacaccaaac agttaagtcc attctctggt 480

actagctaca aattccgttt catattctac ntaacaattt aaattaactg aaatatttct 540
anatggtcta cttctgtcnt ataaaaacna aacttgantt nccaaaaaaa aaaaaaaaaa 600
aaaaaa 606

<210> 56
<211> 183
<212> DNA
<213> Homo sapien

<400> 56
actagtatat ttaaacttac aggcttattt gtaatgtaaa ccaccatttt aatgtactgt 60
aattaacatg gttataatac gtacaatcct tccctcatcc catcacacaa ctttttttgt 120
gtgtgataaa ctgattttgg tttgcaataa aaccttgaaa aataaaaaaa aaaaaaaaaa 180
aaa 183

<210> 57
<211> 622
<212> DNA
<213> Homo sapien

<220>
<221> misc_feature
<222> (1)...(622)
<223> n = A,T,C or G

<400> 57
actagtcact actgtcttct ccttgtagct aatcaatcaa tattcttccc ttgcctgtgg 60
gcagtggaga gtgctgctgg gtgtacgctg cacctgccca ctgagttggg gaaagaggat 120
aatcagtgag cactgttctg ctcagagctc ctgatctacc ccaccccta ggatccagga 180
ctgggtcaaa gctgcatgaa accaggccct ggcagcaacc tgggaatggc tggaggtggg 240
agagaacctg acttctcttt ccctctccct cctccaacat tactggaact ctatcctgtt 300
agggatcttc tgagcttgtt tccctgctgg gtgggacaga agacaaagga gaagggangg 360
tctacaanaa gcagcccttc tttgtcctct ggggttaatg agcttgacct ananttcatg 420
gaganaccan aagcctctga tttttaattt ccntnaaatg tttgaagtnt atatntacat 480
atatatattt ctttnaatnt ttgagtcttt gatatgtctt aaaatccant ccctctgccn 540
gaaacctgaa ttaaaaccat gaanaaaaat gtttncctta aagatgttan taattaattg 600
aaacttgaaa aaaaaaaaa aa 622

<210> 58
<211> 433
<212> DNA
<213> Homo sapien

<400> 58
   gaacaaattc tgattggtta tgtaccgtca aaagacttga agaaatttca tgattttgca 60
   gtgtggaagc gttgaaaatt gaaagttact gcttttccac ttgctcatat agtaaaggga 120
   tcctttcagc tgccagtgtt gaataatgta tcatccagag tgatgttatc tgtgacagtc 180
   accagcttta agctgaacca ttttatgaat accaaataaa tagacctctt gtactgaaaa 240
   catatttgtg actttaatcg tgctgcttgg atagaaatat ttttactggt tcttctgaat 300
   tgacagtaaa cctgtccatt atgaatggcc tactgttcta ttatttgttt tgacttgaat 360
   ttatccacca aagacttcat ttgtgtatca tcaataaagt tgtatgtttc aactgaaaaa 420
   aaaaaaaaaa aaa 433

<210> 59
<211> 649

<212> DNA
<213> Homo sapien

<220>
<221> misc_feature
<222> (1)...(649)
<223> n = A,T,C or G

<400> 59
actagttatt atctgacttt cnggttataa tcattctaat gagtgtgaag tagcctctgg 60
tgtcatttgg atttgcattt ctctgatgag tgatgctatc aagcaccttt gctggtgctg 120
ttggccatat gtgtatgttc cctggagaag tgtctgtgct gagccttggc ccacttttta 180
attaggcgtn tgtcttttta ttactgagtt gtaagantte tttatatatt ctggattcta 240
gacccttatc agatacatgg tttgcaaata ttttctccca ttctgtgggt tgtgttttca 300
ctttatcgat aatgtcctta gacatataat aaatttgtat tttaaaagtg acttgatttg 360
ggctgtgcaa ggtgggctca cgcttgtaat cccagcactt tgggagactg aggtgggtgg 420
atcatatgan gangctagga gttcgaggtc agcctggcca gcatagcgaa aacttgtctc 480
tacnaaaaat acaaaaatta gtcaggcatg gtggtgcacg tctgtaatac cagcttctca 540
ggangctgan gcacaaggat cacttgaacc ccagaangaa gangttgcag tganctgaag 600
atcatgccag ggcaacaaaa atgagaactt gtttaaaaaa aaaaaaaaa 649

<210> 60
<211> 423
<212> DNA
<213> Homo sapien

<220>
<221> misc_feature
<222> (1)...(423)
<223> n = A,T,C or G

<400> 60
actagttcag gccttccagt tcactgacaa acatggggaa gtgtgcccag ctggctggaa 60
acctggcagt gataccatca agcctgatgt ccaaaagagc aaagaatatt tctccaagca 120
gaagtgagcg ctgggctgtt ttagtgccag gctgcggtgg gcagccatga gaacaaaacc 180
tcttctgtat tttttttttc cattagtana acacaagact cngattcagc cgaattgtgg 240
tgtcttacaa ggcagggctt tcctacaggg ggtgganaaa acagcctttc ttcctttggt 300
aggaatggcc tgagttggcg ttgtgggcag gctactggtt tgtatgatgt attagtagag 360
caacccatta atcttttgta gtttgtatna aacttganct gagaccttaa acaaaaaaaa 420
aaa 423

<210> 61
<211> 423
<212> DNA
<213> Homo sapien

<220>
<221> misc_feature
<222> (1)...(423)
<223> n = A,T,C or G

<400> 61
cgggactgga atgtaaagtg aagttcggag ctctgagcac gggctcttcc cgccgggtcc 60
tccctcccca gaccccagag ggagaggccc accccgccca gccccgcccc agcccctgct 120
caggtctgag tatggctggg agtcgggggc cacaggcctc tagctgtgct gctcaagaag 180

actggatcag ggtanctaca agtggccggg ccttgccttt gggattctac cctgttccta 240
atttggtgtt ggggtgcggg gtccctggcc cccttttcca cactncctcc ctccngacag 300
caacctccct tggggcaatt gggcctggnt ctccncccgn tgttgcnacc ctttgttggt 360
ttaaggnctt taaaaatgtt anntttcccc ntgccngggt taaaaaagga aaaaactnaa 420
aaa 423

<210> 62
<211> 683
<212> DNA
<213> Homo sapien

<220>
<221> misc_feature
<222> (1)...(683)
<223> n = A,T,C or G

<400> 62
gctggagagg ggtacggact ttcttggagt tgtcccaggt tggaatgaga ctgaactcaa 60
gaagagaccc taagagactg gggaatggtt cctgccttca ggaaagtgaa agacgcttag 120
gctgtcaaca cttaaaggaa gtccccttga agcccagagt ggacagacta gacccattga 180
tggggccact ggccatggtc cgtggacaag acattccngt gggccatggc acaccggggg 240
ggatcaaaat gtgtacttgt ggggtctcgc cccttgccaa aaccaaacca ntcccactcc 300
tgtcnttgga ctttcttccc attccctcct ccccaaatgc acttcccctc ctccctctgc 360
ccctcctgtg tttttggaat tctgtttccc tcaaaattgt taatttttta nttttngacc 420
atgaacttat gtttggggtc nangttcccc ttnccaatgc atactaatat attaatggtt 480
atttattttt gaaatatttt ttaatgaact tggaaaaaat tnntggaatt tccttncttc 540
cnttttnttt ggggggggtg gggggntggg ttaaaatttt tttggaancc cnatnggaaa 600
ttnttacttg gggcccccct naaaaaantn anttccaatt cttnnatngc ccctnttccn 660
ctaaaaaaa ananannaaa aan 683

<210> 63
<211> 731
<212> DNA
<213> Homo sapien

<220>
<221> misc_feature
<222> (1)...(731)
<223> n = A,T,C or G

<400> 63
actagtcata aagggtgtgc gcgtcttcga cgtggcggtc ttggcgccac tgctgcgaga 60
cccggccctg gacctcaagg tcatccactt ggtgcgtgat ccccgcgcgg tggcgagttc 120
acggatccgc tcgcgccacg gcctcatccg tgagagccta caggtggtgc gcagccgaga 180
ccgcgagctc accgcatgcc cttcttggag gccgcgggcc acaagcttgg cgcccanaaa 240
gaaggcgtng ggggcccgca aantaccacg ctctgggcgc tatggaangt cctcttgcaa 300
taatattggt tnaaaanctg canaanagcc cctgcanccc cctgaactgg gntgcagggc 360
cncttacctn gtttggntgc ggttacaaag aacctgtttn ggaaaccct nccnaaaacc 420
ttccgggaaa attntncaaa tttttnttgg ggaattnttg ggtaaacccc ccnaaaatgg 480
gaaacntttt tgccctnnaa antaaaccat tnggttccgg gggccccccc ncaaaaccct 540
tttttntttt tttntgcccc cantnncccc ccggggcccc tttttttngg ggaaaanccc 600
ccccctnccc nananttta aaagggnggg anaattttn nttnccccc gggnccccn 660
ggngntaaaa nggtttcncc cccccgaggg gnggggnnnc ctcnnaaacc cntntcnnna 720
ccncntttn n 731

<210> 64
<211> 313
<212> DNA
<213> Homo sapien

<220>
<221> misc_feature
<222> (1)...(313)
<223> n = A,T,C or G

<400> 64
actagttgtg caaaccacga ctgaagaaag acgaaaagtg ggaaataact tgcaacgtct 60
gttagagatg gttgctacac atgttgggtc tgtagagaaa catcttgagg agcagattgc 120
taaagttgat agagaatatg aagaatgcat gtcagaagat ctctcggaaa atattaaaga 180
gattagagat aagtatgaga agaaagctac tctaattaag tcttctgaag aatgaagatn 240
aaatgttgat catgtatata tatccatagt gaataaaatt gtctcagtaa agttgtaaaa 300
aaaaaaaaaa aaa 313

<210> 65
<211> 420
<212> DNA
<213> Homo sapien

<220>
<221> misc_feature
<222> (1)...(420)
<223> n = A,T,C or G

<400> 65
actagttccc tggcaggcaa gggcttccaa ctgaggcagt gcatgtgtgg cagagagagg 60
caggaagctg gcagtggcag cttctgtgtc tagggagggg tgtggctccc tccttccctg 120
tctgggaggt tggagggaag aatctaggcc ttagcttgcc ctcctgccac ccttcccctt 180
gtagatactg ccttaacact ccctcctctc tcagctgtgg ctgccaccca agccaggttt 240
ctccgtgctc actaatttat ttccaggaaa ggtgtgtgga agacatgagc cgtgtataat 300
atttgtttta acattttcat tgcaagtatt gaccatcatc cttggttgtg tatcgttgta 360
acacaaatta atgatattaa aaagcatcca aacaaagccn annnnnaana nnannngaaa 420

<210> 66
<211> 676
<212> DNA
<213> Homo sapien

<220>
<221> misc_feature
<222> (1)...(676)
<223> n = A,T,C or G

<400> 66
actagtttcc tatgatcatt aaactcattc tcagggttaa gaaaggaatg taaatttctg 60
cctcaatttg tacttcatca ataagttttt gaagagtgca gatttttagt caggtcttaa 120
aaataaactc acaaatctgg atgcatttct aaattctgca aatgtttcct ggggtgactt 180
aacaaggaat aatcccacaa tatacctagc tacctaatac atggagctgg ggctcaaccc 240
actgttttta aggatttgcg cttacttgtg gctgaggaaa aataagtagt tccgagggaa 300
gtagttttta aatgtgagct tatagatngg aaacagaata tcaacttaat tatggaaatt 360
gttagaaacc tgttctcttg ttatctgaat cttgattgca attactattg tactggatag 420

actccagccc attgcaaagt ctcagatatc ttanctgtgt agttgaattc cttggaaatt 480
ctttttaaga aaaaattgga gtttnaaaga aataaacccc tttgttaaat gaagcttggc 540
tttttggtga aaaanaatca tcccgcaggg cttattgttt aaaaanggaa ttttaagcct 600
ccctggaaaa anttgttaat taaatgggga aaatgntggg naaaaattat ccgttagggt 660
ttaaagggaa aactta 676

<210> 67
<211> 620
<212> DNA
<213> Homo sapien

<220>
<221> misc_feature
<222> (1)...(620)
<223> n = A,T,C or G

<400> 67
caccattaaa gctgcttacc aagaacttcc ccagcatttt gacttccttg tttgatagct 60
gaattgtgag caggtgatag aagagccttt ctagttgaac atacagataa tttgctgaat 120
acattccatt taatgaaggg gttacatctg ttacgaagct actaagaagg agcaagagca 180
taggggaaaa aaatctgatc agaacgcatc aaactcacat gtgccccctc tactacaaac 240
agattgtagt gctgtggtgg tttattccgt tgtgcagaac ttgcaagctg agtcactaaa 300
cccaaagaga ggaaattata ggttagttaa acattgtaat cccaggaact aagtttaatt 360
cacttttgaa gtgttttgtt ttttattttt ggtttgtctg atttactttg ggggaaaang 420
ctaaaaaaaa agggatatca atctctaatt cagtgcccac taaaagttgt ccctaaaaag 480
tctttactgg aanttatggg actttttaag ctccaggtnt tttggtcctc caaattaacc 540
ttgcatgggc cccttaaaat tgttgaangg cattcctgcc tctaagtttg gggaaaattc 600
ccccnttttn aaaatttgga 620

<210> 68
<211> 551
<212> DNA
<213> Homo sapien

<220>
<221> misc_feature
<222> (1)...(551)
<223> n = A,T,C or G

<400> 68
actagtagct ggtacataat cactgaggag ctatttctta acatgctttt atagaccatg 60
ctaatgctag accagtattt aagggctaat ctcacacctc cttagctgta agagtctggc 120
ttagaacaga cctctctgtg caataacttg tggccactgg aaatccctgg gccggcattt 180
gtattggggt tgcaatgact cccaagggcc aaaagagtta aaggcacgac tgggatttct 240
tctgagactg tggtgaaact ccttccaagg ctgagggggt cagtangtgc tctgggaggg 300
actcggcacc actttgatat tcaacaagcc acttgaagcc caattataaa attgttattt 360
tacagctgat ggaactcaat ttgaaccttc aaaactttgt tagtttatcc tattatattg 420
ttaaacctaa ttacatttgt ctagcattgg atttggttcc tgtngcatat gtttttttcn 480
cctatgtgct cccctccccc nnatcttaat ttaaaccnca attttgcnat tcnccnnnnn 540
nannnannna a 551

<210> 69
<211> 396
<212> DNA
<213> Homo sapien

<220>
<221> misc_feature
<222> (1)...(396)
<223> n = A,T,C or G

<400> 69
   cagaaatgga aagcagagtt ttcatttctg tttataaacg tctccaaaca aaaatggaaa 60
   gcagagtttt cattaaatcc ttttaccttt tttttttctt ggtaatcccc tcaaataaca 120
   gtatgtggga tattgaatgt taaagggata tttttttcta ttatttttat aattgtacaa 180
   aattaagcaa atgttaaaag ttttatatgc tttattaatg ttttcaaaag gtatnataca 240
   tgtgatacat tttttaagct tcagttgctt gtcttctggt actttctgtt atgggctttt 300
   ggggagccan aaaccaatct acnatctctt tttgtttgcc aggacatgca ataaaattta 360
   aaaaataaat aaaaactatt nagaaattga aaaaaa 396

<210> 70
<211> 536
<212> DNA
<213> Homo sapien

<220>
<221> misc_feature
<222> (1)...(536)
<223> n = A,T,C or G

<400> 70
   actagtgcaa aagcaaatat aaacatcgaa aaggcgttcc tcacgttagc tgaagatatc 60
   ctttgaaaga cccctgtaaa agagcccaac agtgaaaatg tagatatcag cagtggagga 120
   ggcgtgacag gctggaagag caaatgctgc tgagcattct cctgttccat cagttgccat 180
   ccactacccc gttttctctt cttgctgcaa aataaaccac tctgtccatt tttaactcta 240
   aacagatatt tttgtttctc atcttaacta tccaagccac ctattttatt tgttctttca 300
   tctgtgactg cttgctgact ttatcataat tttcttcaaa caaaaaaatg tatagaaaaa 360
   tcatgtctgt gacttcattt ttaaatgnta cttgctcagc tcaactgcat ttcagttgtt 420
   ttatagtcca gttcttatca acattnaaac ctatngcaat catttcaaat ctattctgca 480
   aattgtataa gaataaaagt tagaatttaa caattaaaaa aaaaaaaaaa aaaaaa 536

<210> 71
<211> 865
<212> DNA
<213> Homo sapien

<220>
<221> misc_feature
<222> (1)...(865)
<223> n = A,T,C or G

<400> 71
gacaaagcgt taggagaaga anagaggcag ggaanactnc ccaggcacga tggccncctt 60
cccaccagca accagcgccc cccaccagcc cccaggcccg gacgacgaag actccatcct 120
ggattaatct nacctctntc gcctgnccca ttcctacctc ggaggtggag gccggaaagg 180
tcncaccaag aganaanctg ctgccaacac caaccgcccc agccctggcg ggcacganag 240
gaaactggtg accaatctgc agaattctna gaggaanaag cnaggggccc cgcgctnaga 300
cagagctgga tatgangcca gaccatggac nctacncccn ncaatncana cgggactgcg 360
gaagatggan gacccncgac nngatcaggc cngctnncca nccccccacc cctatgaatt 420
attcccgctg aangaatctc tganngcctt ccannaaagc gcctccccnc cnaacgnaan 480

tncaacatng ggattanang ctgggaactg naaggggcaa ancctnnaat atccccagaa 540
acaanctctc ccnaanaaac tggggcncct catnggtggn accaactatt aactaaaccg 600
cacgccaagn aantataaaa gggggccccc tccncggnng accccctttt gtcccttaat 660
ganggttatc cnccttgcgt accatggtnc ccnnttctgt ntgnatgttt ccnctcccct 720
ccncctatnt cnagccgaac tcnnatttnc ccgggggtgc natcnantng tncncctttn 780
ttngttgncc cngccctttc cgncggaacn cgtttccccg ttantaacgg cacccggggn 840
aagggtgntt ggccccctcc ctccc 865

<210> 72
<211> 560
<212> DNA
<213> Homo sapien

<220>
<221> misc_feature
<222> (1)...(560)
<223> n = A,T,C or G

<400> 72
cctggacttg tcttggttcc agaacctgac gacccggcga cggcgacgtc tcttttgact 60
aaaagacagt gtccagtgct ccngcctagg agtctacggg gaccgcctcc cgcgccgcca 120
ccatgcccaa cttctctggc aactggaaaa tcatccgatc ggaaaacttc gangaattgc 180
tcnaantgct gggggtgaat gtgatgctna ngaanattgc tgtggctgca gcgtccaagc 240
cagcagtgga gatcnaacag gagggagaca ctttctacat caaaacctcc accaccgtgc 300
gcaccacaaa gattaacttc nnngttgggg agganttga ggancaaact gtggatngga 360
ngcctgtnaa aacctggtga aatgggagaa tganaataaa atggtctgtg anranaaact 420
cctgaaagga gaaggccccc anaactcctg gaccngaaaa actgacccnc cnatngggga 480
actgatnctt gaaccctgaa cgggcgggat ganccttttt tnttgccncc naangggttc 540
tttccntttc cccaaaaaaa 560

<210> 73
<211> 379
<212> DNA
<213> Homo sapien

<220>
<221> misc_feature
<222> (1)...(379)
<223> n = A,T,C or G

<400> 73
ctggggancc ggcggtnngc nccatntcnn gncgcgaagg tggcaataaa aanccnctga 60
aaccgcncaa naaacatgcc naagatatgg acgaggaaga tngngctttc nngnacaanc 120
gnanngagga acanaacaaa ctcnangagc tctcaagcta atgccgcggg gaaggggccc 180
ttggccacnn gtggaattaa gaaatctggc aaanngtann tgttccttgt gcctnangag 240
ataagngacc ctttatttca tctgtattta aacctctctn ttccctgnca taacttcttt 300
tnccacgtan agntggaant anttgttgtc ttggactgtt gtncatttta gannaaactt 360
ttgttcaaaa aaaaaataa 379

<210> 74
<211> 437
<212> DNA
<213> Homo sapien

<220>

<221> misc_feature
<222> (1)...(437)
<223> n = A,T,C or G

<400> 74
actagttcag actgccacgc caaccccaga aaatacccca catgccagaa aagtgaagtc 60
ctaggtgttt ccatctatgt ttcaatctgt ccatctacca ggcctcgcga taaaaacaaa 120
acaaaaaaac gctgccaggt tttanaagca gttctggtct caaaaccatc aggatcctgc 180
caccagggtt cttttgaaat agtaccacat gtaaaaggga atttggcttt cacttcatct 240
aatcactgaa ttgtcaggct ttgattgata attgtagaaa taagtagcct tctgttgtgg 300
gaataagtta taatcagtat tcatctcttt gtttttgtc actcttttct ctctnattgt 360
gtcatttgta ctgtttgaaa aatatttctt ctataaaatt aaactaacct gccttaaaaa 420
aaaaaaaaaa aaaaaaa 437

<210> 75
<211> 579
<212> DNA
<213> Homo sapien

<220>
<221> misc_feature
<222> (1)...(579)
<223> n = A,T,C or G

<400> 75
ctccgtcgcc gccaagatga tgtgcggggc gccctccgcc acgcagccgg ccaccgccga 60
gacccagcac atcgccgacc aggtgaggtc ccagcttgaa gagaaagaaa acaagaagtt 120
ccctgtgttt aaggccgtgt cattcaagag ccaggtggtc gcggggacaa actacttcat 180
caaggtgcac gtcggcgacg aggacttcgt acacctgcga gtgttccaat ctctccctca 240
tgaaaacaag cccttgacct tatctaacta ccagaccaac aaagccaagc atgatgagct 300
gacctatttc tgatcctgac tttggacaag gcccttcagc cagaagactg acaaagtcat 360
cctccgtcta ccagagcgtg cacttgtgat cctaaaataa gcttcatctc cgggctgtgc 420
ccttggggtg gaaggggcan gatctgcact gcttttgcat ttctcttcct aaatttcatt 480
gtgttgattc tttccttcca ataggtgatc ttnattactt tcagaatatt ttccaaatna 540
gatatatttt naaaatcctt aaaaaaaaaa aaaaaaaaa 579

<210> 76
<211> 666
<212> DNA
<213> Homo sapien

<220>
<221> misc_feature
<222> (1)...(666)
<223> n = A,T,C or G

<400> 76
gtttatccta tctctccaac cagattgtca gctccttgag ggcaagagcc acagtatatt 60
tccctgtttc ttccacagtg cctaataata ctgtggaact aggttttaat aatttttaa 120
ttgatgttgt tatgggcagg atggcaacca gaccattgtc tcagagcagg tgctggctct 180
ttcctggcta ctccatgttg gctagcctct ggtaacctct tacttattat cttcaggaca 240
ctcactacag ggaccaggga tgatgcaaca tccttgtctt tttatgacag gatgtttgct 300
cagcttctcc aacaataaaa agcacgtggt aaaacacttg cggatattct ggactgtttt 360
taaaaaatat acagtttacc gaaaatcata ttatcttaca atgaaaagga ntttatagat 420
cagccagtga acaacctttt tccaccatac aaaaattcct tttcccgaan gaaaanggct 480

ttctcaataa ncctcacttt cttaanatct tacaagatag ccccganatc ttatcgaaac 540
tcattttagg caaatatgan ttttattgtn cgttacttgt ttcaaaattt ggtattgtga 600
atatcaatta ccacccccat ctcccatgaa anaaanggga aanggtgaan ttcntaancg 660
cttaaa 666

<210> 77
<211> 396
<212> DNA
<213> Homo sapien

<220>
<221> misc_feature
<222> (1)...(396)
<223> n = A,T,C or G

<400> 77
   ctgcagcccg ggggatccac taatctacca nggttatttg gcagctaatt ctanatttgg 60
   atcattgccc aaagttgcac ttgctggtct cttgggattt ggccttggaa aggtatcata 120
   catanganta tgccanaata aattccattt ttttgaaaat canctccntg gggctggttt 180
   tggtccacag cataacangc actgcctcct tacctgtgag gaatgcaaaa taaagcatgg 240
   attaagtgag aagggagact ctcagccttc agcttcctaa attctgtgtc tgtgactttc 300
   gaagtttttt aaacctctga atttgtacac atttaaattt tcaagtgtac tttaaaataa 360
   aatacttcta atgggaacaa aaaaaaaaaa aaaaaa 396

<210> 78
<211> 793
<212> DNA
<213> Homo sapien

<220>
<221> misc_feature
<222> (1)...(793)
<223> n = A,T,C or G

<400> 78
gcatcctagc cgccgactca cacaaggcag gtgggtgagg aaatccagag ttgccatgga 60
gaaaattcca gtgtcagcat tcttgctcct tgtggccctc tcctacactc tggccagaga 120
taccacagtc aaacctggag ccaaaaagga cacaaaggac tctcgaccca aactgcccca 180
gaccctctcc agaggttggg gtgaccaact catctggact cagacatatg aagaagctct 240
atataaatcc aagacaagca acaaaccctt gatgattatt catcacttgg atgagtgccc 300
acacagtcna gctttaaaga aagtgtttgc tgaaaataaa gaaatccaga aattggcaga 360
gcagtttgtc ctcctcaatc tggtttatga aacaactgac aaacaccttt ctcctgatgg 420
ccagtatgtc ccaggattat gtttgttgac ccatctctga cagttgaagc cgatatcctg 480
ggaagatatt cnaaccgtct ctatgcttac aaactgcaga tacgctctgt tgcttgacac 540
atgaaaaagc tctcaagttg ctnaaaatga attgtaagaa aaaaaatctc cagccttctg 600
tctgtcggct tgaaaattga aaccagaaaa atgtgaaaaa tggctattgt ggaacanatn 660
gacacctgat taggttttgg ttatgttcac cactattttt aanaaaanan nttttaaaat 720
ttggttcaat tntctttttn aaacaatntg tttctacntt gnganctgat ttctaaaaaa 780
aataatnttt ggc 793

<210> 79
<211> 456
<212> DNA
<213> Homo sapien

<220>
<221> misc_feature
<222> (1)...(456)
<223> n = A,T,C or G

<400> 79
actagtatgg ggtgggaggc cccacccttc tcccctaggc gctgttcttg ctccaaaggg 60
ctccgtggag agggactggc agagctgang ccacctgggg ctggggatcc cactcttctt 120
gcagctgttg agcgcaccta accactggtc atgcccccac ccctgctctc cgcacccgct 180
tcctcccgac cccangacca ggctacttct cccctcctct tgcctccctc ctgcccctgc 240
tgcctctgat cgtangaatt gangantgtc ccgccttgtg gctganaatg gacagtggca 300
ggggctggaa atgggtgtgt gtgtgtgtgt gtgtgtgtgt gtgtgtgtgt gcnccccccc 360
tgcaagaccg agattgaggg aaancatgtc tgctgggtgt gaccatgttt cctctccata 420
aantnccccct gtgacnctca naaaaaaaaa aaaaaa 456

<210> 80
<211> 284
<212> DNA
<213> Homo sapien

<220>
<221> misc_feature
<222> (1)...(284)
<223> n = A,T,C or G

<400> 80
ctttgtacct ctagaaaaga taggtattgt gtcatgaaac ttgagtttaa attttatata 60
taaaactaaa agtaatgctc actttagcaa cacatactaa aattggaacc atactgagaa 120
gaatagcatg acctccgtgc aaacaggaca agcaaatttg tgatgtgttg attaaaaaga 180
aataaataaa tgtgtatatg tgtaacttgt atgtttatgt ggaatacaga ttgggaaata 240
aaatgtattt cttactgtga aaaaaaaaaa aaaaaaaaaa aana 284

<210> 81
<211> 671
<212> DNA
<213> Homo sapien

<220>
<221> misc_feature
<222> (1)...(671)
<223> n = A,T,C or G

<400> 81
gccaccaaca ttccaagcta ccctgggtac ctttgtgcag tagaagctag tgagcatgtg 60
agcaagcggt gtgcacacgg agactcatcg ttataattta ctatctgcca agagtagaaa 120
gaaaggctgg ggatatttgg gttggcttgg ttttgatttt ttgcttgttt gtttgttttg 180
tactaaaaca gtattatctt ttgaatatcg tagggacata agtatataca tgttatccaa 240
tcaagatggc tagaatggtg cctttctgag tgtctaaaac ttgacacccc tggtaaatct 300
ttcaacacac ttccactgcc tgcgtaatga agttttgatt catttttaac cactggaatt 360
tttcaatgcc gtcattttca gttagatnat tttgcacttt gagattaaaa tgccatgtct 420
atttgattag tcttattttt ttatttttac aggcttatca gtctcactgt tggctgtcat 480
tgtgacaaag tcaaataaac ccccnaggac aacacacagt atgggatcac atattgtttg 540
acattaagct ttggccaaaa aatgttgcat gtgttttacc tcgacttgct aaatcaatan 600
canaaaggct ggctnataat gttggtggtg aaataattaa tnantaacca aaaaaaaaan 660
aaaaaaaaaa a 671

<210> 82
<211> 217
<212> DNA
<213> Homo sapien

<220>
<221> misc_feature
<222> (1)...(217)
<223> n = A,T,C or G

<400> 82
ctgcagatgt ttcttgaatg ctttgtcaaa ttaanaaagt taaagtgcaa taatgtttga 60
agacaataag tggtggtgta tcttgtttct aataagataa actttttgt ctttgcttta 120
tcttattagg gagttgtatg tcagtgtata aaacatactg tgtggtataa caggcttaat 180
aaattcttta aaggaaaaa aaaaaaaaa aaaaaaa 217

<210> 83
<211> 460
<212> DNA
<213> Homo sapien

<220>
<221> misc_feature
<222> (1)...(460)
<223> n = A,T,C or G

<400> 83
cgcgagtggg agcaccagga tctcgggctc ggaacgagac tgcacggatt gttttaagaa 60
aatggcagac aaaccagaca tgggggaaat cgccagcttc gatnaggcca agctgaanaa 120
aacggagacg caggagaaga acaccctgcc gaccaaagag accattgagc angagaagcg 180
gagtgaaatt tcctaagatc ctggaggatt tcctaccccc gtcctcttcg agaccccagt 240
cgtgatgtgg aggaagagcc acctgcaaga tggacacgag ccacaagctg cactgtgaac 300
ctgggcactc cgcgccgatg ccaccggcct gtgggtctct gaagggaccc cccccaatcg 360
gactgccaaa ttctccggtt tgccccggga tattatacaa nattatttgt atgaataatg 420
annataaaac acacctcgtg gcancaaana aaaaaaaaaa 460

<210> 84
<211> 323
<212> DNA
<213> Homo sapien

<220>
<221> misc_feature
<222> (1)...(323)
<223> n = A,T,C or G

<400> 84
   tggtggatct tggctctgtg gagctgctgg gacgggatct aaaagactat tctggaagct 60
   gtggtccaan gcattttgct ggcttaacgg gtcccggaac aaaggacacc agctctctaa 120
   aattgaagtt tacccganat aacaatcttt tgggcagaga tgcctatttt aacaaacncc 180
   gtccctgcgc aacaacnaac aatctctggg aaataccggc catgaacntg ctgtctcaat 240
   cnancatctc tctagctgac cgatcatatc gtcccagatt actacanatc ataataattg 300
   atttcctgta naaaaaaaaa aaa 323

<210> 85
<211> 771
<212> DNA
<213> Homo sapien

<220>
<221> misc_feature
<222> (1)...(771)
<223> n = A,T,C or G

<400> 85
aaactgggta ctcaacactg agcagatctg ttctttgagc taaaaaccat gtgctgtacc 60
aanagtttgc tcctggctgc tttgatgtca gtgctgctac tccacctctg cggcgaatca 120
gaagcaagca actttgactg ctgtcttgga tacacagacc gtattcttca tcctaaattt 180
attgtgggct tcacacggca gctggccaat gaaggctgtg acatcaatgc tatcatcttt 240
cacacaaaga aaaagttgtc tgtgtgcgca aatccaaaac agacttgggt gaaatatatt 300
gtgcgtctcc tcagtaaaaa agtcaagaac atgtaaaaac tgtggctttt ctggaatgga 360
attggacata gcccaagaac agaaagaact tgctggggtt ggaggtttca cttgcacatc 420
atgganggtt tagtgcttat cttatttgtg cctcctggac ttgtccaatt natgaagtta 480
atcatattgc atcatanttt gctttgttta acatcacatt naaattaaac tgtattttat 540
gttatttata gctntaggtt ttctgtgttt aactttttat acnaantttc ctaaactatt 600
ttggtntant gcaanttaaa aattatattt ggggggggaa taaatattgg antttctgca 660
gccacaagct tttttaaaa aaccantaca nccnngttaa atggtnggtc ccnaatggtt 720
tttgcttttn antagaaaat ttnttagaac natttgaaaa aaaaaaaaaa a 771

<210> 86
<211> 628
<212> DNA
<213> Homo sapien

<220>
<221> misc_feature
<222> (1)...(628)
<223> n = A,T,C or G

<400> 86
actagtttgc tttacatttt tgaaaagtat tatttttgtc caagtgctta tcaactaaac 60
cttgtgttag gtaagaatgg aatttattaa gtgaatcagt gtgacccttc ttgtcataag 120
attatcttaa agctgaagcc aaaatatgct tcaaaagaaa angactttat tgttcattgt 180
agttcataca ttcaaagcat ctgaactgta gtttctatag caagccaatt acatccataa 240
gtggagaang aaatagatta atgtcnaagt atgattggtg gagggagcaa ggttgaagat 300
aatctggggt tgaaattttc tagttttcat tctgtacatt tttagttnga catcagattt 360
gaaatattaa tgtttacctt tcaatgtgtg gtatcagctg gactcantaa cacccctttc 420
ttccctnggg gatggggaat ggattattgg aaaatggaaa gaaaaaagta cttaaagcct 480
tcctttcnca gtttctggct cctaccctac tgatttancc agaataagaa aacattttat 540
catcntctgc tttattccca ttaatnaant tttgatgaat aaatctgctt ttatgcnnac 600
ccaaggaatt nagtggnttc ntcnttgt 628

<210> 87
<211> 518
<212> DNA
<213> Homo sapien

<220>
<221> misc_feature

<222> (1)...(518)
<223> n = A,T,C or G

<400> 87
tttttatttt tttttagaga gtagttcagc ttttatttat aaatttattg cctgttttat 60
tataacaaca ttatactgtt tatggtttaa tacatatggt tcaaaatgta taatacatca 120
agtagtacag ttttaaaatt ttatgcttaa aacaagtttt gtgtaaaaaa tgcagataca 180
ttttacatgg caaatcaatt tttaagtcat cctaaaaatt gatttttttt tgaaatttaa 240
aaacacattt aatttcaatt tctctcttat ataaccttta ttactatagc atggtttcca 300
ctacagttta acaatgcagc aaaattccca tttcacggta aattgggttt taagcggcaa 360
ggttaaaatg ctttgaggat cctnaatacc ctttgaactt caaatgaagg ttatggttgt 420
naatttaacc ctcatgccat aagcagaagc acaagtttag ctgcattttg ctctaaactg 480
taaaancgag ccccccgttg aaaaagcaaa agggaccc 518

<210> 88
<211> 1844
<212> DNA
<213> Homo sapien

<400> 88
gagacagtga atcctagtat caaaggattt ttggcctcag aaaaagttgt tgattatttt 60
tattttattt tatttttcga gactccgtct caaaaaaaaa aaaaaaaaaa agaatcacaa 120
ggtatttgct aaagcatttt gagctgcttg gaaaaaggga agtagttgca gtagagtttc 180
ttccatcttc ttggtgctgg gaagccatat atgtgtcttt tactcaagct aaggggtata 240
agcttatgtg ttgaatttgc tacatctata tttcacatat tctcacaata agagaatttt 300
gaaatagaaa tatcatagaa catttaagaa agtttagtat aaataatatt ttgtgtgttt 360
taatcccttt gaagggatct atccaaagaa aatactttac actgagctcc ttcctacacg 420
tctcagtaac agatcctgtg ttagtctttg aaaatagctc attttttaaa tgtcagtgag 480
tagatgtagc atacatatga tgtataatga cgtgtattat gttaacaatg tctgcagatt 540
ttgtaggaat acaaaacatg gcctttttta taagcaaaac gggccaatga ctagaataac 600
acatagggca atctgtgaat atgtattata agcagcattc cagaaaagta gttggtgaaa 660
taattttcaa gtcaaaaagg gatatggaaa gggaattatg agtaacctct attttttaag 720
ccttgctttt aaattaaacg ctacagccat ttaagccttg aggataataa agcttgagag 780
taataatgtt aggttagcaa aggtttagat gtatcacttc atgcatgcta ccatgatagt 840
aatgcagctc ttcgagtcat ttctggtcat tcaagatatt cacccttttg cccatagaaa 900
gcaccctacc tcacctgctt actgacattg tcttagctga tcacaagatc attatcagcc 960
tccattattc cttactgtat ataaaataca gagttttata ttttcctttc ttcgtttttc 1020
accatattca aaacctaaat ttgtttttgc agatggaatg caaagtaatc aagtgttcgt 1080
gctttcacct agaagggtgt ggtcctgaag gaaagaggtc cctaaatatc ccccaccctg 1140
ggtgctcctc cttccctggt accctgacta ccagaagtca ggtgctagag cagctggaga 1200
agtgcagcag cctgtgcttc cacagatggg ggtgctgctg caacaaggct ttcaatgtgc 1260
ccatcttagg gggagaagct agatcctgtg cagcagcctg gtaagtcctg aggaggttcc 1320
attgctcttc ctgctgctgt cctttgcttc tcaacggggc tcgctctaca gtctagagca 1380
catgcagcta acttgtgcct ctgcttatgc atgagggtta aattaacaac cataaccttc 1440
atttgaagtt caaaggtgta ttcaggatcc tcaaagcatt ttaaccttgc cgcttaaaac 1500
ccaatttacc gtgaaatggg aattttgctg cattgttaaa ctgtagtgga aaccatgcta 1560
tagtaataaa ggttatataa gagagaaatt gaaattaaat gtgtttttaa atttcaaaaa 1620
aaaatcaatc tttaggatga cttaaaaatt gatttgccat gtaaaatgta tctgcatttt 1680
ttacacaaaa cttgttttaa gcataaaatt ttaaaactgt actacttgat gtattataca 1740
ttttgaacca tatgtattaa accataaaca gtataatgtt gttataataa aacaggcaat 1800
aaatttataa ataaaagctg aaaaaaaaaa aaaaaaaaaa aaaa 1844

<210> 89
<211> 523
<212> DNA

<213> Homo sapien

<220>
<221> misc_feature
<222> (1)...(523)
<223> n = A,T,C or G

<400> 89
tttttttttt ttttttagt caatccacat ttattgatca cttattatgt accaggcact 60
gggataaaga tgactgttag tcactcacag taaggaagaa aactagcaaa taagacgatt 120
acaatatgat gtagaaaatg ctaagccaga gatatagaaa ggtcctattg ggtccttctg 180
tcaccttgtc tttccacatc cctacccttc acaggccttc cctccagctt cctgcccccg 240
ctccccactg cagatcccct gggatttgc ctagagctaa acgagganat gggcccctg 300
gccctggcat gacttgaacc caaccacaga ctgggaaagg gagcctttcg anagtggatc 360
actttgatna gaaaacacat agggaattga agagaaantc cccaaatggc cacccgtgct 420
ggtgctcaag aaaagtttgc agaatggata aatgaaggat caagggaatt aatanatgaa 480
taattgaatg gtggctcaat aagaatgact ncnttgaatg acc 523

<210> 90
<211> 604
<212> DNA
<213> Homo sapien

<220>
<221> misc_feature
<222> (1)...(604)
<223> n = A,T,C or G

<400> 90
ccagtgtggt ggaatgcaaa gattaccccg gaagctttcg agaagctggg attccctgca 60
gcaaaggaaa tagccaatat gtgtcgtttc tatgaaatga agccagaccg agatgtcaat 120
ctcacccacc aactaaatcc caaagtcaaa agcttcagcc agtttatctc agagaaccag 180
gggagccttc aagggcatgt agaaaatcag ctgttcagat aggcctctgc accacacagc 240
ctctttcctc tctgatcctt ttcctcttta cggcacaaca ttcatgtttg acagaacatg 300
ctggaatgca attgtttgca acaccgaagg atttcctgcg gtcgcctctt cagtaggaag 360
cactgcattg gtgataggac acggtaattt gattcacatt taacttgcta gttagtgata 420
aggggtggta cacctgtttg gtaaaatgag aagcctcgga aacttgggag cttctctcct 480
accactaatg gggagggcag attattactg ggatttctcc tggggtgaat taatttcaag 540
ccctaattgc tgaaattccc ctnggcaggc tccagttttc tcaactgcat tgcaaaattc 600
cccc 604

<210> 91
<211> 858
<212> DNA
<213> Homo sapien

<220>
<221> misc_feature
<222> (1)...(858)
<223> n = A,T,C or G

<400> 91
   tttttttttt ttttttttta tgattattat tttttttatt gatctttaca tcctcagtgt 60
   tggcagagtt tctgatgctt aataaacatt tgttttgatc agataagtgg aaaaaattgt 120
   catttcctta ttcaagccat gcttttctgt gatattctga tcctagttga acatacagaa 180
   ataaatgtct aaaacagcac ctcgattctc gtctataaca ggactaagtt cactgtgatc 240
   ttaaataagc ttggctaaaa tgggacatga gtggaggtag tcacacttca gcgaagaaag 300
   agaatctcct gtataatctc accaggagat tcaacgaatt ccaccacact ggactagtgg 360
   atcccccggg ctgcaggaat tcgatatcaa gcttatcgat accgtcgacc tcgagggggg 420
   gcccggtacc caattcgccc tatagtgagt cgtattacgc gcgctcactg gccgtcgttt 480
   tacaacgtcg tgactgggaa aaccctggcg ttacccaact taatcgcctt gcagcacatc 540
   cccctttcgc cagctggcgt aatagcgaan agcccgcacc gatcgccctt ncaacagttg 600
   cgcagcctga atggcgaatg ggacgcgccc tgtagcggcg cattaagcgc cggcngggtg 660
   tggnggntcc cccacgtgac cgntacactt ggcagcgcct tacgccggtc nttcgctttc 720
   ttcccttcct ttctcgcacc gttcgccggg tttccccgnn agctnttaat cgggggnctc 780
   cctttanggg tncnaattaa nggnttacng gaccttngan cccaaaaact ttgattaggg 840
   ggaaggtccc cgaagggg 858

<210> 92
<211> 585
<212> DNA
<213> Homo sapien

<220>
<221> misc_feature
<222> (1)...(585)
<223> n = A,T,C or G

<400> 92
gttgaatctc ctggtgagat tatacaggag attctctttc ttcgctgaag tgtgactacc 60
tccactcatg tcccatttta gccaagctta tttaagatca cagtgaactt agtcctgtta 120
tagacgagaa tcgaggtgct gttttagaca tttatttctg tatgttcaac taggattaga 180
atattacaga aaagcatggc ttgaataagg aaatgacaat tttttccact tatctgatca 240
gaacaaatgt ttattaagca tcagaaactc tgccaacact gaggatgtaa agatcaataa 300
aaaaaataat aatcatnann naaanannan nngaagggcg gccgccaccg cggtggagct 360
ccagcttttg ttccctttag tgagggttaa ttgcgcgctt ggcgttaatc atggtcatag 420
ctgtttcctg tgtgaaattg ttatccgctc cacaattccn cncaacatac gagccgggaa 480
gcntnangtg taaaagcctg gggtgcctaa ttgagtgagc tnactcacat taattgngt 540
tgcgctccac ttgcccgctt ttccantccg ggaaacctgt tcgnc 585

<210> 93
<211> 567
<212> DNA
<213> Homo sapien

<220>
<221> misc_feature
<222> (1)...(567)
<223> n = A,T,C or G

<400> 93
cggcagtgtt gctgtctgcg tgtccacctt ggaatctggc tgaactggct gggaggacca 60
agactgcggc tggggtgggc anggaaggga accgggggct gctgtgaagg atcttggaac 120
ttccctgtac ccaccttccc cttgcttcat gtttgtanag gaaccttgtg ccggccaagc 180
ccagtttcct tgtgtgatac actaatgtat ttgctttttt tgggaaatan anaaaaatca 240
attaaattgc tantgtttct ttgaannnnn nnnnnnnnnn nnnnnnnggg ggggncgccc 300
ccncggngga aacnccccct tttgttccct ttaattgaaa ggttaattng cncncntggc 360
gttaanccnt gggccaaanc tngttncccg tgntgaaatt gttnatcccc tcccaaattc 420
ccccccnncc ttccaaaccc ggaaancctn annntgttna ancccggggg gttgcctaan 480
ngnaattnaa ccnaaccccc ntttaaatng nntttgcncn ccacnngccc cnctttccca 540

nttcggggaa aaccctntcc gtgccca 567

<210> 94
<211> 620
<212> DNA
<213> Homo sapien

<220>
<221> misc_feature
<222> (1)...(620)
<223> n = A,T,C or G

<400> 94
actagtcaaa aatgctaaaa taatttggga gaaaatattt tttaagtagt gttatagttt 60
catgtttatc ttttattatg ttttgtgaag ttgtgtcttt tcactaatta cctatactat 120
gccaatattt ccttatatct atccataaca tttatactac atttgtaana naatatgcac 180
gtgaaactta acactttata aggtaaaaat gaggtttcca anatttaata atctgatcaa 240
gttcttgtta tttccaaata gaatggactt ggtctgttaa gggctaagga gaagaggaag 300
ataaggttaa aagttgttaa tgaccaaaca ttctaaaaga aatgcaaaaa aaaagtttat 360
tttcaagcct tcgaactatt taaggaaagc aaaatcattt cctaaatgca tatcatttgt 420
gagaatttct cattaatatc ctgaatcatt catttcacta aggctcatgt tnactccgat 480
atgtctctaa gaaagtacta tttcatggtc caaacctggt tgccatantt gggtaaaggc 540
tttcccttaa gtgtgaaant atttaaaatg aaattttcct ctttttaaaa attctttana 600
agggttaagg gtgttgggga 620

<210> 95
<211> 470
<212> DNA
<213> Homo sapien

<220>
<221> misc_feature
<222> (1)...(470)
<223> n = A,T,C or G

<400> 95
ctcgaccttc tctgcacagc ggatgaaccc tgagcagctg aagaccagaa aagccactat 60
nactttntgc ttaattcang agcttacang attcttcaaa gagtgngtcc agcatccttt 120
gaaacatgag ttcttaccag cagaagcaga cctttacccc accacctcag cttcaacagc 180
agcaggtgaa acaacccatc cagcctccac ctnaggaaat atttgttccc acaaccaagg 240
agccatgcca ctcaaaggtt ccacaacctg naaacacaaa nattccagag ccaggctgta 300
ccaaggtccc tgagccaggg ctgtaccaan gtccctgagc caggttgtac caangtccct 360
gagccaggat gtaccaaggt ccctgancca ggttgtccaa ggtccctgag ccaggctaca 420
ccaagggcct gngccaggca gcatcaangt ccctgaccaa ggcttatcaa 470

<210> 96
<211> 660
<212> DNA
<213> Homo sapien

<220>
<221> misc_feature
<222> (1)...(660)
<223> n = A,T,C or G

<400> 96
tttttttttt tttttttttt ggaattaaaa gcaatttaat gagggcagag caggaaacat 60
gcatttcttt tcattcgaat cttcagatga accctgagca gccgaagacc agaaaagcca 120
tgaagacttt ctgcttaatt caggggctta caggattctt cagagtgtgt gtgaacaaaa 180
gctttatagt acgtattttt aggatacaaa taagagagag actatggctt ggggtgagaa 240
tgtactgatt acaaggtcta cagacaatta agacacagaa acagatggga agagggtgnc 300
cagcatctgg nggttggctt ctcaagggct tgtctgtgca ccaaattact tctgcttggn 360
cttctgctga gctgggcctg gagtgaccgt tgaaggacat ggctctggta cctttgtgta 420
gcctgncaca ggaactttgg tgtatccttg ctcaggaact ttgatggcac ctggctcagg 480
aaacttgatg aagccttggt caagggacct tgatgcttgc tggctcaggg accttggngn 540
ancctgggct canggacctt tgncncaacc ttggcttcaa gggacccttg gnacatcctg 600
gcnnagggac ccttgggncc aaccctgggc ttnagggacc ctttggntnc nanccttggc 660

<210> 97
<211> 441
<212> DNA
<213> Homo sapien

<220>
<221> misc_feature
<222> (1)...(441)
<223> n = A,T,C or G

<400> 97
gggaccatac anagtattcc tctcttcaca ccaggaccag ccactgttgc agcatgagtt 60
cccagcagca gaagcagccc tgcatcccac cccctcagct tcagcagcag caggtgaaac 120
agccttgcca gcctccacct caggaaccat gcatccccaa aaccaaggag ccctgccacc 180
ccaaggtgcc tgagccctgc caccccaaag tgcctgagcc ctgccagccc aaggttccag 240
agccatgcca ccccaaggtg cctgagccct gcccttcaat agtcactcca gcaccagccc 300
agcagaanac caagcagaag taatgtggtc cacagccatg cccttgagga gccggccacc 360
agatgctgaa tcccctatcc cattctgtgt atgagtccca tttgccttgc aattagcatt 420
ctgtctcccc caaaaaaaaa a 441

<210> 98
<211> 600
<212> DNA
<213> Homo sapien

<220>
<221> misc_feature
<222> (1)...(600)
<223> n = A,T,C or G

<400> 98
gtattcctct cttcacacca ggaccagcca ctgttgcagc atgagttccc agcagcagaa 60
gcagccctgc atcccacccc ctcagcttca gcagcagcag gtgaaacagc cttgccagcc 120
tccacctcag gaaccatgca tccccaaaac caaggagccc tgccacccca aggtgcctga 180
gccctgccac cccaaagtgc ctgagccctg ccagcccaag gttccagagc catgccaccc 240
caaggtgcct gagccctgcc cttcaatagt cactccagca ccagcccagc agaanaccaa 300
gcagaagtaa tgtggtccac agccatgccc ttgaggagcc ggccaccana tgctgaatcc 360
cctatcccat tctgtgtatg agtcccattt gccttgcaat tagcattctg tctcccccaa 420
aaaagaatgt gctatgaagc tttctttcct acacactctg agtctctgaa tgaagctgaa 480
ggtcttaant acaganctag ttttcagctg ctcagaattc tctgaagaaa agatttaaga 540
tgaaaggcaa atgattcagc tccttattac cccattaaat tcnctttcaa tttcaaaaaa 600

<210> 99
<211> 667
<212> DNA
<213> Homo sapien

<220>
<221> misc_feature
<222> (1)...(667)
<223> n = A,T,C or G

<400> 99
actagtgact gagttcctgg caaagaaatt tgacctggac cagttgataa ctcatgtttt 60
accatttaaa aaaatcagtg aaggatttga gctgctcaat tcaggacaaa gcattcgaac 120
ggtcctgacg ttttgagatc caaagtggca ggaggtctgt gttgtcatgg tgaactggag 180
tttctcttgt gagagttccc tcatctgaaa tcatgtatct gtctcacaaa tacaagcata 240
agtagaagat ttgttgaaga catagaaccc ttataaagaa ttattaacct ttataaacat 300
ttaaagtctt gtgagcacct gggaattagt ataataacaa tgttnatatt tttgatttac 360
attttgtaag gctataattg tatcttttaa gaaaacatac cttggatttc tatgttgaaa 420
tggagatttt taagagtttt aaccagctgc tgcagatata ttactcaaaa cagatatagc 480
gtataaagat atagtaaatg catctcctag agtaatattc acttaacaca ttggaaacta 540
ttatttttta gatttgaata tnaatgttat tttttaaaca cttgttatga gttacttggg 600
attacatttt gaaatcagtt cattccatga tgcanattac tgggattaga ttaagaaaga 660
cggaaaa 667

<210> 100
<211> 583
<212> DNA
<213> Homo sapien

<220>
<221> misc_feature
<222> (1)...(583)
<223> n = A,T,C or G

<400> 100
gttttgtttg taagatgatc acagtcatgt tacactgatc taaaggacat atatataacc 60
ctttaaaaaa aaaatcactg cctcattctt atttcaagat gaatttctat acagactaga 120
tgtttttctg aagatcaatt agacattttg aaaatgattt aaagtgtttt ccttaatgtt 180
ctctgaaaac aagtttcttt tgtagtttta accaaaaaag tgcccttttt gtcactggat 240
tctcctagca ttcatgattt tttttcata caatgaaatt aaaattgcta aaatcatgga 300
ctggctttct ggttggattt caggtaagat gtgtttaagg ccagagcttt tctcagtatt 360
tgattttttt ccccaatatt tgatttttta aaaatataca catnggtgct gcatttatat 420
ctgctggttt aaaattctgt catatttcac ttctagcctt ttagttatgg caaatcatat 480
tttactttta cttaaagcat ttggtnattt ggantatctg gttctannct aaaaaaanta 540
attctatnaa ttgaantttt ggtactcnnc catatttgga tcc 583

<210> 101
<211> 592
<212> DNA
<213> Homo sapien

<220>
<221> misc_feature
<222> (1)...(592)
<223> n = A,T,C or G

<400> 101
   gtggagacgt acaaagagca gccgctcaag acacctggga agaaaaagaa aggcaagccc 60
   gggaaacgca aggagcagga aaagaaaaaa cggcgaactc gctctgcctg gttagactct 120
   ggagtgactg ggagtgggct agaaggggac cacctgtctg acacctccac aacgtcgctg 180
   gagctcgatt cacggaggca ttgaaatttt cagcaganac cttccaagga catattgcag 240
   gattctgtaa tagtgaacat atggaaagta ttagaaatat ttattgtctg taaatactgt 300
   aaatgcattg gaataaaact gtctccccca ttgctctatg aaactgcaca ttggtcattg 360
   tgaatatttt tttttttgcc aaggctaatc caattattat tatcacattt accataattt 420
   attttgtcca ttgatgtatt tattttgtaa atgtatcttg gtgctgctga atttctatat 480
   tttttgtaca taatgcnttt anatatacct atcaagtttg ttgataaatg acncaatgaa 540
   gtgncncnan ttggnggttg aatttaatga atgcctaatt ttattatccc aa 592

<210> 102
<211> 587
<212> DNA
<213> Homo sapien

<220>
<221> misc_feature
<222> (1)...(587)
<223> n = A,T,C or G

<400> 102
cgtcctaagc acttagacta catcagggaa gaacacagac cacatccctg tcctcatgcg 60
gcttatgttt tctggaagaa agtggagacc nagtccttgg ctttagggct ccccggctgg 120
gggctgtgca ntccggtcag ggcgggaagg gaaatgcacc gctgcatgtg aacttacagc 180
ccaggcggat gccccttccc ttagcactac ctggcctcct gcatcccctc gcctcatgtt 240
cctcccacct tcaaanaatg aanaacccca tgggcccagc cccttgccct ggggaaccaa 300
ggcagccttc caaaactcag gggctgaagc anactattag ggcaggggct gactttgggt 360
gacactgccc attccctctc agggcagctc angtcacccn ggnctcttga acccagcctg 420
ttcctttgaa aaagggcaaa actgaaaagg gcttttccta naaaaagaaa aaccagggaa 480
ctttgccagg gcttcnntnt taccaaaacn ncttctcnng gatttttaat tccccattng 540
gcctccactt accnggggcn atgccccaaa attaanaatt tcccatc 587

<210> 103
<211> 496
<212> DNA
<213> Homo sapien

<220>
<221> misc_feature
<222> (1)...(496)
<223> n = A,T,C or G

<400> 103
anaggactgg ccctacntgc tctctctcgt cctacctatc aatgcccaac atggcagaac 60
ctgcanccct tggncactgc anatggaaac ctctcagtgt cttgacatca ccctacccnt 120
gcggtgggtc tccaccacaa ccactttgac tctgtggtcc ctgnanggtg gnttctcctg 180
actggcagga tggaccttan ccnacatatc cctctgttcc ctctgctnag anaaagaatt 240
cccttaacat gatataatcc acccatgcaa ntngctactg gcccagctac catttaccat 300
ttgcctacag aatttcattc agtctacact ttggcattct ctctggcgat agagtgtggc 360
tgggctgacc gcaaaaggtg ccttacacac tggcccccac cctcaaccgt tgacncatca 420
gangcttgcc tcctccttct gattnncccc catgttggat atcagggtgc tcnagggatt 480
ggaaaagaaa caaaac 496

<210> 104
<211> 575
<212> DNA
<213> Homo sapien

<220>
<221> misc_feature
<222> (1)...(575)
<223> n = A,T,C or G

<400> 104
gcacctgctc tcaatccnnc tctcaccatg atcctccgcc tgcanaaact cctctgccaa 60
ctatggangt ggtttcnggg gtggctcttg ccaactggga agaagccgtg gtgtctctac 120
ctgttcaact cngtttgtgt ctgggggatc aactnggggc tatggaagcg gctnaactgt 180
tgttttggtg gaagggctgg taattggctt tgggaagtng cttatngaag ttggcctngg 240
gaagttgcta ttgaaagtng ccntggaagt ngntttggtg gggggttttg ctggtggcct 300
ttgttnaatt tgggtgcttt gtnaatggcg gccccctcnc ctgggcaatg aaaaaaatca 360
ccnatgcngn aaacctcnac nnaacagcct gggcttccct cacctcgaaa aaagttgctc 420
cccccccaaa aaaggncaan cccctcaann tggaangttg aaaaaatcct cgaatgggga 480
ncccnaaaac aaaaancccc ccntttcccn gnaanggggg aaataccncc cccccactta 540
cnaaaaccct tntaaaaaac cccccgggaa aaaaa 575

<210> 105
<211> 619
<212> DNA
<213> Homo sapien

<220>
<221> misc_feature
<222> (1)...(619)
<223> n = A,T,C or G

<400> 105
cactagtagg atagaaacac tgtgtcccga gagtaaggag agaagctact attgattaga 60
gcctaaccca ggttaactgc aagaagaggc gggatacttt cagctttcca tgtaactgta 120
tgcataaagc caatgtagtc cagtttctaa gatcatgttc caagctaact gaatcccact 180
tcaatacaca ctcatgaact cctgatggaa caataacagg cccaagcctg tggtatgatg 240
tgcacacttg ctagactcan aaaaaatact actctcataa atgggtggga gtattttggt 300
gacaacctac tttgcttggc tgagtgaagg aatgatattc atatattcat ttattccatg 360
gacatttagt tagtgctttt tatataccag gcatgatgct gagtgacact cttgtgtata 420
tttccaaatt tttgtacagt cgctgcacat atttgaaatc atatattaag acttccaaaa 480
aatgaagtcc ctggtttttc atggcaactt gatcagtaaa ggattcncct ctgtttggta 540
cttaaaacat ctactatatn gttnanatga aattcctttt ccccncctcc cgaaaaaana 600
aagtggtggg gaaaaaaaa 619

<210> 106
<211> 506
<212> DNA
<213> Homo sapien

<220>
<221> misc_feature
<222> (1)...(506)
<223> n = A,T,C or G

<400> 106
cattggtnct ttcatttgct ntggaagtgt nnatctctaa cagtggacaa agttcccngt 60
gccttaaact ctgtnacact tttgggaant gaaaanttng tantatgata ggttattctg 120
angtanagat gttctggata ccattanatn tgcccccngt gtcagaggct catattgtgt 180
tatgtaaatg gtatntcatt cgctactatn antcaattng aaatanggtc tttgggttat 240
gaatantnng cagcncanct nanangctgt ctgtngtatt cattgtggtc atagcacctc 300
acancattgt aacctcnatc nagtgagaca nactagnaan ttcctagtga tggctcanga 360
ttccaaatgg nctcatntcn aatgtttaaa agttanttaa gtgtaagaaa tacagactgg 420
atgttccacc aactagtacc tgtaatgacn ggcctgtccc aacacatctc ccttttccat 480
gactgtggta ncccgcatcg gaaaaa 506

<210> 107
<211> 452
<212> DNA
<213> Homo sapien

<220>
<221> misc_feature
<222> (1)...(452)
<223> n = A,T,C or G

<400> 107
gttgagtctg tactaaacag taagatatct caatgaacca taaattcaac tttgtaaaaa 60
tcttttgaag catagataat attgtttggt aaatgtttct tttgtttggt aaatgtttct 120
tttaaagacc ctcctattct ataaaactct gcatgtagag gcttgtttac ctttctctct 180
ctaaggttta caataggagt ggtgatttga aaaatataaa attatgagat tggttttcct 240
gtggcataaa ttgcatcact gtatcatttt cttttttaac cggtaagant ttcagtttgt 300
tggaaagtaa ctgtganaac ccagtttccc gtccatctcc cttagggact acccatagaa 360
catgaaaagg tccccacnga agcaagaaga taagtctttc atggctgctg gttgcttaaa 420
ccactttaaa accaaaaaat tccccttgga aa 452

<210> 108
<211> 502
<212> DNA
<213> Homo sapien

<220>
<221> misc_feature
<222> (1)...(502)
<223> n = A,T,C or G

<400> 108
atcttcttcc cttaattagt tnttatttat ntattaaatt ttattgcatg tcctggcaaa 60
caaaaagaga ttgtagattg gcttctggct ccccaaaagc ccataacaga aagtaccaca 120
agaccncaac tgaagcttaa aaaatctatc acatgtataa tacctttnga agaacattaa 180
tanagcatat aaaactttta acatntgctt aatgttgtnc aattataaaa ntaatngaaa 240
aaaatgtccc tttaacatnc aatatcccac atagtgttat ttnaggggat taccnngnaa 300
naaaaaaagg gtagaaggga tttaatgaaa actctgcttn ccatttctgt ttanaaacgt 360
ctccagaaca aaaacttntc aantctttca gctaaccgca tttgagctna ggccactcaa 420
aaactccatt agncccactt tctaanggtc tctanagctt actaancctt ttgacccctt 480
accctggnta ctcctgccct ca 502

<210> 109
<211> 1308

<212> DNA
<213> Homo sapien

<400> 109
acccgaggtc tcgctaaaat catcatggat tcacttggcg ccgtcagcac tcgacttggg 60
tttgatcttt tcaaagagct gaagaaaaca aatgatggca acatcttctt ttcccctgtg 120
ggcatcttga ctgcaattgg catggtcctc ctggggaccc gaggagccac cgcttcccag 180
ttggaggagg tgtttcactc tgaaaaagag acgaagagct caagaataaa ggctgaagaa 240
aaagaggtga ttgagaacac agaagcagta catcaacaat tccaaaagtt tttgactgaa 300
ataagcaaac tcactaatga ttatgaactg aacataacca acaggctgtt tggagaaaaa 360
acatacctct tccttcaaaa atacttagat tatgttgaaa aatattatca tgcatctctg 420
gaacctgttg attttgtaaa tgcagccgat gaaagtcgaa agaagattaa ttcctgggtt 480
gaaagcaaaa caaatgaaaa aatcaaggac ttgttcccag atggctctat tagtagctct 540
accaagctgg tgctggtgaa catggtttat tttaaagggc aatgggacag ggagtttaag 600
aaagaaaata ctaaggaaga gaaattttgg atgaataaga gcacaagtaa atctgtacag 660
atgatgacac agagccattc ctttagcttc actttcctgg aggacttgca ggccaaaatt 720
ctagggattc catataaaaa caacgaccta agcatgtttg tgcttctgcc caacgacatc 780
gatggcctgg agaagataat agataaaata agtcctgaga aattggtaga gtggactagt 840
ccagggcata tggaagaaag aaaggtgaat ctgcacttgc cccggtttga ggtggaggac 900
agttacgatc tagaggcggt cctggctgcc atggggatgg gcgatgcctt cagtgagcac 960
aaagccgact actcgggaat gtcgtcaggc tccgggttgt acgcccagaa gttcctgcac 1020
agttcctttg tggcagtaac tgaggaaggc accgaggctg cagctgccac tggcataggc 1080
tttactgtca catccgcccc aggtcatgaa aatgttcact gcaatcatcc cttcctgttc 1140
ttcatcaggc acaatgaatc caacagcatc ctcttcttcg gcagattttc ttctccttaa 1200
gatgatcgtt gccatggcat tgctgctttt agcaaaaaac aactaccagt gttactcata 1260
tgattatgaa aatcgtccat tcttttaaat ggtggctcac ttgcattt 1308

<210> 110
<211> 391
<212> PRT
<213> Homo sapien

<400> 110

<210> 111
<211> 1419
<212> DNA
<213> Homo sapien

<400> 111
ggagaactat aaattaagga tcccagctac ttaattgact tatgcttcct agttcgttgc 60
ccagccacca ccgtctctcc aaaaacccga ggtctcgcta aaatcatcat ggattcactt 120
ggcgccgtca gcactcgact tgggtttgat cttttcaaag agctgaagaa aacaaatgat 180
ggcaacatct tcttttcccc tgtgggcatc ttgactgcaa ttggcatggt cctcctgggg 240
acccgaggag ccaccgcttc ccagttggag gaggtgtttc actctgaaaa agagacgaag 300
agctcaagaa taaaggctga agaaaaagag gtggtaagaa taaaggctga aggaaaagag 360
attgagaaca cagaagcagt acatcaacaa ttccaaaagt ttttgactga aataagcaaa 420
ctcactaatg attatgaact gaacataacc aacaggctgt ttggagaaaa aacatacctc 480
ttccttcaaa aatacttaga ttatgttgaa aaatattatc atgcatctct ggaacctgtt 540
gattttgtaa atgcagccga tgaaagtcga aagaagatta attcctgggt tgaaagcaaa 600
acaaatgaaa aaatcaagga cttgttccca gatggctcta ttagtagctc taccaagctg 660
gtgctggtga acatggttta ttttaaaggg caatgggaca gggagtttaa gaaagaaaat 720
actaaggaag agaaattttg gatgaataag agcacaagta aatctgtaca gatgatgaca 780
cagagccatt cctttagctt cactttcctg gaggacttgc aggccaaaat tctagggatt 840
ccatataaaa acaacgacct aagcatgttt gtgcttctgc ccaacgacat cgatggcctg 900
gagaagataa tagataaaat aagtcctgag aaattggtag agtggactag tccagggcat 960
atggaagaaa gaaaggtgaa tctgcacttg ccccggtttg aggtggagga cagttacgat 1020
ctagaggcgg tcctggctgc catggggatg ggcgatgcct tcagtgagca caaagccgac 1080
tactcgggaa tgtcgtcagg ctccgggttg tacgcccaga agttcctgca cagttccttt 1140
gtggcagtaa ctgaggaagg caccgaggct gcagctgcca ctggcatagg ctttactgtc 1200

acatccgccc caggtcatga aaatgttcac tgcaatcatc ccttcctgtt cttcatcagg 1260
cacaatgaat ccaacagcat cctcttcttc ggcagatttt cttctcctta agatgatcgt 1320
tgccatggca ttgctgcttt tagcaaaaaa caactaccag tgttactcat atgattatga 1380
aaatcgtcca ttcttttaaa tggtggctca cttgcattt 1419

<210> 112
<211> 400
<212> PRT
<213> Homo sapien

<400> 112

<210> 113
<211> 957
<212> DNA
<213> Homo sapien

<400> 113
ctcgaccttc tctgcacagc ggatgaaccc tgagcagctg aagaccagaa aagccactat 60
gactttctgc ttaattcagg agcttacagg attcttcaaa gagtgtgtcc agcatccttt 120
gaaacatgag ttcttaccag cagaagcaga cctttacccc accacctcag cttcaacagc 180
agcaggtgaa acaacccagc cagcctccac ctcaggaaat atttgttccc acaaccaagg 240
agccatgcca ctcaaaggtt ccacaacctg gaaacacaaa gattccagag ccaggctgta 300
ccaaggtccc tgagccaggc tgtaccaagg tccctgagcc aggttgtacc aaggtccctg 360
agccaggatg taccaaggtc cctgagccag gttgtaccaa ggtccctgag ccaggctaca 420
ccaaggtccc tgagccaggc agcatcaagg tccctgacca aggcttcatc aagtttcctg 480
agccaggtgc catcaaagtt cctgagcaag gatacaccaa agttcctgtg ccaggctaca 540
caaaggtacc agagccatgt ccttcaacgg tcactccagg cccagctcag cagaagacca 600
agcagaagta atttggtgca cagacaagcc cttgagaagc caaccaccag atgctggaca 660
ccctcttccc atctgtttct gtgtcttaat tgtctgtaga ccttgtaatc agtacattct 720
caccccaagc catagtctct ctcttatttg tatcctaaaa atacggtact ataaagcttt 780
tgttcacaca cactctgaag aatcctgtaa gcccctgaat taagcagaaa gtcttcatgg 840
cttttctggt ctttggctgc tcagggttca tctgaagatt cgaatgaaaa gaaatgcatg 900
tttcctgctc tgccctcatt aaattgcttt taattccaaa aaaaaaaaaa aaaaaaa 957

<210> 114
<211> 161
<212> PRT
<213> Homo sapien

<400> 114
Met Ser Ser Tyr Gln Gln Lys Gln Thr Phe Thr Pro Pro Pro Gln Leu
1               5                   10                  15
Gln Gln Gln Gln Val Lys Gln Pro Ser Gln Pro Pro Pro Gln Glu Ile
            20                  25                  30
Phe Val Pro Thr Thr Lys Glu Pro Cys His Ser Lys Val Pro Gln Pro
        35                  40                  45
Gly Asn Thr Lys Ile Pro Glu Pro Gly Cys Thr Lys Val Pro Glu Pro
    50                  55                  60
Gly Cys Thr Lys Val Pro Glu Pro Gly Cys Thr Lys Val Pro Glu Pro
65                  70                  75                  80
Gly Cys Thr Lys Val Pro Glu Pro Gly Cys Thr Lys Val Pro Glu Pro
                85                  90                  95
Gly Tyr Thr Lys Val Pro Glu Pro Gly Ser Ile Lys Val Pro Asp Gln
            100                 105                 110
Gly Phe Ile Lys Phe Pro Glu Pro Gly Ala Ile Lys Val Pro Glu Gln
        115                 120                 125
Gly Tyr Thr Lys Val Pro Val Pro Gly Tyr Thr Lys Val Pro Glu Pro
    130                 135                 140
Cys Pro Ser Thr Val Thr Pro Gly Pro Ala Gln Gln Lys Thr Lys Gln

145                 150                 155                 160
Lys

<210> 115
<211> 506
<212> DNA
<213> Homo sapien

<220>
<221> misc_feature
<222> (1)...(506)
<223> n = A,T,C or G

<400> 115
cattggtnct ttcatttgct ntggaagtgt nnatctctaa cagtggacaa agttcccngt      60
gccttaaact ctgtnacact tttgggaant gaaaanttng tantatgata ggttattctg     120
angtanagat gttctggata ccattanatn tgcccccngt gtcagaggct catattgtgt     180
tatgtaaatg gtatntcatt cgctactatn antcaattng aaatanggtc tttgggttat     240
gaatantnng cagcncanct nanangctgt ctgtngtatt cattgtggtc atagcacctc     300
acancattgt aacctcnatc nagtgagaca nactagnaan ttcctagtga tggctcanga     360
ttccaaatgg nctcatntcn aatgtttaaa agttanttaa gtgtaagaaa tacagactgg     420
atgttccacc aactagtacc tgtaatgacn ggcctgtccc aacacatctc ccttttccat     480
gactgtggta ncccgcatcg gaaaaa                                          506

<210> 116
<211> 3079
<212> DNA
<213> Homo sapien

<400> 116
ggatccccgg gtttcctaaa ccccccacag agtcctgccc aggccaaaga gcaaggaaaa      60
ggtcaaaggg cagaaaaaat gctgagttag gaggagctat ggaaggataa acctggcctt     120
aaagaggtca aagtggttta tagggggcgc tgagggcttc ccacattctc tggcctaaac     180
cttgcaggca gatctgccca gtgggctctg ggatagctgt gccttcccta acaaaaaaat     240
tgtgcacaaa aggatgaaac tctattttcc ctctagcaca taaccaagaa tataaggcta     300
cagattgcct ttcccagagg gaaaaccctg cagcaacctg ctgcctggaa aagtgtaaga     360
gcagatcact ggggaatcgt ttgccccccg ctgatggaca gcttccccaa gctccaaggg     420
caggtgctca gcatgtaccg tactgggatg gttgtcaata ctcctggtcc tgtaagagtc     480
ccaggacact gccatgccaa tgccccctca gttcctggca tcctttttgg gctgctcaca     540
gccccagcct ctatggtgaa gacatacttg ctagcagcgt caccaacttg ttgccaagag     600
atcagtgctc gaaggcaagg ttatttctaa ctgagcagag cctgccagga agaaagcgtt     660
tgcaccccac accactgtgc aggtgtgacc ggtgagctca cagctgcccc ccaggcatgc     720
ccagcccact taatcatcac agctcgacag ctctctcgcc cagcccagtt ctggaaggga     780
taaaaagggg catcaccgtt cctgggtaac agagccacct tctgcgtcct gctgagctct     840
gttctctcca gcacctccca acccactagt gcctggttct cttgctccac caggaacaag     900
ccaccatgtc tcgccagtca agtgtgtctt ccggagcggg gggcagtcgt agcttcagca     960
ccgcctctgc catcaccccg tctgtctccc gcaccagctt cacctccgtg tcccggtccg    1020
ggggtggcgg tggtggtggc ttcggcaggg tcagccttgc gggtgcttgt ggagtgggtg    1080
gctatggcag ccggagcctc tacaacctgg ggggctccaa gaggatatcc atcagcacta    1140
gtggtggcag cttcaggaac cggtttggtg ctggtgctgg aggcggctat ggctttggag    1200
gtggtgccgg tagtggattt ggtttcggcg gtggagctgg tggtggcttt gggctcggtg    1260
gcggagctgg ctttggaggt ggcttcggtg gccctggctt tcctgtctgc cctcctggag    1320
gtatccaaga ggtcactgtc aaccagagtc tcctgactcc cctcaacctg caaatcgacc    1380
ccagcatcca gagggtgagg accgaggagc gcgagcagat caagaccctc aacaataagt    1440

ttgcctcctt catcgacaag gtgcggttcc tggagcagca gaacaaggtt ctggaaacaa 1500
agtggaccct gctgcaggag cagggcacca agactgtgag gcagaacctg gagccgttgt 1560
tcgagcagta catcaacaac ctcaggaggc agctggacag catcgtgggg gaacggggcc 1620
gcctggactc agagctgaga aacatgcagg acctggtgga agacttcaag aacaagtatg 1680
aggatgaaat caacaagcgt accactgctg agaatgagtt tgtgatgctg aagaaggatg 1740
tagatgctgc ctacatgaac aaggtggagc tggaggccaa ggttgatgca ctgatggatg 1800
agattaactt catgaagatg ttctttgatg cggagctgtc ccagatgcag acgcatgtct 1860
ctgacacctc agtggtcctc tccatggaca acaaccgcaa cctggacctg gatagcatca 1920
tcgctgaggt caaggcccag tatgaggaga ttgccaaccg cagccggaca gaagccgagt 1980
cctggtatca gaccaagtat gaggagctgc agcagacagc tggccggcat ggcgatgacc 2040
tccgcaacac caagcatgag atctctgaga tgaaccggat gatccagagg ctgagagccg 2100
agattgacaa tgtcaagaaa cagtgcgcca atctgcagaa cgccattgcg gatgccgagc 2160
agcgtgggga gctggccctc aaggatgcca ggaacaagct ggccgagctg gaggaggccc 2220
tgcagaaggc caagcaggac atggcccggc tgctgcgtga gtaccaggag ctcatgaaca 2280
ccaagctggc cctggacgtg gagatcgcca cttaccgcaa gctgctggag ggcgaggaat 2340
gcagactcag tggagaagga gttggaccag tcaacatctc tgttgtcaca agcagtgttt 2400
cctctggata tggcagtggc agtggctatg gcggtggcct cggtggaggt cttggcggcg 2460
gcctcggtgg aggtcttgcc ggaggtagca gtggaagcta ctactccagc agcagtgggg 2520
gtgtcggcct aggtggtggg ctcagtgtgg ggggctctgg cttcagtgca agcagtagcc 2580
gagggctggg ggtgggcttt ggcagtggcg ggggtagcag ctccagcgtc aaatttgtct 2640
ccaccacctc ctcctcccgg aagagcttca agagctaaga acctgctgca agtcactgcc 2700
ttccaagtgc agcaacccag cccatggaga ttgcctcttc taggcagttg ctcaagccat 2760
gttttatcct tttctggaga gtagtctaga ccaagccaat tgcagaacca cattctttgg 2820
ttcccaggag agccccattc ccagcccctg gtctcccgtg ccgcagttct atattctgct 2880
tcaaatcagc cttcaggttt cccacagcat ggcccctgct gacacgagaa cccaaagttt 2940
tcccaaatct aaatcatcaa aacagaatcc ccaccccaat cccaaatttt gttttggttc 3000
taactacctc cagaatgtgt tcaataaaat gttttataat ataagctggt gtgcagaatt 3060
gttttttttt tctacccaa 3079

<210> 117
<211> 6921
<212> DNA
<213> Homo sapien

<400> 117
gaattctgac tgtccactca aaacttctat tccgatcaaa gctatctgtg actacagaca 60
aattgagata accatttaca aagacgatga atgtgttttg gcgaataact ctcatcgtgc 120
taaatggaag gtcattagtc ctactgggaa tgaggctatg gtcccatctg tgtgcttcac 180
cgttcctcca ccaaacaaag aagcggtgga ccttgccaac agaattgagc aacagtatca 240
gaatgtcctg actctttggc atgagtctca cataaacatg aagagtgtag tatcctggca 300
ttatctcatc aatgaaattg atagaattcg agctagcaat gtggcttcaa taaagacaat 360
gctacctggt gaacatcagc aagttctaag taatctacaa tctcgttttg aagattttct 420
ggaagatagc caggaatccc aagtcttttc aggctcagat ataacacaac tggaaaagga 480
ggttaatgta tgtaagcagt attatcaaga acttcttaaa tctgcagaaa gagaggagca 540
agaggaatca gtttataatc tctacatctc tgaagttcga aacattagac ttcggttaga 600
gaactgtgaa gatcggctga ttagacagat tcgaactccc ctggaaagag atgatttgca 660
tgaaagtgtg ttcagaatca cagaacagga gaaactaaag aaagagctgg aacgacttaa 720
agatgatttg ggaacaatca caaataagtg tgaggagttt ttcagtcaag cagcagcctc 780
ttcatcagtc cctaccctac gatcagagct taatgtggtc cttcagaaca tgaaccaagt 840
ctattctatg tcttccactt acatagataa gttgaaaact gttaacttgg tgttaaaaaa 900
cactcaagct gcagaagccc tcgtaaaact ctatgaaact aaactgtgtg aagaagaagc 960
agttatagct gacaagaata atattgagaa tctaataagt actttaaagc aatggagatc 1020
tgaagtagat gaaaagagac aggtattcca tgccttagag gatgagttgc agaaagctaa 1080
agccatcagt gatgaaatgt ttaaaacgta taaagaacgg gaccttgatt ttgactggca 1140
caaagaaaaa gcagatcaat tagttgaaag gtggcaaaat gttcatgtgc agattgacaa 1200

caggttacgg gacttagagg gcattggcaa atcactgaag tactacagag acacttacca 1260
tcctttagat gattggatcc agcaggttga aactactcag agaaagattc aggaaaatca 1320
gcctgaaaat agtaaaaccc tagccacaca gttgaatcaa cagaagatgc tggtgtccga 1380
aatagaaatg aaacagagca aaatggacga gtgtcaaaaa tatgcagaac agtactcagc 1440
tacagtgaag gactatgaat tacaaacaat gacctaccgg gccatggtag attcacaaca 1500
aaaatctcca gtgaaacgcc gaagaatgca gagttcagca gatctcatta ttcaagagtt 1560
catggaccta aggactcgat atactgccct ggtcactctc atgacacaat atattaaatt 1620
tgctggtgat tcattgaaga ggctggaaga ggaggagatt aaaaggtgta aggagacttc 1680
tgaacatggg gcatattcag atctgcttca gcgtcagaag gcaacagtgc ttgagaatag 1740
caaacttaca ggaaagataa gtgagttgga aagaatggta gctgaactaa agaaacaaaa 1800
gtcccgagta gaggaagaac ttccgaaggt cagggaggct gcagaaaatg aattgagaaa 1860
gcagcagaga aatgtagaag atatctctct gcagaagata agggctgaaa gtgaagccaa 1920
gcagtaccgc agggaacttg aaaccattgt gagagagaag gaagccgctg aaagagaact 1980
ggagcgggtg aggcagctca ccatagaggc cgaggctaaa agagctgccg tggaagagaa 2040
cctcctgaat tttcgcaatc agttggagga aaacaccttt accagacgaa cactggaaga 2100
tcatcttaaa agaaaagatt taagtctcaa tgatttggag caacaaaaaa ataaattaat 2160
ggaagaatta agaagaaaga gagacaatga ggaagaactc ttgaagctga taaagcagat 2220
ggaaaaagac cttgcatttc agaaacaggt agcagagaaa cagttgaaag aaaagcagaa 2280
aattgaattg gaagcaagaa gaaaaataac tgaaattcag tatacatgta gagaaaatgc 2340
attgccagtg tgtccgatca cacaggctac atcatgcagg gcagtaacgg gtctccagca 2400
agaacatgac aagcagaaag cagaagaact caaacagcag gtagatgaac taacagctgc 2460
caatagaaag gctgaacaag acatgagaga gctgacatat gaacttaatg ccctccagct 2520
tgaaaaaacg tcatctgagg aaaaggctcg tttgctaaaa gataaactag atgaaacaaa 2580
taatacactc agatgcctta agttggagct ggaaaggaag gatcaggcgg agaaagggta 2640
ttctcaacaa ctcagagagc ttggtaggca attgaatcaa accacaggta aagctgaaga 2700
agccatgcaa gaagctagtg atctcaagaa aataaagcgc aattatcagt tagaa[?]taga 2760
atctcttaat catgaaaaag ggaaactaca aagagaagta.gacagaatca caaggcaca 2820
tgctgtagct gagaagaata ttcagcattt aaattcacaa attcattctt ttcgagatga 2830
gaaagaatta gaaagactac aaatctgcca gagaaaatca gatcatctaa aagaacaatt 2940
tgagaaaagc catgagcagt tgcttcaaaa tatcaaagct gaaaaagaaa ataatgataa 3000
aatccaaagg ctcaatgaag aattggagaa aagtaatgag tgtgcagaga tgctaaaaca 3060
aaaagtagag gagcttacta ggcagaataa tgaaaccaaa ttaatgatgc agagaattca 3120
ggcagaatca gagaatatag ttttagagaa acaaactatc cagcaaagat gtgaagcact 3180
gaaaattcag gcagatggtt ttaaagatca gctacgcagc acaaatgaac acttgcataa 3240
acagacaaaa acagagcagg attttcaaag aaaaattaaa tgcctagaag aagacctggc 3300
gaaaagtcaa aatttggtaa gtgaatttaa gcaaaagtgt gaccaacaga acattatcat 3360
ccagaatacc aagaaagaag ttagaaatct gaatgcggaa ctgaatgctt ccaaagaaga 3420
gaagcgacgc ggggagcaga aagttcagct acaacaagct caggtgcaag agttaaataa 3480
caggttgaaa aaagtacaag acgaattaca cttaaagacc atagaggagc agatgaccca .3540
cagaaagatg gttctgtttc aggaagaatc tggtaaattc aaacaatcag cagaggagtt 3600
tcggaagaag atggaaaaat taatggagtc caaagtcatc actgaaaatg atatttcagg 3660
cattaggctt gactttgtgt ctcttcaaca agaaaactct agagcccaag aaaatgctaa 3720
gctttgtgaa acaaacatta aagaacttga aagacagctt caacagtatc gtgaacaaat 3780
gcagcaaggg cagcacatgg aagcaaatca ttaccaaaaa tgtcagaaac ttgaggatga 3840
gctgatagcc cagaagcgtg aggttgaaaa cctgaagcaa aaaatggacc aacagatcaa 3900
agagcatgaa catcaattag ttttgctcca gtgtgaaatt caaaaaaaga gcacagccaa 3960
agactgtacc ttcaaaccag attttgagat gacagtgaag gagtgccagc actctggaga 4020
gctgtcctct agaaacactg gacaccttca cccaacaccc agatcccctc tgttgagatg 4080
gactcaagaa ccacagccat tggaagagaa gtggcagcat cgggttgttg aacagatacc 4140
caaagaagtc caattccagc caccaggggc tccactcgag aaagagaaaa gccagcagtg 4200
ttactctgag tacttttctc agacaagcac cgagttacag ataacttttg atgagacaaa 4260
ccccattaca agactgtctg aaattgagaa gataagagac caagccctga acaattctag 4320
accacctgtt aggtatcaag ataacgcatg tgaaatggaa ctggtgaagg ttttgacacc 4380
cttagagata gctaagaaca agcagtatga tatgcataca gaagtcacaa cattaaaaca 4440
agaaaagaac ccagttccca gtgctgaaga atggatgctt gaagggtgca gagcatctgg 4500

tggactcaag aaaggggatt tccttaagaa gggcttagaa ccagagacct tccagaactt 4560
tgatggtgat catgcatgtt cagtcaggga tgatgaattt aaattccaag ggcttaggca 4620
cactgtgact gccaggcagt tggtggaagc taagcttctg gacatgagaa caattgagca 4680
gctgcgactc ggtcttaaga ctgttgaaga agttcagaaa actcttaaca agtttctgac 4740
gaaagccacc tcaattgcag ggctttacct agaatctaca aaagaaaaga tttcatttgc 4800
ctcagcggcc gagagaatca taatagacaa aatggtggct ttggcatttt tagaagctca 4860
ggctgcaaca ggttttataa ttgatcccat ttcaggtcag acatattctg ttgaagatgc 4920
agttcttaaa ggagttgttg accccgaatt cagaattagg cttcttgagg cagagaaggc 4980
agctgtggga tattcttatt cttctaagac attgtcagtg tttcaagcta tggaaaatag 5040
aatgcttgac agacaaaaag gtaaacatat cttggaagcc cagattgcca gtgggggtgt 5100
cattgaccct gtgagaggca ttcgtgttcc tccagaaatt gctctgcagc aggggttgtt 5160
gaataatgcc atcttacagt ttttacatga gccatccagc aacacaagag ttttccctaa 5220
tcccaataac aagcaagctc tgtattactc agaattactg cgaatgtgtg tatttgatgt 5280
agagtcccaa tgctttctgt ttccatttgg ggagaggaac atttccaatc tcaatgtcaa 5340
gaaaacacat agaatttctg tagtagatac taaaacagga tcagaattga ccgtgtatga 5400
ggctttccag agaaacctga ttgagaaaag tatatatctt gaactttcag ggcagcaata 5460
tcagtggaag gaagctatgt tttttgaatc ctatgggcat tcttctcata tgctgactga 5520
tactaaaaca ggattacact tcaatattaa tgaggctata gagcagggaa caattgacaa 5580
agccttggtc aaaaagtatc aggaaggcct catcacactt acagaacttg ctgattcttt 5640
gctgagccgg ttagtcccca agaaagattt gcacagtcct gttgcagggt attggctgac 5700
tgctagtggg gaaaggatct ctgtactaaa agcctcccgt agaaatttgg ttgatcggat 5760
tactgccctc cgatgccttg aagcccaagt cagtacaggg ggcataattg atcctcttac 5820
tggcaaaaag taccgggtgg ccgaagcttt gcatagaggc ctggttgatg aggggtttgc 5880
ccagcagctg cgacagtgtg aattagtaat cacagggatt ggccatccca tcactaacaa 5940
aatgatgtca gtggtggaag ctgtgaatgc aaatattata aataaggaaa tgggaatccg 6000
atgtttggaa tttcagtact tgacaggagg gttgatagag ccacaggttc actctcggtt 6060
atcaatagaa gaggctctcc aagtaggtat tatagatgtc ctcattgcca caaaactcaa 6120
agatcaaaag tcatatgtca gaaatataat atgccctcag acaaaaagaa agttgacata 6180
taaagaagcc ttagaaaaag ctgattttga tttccacaca ggacttaaac tgttagaagt 6240
atctgagccc ctgatgacag gaatttctag cctctactat tcttcctaat gggacatgtt 6300
taaataactg tgcaaggggt gatgcaggct ggttcatgcc actttttcag agtatgatga 6360
tatcggctac atatgcagtc tgtgaattat gtaacatact ctatttcttg agggctgcaa 6420
attgctaagt gctcaaaata gagtaagttt taaattgaaa attacataag atttaatgcc 6480
cttcaaatgg tttcatttag ccttgagaat ggttttttga aacttggcca cactaaaatg 6540
tttttttttt tttacgtaga atgtgggata aacttgatga actccaagtt cacagtgtca 6600
tttcttcaga actccccttc attgaatagt gatcatttat taaatgataa attgcactcg 6660
ctgaaagagc acgtcatgaa gcaccatgga atcaaagaga aagatataaa ttcgttccca 6720
cagccttcaa gctgcagtgt tttagattgc ttcaaaaaat gaaaaagttt tgcctttttc 6780
gatatagtga ccttctttgc atattaaaat gtttaccaca atgtcccatt tctagttaag 6840
tcttcgcact tgaaagctaa cattatgaat attatgtgtt ggaggagggg aaggattttc 6900
ttcattctgt gtatttccg g 6921

<210> 118
<211> 946
<212> DNA
<213> Homo sapien

<400> 118
cttctgactg ggctcaggct gacaggtaga gctcaccatg gcttcttgtg tccttgtccc 60
ctccccatca cagctgtggt gcagtccacc gtctccagtg gctatggcgg tgccagtggt 120
gtcggcagtg gcttaggcct gggtggagga agcagctact cctatggcag tggtcttggc 180
gttggaggtg gcttcagttc cagcagtggc agagccattg ggggtggcct cagctctgtt 240
ggaggcggca gttccaccat caagtacacc accacctcct cctccagcag gaagagctat 300
aagcactaaa gtgcgtctgc tagctctcgg tcccacagtc ctcaggcccc tctctggctg 360
cagagccctc tcctcaggtt gcctgtcctc tcctggcctc cagtctcccc tgctgtccca 420

ggtagagctg gggatgaatg cttagtgccc tcacttcttc tctctctctc tataccatct 480
gagcacccat tgcttaccat cagatcaacc tctgatttta catcatgatg taatcaccac 540
tggagcttca ctgttactaa attattaatt tcttgcctcc agtgttctat ctctgaggct 600
gagcattata agaaaatgac ctctgctcct tttcattgca gaaaattgcc aggggcttat 660
ttcagaacaa ctttccactta ctttccactg gctctcaaac tctctaactt ataagtgttg 720
tgaaccccca cccaggcagt atccatgaaa gcacaagtga ctagtcctat gatgtacaaa 780
gcctgtatct ctgtgatgat ttctgtgctc ttcactgttt gcaattgcta aataaagcag 840
atttataata catatattct tttactttgc cttgctttgg ggccaaagtt ttgggcttaa 900
actttttttat ctgataagtg aatagttgtt tttaaaagat aatcta 946

<210> 119
<211> 8948
<212> DNA
<213> Homo sapien

<400> 119
   tcaacagccc ctgctccttg ggcccctcca tgccatgccg taatctctcc cacccgacca 60
   acaccaacac ccagctccga cgcagctcct ctcgcccctt gccgccctcc gagccacagc 120
   tttcctcccg ctcctgcccc cggcccgtcg ccgtctccgc gctcgcagcg gcctcgggag 180
   ggcccaggta gcgagcagcg acctcgcgag ccttccgcac tcccgcccgg ttccccggcc 240
   gtccgcctat ccttggcccc ctccgctttc tccgcgccgg cccgcctcgc ttatgcctcg 300
   gcgctgagcc gctctcccga ttgcccgccg acatgagctg caacggaggc tcccacccgc 360
   ggatcaacac tctgggccgc atgatccgcg ccgagtctgg cccggacctg cgctacgagg 420
   tgaccagcgg cggcgggggc accagcagga tgtactattc tcggcgcggc gtgatcaccg 480
   accagaactc ggacggctac tgtcaaaccg gcacgatgtc caggcaccag aaccagaaca 540
   ccatccagga gctgctgcag aactgctccg actgcttgat gcgagcagag ctcatcgtgc 600
   agcctgaatt gaagtatgga gatggaatac aactgactcg gagtcgagaa ttggatgagt 660
   gttttgccca ggccaatgac caaatggaaa tcctcgacag cttgatcaga gagatgcggc 720
   agatggccca gccctgtgat gcttaccaga aaaggcttct tcagctccaa gagcaaatgc 780
   gagcccttta taaagccatc agtgtccctc gagtccgcag ggccagctcc aagggtggtg 840
   gaggctacac ttgtcagagt ggctctggct gggatgagtt caccaaacat gtcaccagtg 900
   aatgtttggg gtggatgagg cagcaaaggg cggagatgga catggtggcc tggggtgtgg 960
   acctggcctc agtggagcag cacattaaca gccaccgggg catccacaac tccatcggcg 1020
   actatcgctg gcagctggac aaaatcaaag ccgacctgcg cgagaaatct gcgatctacc 1080
   agttggagga ggagtatgaa aacctgctga aagcgtcctt tgagaggatg gatcacctgc 1140
   gacagctgca gaacatcatt caggccacgt ccagggagat catgtggatc aatgactgcg 1200
   aggaggagga gctgctgtac gactggagcg acaagaacac caacatcgct cagaaacagg 1260
   aggccttctc catacgcatg agtcaactgg aagttaaaga aaaagagctc aataagctga 1320
   aacaagaaag tgaccaactt gtcctcaatc agcatccagc ttcagacaaa attgaggcct 1380
   atatggacac tctgcagacg cagtggagtt ggattcttca gatcaccaag tgcattgatg 1440
   ttcatctgaa agaaaatgct gcctactttc agttttttga agaggcgcag tctactgaag 1500
   catacctgaa ggggctccag gactccatca ggaagaagta cccctgcgac aagaacatgc 1560
   ccctgcagca cctgctggaa cagatcaagg agctggagaa agaacgagag aaaatccttg 1620
   aatacaagcg tcaggtgcag aacttggtaa acaagtctaa gaagattgta cagctgaagc 1680
   ctcgtaaccc agactacaga agcaataaac ccattattct cagagctctc tgtgactaca 1740
   aacaagatca gaaaatcgtg cataaggggg atgagtgtat cctgaaggac aacaacgagc 1800
   gcagcaagtg gtacgtgacg ggcccgggag gcgttgacat gcttgttccc tctgtggggc 1860
   tgatcatccc tcctccgaac ccactggccg tggacctctc ttgcaagatt gagcagtact 1920
   acgaagccat cttggctctg tggaaccagc tctacatcaa catgaagagc ctggtgtcct 1980
   ggcactactg catgattgac atagagaaga tcagggccat gacaatcgcc aagctgaaaa 2040
   caatgcggca ggaagattac atgaagacga tagccgacct tgagttacat taccaagagt 2100
   tcatcagaaa tagccaaggc tcagagatgt ttggagatga tgacaagcgg aaaatacagt 2160
   ctcagttcac cgatgcccag aagcattacc agaccctggt cattcagctc cctggctatc 2220
   cccagcacca gacagtgacc acaactgaaa tcactcatca tggaacctgc caagatgtca 2280
   accataataa agtaattgaa accaacagag aaaatgacaa gcaagaaaca tggatgctga 2340
   tggagctgca gaagattcgc aggcagatag agcactgcga gggcaggatg actctcaaaa 2400
   acctccctct agcagaccag gggtcttctc accacatcac agtgaaaatt aacgagctta 2460
   agagtgtgca gaatgattca caagcaattg ctgaggttct caaccagctt aaagatatgc 2520
   ttgccaactt cagaggttct gaaaagtact gctatttaca gaatgaagta tttggactat 2580
   ttcagaaact ggaaaatatc aatggtgtta cagatggcta cttaaatagc ttatgcacag 2640
   taagggcact gctccaggct attctccaaa cagaagacat gttaaaggtt tatgaagcca 2700
   ggctcactga ggaggaaact gtctgcctgg acctggataa agtggaagct taccgctgtg 2760
   gactgaagaa aataaaaaat gacttgaact tgaagaagtc gttgttggcc actatgaaga 2820
   cagaactaca gaaagcccag cagatccact ctcagacttc acagcagtat ccactttatg 2880
   atctggactt gggcaagttc ggtgaaaaag tcacacagct gacagaccgc tggcaaagga 2940
   tagataaaca gatcgacttt agattatggg acctggagaa acaaatcaag caattgagga 3000
   attatcgtga taactatcag gctttctgca agtggctcta tgatcgtaaa cgccgccagg 3060
   attccttaga atccatgaaa tttggagatt ccaacacagt catgcggttt ttgaatgagc 3120
   agaagaactt gcacagtgaa atatctggca aacgagacaa atcagaggaa gtacaaaaaa 3180
   ttgctgaact ttgcgccaat tcaattaagg attatgagct ccagctggcc tcatacacct 3240
   caggactgga aactctgctg aacataccta tcaagaggac catgattcag tccccttctg 3300
   gggtgattct gcaagaggct gcagatgttc atgctcggta cattgaacta cttacaagat 3360
   ctggagacta ttacaggttc ttaagtgaga tgctgaagag tttggaagat ctgaagctga 3420
   aaaataccaa gatcgaagtt ttggaagagg agctcagact ggcccgagat gccaactcgg 3480
   aaaactgtaa taagaacaaa ttcctggatc agaacctgca gaaataccag gcagagtgtt 3540
   cccagttcaa agcgaagctt gcgagcctgg aggagctgaa gagacaggct gagctggatg 3600
   ggaagtcggc taagcaaaat ctagacaagt gctacggcca aataaaagaa ctcaatgaga 3660
   agatcacccg actgacttat gagattgaag atgaaaagag aagaagaaaa tctgtggaag 3720
   acagatttga ccaacagaag aatgactatg accaactgca gaaagcaagg caatgtgaaa 3780
   aggagaacct tggttggcag aaattagagt ctgagaaagc catcaaggag aaggagtacg 3840
   agattgaaag gttgagggtt ctactgcagg aagaaggcac ccggaagaga gaatatgaaa 3900
   atgagctggc aaaggtaaga aaccactata atgaggagat gagtaattta aggaacaagt 3960
   atgaaacaga gattaacatt acgaagacca ccatcaagga gatatccatg caaaaagagg 4020
   atgattccaa aaatcttaga aaccagcttg atagactttc aagggaaaat cgagatctga 4080
   aggatgaaat tgtcaggctc aatgacagca tcttgcaggc cactgagcag cgaaggcgag 4140
   ctgaagaaaa cgcccttcag caaaaggcct gtggctctga gataatgcag aagaagcagc 4200
   atctggagat agaactgaag caggtcatgc agcagcgctc tgaggacaat gcccggcaca 4260
   agcagtccct ggaggaggct gccaagacca ttcaggacaa aaataaggag atcgagagac 4320
   tcaaagctga gtttcaggag gaggccaagc gccgctggga atatgaaaat gaactgagta 4380
   aggtaagaaa caattatgat gaggagatca ttagcttaaa aaatcagttt gagaccgaga 4440
   tcaacatcac caagaccacc atccaccagc tcaccatgca gaaggaagag gataccagtg 4500
   gctaccgggc tcagatagac aatctcaccc gagaaaacag gagcttatct gaagaaataa 4560
   agaggctgaa gaacactcta acccagacca cagagaatct caggagggtg gaagaagaca 4620
   tccaacagca aaaggccact ggctctgagg tgtctcagag gaaacagcag ctggaggttg 4680
   agctgagaca agtcactcag atgcgaacag aggagagcgt aagatataag caatctcttg 4740
   atgatgctgc caaaaccatc caggataaaa acaaggagat agaaaggtta aaacaactga 4800
   tcgacaaaga aacaaatgac cggaaatgcc tggaagatga aaacgcgaga ttacaaaggg 4860
   tccagtatga cctgcagaaa gcaaacagta gtgcgacgga gacaataaac aaactgaagg 4920
   ttcaggagca agaactgaca cgcctgagga tcgactatga aagggtttcc caggagagga 4980
   ctgtgaagga ccaggatatc acgcggttcc agaactctct gaaagagctg cagctgcaga 5040
   agcagaaggt ggaagaggag ctgaatcggc tgaagaggac cgcgtcagaa gactcctgca 5100
   agaggaagaa gctggaggaa gagctggaag gcatgaggag gtcgctgaag gagcaagcca 5160
   tcaaaatcac caacctgacc cagcagctgg agcaggcatc cattgttaag aagaggagtg 5220
   aggatgacct ccggcagcag agggacgtgc tggatggcca cctgagggaa aagcagagga 5280
   cccaggaaga gctgaggagg ctctcttctg aggtcgaggc cctgaggcgg cagttactcc 5340
   aggaacagga aagtgtcaaa caagctcact tgaggaatga gcatttccag aaggcgatag 5400
   aagataaaag cagaagctta aatgaaagca aaatagaaat tgagaggctg cagtctctca 5460
   cagagaacct gaccaaggag cacttgatgt tagaagaaga actgcggaac ctgaggctgg 5520
   agtacgatga cctgaggaga ggacgaagcg aagcggacag tgataaaaat gcaaccatct 5580
   tggaactaag gagccagctg cagatcagca acaaccggac cctggaactg caggggctga 5640
   ttaatgattt acagagagag agggaaaatt tgagacagga aattgagaaa ttccaaaagc 5700
   aggctttaga ggcatctaat aggattcagg aatcaaagaa tcagtgtact caggtggtac 5760
   aggaaagaga gagccttctg gtgaaaatca aagtcctgga gcaagacaag gcaaggctgc 5820
   agaggctgga ggatgagctg aatcgtgcaa aatcaactct agaggcagaa accagggtga 5880
   aacagcgcct ggagtgtgag aaacagcaaa ttcagaatga cctgaatcag tggaagactc 5940
   aatattcccg caaggaggag gctattagga agatagaatc ggaaagagaa aagagtgaga 6000
   gagagaagaa cagtcttagg agtgagatcg aaagactcca agcagagatc aagagaattg 6060
   aagagaggtg caggcgtaag ctggaggatt ctaccaggga gacacagtca cagttagaaa 6120
   cagaacgctc ccgatatcag agggagattg ataaactcag acagcgccca tatgggtccc 6180
   atcgagagac ccagactgag tgtgagtgga ccgttgacac ctccaagctg gtgtttgatg 6240
   ggctgaggaa gaaggtgaca gcaatgcagc tctatgagtg tcagctgatc gacaaaacaa 6300
   ccttggacaa actattgaag gggaagaagt cagtggaaga agttgcttct gaaatccagc 6360
   cattccttcg gggtgcagga tctatcgctg gagcatctgc ttctcctaag gaaaaatact 6420
   ctttggtaga ggccaagaga aagaaattaa tcagcccaga atccacagtc atgcttctgg 6480
   aggcccaggc agctacaggt ggtataattg atccccatcg gaatgagaag ctgactgtcg 6540
   acagtgccat agctcgggac ctcattgact tcgatgaccg tcagcagata tatgcagcag 6600
   aaaaagctat cactggtttt gatgatccat tttcaggcaa gacagtatct gtttcagaag 6660
   ccatcaagaa aaatttgatt gatagagaaa ccggaatgcg cctgctggaa gcccagattg 6720
   cttcaggggg tgtagtagac cctgtgaaca gtgtcttttt gccaaaagat gtcgccttgg 6780
   cccgggggct gattgataga gatttgtatc gatccctgaa tgatccccga gatagtcaga 6840
   aaaactttgt ggatccagtc accaaaaaga aggtcagtta cgtgcagctg aaggaacggt 6900
   gcagaatcga accacatact ggtctgctct tgctttcagt acagaagaga agcatgtcct 6960
   tccaaggaat cagacaacct gtgaccgtca ctgagctagt agattctggt atattgagac 7020
   cgtccactgt caatgaactg gaatctggtc agatttctta tgacgaggtt ggtgagagaa 7080
   ttaaggactt cctccagggt tcaagctgca tagcaggcat atacaatgag accacaaaac 7140
   agaagcttgg catttatgag gccatgaaaa ttggcttagt ccgacctggt actgctctgg 7200
   agttgctgga agcccaagca gctactggct ttatagtgga tcctgttagc aacttgaggt 7260
   taccagtgga ggaagcctac aagagaggtc tggtgggcat tgagttcaaa gagaagctcc 7320
   tgtctgcaga acgagctgtc actgggtata atgatcctga aacaggaaac atcatctctt 7380
   tgttccaagc catgaataag gaactcatcg aaaagggcca cggtattcgc ttattagaag 7440
   cacagatcgc aaccgggggg atcattgacc caaaggagag ccatcgttta ccagttgaca 7500
   tagcatataa gaggggctat ttcaatgagg aactcagtga gattctctca gatccaagtg 7560
   atgataccaa aggatttttt gaccccaaca ctgaagaaaa tcttacctat ctgcaactaa 7620
   aagaaagatg cattaaggat gaggaaacag ggctctgtct tctgcctctg aaagaaaaga 7680
   agaaacaggt gcagacatca caaaagaata ccctcaggaa gcgtagagtg gtcatagttg 7740
   acccagaaac caataaagaa atgtctgttc aggaggccta caagaagggc ctaattgatt 7800
   atgaaacctt caaagaactg tgtgagcagg aatgtgaatg ggaagaaata accatcacgg 7860
   gatcagatgg ctccaccagg gtggtcctgg tagatagaaa gacaggcagt cagtatgata 7920
   ttcaagatgc tattgacaag ggccttgttg acaggaagtt ctttgatcag taccgatccg 7980
   gcagcctcag cctcactcaa tttgctgaca tgatctcctt gaaaaatggt gtcggcacca 8040
   gcagcagcat gggcagtggt gtcagcgatg atgtttttag cagctcccga catgaatcag 8100
   taagtaagat ttccaccata tccagcgtca ggaatttaac cataaggagc agctcttttt 8160
   cagacaccct ggaagaatcg agccccattg cagccatctt tgacacagaa aacctggaga 8220
   aaatctccat tacagaaggt atagagcggg gcatcgttga cagcatcacg ggtcagaggc 8280
   ttctggaggc tcaggcctgc acaggtggca tcatccaccc aaccacgggc cagaagctgt 8340
   cacttcagga cgcagtctcc cagggtgtga ttgaccaaga catggccacc agcgtgaagc 8400
   ctgctcagaa agccttcata ggcttcgagg gtgtgaaggg aaagaagaag atgtcagcag 8460
   cagaggcagt gaaagaaaaa tggctcccgt atgaggctgg ccagcgcttc ctggagttcc 8520
   agtacctcac gggaggtctt gttgacccgg aagtgcatgg gaggataagc accgaagaag 8580
   ccatccggaa ggggttcata gatggccgcg ccgcacagag gctgcaagac accagcagct 8640
   atgccaaaat cctgacctgc cccaaaacca aattaaaaat atcctataag gatgccataa 8700
   atcgctccat ggtagaagat atcactgggc tgcgccttct ggaagccgcc tccgtgtcgt 8760
   ccaagggctt acccagccct tacaacatgt cttcggctcc ggggtcccgc tccggctccc 8820
   gctcgggatc tcgctccgga tctcgctccg ggtcccgcag tgggtcccgg agaggaagct 8880
   ttgacgccac agggaattct tcctactctt attcctactc atttagcagt agttctattg 8940
   ggcactag                                                          8948

<210> 120
<211> 587
<212> DNA
<213> Homo sapien

<220>
<221> misc_feature
<222> (1)...(587)
<223> n = A,T,C or G

<400> 120
cgtcctaagc acttagacta catcagggaa gaacacagac cacatccctg tcctcatgcg      60
gcttatgttt tctggaagaa agtggagacc nagtccttgg ctttagggct ccccggctgg     120
gggctgtgca ntccggtcag ggcgggaagg gaaatgcacc gctgcatgtg aacttacagc     180
ccaggcggat gccccttccc ttagcactac ctggcctcct gcatcccctc gcctcatgtt     240
cctcccacct tcaaanaatg aanaacccca tgggcccagc cccttgccct ggggaaccaa     300
ggcagccttc caaaactcag gggctgaagc anactattag ggcaggggct gactttgggt      360
gacactgccc attccctctc agggcagctc angtcacccn ggnctcttga acccagcctg     420
ttcctttgaa aaagggcaaa actgaaaagg gcttttccta naaaaagaaa aaccagggaa     480
ctttgccagg gcttcnntnt taccaaaacn ncttctcnng gatttttaat tccccattng     540
gcctccactt accnggggcn atgccccaaa attaanaatt tcccatc                   587

<210> 121
<211> 619
<212> DNA
<213> Homo sapien

<220>
<221> misc_feature
<222> (1)...(619)
<223> n = A,T,C or G

<400> 121
cactagtagg atagaaacac tgtgtcccga gagtaaggag agaagctact attgattaga      60
gcctaaccca ggttaactgc aagaagaggc gggatacttt cagctttcca tgtaactgta     120
tgcataaagc caatgtagtc cagtttctaa gatcatgttc caagctaact gaatcccact     180
tcaatacaca ctcatgaact cctgatggaa caataacagg cccaagcctg tggtatgatg     240
tgcacacttg ctagactcan aaaaaatact actctcataa atgggtggga gtattttggt     300
gacaacctac tttgcttggc tgagtgaagg aatgatattc atatattcat ttattccatg     360
gacatttagt tagtgctttt tatataccag gcatgatgct gagtgacact cttgtgtata     420
tttccaaatt tttgtacagt cgctgcacat atttgaaatc atatattaag acttccaaaa     480
aatgaagtcc ctggtttttc atggcaactt gatcagtaaa ggattcncct ctgtttggta     540
cttaaaacat ctactatatn gttnanatga aattcctttt ccccncctcc cgaaaaaana     600
aagtggtggg gaaaaaaaa                                                  619

<210> 122
<211> 1475
<212> DNA
<213> Homo sapien

<400> 122
   tccacctgtc cccgcagcgc cggctcgcgc cctcctgccg cagccaccga gccgccgtct      60
   agcgccccga cctcgccacc atgagagccc tgctggcgcg cctgcttctc tgcgtcctgg     120
   tcgtgagcga ctccaaaggc agcaatgaac ttcatcaagt tccatcgaac tgtgactgtc 180
   taaatggagg aacatgtgtg tccaacaagt acttctccaa cattcactgg tgcaactgcc 240
   caaagaaatt cggagggcag cactgtgaaa tagataagtc aaaaacctgc tatgagggga 300
   atggtcactt ttaccgagga aaggccagca ctgacaccat gggccggccc tgcctgccct 360
   ggaactctgc cactgtcctt cagcaaacgt accatgccca cagatctgat gctcttcagc 420
   tgggcctggg gaaacataat tactgcagga acccagacaa ccggaggcga ccctggtgct 480
   atgtgcaggt gggcctaaag ccgcttgtcc aagagtgcat ggtgcatgac tgcgcagatg 540
   gaaaaaagcc ctcctctcct ccagaagaat taaaatttca gtgtggccaa aagactctga 600
   ggccccgctt taagattatt gggggagaat tcaccaccat cgagaaccag ccctggtttg 660
   cggccatcta caggaggcac cgggggggct ctgtcaccta cgtgtgtgga ggcagcctca 720
   tcagcccttg ctgggtgatc agcgccacac actgcttcat tgattaccca aagaaggagg 780
   actacatcgt ctacctgggt cgctcaaggc ttaactccaa cacgcaaggg gagatgaagt 840
   ttgaggtgga aaacctcatc ctacacaagg actacagcgc tgacacgctt gctcaccaca 900
   acgacattgc cttgctgaag atccgttcca aggagggcag gtgtgcgcag ccatcccgga 960
   ctatacagac catctgcctg ccctcgatgt ataacgatcc ccagtttggc acaagctgtg 1020
   agatcactgg ctttggaaaa gagaattcta ccgactatct ctatccggag cagctgaaga 1080
   tgactgttgt gaagctgatt tcccaccggg agtgtcagca gccccactac tacggctctg 1140
   aagtcaccac caaaatgctg tgtgctgctg acccacagtg gaaaacagat tcctgccagg 1200
   gagactcagg gggacccctc gtctgttccc tccaaggccg catgactttg actggaattg 1260
   tgagctgggg ccgtggatgt gccctgaagg acaagccagg cgtctacacg agagtctcac 1320
   acttcttacc ctggatccgc agtcacacca aggaagagaa tggcctggcc ctctgagggt 1380
   ccccagggag gaaacgggca ccacccgctt tcttgctggt tgtcattttt gcagtagagt 1440
   catctccatc agctgtaaga agagactggg aagat 1475

<210> 123
<211> 2294
<212> DNA
<213> Homo sapien

<400> 123
cagcgccggc tcgcgccctc ctgccgcagc caccgagccg ccgtctagcg ccccgacctc 60
gccaccatga gagccctgct ggcgcgcctg cttctctgcg tcctggtcgt gagcgactcc 120
aaaggcagca atgaacttca tcaagttcca tcgaactgtg actgtctaaa tggaggaaca 180
tgtgtgtcca acaagtactt ctccaacatt cactggtgca actgcccaaa gaaattcgga 240
gggcagcact gtgaaataga taagtcaaaa acctgctatg aggggaatgg tcacttttac 300
cgaggaaagg ccagcactga caccatgggc cggccctgcc tgccctggaa ctctgccact 360
gtccttcagc aaacgtacca tgcccacaga tctgatgctc ttcagctggg cctggggaaa 420
cataattact gcaggaaccc agacaaccgg aggcgaccct ggtgctatgt gcaggtgggc 480
ctaaagccgc ttgtccaaga gtgcatggtg catgactgcg cagatggaaa aaagccctcc 540
tctcctccag aagaattaaa atttcagtgt ggccaaaaga ctctgaggcc ccgctttaag 600
attattgggg gagaattcac caccatcgag aaccagccct ggtttgcggc catctacagg 660
aggcaccggg ggggctctgt cacctacgtg tgtggaggca gcctcatcag cccttgctgg 720
gtgatcagcg ccacacactg cttcattgat tacccaaaga aggaggacta catcgtctac 780
ctgggtcgct caaggcttaa ctccaacacg caaggggaga tgaagtttga ggtggaaaac 840
ctaatcctac acaaggacta cagcgctgac acgcttgctc accacaacga cattgccttg 900
ctgaagatcc gttccaagga gggcaggtgt gcgcagccat cccggactat acagaccatc 960
tgcctgccct cgatgtataa cgatccccag tttggcacaa gctgtgagat cactggcttt 1020
ggaaaagaga attctaccga ctatctctat ccggagcagc tgaaaatgac tgttgtgaag 1080
ctgatttccc accgggagtg tcagcagccc cactactacg gctctgaagt caccaccaaa 1140
atgctgtgtg ctgctgaccc acagtggaaa acagattcct gccagggaga ctcaggggga 1200
cccctcgtct gttccctcca aggccgcatg actttgactg gaattgtgag ctggggccgt 1260
ggatgtgccc tgaaggacaa gccaggcgtc tacacgagag tctcacactt cttaccctgg 1320
atccgcagtc acaccaagga agagaatggc ctggccctct gagggtcccc agggaggaaa 1380
cgggcaccac ccgctttctt gctggttgct attttgcagt agagtcatct ccatcagctg 1440
taagaagagc tgggaatata ggctctgcac agatggattt gcctgtgcca ccaccagggc 1500

gaacgacaat agctttaccc tcaggcatag gcctgggtgc tggctgccca gacccctctg 1560
gccaggatgg aggggtggtc ctgactcaac atgttactga ccagcaactt gtcttttcct 1620
ggactgaagc ctgcaggagt taaaaagggc agggcatctc ctgtgcatgg gctcgaaggg 1680
agagccagct cccccgaccg gtgggcattt gtgaggccca tggttgagaa atgaataatt 1740
tcccaattag gaagtgtaag cagctgaggt ctcttgaggg agcttagcca atgtgggagc 1800
agcggtttgg ggagcagaga cactaacgac ttcagggcag ggctctgata ttccatgaat 1860
gtatcaggaa atatatatgt gtgtgtatgt ttgcacactt gtgtgtgggc tgtgagtgta 1920
agtgtgagta agagctggtg tctgattgtt aagtctaaat atttccttaa actgtgtgga 1980
ctgtgatgcc acacagagtg gtctttctgg agaggttata ggtcactcct ggggcctctt 2040
gggtccccca cgtgacagtg cctgggaatg tattattctg cagcatgacc tgtgaccagc 2100
actgtctcag tttcactttc acatagatgt ccctttcttg gccagttatc ccttcctttt 2160
agcctagttc atccaatcct cactgggtgg ggtgaggacc actcctgtac actgaatatt 2220
tatatttcac tatttttatt tatatttttg taattttaaa taaaagtgat caataaaatg 2280
tgatttttct gatg 2294

<210> 124
<211> 956
<212> DNA
<213> Homo sapien

<400> 124
gatgagttcc gcaccaagtt tgagacagac caggccctgc gcctgagtgt ggaggccgac 60
atcaatggcc tgcgcagggt gctggatgag ctgaccctgg ccagagccga cctggagatg 120
cagattgaga acctcaagga ggagctggcc tacctgaaga agaaccacga ggaggagatg 180
aacgccctgc gaggccaggt gggtggtgag atcaatgtgg agatggacgc tgccccaggc 240
gtggacctga gccgcatcct caacgagatg cgtgaccagt atgagaagat ggcagagaag 300
aaccgcaagg atgccgagga ttggttcttc agcaagacag aggaactgaa ccgcgaggtg 360
gccaccaaca gtgagctggt gcagagtggc aagagtgaga tctcggagct ccggcgcacc 420
atgcaggcct tggagataga gctgcagtcc cagctcagca tgaaagcatc cctggagggc 480
aacctggcgg agacagagaa ccgctactgc gtgcagctgt cccagatcca ggggctgatt 540
ggcagcgtgg aggagcagct ggcccagctt cgctgcgaga tggagcagca gaaccaggaa 600
tacaaaatcc tgctggatgt gaagacgcgg ctggagcagg agattgccac ctaccgccgc 660
ctgctggagg gagaggatgc ccacctgact cagtacaaga aagaaccggt gaccacccgt 720
caggtgcgta ccattgtgga agaggtccag gatggcaagg tcatctcctc ccgcgagcag 780
gtccaccaga ccacccgctg aggactcagc taccccggcc ggccacccag gaggcaggga 840
cgcagccgcc ccatctgccc cacagtctcc ggcctctcca gcctcagccc cctgcttcag 900
tcccttcccc atgcttcctt gcctgatgac aataaaagct tgttgactca gctatg 956

<210> 125
<211> 486
<212> DNA
<213> Homo sapien

<220>
<221> misc_feature
<222> (1)...(486)
<223> n = A,T,C or G

<400> 125
aaattatata tagtgnttca gctcccattg tggtgttcat agtcttctag gaacagataa 60
acttaagtat tcaattcact cttggcattt tttctttaat ataggctttt tagcctattt 120
ttggaaaact gcttttcttc tgagaacctt attctgaatg tcatcaactt taccaaacct 180
tctaagtcca gagctaactt agtactgttt aagttactat tgactgaatt ttcttcattt 240
tctgtttagc cagtgttacc aaggtaagct ggggaatgaa gtataccaac ttctttcaga 300
gcattttagg acattatggc agctttagaa ggctgtcttg tttctagcca agggagagct 360

agcgcaggtt ttggatacta gagaaagtca tttgcttgta ctattgccat tttagaaagc 420
tctgatgtga attcaaattt tacctctgtt acttaaagcc aacaatttta aggcagtagt 480
tttact 486

<210> 126
<211> 3552
<212> DNA
<213> Homo sapien

<400> 126
cggcaggcag gtctcgtctc ggcaccctcc cggcgcccgc gttctcctgg ccctgcccgg 60
catcccgatg gccgccgctg ggccccggcg ctccgtgcgc ggagccgtct gcctgcatct 120
gctgctgacc ctcgtgatct tcagtcgtgc tggtgaagcc tgcaaaaagg tgatacttaa 180
tgtaccttct aaactagagg cagacaaaat aattggcaga gttaatttgg aagagtgctt 240
caggtctgca gacctcatcc ggtcaagtga tcctgatttc agagttctaa atgatgggtc 300
agtgtacaca gccagggctg ttgcgctgtc tgataagaaa agatcattta ccatatggct 360
ttctgacaaa aggaaacaga cacagaaaga ggttactgtg ctgctagaac atcagaagaa 420
ggtatcgaag acaagacaca ctagagaaac tgttctcagg cgtgccaaga ggagatgggc 480
acctattcct tgctctatgc aagagaattc cttgggccct ttcccattgt ttcttcaaca 540
agttgaatct gatgcagcac agaactatac tgtcttctac tcaataagtg gacgtggagt 600
tgataaagaa cctttaaatt tgttttatat agaaagagac actggaaatc tattttgcac 660
tcggcctgtg gatcgtgaag aatatgatgt ttttgatttg attgcttatg cgtcaactgc 720
agatggatat tcagcagatc tgcccctccc actacccatc agggtagagg atgaaaatga 780
caaccaccct gttttcacag aagcaattta taattttgaa gttttggaaa gtagtagacc 840
tggtactaca gtgggggtgg tttgtgccac agacagagat gaaccggaca caatgcatac 900
gcgcctgaaa tacagcattt tgcagcagac accaaggtca cctgggctct tttctgtgca 960
tcccagcaca ggcgtaatca ccacagtctc tcattatttg gacagagagg ttgtagacaa 1020
gtactcattg ataatgaaag tacaagacat ggatggccag ttttttggat tgataggcac 1080
atcaacttgt atcataacag taacagattc aaatgataat gcacccactt tcagacaaaa 1140
tgcttatgaa gcatttgtag aggaaaatgc attcaatgtg gaaatcttac gaatacctat 1200
agaagataag gatttaatta acactgccaa ttggagagtc aattttacca ttttaaaggg 1260
aaatgaaaat ggacatttca aaatcagcac agacaaagaa actaatgaag gtgttctttc 1320
tgttgtaaag ccactgaatt atgaagaaaa ccgtcaagtg aacctggaaa ttggagtaaa 1380
caatgaagcg ccatttgcta gagatattcc cagagtgaca gccttgaaca gagccttggt 1440
tacagttcat gtgagggatc tggatgaggg gcctgaatgc actcctgcag cccaatatgt 1500
gcggattaaa gaaaacttag cagtggggtc aaagatcaac ggctataagg catatgaccc 1560
cgaaaataga aatggcaatg gtttaaggta caaaaaattg catgatccta aaggttggat 1620
caccattgat gaaatttcag ggtcaatcat aacttccaaa atcctggata gggaggttga 1680
aactcccaaa aatgagttgt ataatattac agtcctggca atagacaaag atgatagatc 1740
atgtactgga acacttgctg tgaacattga agatgtaaat gataatccac cagaaatact 1800
tcaagaatat gtagtcattt gcaaaccaaa aatggggtat accgacattt tagctgttga 1860
tcctgatgaa cctgtccatg gagctccatt ttatttcagt ttgcccaata cttctccaga 1920
aatcagtaga ctgtggagcc tcaccaaagt taatgataca gctgcccgtc tttcatatca 1980
gaaaaatgct ggatttcaag aatataccat tcctattact gtaaaagaca gggccggcca 2040
agctgcaaca aaattattga gagttaatct gtgtgaatgt actcatccaa ctcagtgtcg 2100
tgcgacttca aggagtacag gagtaatact tggaaaatgg gcaatccttg caatattact 2160
gggtatagca ctgctctttt ctgtattgct aactttagta tgtggagttt ttggtgcaac 2220
taaagggaaa cgttttcctg aagatttagc acagcaaaac ttaattatat caaacacaga 2280
agcacctgga gacgatagag tgtgctctgc caatggattt atgacccaaa ctaccaacaa 2340
ctctagccaa ggtttttgtg gtactatggg atcaggaatg aaaaatggag ggcaggaaac 2400
cattgaaatg atgaaaggag gaaaccagac cttggaatcc tgccgggggg ctgggcatca 2460
tcataccctg gactcctgca ggggaggaca cacggaggtg gacaactgca gatacactta 2520
ctcggagtgg cacagtttta ctcaaccccg tctcggtgaa aaattgcatc gatgtaatca 2580
gaatgaagac cgcatgccat cccaagatta tgtcctcact tataactatg agggaagagg 2640
atctccagct ggttctgtgg gctgctgcag tgaaaagcag gaagaagatg gccttgactt 2700

tttaaataat ttggaaccca aatttattac attagcagaa gcatgcacaa agagataatg 2760
tcacagtgct acaattaggt ctttgtcaga cattctggag gtttccaaaa ataatattgt 2820
aaagttcaat ttcaacatgt atgtatatga tgattttttt ctcaattttg aattatgcta 2880
ctcaccaatt tatattttta aagcaagttg ttgcttatct tttccaaaaa gtgaaaaatg 2940
ttaaaacaga caactggtaa atctcaaact ccagcactgg aattaaggtc tctaaagcat 3000
ctgctctttt tttttttac agatatttta gtaataaata tgctggataa atattagtcc 3060
aacaatagct aagttatgct aatatcacat tattatgtat tcactttaag tgatagttta 3120
aaaaataaac aagaaatatt gagtatcact atgtgaagaa agttttggaa aagaaacaat 3180
gaagactgaa ttaaattaaa aatgttgcag ctcataaaga attggactca cccctactgc 3240
actaccaaat tcatttgact ttggaggcaa aatgtgttga agtgccctat gaagtagcaa 3300
ttttctatag gaatatagtt ggaaataaat gtgtgtgtgt atattattat taatcaatgc 3360
aatatttaaa tgaaatgaga acaaagagga aaatggtaaa aacttgaaat gaggctgggg 3420
tatagtttgt cctacaatag aaaaaagaga gagcttccta ggcctgggct cttaaatgct 3480
gcattataac tgagtctatg aggaaatagt tcctgtccaa tttgtgtaat ttgtttaaaa 3540
ttgtaaataa at 3552

<210> 127
<211> 754
<212> DNA
<213> Homo sapien

<400> 127
tttttttttt ttgtcattgt tcattgattt taatgagaaa gctaagagag gaaataagta 60
gcctttcaaa ggtcacacag aagtaagtga cagatccagg atttatatcc aagcattctg 120
gctctagtgt ccatgcttct caaccattat gacccaatat tcaaccaaat caatactgaa 180
ggacacgtga aatgtatccg gtatttactt attacaaaca aaaatccaat gaacattctt 240
gaagacatac acaaaaataa tggttacaat agaagttact ggaattgaaa ttttggttca 300
acctatatta aaatgtaagg cttttgatat agctaataga tttttgaaat gatcagtctt 360
aacgtttgta ggggagcaca ctcctgcatg ggaaaagat tcactgtgaa gcacagagca 420
cctttatggt tggatcatct tgtcattaaa gttcaggcgt tatctatcct gtaagtggca 480
gaatcaagac tgcaatatcg cctgcttttc ttttaactc atgtttcccc ttgactacac 540
tggtcctcaa agtaaaaccc ctgtgtcagt gtactattca tggaatactc tgcaattata 600
accaccttct aatactttta atacccaatc aaaatttatt atacatatgt atcatagata 660
ctcatctgta aagctgtgct tcaaaatagt gatctcttcc caacattaca atatatatta 720
atgatgtcga acctgcccgg gcggccgctc gaag 754

<210> 128
<211> 374
<212> DNA
<213> Homo sapien

<400> 128
aggttttgat taaaaaggca aatgatttta ttgttcgata atcttttaaa aaaataagag 60
gaaggagtaa aattaaagat gaaagatgat ttttatttcc ttgtgacctc tatatccccc 120
ttcccctgcc cttggtaagt aactcttgat ggagaaagga ttaaagactc ttatttaacc 180
aaaaaacaga gccagctaat catttccaaa ggttagtatc tccctgctga cctcttcttt 240
ggtttaattg aataaaacta tatgttcata tatgtattaa aacaactcag aataacatct 300
tttcttcctt agttaaggca ttataagggc tatactatca tccataataa ccaaggcaat 360
aacttaaaaa gctg 374

<210> 129
<211> 546
<212> DNA
<213> Homo sapien

<400> 129
agtgtgatgg atatctgcag aattcgggct aagcgtggtc gcggcccgag gtctggaact 60
tcccagcacy tgaaaaggag cctcctgagc tgactcggct aaagccccac tttcgctcct 120
cctcatttct gcctactgat ttccttggag cattcatctg aatattaccg tttgctgtgt 180
aacctggtac atacatagca tgactccctg gaatagagtg ggctggggtg cttatgctgg 240
gagagtgatt gacatgcact ttcaagctat atctaccatt tgcagcaaag gagaaaaaat 300
acctcgagta aattccatca ttttttataa catcagcacc tgctccatca tcaaggagtc 360
tcagcgtaac aggatctcca gtctctggct caactgtggc agtgacagtg gcattaagaa 420
tgggataaaa tccctgtttc acattggcat aaatcatcac aggatgagga aaatggaggc 480
tgtctctttc cacaaaggct tccacagtgg ctgggggcac agacctgccc gggcggccgc 540
tcgaaa 546

<210> 130
<211> 5156
<212> DNA
<213> Homo sapien

<400> 130
accaaccgag gcgccgggca gcgacccctg cagcggagac agagactgag cggcccggca 60
ccgccatgcc tgcgctctgg ctgggctgct gcctctgctt gtcgctcctc ctgcccgcag 120
cccgggccac ctccaggagg gaagtctgtg attgcaatgg gaagtccagg cagtgtatct 180
ttgatcggga actttacaga caaactggta atggattccg ctgcctcaac tgcaatgaca 240
acactgatgg cattcactgc gagaagtgca agaatggctt ttaccggcac agagaaaggg 300
accgctgttt gccctgcaat tgtaactcca aaggttctct tagtgctcga tgtgacaact 360
ccggacggtg cagctgtaaa ccaggtgtga caggagccag atgcgaccga tgtctgccag 420
gcttccacat gctcacggat gcggggtgca cccaagacca gagactgcta gactccaagt 480
gtgactgtga cccagctggc atcgcagggc cctgtgacgc gggccgctgt gtctgcaagc 540
cagctgtcac tggagaacgc tgtgataggt gtcgatcagg ttactataat ctggatgggg 600
ggaaccctga gggctgtacc cagtgtttct gctatgggca ttcagccagc tgccgcagct 660
ctgcagaata cagtgtccat aagatcacct ctacctttca tcaagatgtt gatggctgga 720
aggctgtcca acgaaatggg tctcctgcaa agctccaatg gtcacagcgc catcaagatg 780
tgtttagctc agcccaacga ctagaccctg tctattttgt ggctcctgcc aaatttcttg 840
ggaatcaaca ggtgagctat ggtcaaagcc tgtcctttga ctaccgtgtg gacagaggag 900
gcagacaccc atctgcccat gatgtgattc tggaaggtgc tggtctacgg atcacagctc 960
ccttgatgcc acttggcaag acactgcctt gtgggctcac caagacttac acattcaggt 1020
taaatgagca tccaagcaat aattggagcc cccagctgag ttactttgag tatcgaaggt 1080
tactgcggaa tctcacagcc ctccgcatcc gagctacata tggagaatac agtactgggt 1140
acattgacaa tgtgaccctg atttcagccc gccctgtctc tggagcccca gcaccctggg 1200
ttgaacagtg tatatgtcct gttgggtaca aggggcaatt ctgccaggat tgtgcttctg 1260
gctacaagag agattcagcg agactggggc cttttggcac ctgtattcct tgtaactgtc 1320
aaggggggag ggcctgtgat ccagacacag gagattgtta ttcaggggat gagaatcctg 1380
acattgagtg tgctgactgc ccaattggtt tctacaacga tccgcacgac ccccgcagct 1440
gcaagccatg tccctgtcat aacgggttca gctgctcagt gatgccggag acggaggagg 1500
tggtgtgcaa taactgccct cccggggtca ccggtgcccg ctgtgagctc tgtgctgatg 1560
gctactttgg ggaccccttt ggtgaacatg gcccagtgag gccttgtcag ccctgtcaat 1620
gcaacaacaa tgtggacccc agtgcctctg ggaattgtga ccggctgaca ggcaggtgtt 1680
tgaagtgtat ccacaacaca gccggcatct actgcgacca gtgcaaagca ggctacttcg 1740
gggacccatt ggctcccaac ccagcagaca agtgtcgagc ttgcaactgt aaccccatgg 1800
gctcagagcc tgtaggatgt cgaagtgatg gcacctgtgt ttgcaagcca ggatttggtg 1860
gccccaactg tgagcatgga gcattcagct gtccagcttg ctataatcaa gtgaagattc 1920
agatggatca gtttatgcag cagcttcaga gaatggaggc cctgatttca aaggctcagg 1980
gtggtgatgg agtagtacct gatacagagc tggaaggcag gatgcagcag gctgagcagg 2040
cccttcagga cattctgaga gatgcccaga tttcagaagg tgctagcaga tcccttggtc 2100
tccagttggc caaggtgagg agccaagaga acagctacca gagccgcctg gatgacctca 2160
agatgactgt ggaaagagtt cgggctctgg gaagtcagta ccagaaccga gttcgggata 2220

ctcacaggct catcactcag atgcagctga gcctggcaga aagtgaagct tccttgggaa 2280
acactaacat tcctgcctca gaccactacg tggggccaaa tggctttaaa agtctggctc 2340
aggaggccac aagattagca gaaagccacg ttgagtcagc cagtaacatg gagcaactga 2400
caagggaaac tgaggactat tccaaacaag ccctctcact ggtgcgcaag gccctgcatg 2460
aaggagtcgg aagcggaagc ggtagcccgg acggtgctgt ggtgcaaggg cttgtggaaa 2520
aattggagaa aaccaagtcc ctggcccagc agttgacaag ggaggccact caagcggaaa 2580
ttgaagcaga taggtcttat cagcacagtc tccgcctcct ggattcagtg tctcggcttc 2640
agggagtcag tgatcagtcc tttcaggtgg aagaagcaaa gaggatcaaa caaaaagcgg 2700
attcactctc aagcctggta accaggcata tggatgagtt caagcgtaca cagaagaatc 2760
tgggaaactg gaaagaagaa gcacagcagc tcttacagaa tggaaaaagt gggagagaga 2820
aatcagatca gctgctttcc cgtgccaatc ttgctaaaag cagagcacaa gaagcactga 2880
gtatgggcaa tgccactttt tatgaagttg agagcatcct taaaaacctc agagagtttg 2940
acctgcaggt ggacaacaga aaagcagaag ctgaagaagc catgaagaga ctctcctaca 3000
tcagccagaa ggtttcagat gccagtgaca agacccagca agcagaaaga gccctgggga 3060
gcgctgctgc tgatgcacag agggcaaaga atggggccgg ggaggccctg gaaatctcca 3120
gtgagattga acaggagatt gggagtctga acttggaagc caatgtgaca gcagatggag 3180
ccttggccat ggaaaaggga ctggcctctc tgaagagtga gatgagggaa gtggaaggag 3240
agctggaaag gaaggagctg gagtttgaca cgaatatgga tgcagtacag atggtgatta 3300
cagaagccca gaaggttgat accagagcca agaacgctgg ggttacaatc caagacacac 3360
tcaacacatt agacggcctc ctgcatctga tggaccagcc tctcagtgta gatgaagagg 3420
ggctggtctt actggagcag aagctttccc gagccaagac ccagatcaac agccaactgc 3480
ggcccatgat gtcagagctg gaagagaggg cacgtcagca gaggggccac ctccatttgc 3540
tggagacaag catagatggg attctggctg atgtgaagaa cttggagaac attagggaca 3600
acctgccccc aggctgctac aatacccagg ctcttgagca acagtgaagc tgccataaat 3660
atttctcaac tgaggttctt gggatacaga tctcagggct cgggagccat gtcatgtgag 3720
tgggtgggat ggggacattt gaacatgttt aatgggtatg ctcaggtcaa ctgacctgac 3780
cccattcctg atcccatggc caggtggttg tcttattgca ccatactcct tgcttcctga 3840
tgctgggcaa tgaggcagat agcactgggt gtgagaatga tcaaggatct ggaccccaaa 3900
gaatagactg gatggaaaga caaactgcac aggcagatgt ttgcctcata atagtcgtaa 3960
gtggagtcct ggaatttgga caagtgctgt tgggatatag tcaacttatt ctttgagtaa 4020
tgtgactaaa ggaaaaaact ttgactttgc ccaggcatga aattcttcct aatgtcagaa 4080
cagagtgcaa cccagtcaca ctgtggccag taaaatacta ttgcctcata ttgtcctctg 4140
caagcttctt gctgatcaga gttcctccta cttacaaccc agggtgtgaa catgttctcc 4200
attttcaagc tggaagaagt gagcagtgtt ggagtgagga cctgtaaggc aggcccattc 4260
agagctatgg tgcttgctgg tgcctgccac ctttaagttc tggacctggg catgacatcc 4320
tttcttttaa tgatgccatg gcaacttaga gattgcattt ttattaaagc atttcctacc 4380
agcaaagcaa atgttgggaa agtatttact ttttcggttt caaagtgata gaaaagtgtg 4440
gcttgggcat tgaaagaggt aaaattctct agatttatta gtcctaattc aatcctactt 4500
ttagaacacc aaaaatgatg cgcatcaatg tattttatct tattttctca atctcctctc 4560
tctttcctcc acccataata agagaatgtt cctactcaca cttcagctgg gtcacatcca 4620
tccctccatt catccttcca tccatctttc catccattac ctccatccat ccttccaaca 4680
tatatttatt gagtacctac tgtgtgccag gggctggtgg gacagtggtg acatagtctc 4740
tgccctcata gagttgattg tctagtgagg aagacaagca tttttaaaaa ataaatttaa 4800
acttacaaac tttgtttgtc acaagtggtg tttattgcaa taaccgcttg gtttgcaacc 4860
tctttgctca acagaacata tgttgcaaga ccctcccatg ggggcacttg agttttggca 4920
aggctgacag agctctgggt tgtgcacatt tctttgcatt ccagctgtca ctctgtgcct 4980
ttctacaact gattgcaaca gactgttgag ttatgataac accagtggga attgctggag 5040
gaaccagagg cacttccacc ttggctggga agactatggt gctgccttgc ttctgtattt 5100
ccttggattt tcctgaaagt gtttttaaat aaagaacaat tgttagaaaa aaaaaa 5156

<210> 131
<211> 671
<212> DNA
<213> Homo sapien

<400> 131
aggtctggag ggcccacagc cggatgtggg acaccgggaa aaagtggtca tagcacacat 60
ttttgcatcc cggttgcagt gtgttgcaga cgaagtcctc ttgctcgtca ccccacactt 120
cctgggcagc caycacgagg atcatgactc ggaaaataaa gatgactgtg atccacacct 180
tcccgatgct ggtggagtgt ttgttgacac ccccgatgaa agtgtgcagc gtcccccaat 240
ccattgcgct ggtttatccc tgagtcctgt ttccaacgac tgccagtgtt tcagacccaa 300
agaatgaggg caagatccct ctgcgagggt ttcagacctc cttctcctac cccactggag 360
tgcctagaag ccaatgggtg cacagtgatg atacgaatgt caatctttgc tcggtcagtg 420
aggatgtcgc ctggaatatt caaattgaat tacagatgca tgaagagggc gtacaagtta 480
gaatttttct ttcgccatac agaaattgtt tagccagatc ttctgtactt cttttccttc 540
cctgaccctt cctgctcccc aggaagggag gtcagccccg tttgcaaaac acaggatgcc 600
cgtgacaccg gagacaggtc ttcttcaccg acaggaagtg ccttctggtg cctgcacgtt 660
ttaactgcta t 671

<210> 132
<211> 590
<212> DNA
<213> Homo sapien

<400> 132
ctgaatggaa aagcttatgg ctctgtgatg atattagtga ccagcggaga tgataagctt 60
cttggcaatt gcttacccac tgtgctcagc agtggttcaa caattcactc cattgccctg 120
ggttcatctg cagccccaaa tctggaggaa ttatcacgtc ttacaggagg tttaaagttc 180
tttgttccag atatatcaaa ctccaatagc atgattgatg ctttcagtag aatttcctct 240
ggaactggag acattttcca gcaacatatt cagcttgaaa gtacaggtga aaatgtcaaa 300
cctcaccatc aattgaaaaa cacagtgact gtggataata ctgtgggcaa cgacactatg 360
tttctagtta cgtggcaggc cagtggtcct cctgagatta tattatttga tcctgatgga 420
cgaaaatact acacaaataa ttttatcacc aatctaactt ttcggacagc tagtttttgg 480
attccaggaa cagctaagcc tgggcactgg acttacaccc tgaacaatac ccatcattct 540
ctgcaagccc tgaaagtgac agtgacctct cgcgcctcca actcagacct 590

<210> 133
<211> 581
<212> DNA
<213> Homo sapien

<400> 133
aggtcctgtc cgggggcact gagaactccc tctggaattc ttggggggtg ttggggagag 60
actgtgggcc tggagataaa acttgtctcc tctaccacca ccctgtaccc tagcctgcac 120
ctgtcctcat ctctgcaaag ttcagcttcc ttccccaggt ctctgtgcac tctgtcttgg 180
atgctctggg gagctcatgg gtggaggagt ctccaccaga gggaggctca ggggactggt 240
tgggccaggg atgaatattt gagggataaa aattgtgtaa gagccaaaga attggtagta 300
gggggagaac agagaggagc tgggctatgg gaaatgattt gaataatgga gctgggaata 360
tggctggata tctggtacta aaaaagggtc tttaagaacc tacttcctaa tctcttcccc 420
aatccaaacc atagctgtct gtccagtgct ctcttcctgc ctccagctct gccccaggct 480
ccccctagac tctgtccctg ggctagggca ggggaggagg gagagcaggg ttgggggaga 540
ggctgaggag agtgtgacat gtggggagag gaccagacct c 581

<210> 134
<211> 4797
<212> DNA
<213> Homo sapien

<220>
<221> misc_feature

<222> (1)...(4797)
<223> n = A,T,C or G

<400> 134
cctgggacca aagtgctgcc cagagctgag ggtcctggag ccacatgaga aggcttctcc 60
ctgtgtacct gtgcagcaca gggtagggtg agtccactca gctgtctagg agaggaccca 120
ggagcagcag agacncgcca agcctttact cataccatat tctgatcctt ttccagcaaa 180
ttgtggctac taatttgccc cctgaagatc aagatggctc tggggatgac tctgacaact 240
tctccggctc aggtgcaggt gaggttgtca tgggggcccc ccccacccaa gacggcaaca 300
ggtcatgcct gggggcagtg gtcaggcagt ctcctgtgtt tactgagcat gtactgagtg 360
caccctgcct gccctgtctc cacccagctg gctccaaagg gcaatgctga ggagaggaat 420
ggggtcgtga gctgctgtta aggagagctc atgcttggag gtgaggtgaa ggctgtgagc 480
tccagaaggc cccagggcgc nctgctgcac gcaggctcat attcactagg aatagcttta 540
ctcactaaga aacctctgga acccccttca gaaggttatt tgactcctga gcctctattt 600
tctcatctgc aaaatgggaa taataccttg acctgataag cttgtggagc tgtaaggcag 660
cacagagcca gctggggtgt agctcttcca tccaagctcc cttccttact tcccctttcc 720
tgtggggact gggggagaga agtccctgag ctggaggtgg tcagggaagc ttcacagagg 780
aggtggctct tgagtggacc tcaggaagag gggtgagaga gctaaggaag gaggctgagg 840
tcatccctgg ggaagtgacc tagcggaggc ctgagagctg caaggtagga tatctgttgt 900
tggaagtgtc tgttgttgga agtgggggcc tttttttcag ggagggtggg gccagagaag 960
tgtgtgccct gggataagta ggataaccac agtagttatg cccctaaggg atgcccaccc 1020
cacccctgtg gtcacagaaa agctttccca ggtggcctag gcacctgtct cgtggctcca 1080
gagacaggct gcacctgaca cacacaatgg aaggacagct ctccttgtcc attttccaag 1140
gagcttagcc tcagctgcct tgtccaggta ctagcctccc tcatagcttg agcttggcca 1200
gcccaggtgc tctggagcct cccccgaccc acccaacaca ctctgcttct ggtcctcccc 1260
accccccacc tccccaacac actctgcttc tggtcctgca ggtgctttgc aagatatcac 1320
cttgtcacag cagaccccct ccacttggaa ggacacgcag ctcctgacgg ctattcccac 1380
gtctccagaa cccaccggcc tggaggctac agctgcctcc acctccaccc tgccggctgg 1440
agaggggccc aaggagggag aggctgtagt cctgccagaa gtggagcctg gcctcaccgc 1500
ccgggagcag gaggccaccc cccgacccag ggagaccaca cagctcccga ccactcatca 1560
ggcctcaacg accacagcca ccacggccca ggagcccgcc acctcccacc cccacaggga 1620
catgcagcct ggccaccatg agacctcaac ccctgcagga cccagccaag ctgaccttca 1680
cactccccac acagaggatg gaggtccttc tgccaccgag agggctgctg aggatggagc 1740
ctccagtcag ctcccagcag cagagggctc tggggagcag gtgagtggcc tctgcattcc 1800
ttgggaaatt gagtgggttg gtcctaatgc ctggcacttg gcaggcccta cacctgtgcc 1860
ctgcgcgatc tcgtattcct caccaggaag acagggcaca ggggccgcct tcccctaccc 1920
ccagggcctc gcagagcagg acagactaac tatgagatca gagcagaagc acccttaaag 1980
atcacccaag agagggctcc caaactcaca atccaaactt gcagccctcg tcgaagagtg 2040
aacgttatac cagtcatttt atttatagct tcgtggattt acgcttacac taaatagtct 2100
gctattcata caaaatgtgt gctttgtatc actttttgtg atatccatgc catggtccag 2160
ccagggtccg gagttgatgt ggcaagaagg cctggctttc gggccctgtg cgatcctggt 2220
ttgggtgcat ctgagtgggt ggtggcaaag atcagggagg caggagctgc ttctgggtct 2280
gtagtggagc tggttgctgc tgctggcggt gacctggcca acccaatctg cccctgccct 2340
cccacaggac ttcacctttg aaacctcggg ggagaatacg gctgtagtgg ccgtggagcc 2400
tgaccgccgg aaccagtccc cagtggatca gggggccacg ggggcctcac agggcctcct 2460
ggacaggaaa gaggtgctgg gaggtgagtt ttctttcagg ggggtagttt ggggtgaatt 2520
gctgctgtgg ggtcagggtg gggctgacca cagccaaggc cactgctttg ggagggtctg 2580
cacgagagcc caaggagccg ctgagctgag ctggccccgt ctacctgccc taggggtcat 2640
tgccggaggc ctcgtggggc tcatctttgc tgtgtgcctg gtgggtttca tgctgtaccg 2700
catgaagaag aaggacgaag gcagctactc cttggaggag ccgaaacaag ccaacggcgg 2760
ggcctaccag aagcccacca aacaggagga attctatgcc tgacgcggga gccatgcgcc 2820
cccctcgccc tgccactcac taggccccca cttgcctctt ccttgaagaa ctgcaggccc 2880
tggcctcccc tgccaccagg ccacctcccc agcattccag cccctctggt cgctcctgcc 2940
cacggagtcg tgggtgtgct gggagctcca ctctgcttct ctgacttctg cctggagact 3000
tagggcacca ggggtttctc gcataggacc tttccaccac agccagcacc tggcatcgca 3060

ccattctgac tcggtttctc caaactgaag cagcctctcc ccaggtccag ctctggaggg 3120
gagggggatc cgactgcttt ggacctaaat ggcctcatgt ggctggaaga tcctgcgggt 3180
ggggcttggg gctcacacac ctgtagcact tactggtagg accaagcatc ttgggggggt 3240
ggccgctgag tggcagggga caggagtcac tttgtttcgt ggggaggtct aatctagata 3300
tcgacttgtt tttgcacatg tttcctctag ttctttgttc atagcccagt agaccttgtt 3360
acttctgagg taagttaagt aagttgattc ggtatccccc catcttgctt ccctaatcta 3420
tggtcgggag acagcatcag ggttaagaag actttttttt ttttttttaa actaggagaa 3480
ccaaatctgg aagccaaaat gtaggcttag tttgtgtgtt gtctcttgag tttgtcgctc 3540
atgtgtgcaa cagggtatgg actatctgtc tggtggcccc gttctggtgg tctgttggca 3600
ggctggccag tccaggctgc cgtggggccg ccgcctcttt caagcagtcg tgcctgtgtc 3660
catgcgctca gggccatgct gaggcctggg ccgctgccac gttggagaag cccgtgtgag 3720
aagtgaatgc tgggactcag ccttcagaca gagaggactg tagggagggc ggcaggggcc 3780
tggagatcct cctgcaggct cacgcccgtc ctcctgtggc gccgtctcca ggggctgctt 3840
cctcctggaa attgacgagg ggtgtcttgg gcagagctgg ctctgagcgc ctccatccaa 3900
ggccaggttc tccgttagct cctgtggccc caccctgggc cctgggctgg aatcaggaat 3960
attttccaaa gagtgatagt cttttgcttt tggcaaaact ctacttaatc caatgggttt 4020
ttccctgtac agtagatttt ccaaatgtaa taaactttaa tataaagtag tctgtgaatg 4080
ccactgcctt cgcttcttgc ctctgtgctg tgtgtgacgt gaccggactt ttctgcaaac 4140
accaacatgt tgggaaactt ggctcgaatc tctgtgcctt cgtctttccc atggggaggg 4200
attctggttc cagggtccct ctgtgtattt gcttttttgt tttggctgaa attctcctgg 4260
aggtcggtag gttcagccaa ggttttataa ggctgatgtc aatttctgtg ttgccaagct 4320
ccaagcccat cttctaaatg gcaaaggaag gtggatggcc ccagcacagc ttgacctgag 4380
gctgtggtca cagcggaggt gtggagccga ggcctacccc ncagacacct tggacatcct 4440
cctcccaccc ggctgcagag gccaganncc agcccagggt cctgcactta cttgcttatt 4500
tgacaacgtt tcagcgactc cgttggccac tccgagagtg ggccagtctg tggatcagag 4560
atgcaccacc aagccaaggg aacctgtgtc cggtattcga tactgcgact ttctgcctgg 4620
agtgtatgac tgcacatgac tcgggggtgg ggaaaggggt cggctgacca tgctcatctg 4680
ctggtccgtg ggacggtncc caagccagag gtgggttcat ttgtgtaacg acaataaacg 4740
gtacttgtca tttcgggcaa cggctgctgt ggtggtggtt gagtctcttc ttggctt 4797

<210> 135
<211> 2856
<212> DNA
<213> Homo sapien

<400> 135
tagtcgcggg tccccgagtg agcacgccag ggagcaggag accaaacgac gggggtcgga 60
gtcagagtcg cagtgggagt ccccggaccg gagcacgagc ctgagcggga gagcgccgct 120
cgcacgcccg tcgccacccg cgtacccggc gcagccagag ccaccagcgc agcgctgcca 180
tggagcccag cagcaagaag ctgacgggtc gcctcatgct ggctgtggga ggagcagtgc 240
ttggctccct gcagtttggc tacaacactg gagtcatcaa tgccccccag aaggtgatcg 300
aggagttcta caaccagaca tgggtccacc gctatgggga gagcatcctg cccaccacgc 360
tcaccacgct ctggtccctc tcagtggcca tcttttctgt tgggggcatg attggctcct 420
tctctgtggg cctttttcgtt aaccgctttg gccggcggaa ttcaatgctg atgatgaacc 480
tgctggcctt cgtgtccgcc gtgctcatgg gcttctcgaa actgggcaag tcctttgaga 540
tgctgatcct gggccgcttc atcatcggtg tgtactgcgg cctgaccaca ggcttcgtgc 600
ccatgtatgt gggtgaagtg tcacccacag cctttcgtgg ggccctgggc accctgcacc 660
agctgggcat cgtcgtcggc atcctcatcg cccaggtgtt cggcctggac tccatcatgg 720
gcaacaagga cctgtggccc ctgctgctga gcatcatctt catcccggcc ctgctgcagt 780
gcatcgtgct gcccttctgc cccgagagtc cccgcttcct gctcatcaac cgcaacgagg 840
agaaccgggc caagagtgtg ctaaagaagc tgcgcgggac agctgacgtg acccatgacc 900
tgcaggagat gaaggaagag agtcggcaga tgatgcggga gaagaaggtc accatcctgg 960
agctgttccg ctcccccgcc taccgccagc ccatcctcat cgctgtggtg ctgcagctgt 1020
cccagcagct gtctggcatc aacgctgtct tctattactc cacgagcatc ttcgagaagg 1080
cgggggtgca gcagcctgtg tatgccacca ttggctccgg tatcgtcaac acggccttca 1140

ctgtcgtgtc gctgtttgtg gtggagcgag caggccggcg gaccctgcac ctcataggcc 1200
tcgctggcat ggcgggttgt gccatactca tgaccatcgc gctagcactg ctggagcagc 1260
taccctggat gtcctatctg agcatcgtgg ccatctttgg ctttgtggcc ttctttgaag 1320
tgggtcctgg ccccatccca tggttcatcg tggctgaact cttcagccag ggtccacgtc 1380
cagctgccat tgccgttgca ggcttctcca actggacctc aaatttcatt gtgggcatgt 1440
gcttccagta tgtggagcaa ctgtgtggtc cctacgtctt catcatcttc actgtgctcc 1500
tggttctgtt cttcatcttc acctacttca aagttcctga gactaaaggc cggaccttcg 1560
atgagatcgc ttccggcttc cggcaggggg gagccagcca aagtgataag acacccgagg 1620
agctgttcca tcccctgggg gctgattccc aagtgtgagt cgccccagat caccagcccg 1680
gcctgctccc agcagcccta aggatctctc aggagcacag gcagctggat gagacttcca 1740
aacctgacag atgtcagccg agccgggcct ggggctcctt tctccagcca gcaatgatgt 1800
ccagaagaat attcaggact taacggctcc aggattttaa caaaagcaag actgttgctc 1860
aaatctattc agacaagcaa caggttttat aattttttta ttactgattt tgttattttt 1920
atatcagcct gagtctcctg tgcccacatc ccaggcttca ccctgaatgg ttccatgcct 1980
gagggtggag actaagccct gtcgagacac ttgccttctt cacccagcta atctgtaggg 2040
ctggacctat gtcctaagga cacactaatc gaactatgaa ctacaaagct tctatcccag 2100
gaggtggcta tggccacccg ttctgctggc ctggatctcc ccactctagg ggtcaggctc 2160
cattaggatt tgccccttcc catctcttcc tacccaacca ctcaaattaa tctttcttta 2220
cctgagacca gttgggagca ctggagtgca gggaggagag gggaagggcc agtctgggct 2280
gccgggttct agtctccttt gcactgaggg ccacactatt accatgagaa gagggcctgt 2340
gggagcctgc aaactcactg ctcaagaaga catggagact cctgccctgt tgtgtataga 2400
tgcaagatat ttatatatat ttttggttgt caatattaaa tacagacact aagttatagt 2460
atatctggac aagccaactt gtaaatacac cacctcactc ctgttactta cctaaacaga 2520
tataaatggc tggtttttag aaacatggtt ttgaaatgct tgtggattga gggtaggagg 2580
tttggatggg agtgagacag aagtaagtgg ggttgcaacc actgcaacgg cttagacttc 2640
gactcaggat ccagtccctt acacgtacct ttcatcagtg tcctcttgct caaaaatctg 2700
tttgatccct gttacccaga gaatatatac attctttatc ttgacattca aggcatttct 2760
atcacatatt tgatagttgg tgttcaaaaa aacactagtt ttgtgccagc cgtgatgctc 2820
aggcttgaaa tcgcattatt ttgaatgtga agggaa 2856

<210> 136
<211> 356
<212> DNA
<213> Homo sapien

<400> 136
ggtggagcca aatgaagaaa atgaagatga aagagacaga cacctcagtt tttctggatc 60
aggcattgat gatgatgaag attttatctc cagcaccatt tcaaccacac cacgggcttt 120
tgaccacaca aaacagaacc aggactggac tcagtggaac ccaagccatt caaatccgga 180
agtgctactt cagacaacca caaggatgac tgatgtagac agaaatggca ccactgctta 240
tgaaggaaac tggaacccag aagcacaccc tcccctcatt caccatgagc atcatgagga 300
agaagagacc ccacattcta caagcacaat ccaggcaact cctagtagta caacgg 356

<210> 137
<211> 356
<212> DNA
<213> Homo sapien

<220>
<221> misc_feature
<222> (1)...(356)
<223> n = A,T,C or G

<400> 137
gcaggtggag aagacatttt attgttcctg gggtctctgg aggcccattg gtggggctgg 60

gtcactggct gcccccggaa cagggcgctg ctccatggct ctgcttgtgg tagtctgtgg 120
ctatgtctcc cagcaaggac agaaactcag aaaaatcaat cttcttatcc tcattcttgt 180
ccttttctc aaagacatcg gcgaggtaat ttgtgccctt tttacctcgg cccgcgacca 240
cgctaaggcc aaanttccag acanayggcc gggccggtnc nataggggan cccaacttgg 300
ggacccaaac tctggcgcgg aaacacangg gcataagctt gnttcctgtg gggaaa 356

<210> 138
<211> 353
<212> DNA
<213> Homo sapien

<400> 138
aggtccagtc ctccacttgg cctgatgaga gtggggagtg gcaagggacg tttctcctgc 60
aatagacact tagatttctc tcttgtggga agaaaccacc tgtccatcca ctgactcttc 120
tacattgatg tggaaattgc tgctgctacc accacctcct gaagaggctt ccctgatgcc 180
aatgccagcc atcttggcat cctggccctc gagcaggctg cggtaagtag cgatctcctg 240
ctccagccgt gtctttatgt caagcagcat cttgtactcc tggttctgag cctccatctc 300
gcatcggagc tcactcagac ctcgsccgsg mssmcgctam gccgaattcc agc 353

<210> 139
<211> 371
<212> DNA
<213> Homo sapien

<400> 139
agcgtggtcg cggccgaggt ccatccgaag caagattgca gatggcagtg tgaagagaga 60
agacatattc tacacttcaa agctttggtg caattcccat cgaccagagt tggtccgacc 120
agccttggaa aggtcactga aaaatcttca attggattat gttgacctct accttattca 180
ttttccagtg tctgtaaagc caggtgagga agtgatccca aaagatgaaa atggaaaaat 240
actatttgac acagtggatc tctgtgccac gtgggaggcc gtggagaagt gtaaagatgc 300
aggattggac ctgcccgggc ggccgctcga aagccgaatt ccagcacact ggcggccgtt 360
actagtggat c 371

<210> 140
<211> 370
<212> DNA
<213> Homo sapien

<400> 140
tagcgtggtc gcggccgagg tccatctccc tttgggaact agggggctgc tggtgggaaa 60
tgggagccag ggcagatgtt gcattccttt gtgtccctgt aaatgtggga ctacaagaag 120
aggagctgcc tgagtggtac tttctcttcc tggtaatcct ctggcccagc ctcatggcag 180
aatagaggta tttttaggct atttttgtaa tatggcttct ggtcaaaatc cctgtgtagc 240
tgaattccca agccctgcat tgtacagccc cccactcccc tcaccaccta ataaaggaat 300
agttaacact caaaaaaaaa aaaaaacctg cccgggcggc cgctcgaaag ccgaattcca 360
gcacactggc 370

<210> 141
<211> 371
<212> DNA
<213> Homo sapien

<400> 141
tagcgtggtc gcggccgagg tcctctgtgc tgcctgtcac agcccgatgg taccagcgca 60
gggtgtaggc agtgcaggag ccctcatcca gtggcaggga acaggggtca tcactatccc 120

aaggagcttc agggtcctgg tactcctcca cagaatactc ggagtattca gagtactcat 180
catcctcagg gggtacccgc tcttcctcct ctgcatgaga gacgcggagc acaggcacag 240
catggagctg ggagccggca gtgtctgcag cataactagg gaggggtcgt gatccagatg 300
cgatgaactg gccctggcag gcacagtgct gactcatctc ttggcgacct gcccgggcgg 360
ccgctcgaag c 371

<210> 142
<211> 343
<212> DNA
<213> Homo sapien

<400> 142
gcgttttgag gccaatggtg taaaaggaaa tatcttcaca taaaaactag atggaagcat 60
tgtcagaaac ctctttgtga tgtttgcttt caactcacag agttgaacat tccttttcat 120
agagcagttt tgaaacactc ttttgtagaa tttgcaagcg gatgattgga tcgctatgag 180
gtcttcattg gaaacgggat acctttacat aaaaactaga cagtagcatt ctcagaaatt 240
tctttgggat gtgggcattc aacccacaga ggagaacttc atttgataga gcagttttga 300
aacacccttt ttgtagaatc tacaggtgga catttagagt gct 343

<210> 143
<211> 354
<212> DNA
<213> Homo sapien

<400> 143
aggtctgatg gcagaaaaac tcagactgtc tgcaacttta cagatggtgc attggttcag 60
catcaggagt gggatgggaa ggaaagcaca ataacaagaa aattgaaaga tgggaaatta 120
gtggtggagt gtgtcatgaa caatgtcacc tgtactcgga tctatgaaaa agtagaataa 180
aaattccatc atcactttgg acaggagtta attaagagaa tgaccaagct cagttcaatg 240
agcaaatctc catactgttt ctttcttttt tttttcatta ctgtgttcaa ttatctttat 300
cataaacatt ttacatgcag ctatttcaaa gtgtgttgga ttaattagga tcat 354

<210> 144
<211> 353
<212> DNA
<213> Homo sapien

<400> 144
ggtcaaggac ctgggggacc cccaggtcca gcagccacat gattctgcag cagacaggga 60
cctagagcac atctggatct cagccccacc cctggcaacc tgcctgccta gagaactccc 120
aagatgacag actaagtagg attctgccat ttagaataat tctggtatcc tgggcgttgc 180
gttaagttgc ttaactttca ttctgtctta cgatagtctt cagaggtggg aacagatgaa 240
gaaaccatgc cccagagaag gttaagtgac ttcctcttta tggagccagt gttccaacct 300
aggtttgcct gataccagac ctgtggcccc acctcccatg caggtctctg tgg 353

<210> 145
<211> 371
<212> DNA
<213> Homo sapien

<400> 145
caggtctgtc ataaactggt ctggagtttc tgacgactcc ttgttcacca aatgcaccat 60
ttcctgagac ttgctggcct ctccgttgag tccacttggc tttctgtcct ccacagctcc 120
attgccactg ttgatcacta gctttttctt ctgcccacac ctttttcgac tgttgactgc 180
aatgcaaact gcaagaatca aagccaaggc caagagggat gccaagatga tcagccattc 240

tggaatttgg ggtgtcctta taggaccaga ggttgtgttt gctccacctt cttgactccc 300
atgtgagacc tcggccgcga ccacgctaag ccgaattcca gcacactggc ggccgttac 360
tagtggatcc g 371

<210> 146
<211> 355
<212> DNA
<213> Homo sapien

<400> 146
ggtcctccgt cctcttccca gaggtgtcgg ggcttggccc cagcctccat cttcgtctct 60
caggatggcg agtagcagcg gctccaaggc tgaattcatt gtcggaggga aatataaact 120
ggtacggaag atcgggtctg gctccttcgg ggacatctat ttggcgatca acatcaccaa 180
cggcgaggaa gtggcagtga agctagaatc tcagaaggcc aggcatcccc agttgctgta 240
cgagagcaag ctctataaga ttcttcaagg tggggttggc atcccccaca tacggtggta 300
tggtcaggaa aaagactaca atgtactagt catggatctt ctgggaccta gcctc 355

<210> 147
<211> 355
<212> DNA
<213> Homo sapien

<400> 147
ggtctgttac aaaatgaaga cagacaacac aacatttact ctgtggagat atcctactca 60
tactatgcac gtgctgtgat tttgaacata actcgtccca aaaacttgtc acgatcatcc 120
tgacttttta ggttggctga tccatcaatc ttgcactcaa ctgttacttc tttcccagtg 180
ttgttaggag caaagctgac ctgaacagca accaatggct gtagataccc aacatgcagt 240
tttttcccat aatatgggaa atattttaag tctatcattc cattatgagg ataaactgct 300
acatttggta tatcttcatt ctttgaaaca caatctatcc ttggcactcc ttcag 355

<210> 148
<211> 369
<212> DNA
<213> Homo sapien

<400> 148
aggtctctct ccccctctcc ctctcctgcc agccaagtga agacatgctt acttcccctt 60
caccttcctt catgatgtgg gaagagtgct gcaacccagc cctagccaac accgcatgag 120
agggagtgtg ccgagggctt ctgagaaggt ttctctcaca tctagaaaga agcgcttaag 180
atgtggcagc ccctcttctt caagtggctc ttgtcctgtt gccctgggag ttctcaaatt 240
gctgcagcag cctccatcca gcctgaggat gacatcaata cacagaggaa gaagagtcag 300
gaaaagatga gagaagttac agactctcct gggcgacccc gagagcttac cattcctcag 360
acttcttca 369

<210> 149
<211> 620
<212> DNA
<213> Homo sapien

<220>
<221> misc_feature
<222> (1)...(620)
<223> n = A,T,C or G

<400> 149

actagtcaaa aatgctaaaa taatttggga gaaaatattt tttaagtagt gttatagttt 60
catgtttatc ttttattatg ttttgtgaag ttgtgtcttt tcactaatta cctatactat 120
gccaatattt ccttatatct atccataaca tttatactac atttgtaana naatatgcac 180
gtgaaactta acactttata aggtaaaaat gaggtttcca anatttaata atctgatcaa 240
gttcttgtta tttccaaata gaatggactt ggtctgttaa gggctaagga gaagaggaag 300
ataaggttaa aagttgttaa tgaccaaaca ttctaaaaga aatgcaaaaa aaaagtttat 360
tttcaagcct tcgaactatt taaggaaagc aaaatcattt cctaaatgca tatcatttgt 420
gagaatttct cattaatatc ctgaatcatt catttcacta aggctcatgt tnactccgat 480
atgtctctaa gaaagtacta tttcatggtc caaacctggt tgccatantt gggtaaaggc 540
tttcccttaa gtgtgaaant atttaaaatg aaattttcct ctttttaaaa attctttana 600
agggttaagg gtgttgggga 620

<210> 150
<211> 371
<212> DNA
<213> Homo sapien

<400> 150
ggtccgatca aaacctgcta cctccccaag actttactag tgccgataaa ctttctcaaa 60
gagcaaccag tatcacttcc ctgtttataa aacctctaac catctctttg ttctttgaac 120
atgctgaaaa ccacctggtc tgcatgtatg cccgaatttg yaattctttt ctctcaaatg 180
aaaatttaat tttagggatt catttctata ttttcacata tgtagtatta ttatttcctt 240
atatgtgtaa ggtgaaattt atggtatttg agtgtgcaag aaaatatatt tttaaagctt 300
tcatttttcc cccagtgaat gatttagaat tttttatgta aatatacaga atgttttttc 360
ttacttttat a 371

<210> 151
<211> 4655
<212> DNA
<213> Homo sapien

<400> 151
gggacttgag ttctgttatc ttcttaagta gattcatatt gtaagggtct cggggtgggg 60
gggttggcaa aatcctggag ccagaagaaa ggacagcagc attgatcaat cttacagcta 120
acatgttgta cctggaaaac aatgcccaga ttcaatttag tgagccacag tacacgaacc 180
tggggctcct gaacagcatg gaccagcaga ttcagaacgg ctcctcgtcc accagtccct 240
ataacacaga ccacgcgcag aacagcgtca cggcgccctc gccctacgca cagcccagct 300
ccaccttcga tgctctctct ccatcacccg ccatcccctc caacaccgac tacccaggcc 360
cgcacagttt cgacgtgtcc ttccagcagt cgagcaccgc caagtcggcc acctggacgt 420
attccactga actgaagaaa ctctactgcc aaattgcaaa gacatgcccc atccagatca 480
aggtgatgac cccacctcct cagggagctg ttatccgcgc catgcctgtc tacaaaaaag 540
ctgagcacgt cacggaggtg gtgaagcggt gccccaacca tgagctgagc cgtgaattca 600
acgagggaca gattgcccct yctagtcatt tgattcgagt agaggggaac agccatgccc 660
agtatgtaga agatcccatc acaggaagac agagtgtgct ggtaccttat gagccacccc 720
aggttggcac tgaattcacg acagtcttgt acaatttcat gtgtaacagc agttgtgttg 780
gagggatgaa ccgccgtcca attttaatca ttgttactct ggaaaccaga gatgggcaag 840
tcctgggccg acgctgcttt gaggcccgga tctgtgcttg cccaggaaga gacaggaagg 900
cggatgaaga tagcatcaga aagcagcaag tttcggacag tacaaagaac ggtgatggta 960
cgaagcgccc gtttcgtcag aacacacatg gtatccagat gacatccatc aagaaacgaa 1020
gatccccaga tgatgaactg gtatacttac cagtgagggg ccgtgagact tatgaaatgc 1080
tggtgaagat caaagagtcc ctggaactca tgcagtacct tcttcagcac acaattgaaa 1140
cgtacaggca acagcaacag cagcagcacc agcacttact tcagaaacag acctcaatac 1200
agtctccatc ttcatatggt aacagctccc cacctctgaa caaaatgaac agcatgaaca 1260
agctgccttc tgtgagccag cttatcaacc ctcagcagcg caacgccctc actcctacaa 1320
ccattcctga tggcatggga gccaacattc ccatgatggg cacccacatg ccaatggctg 1380

gagacatgaa tggactcagc cccacccagg cactccctcc cccactctcc atgccatcca 1440
cctcccactg cacaccccca cctccgtatc ccacagattg cagcattgtc agtttcttag 1500
cgaggttggg ctgttcatca tgtctggact atttcacgac ccaggggctg accaccatct 1560
atcagattga gcattactcc atggatgatc tggcaagtct gaaaatccct gagcaatttc 1620
gacatgcgat ctggaagggc atcctggacc accggcagct ccacgaattc tcctcccctt 1680
ctcatctcct gcggacccca agcagtgcct ctacagtcag tgtgggctcc agtgagaccc 1740
ggggtgagcg tgttattgat gctgtgcgat tcaccctccg ccagaccatc tctttcccac 1800
cccgagatga gtggaatgac ttcaactttg acatggatgc tcgccgcaat aagcaacagc 1860
gcatcaaaga ggagggggag tgagcctcac catgtgagct cttcctatcc ctctcctaac 1920
tgccagcccc ctaaaagcac tcctgcttaa tcttcaaagc cttctcccta gctcctcccc 1980
ttcctcttgt ctgatttctt aggggaagga gaagtaagag gcttacttct taccctaacc 2040
atctgacctg gcatctaatt ctgattctgg ctttaagcct tcaaaactat agcttgcaga 2100
actgtagctt gccatggcta ggtagaagtg agcaaaaaag agttgggtgt ctccttaagc 2160
tgcagagatt tctcattgac ttttataaag catgttcacc cttatagtct aagactatat 2220
atataaatgt ataaatatac agtatagatt tttgggtggg gggcattgag tattgtttaa 2280
aatgtaattt aaatgaaaga aaattgagtt gcacttattg accatttttt aatttacttg 2340
ttttggatgg cttgtctata ctccttccct taaggggtat catgtatggt gataggtatc 2400
tagagcttaa tgctacatgt gagtgacgat gatgtacaga ttctttcagt tctttggatt 2460
ctaaatacat gccacatcaa acctttgagt agatccattt ccattgctta ttatgtaggt 2520
aagactgtag atatgtattc ttttctcagt gttggtatat tttatattac tgacatttct 2580
tctagtgatg atggttcacg ttggggtgat ttaatccagt tataagaaga agttcatgtc 2640
caaacgtcct ctttagtttt tggttgggaa tgaggaaaat tcttaaaagg cccatagcag 2700
ccagttcaaa aacacccgac gtcatgtatt tgagcatatc agtaaccccc ttaaatttaa 2760
taccagatac cttatcttac aatattgatt gggaaaacat ttgctgccat tacagaggta 2820
ttaaaactaa atttcactac tagattgact aactcaaata cacatttgct actgttgtaa 2880
gaattctgat tgatttgatt gggatgaatg ccatctatct agttctaaca gtgaagtttt 2940
actgtctatt aatattcagg gtaaatagga atcattcaga aatgttgagt ctgtactaaa 3000
cagtaagata tctcaatgaa ccataaattc aactttgtaa aaatcttttg aagcatagat 3060
aatattgttt ggtaaatgtt tcttttgttt ggtaaatgtt tcytttaaag accctcctat 3120
tctataaaac tctgcatgta gaggcttgtt tacctttctc tctctaaggt ttacaatagg 3180
agtggtgatt tgaaaaatat aaaattatga gattggtttt cctgtggcat aaattgcatc 3240
actgtatcat tttctttttt aaccggtaag agtttcagtt tgttggaaag taactgtgag 3300
aacccagttt cccgtccatc tcccttaggg actacccata gacatgaaag gtccccacag 3360
agcaagagat aagtctttca tggctgctgt tgcttaaacc acttaaacga agagttccct 3420
tgaaactttg ggaaaacatg ttaatgacaa tattccagat ctttcagaaa tataacacat 3480
ttttttgcat gcatgcaaat gagctctgaa atcttcccat gcattctggt caagggctgt 3540
cattgcacat aagcttccat tttaatttta aagtgcaaaa gggccagcgt ggctctaaaa 3600
ggtaatgtgt ggattgcctc tgaaaagtgt gtatatattt tgtgtgaaat tgcatacttt 3660
gtattttgat tatttttttt ttcttcttgg gatagtggga tttccagaac cacacttgaa 3720
accttttttt atcgtttttg tattttcatg aaaataccat ttagtaagaa taccacatca 3780
aataagaaat aatgctacaa ttttaagagg ggagggaagg gaaagttttt ttttttatta 3840
tttttttaaa attttgtatg ttaaagagaa tgagtccttg atttcaaagt tttgttgtac 3900
ttaaatggta ataagcactg taaacttctg caacaagcat gcagctttgc aaacccatta 3960
aggggaagaa tgaaagctgt tccttggtcc tagtaagaag acaaactgct tcccttactt 4020
tgctgagggt ttgaataaac ctaggacttc cgagctatgt cagtactatt caggtaacac 4080
tagggccttg gaaatccctg tactgtgtct catggatttg gcactagcca aagcgaggca 4140
ccccttactg gcttacctcc tcatggcagc ctactctcct tgagtgtatg agtagccagg 4200
gtaaggggta aaaggatagt aagcatagaa accactagaa agtgggctta atggagttct 4260
tgtggcctca gctcaatgca gttagctgaa gaattgaaaa gtttttgttt ggagacgttt 4320
ataaacagaa atggaaagca gagttttcat taaatccttt tacctttttt ttttcttggt 4380
aatcccctaa aataacagta tgtgggatat tgaatgttaa agggatattt ttttctatta 4440
tttttataat tgtacaaaat taagcaaatg ttaaaagttt tatatgcttt attaatgttt 4500
tcaaaaggta ttatacatgt gatacatttt ttaagcttca gttgcttgtc ttctggtact 4560
ttctgttatg ggcttttggg gagccagaag ccaatctaca atctcttttt gtttgccagg 4620
acatgcaata aaatttaaaa aataaataaa aacta 4655

<210> 152
<211> 586
<212> PRT
<213> Homo sapien

<400> 152
Met Leu Tyr Leu Glu Asn Asn Ala Gln Thr Gln Phe Ser Glu Pro Gln
1               5                   10                  15
Tyr Thr Asn Leu Gly Leu Leu Asn Ser Met Asp Gln Gln Ile Gln Asn
            20                  25                  30
Gly Ser Ser Ser Thr Ser Pro Tyr Asn Thr Asp His Ala Gln Asn Ser
        35                  40                  45
Val Thr Ala Pro Ser Pro Tyr Ala Gln Pro Ser Ser Thr Phe Asp Ala
    50                  55                  60
Leu Ser Pro Ser Pro Ala Ile Pro Ser Asn Thr Asp Tyr Pro Gly Pro
65                  70                  75                  80
His Ser Phe Asp Val Ser Phe Gln Gln Ser Ser Thr Ala Lys Ser Ala
                85                  90                  95
Thr Trp Thr Tyr Ser Thr Glu Leu Lys Lys Leu Tyr Cys Gln Ile Ala
            100                 105                 110
Lys Thr Cys Pro Ile Gln Ile Lys Val Met Thr Pro Pro Pro Gln Gly
        115                 120                 125
Ala Val Ile Arg Ala Met Pro Val Tyr Lys Lys Ala Glu His Val Thr
    130                 135                 140
Glu Val Val Lys Arg Cys Pro Asn His Glu Leu Ser Arg Glu Phe Asn
145                 150                 155                 160
Glu Gly Gln Ile Ala Pro Ser Ser His Leu Ile Arg Val Glu Gly Asn
                165                 170                 175
Ser His Ala Gln Tyr Val Glu Asp Pro Ile Thr Gly Arg Gln Ser Val
            180                 185                 190
Leu Val Pro Tyr Glu Pro Pro Gln Val Gly Thr Glu Phe Thr Thr Val
        195                 200                 205
Leu Tyr Asn Phe Met Cys Asn Ser Ser Cys Val Gly Gly Met Asn Arg
    210                 215                 220
Arg Pro Ile Leu Ile Ile Val Thr Leu Glu Thr Arg Asp Gly Gln Val
225                 230                 235                 240
Leu Gly Arg Arg Cys Phe Glu Ala Arg Ile Cys Ala Cys Pro Gly Arg
                245                 250                 255
Asp Arg Lys Ala Asp Glu Asp Ser Ile Arg Lys Gln Gln Val Ser Asp
            260                 265                 270
Ser Thr Lys Asn Gly Asp Gly Thr Lys Arg Pro Phe Arg Gln Asn Thr
        275                 280                 285
His Gly Ile Gln Met Thr Ser Ile Lys Lys Arg Arg Ser Pro Asp Asp
    290                 295                 300
Glu Leu Val Tyr Leu Pro Val Arg Gly Arg Glu Thr Tyr Glu Met Leu
305                 310                 315                 320
Val Lys Ile Lys Glu Ser Leu Glu Leu Met Gln Tyr Leu Leu Gln His
                325                 330                 335
Thr Ile Glu Thr Tyr Arg Gln Gln Gln Gln Gln His Gln His Leu
            340                 345                 350
Leu Gln Lys Gln Thr Ser Ile Gln Ser Pro Ser Ser Tyr Gly Asn Ser
        355                 360                 365
Ser Pro Pro Leu Asn Lys Met Asn Ser Met Asn Lys Leu Pro Ser Val
    370                 375                 380

Ser Gln Leu Ile Asn Pro Gln Gln Arg Asn Ala Leu Thr Pro Thr Thr
385                 390                 395                 400
Ile Pro Asp Gly Met Gly Ala Asn Ile Pro Met Met Gly Thr His Met
                405                 410                 415
Pro Met Ala Gly Asp Met Asn Gly Leu Ser Pro Thr Gln Ala Leu Pro
            420                 425                 430
Pro Pro Leu Ser Met Pro Ser Thr Ser His Cys Thr Pro Pro Pro Pro
        435                 440                 445
Tyr Pro Thr Asp Cys Ser Ile Val Ser Phe Leu Ala Arg Leu Gly Cys
    450                 455                 460
Ser Ser Cys Leu Asp Tyr Phe Thr Thr Gln Gly Leu Thr Thr Ile Tyr
465                 470                 475                 480
Gln Ile Glu His Tyr Ser Met Asp Asp Leu Ala Ser Leu Lys Ile Pro
                485                 490                 495
Glu Gln Phe Arg His Ala Ile Trp Lys Gly Ile Leu Asp His Arg Gln
            500                 505                 510
Leu His Glu Phe Ser Ser Pro Ser His Leu Leu Arg Thr Pro Ser Ser
        515                 520                 525
Ala Ser Thr Val Ser Val Gly Ser Ser Glu Thr Arg Gly Glu Arg Val
    530                 535                 540
Ile Asp Ala Val Arg Phe Thr Leu Arg Gln Thr Ile Ser Phe Pro Pro
545                 550                 555                 560
Arg Asp Glu Trp Asn Asp Phe Asn Phe Asp Met Asp Ala Arg Arg Asn
                565                 570                 575
Lys Gln Gln Arg Ile Lys Glu Glu Gly Glu
            580                 585

<210> 153
<211> 2007
<212> DNA
<213> Homo sapien

<400> 153
gaattcgtcg ctgctccagg gaaagttctg ttactccact gactctctct tttcctgata      60
acatggccag caagaaagta attacagtgt ttggagcaac aggagctcaa ggtggctctg     120
tggccagggc aattttggag agcaaaaaat ttgcagtgag agcagtgacc agggatgtga     180
cttgaccaaa tgccctggag ctccagcgcc ttggagctga ggtggtcaaa ggtgacctga     240
atgataaagc atcggtggac agtgccttaa aaggtgtcta tggggccttc ttggtgacca     300
acttctggga ccctctcaac caagataagg aagtgtgtcg ggggaagctg gtggcagact     360
ccgccaagca cctgggtctg aagcacgtgg tgtacagcgg cctggagaac gtcaagcgac     420
tgacggatgg caagctggag gtgccgcact ttgacagcaa gggcgaggtg gaggagtact     480
tctggtccat tggcatcccc atgaccagtg tccgcgtggc ggcctacttt gaaaactttc     540
tcgcggcgtg gcggcccgtg aaagcctctg atggagatta ctacaccttg gctgtaccga     600
tgggagatgt accaatggat ggtatctctg ttgctgatat tggagcagcc gtctctagca     660
tttttaattc tccagaggaa tttttaggca aggccgtggg gctcagtgca gaagcactaa     720
caatacagca atatgctgat gttttgtcca aggctttggg gaaagaagtc cgagatgcaa     780
agattacccc ggaagctttc gagaagctgg gattccctgc agcaaaggaa atagccaata     840
tgtgtcgttt ctatgaaatg aagccagacc gagatgtcaa tctcacccac caactaaatc     900
ccaaagtcaa aagcttcagc cagtttatct cagagaacca gggagccttc aagggcatgt     960
agaaaatcag ctgttcagat aggcctctgc accacacagc ctctttcctc tctgatcctt    1020
ttcctcttta cggcacaaca ttcatgttga cagaacatgc tggaatgcaa ttgtttgcaa    1080
caccgaagga tttcctgcgg tcgcctcttc agtaggaagc actgcattgg tgataggaca    1140
cggtaatttg attcacattt aacttgctag ttagtgataa gggtggtaca actgtttggt    1200
aaaatgagaa gcctcggaac ttggagcttc tctcctacca ctaatgggag ggcagattat    1260
actgggattt ctcctgggtg agtaatttca agccctaatg ctgaaattcc cctaggcagc    1320

tccagttttc tcaactgcat tgcaaaattc ccagtgaact tttaagtact tttaacttaa 1380
aaaaatgaac atctttgtag agaattttct ggggaacatg gtgttcaatg aacaagcaca 1440
agcattggaa atgctaaaat tcagttttgc ctcaagattg gaagtttatt ttctgactca 1500
ttcatgaagt catctattga gccaccattc aattattcat ctattaattc cttgatcctt 1560
catttatcca ttctgcaaac ttttcttgag caccagcacg ggtggccatt tgtggacttc 1620
tcttcattcc tatgtgtttt cttatcaaag tgatccactc tcgaaaggct cctttccagt 1680
ctgtggttgg gttcaagtca tgccagggcc agggggccca tctcctcgtt tagctctagg 1740
caaaatccag gggatctgca gtggggagcg ggggcaggaa gctggaggga aggcctgtga 1800
agggtaggga tgtggaaaga caaggtgaca gaaggaccca ataggacctt tctatatctc 1860
tggcttagca ttttctacat catattgtaa tcgtcttatt tgctagtttt cttccttact 1920
gtgagtgact aacagtcatc tttatcccag tgcctggtac ataataagtg atcaataaat 1980
gttgattgac taaaaaaaaa aaaaaaa 2007

<210> 154
<211> 2148
<212> DNA
<213> Homo sapien

<400> 154
gaattcgtcg ctgctccagg gaaagttctg ttactccact gactctctct tttcctgata 60
acatggccag caagaaagta attacagtgt ttggagcaac aggagctcaa ggtggctctg 120
tggccagggc aattttggag agcaaaaaat ttgcagtgag agcagtgacc agggatgtga 180
cttgaccaaa tgccctggag ctccagcgcc ttggagctga ggtggtcaaa ggtgacctga 240
atgataaagc atcggtggac agtgccttaa aaggggaagc tggtggcaga ctccgccaag 300
cacctgggtc tgaagcacgt ggtgtacagc ggcctggaga acgtcaagcg actgacggat 360
ggcaagctgg aggtgccgca ctttgacagc aagggcgagg tggaggagta cttctggtcc 420
attggcatcc ccatgaccag tgtccgcgtg gcggcctact ttgaaaactt tctcgcggcg 480
tggcggcccg tgaaagcctc tgatggagat tactacacct tggctgtacc gatgggagat 540
gtaccaatgg atggtatctc tgttgctgat attggagcag ccgtctctag catttttaat 600
tctccagagg aatttttagg caaggccgtg gggctcagtg cagaagcact aacaatacag 660
caatatgctg atgttttgtc caaggctttg gggaaagaag tccgagatgc aaagactatc 720
tgtgctatag atgaccagaa aacagtggaa gaaggtttca tggaagacgt gggcttgagt 780
tggtccttga gggaacatga ccatgtatag acagaggagg catcaagaag gctggcctgg 840
ctaattctgg aataaacacg acaaaccaga ggcagtacgg gaaggaggca aattctggct 900
ctgcctctat ccttgattac cccggaagct ttcgagaagc tgggattccc tgcagcaaag 960
gaaatagcca atatgtgtcg tttctatgaa atgaagccag accgagatgt caatctcacc 1020
caccaactaa atcccaaagt caaaagcttc agccatttta tctcagagaa ccagggagcc 1080
ttcaagggca tgtagaaaat cagctgttca gataggcctc tgcaccacac agcctctttc 1140
ctctctgatc cttttcctct ttacggcaca acattcatgt tgacagaaca tgctggaatg 1200
caattgtttg caacaccgaa ggatttcctg cggtcgcctc ttcagtagga agcactgcat 1260
tggtgatagg acacggtaat ttgattcaca tttaacttgc tagttagtga taagggtggt 1320
acaactgttt ggtaaaatga gaagcctcgg aacttggagc ttctctccta ccactaatgg 1380
gagggcagat tatactggga tttctcctgg gtgagtaatt tcaagcccta atgctgaaat 1440
tcccctaggc agctccagtt ttctcaactg cattgcaaaa ttcccagtga acttttaagt 1500
acttttaact taaaaaaatg aacatctttg tagagaattt tctggggaac atggtgttca 1560
atgaacaagc acaagcattg gaaatgctaa aattcagttt tgcctcaaga ttggaagttt 1620
attttctgac tcattcatga agtcatctat tgagccacca ttcaattatt catctattaa 1680
ttccttgatc cttcatttat ccattctgca aacttttctt gagcaccagc acgggtggcc 1740
atttgtggac ttctcttcat tcctatgtgt tttcttatca aagtgatcca ctctcgaaag 1800
gctcctttcc agtctgtggt tgggttcaag tcatgccagg gccagggggc ccatctcctc 1860
gtttagctct aggcaaaatc caggggatct gcagtgggga gcgggggcag gaagctggag 1920
ggaaggcctg tgaagggtag ggatgtggaa agacaaggtg acagaaggac ccaataggac 1980
ctttctatat ctctggctta gcattttcta catcatattg taatcgtctt atttgctagt 2040
tttcttcctt actgtgagtg actaacagtc atctttatcc cagtgcctgg tacataataa 2100
gtgatcaata aatgttgatt gactaaatga aaaaaaaaaa aaaaaaa 2148

<210> 155
<211> 153
<212> PRT
<213> Homo sapien

<400> 155
Met Thr Ser Val Arg Val Ala Ala Tyr Phe Glu Asn Phe Leu Ala Ala
1               5                   10                  15
Trp Arg Pro Val Lys Ala Ser Asp Gly Asp Tyr Tyr Thr Leu Ala Val
            20                  25                  30
Pro Met Gly Asp Val Pro Met Asp Gly Ile Ser Val Ala Asp Ile Gly
        35                  40                  45
Ala Ala Val Ser Ser Ile Phe Asn Ser Pro Glu Glu Phe Leu Gly Lys
    50                  55                  60
Ala Val Gly Leu Ser Ala Glu Ala Leu Thr Ile Gln Gln Tyr Ala Asp
65                  70                  75                  80
Val Leu Ser Lys Ala Leu Gly Lys Glu Val Arg Asp Ala Lys Ile Thr
                85                  90                  95
Pro Glu Ala Phe Glu Lys Leu Gly Phe Pro Ala Ala Lys Glu Ile Ala
            100                 105                 110
Asn Met Cys Arg Phe Tyr Glu Met Lys Pro Asp Arg Asp Val Asn Leu
        115                 120                 125
Thr His Gln Leu Asn Pro Lys Val Lys Ser Phe Ser Gln Phe Ile Ser
    130                 135                 140
Glu Asn Gln Gly Ala Phe Lys Gly Met
145                 150

<210> 156
<211> 128
<212> PRT
<213> Homo sapien

<400> 156
Met Thr Ser Val Arg Val Ala Ala Tyr Phe Glu Asn Phe Leu Ala Ala
1               5                   10                  15
Trp Arg Pro Val Lys Ala Ser Asp Gly Asp Tyr Tyr Thr Leu Ala Val
            20                  25                  30
Pro Met Gly Asp Val Pro Met Asp Gly Ile Ser Val Ala Asp Ile Gly
        35                  40                  45
Ala Ala Val Ser Ser Ile Phe Asn Ser Pro Glu Glu Phe Leu Gly Lys
    50                  55                  60
Ala Val Gly Leu Ser Ala Glu Ala Leu Thr Ile Gln Gln Tyr Ala Asp
65                  70                  75                  80
Val Leu Ser Lys Ala Leu Gly Lys Glu Val Arg Asp Ala Lys Thr Ile
                85                  90                  95
Cys Ala Ile Asp Asp Gln Lys Thr Val Glu Glu Gly Phe Met Glu Asp
            100                 105                 110
Val Gly Leu Ser Trp Ser Leu Arg Glu His Asp His Val Ala Gly Ala
        115                 120                 125

<210> 157
<211> 424
<212> DNA
<213> Homo sapien

<220>
<221> misc_feature
<222> (1)...(424)
<223> n = A,T,C or G

<400> 157
ctgcagcccg ggggatccac tagtccagtg tggtggaatt cattggtctt tacaagactt 60
ggatacatta cagcagacat ggaaatataa ttttaaaaaa tttctctcca acctccttca 120
aattcagtca ccactgttat attaccttct ccaggaaccc tccagtgggg aaggctgcga 180
tattagattt ccttgtatgc aaagtttttg ttgaaagctg tgctcagagg aggtgagagg 240
agaggaagga gaaaactgca tcataacttt acagaattga atctagagtc ttccccgaaa 300
agcccagaaa cttctctgcn gnatctggct tgtccatctg gtctaaggtg gctgcttctt 360
ccccagccat cgagtcagtt tgtgcccatg aataatacac gacctgctat ttcccatgat 420
tgct 424

<210> 158
<211> 2099
<212> DNA
<213> Homo sapien

<400> 158
ccgcggttaa aaggcgcagc aggtgggagc cggggccttc acccgaaacc cgacgagagc 60
ccgacagccg gcggcgcccg agcccgacct gcctgcccag ccggagcgaa gggcgccgcc 120
ccgcgcagag cccgcgccag ggccgccggc cgcagagcag ttaaaacgtg caggcaccag 180
aaggcacttc ctgtcggtga agaagacctg tctccggtgt cacgggcatc ctgtgttttg 240
caaacggggc tgacctccct tcctggggag caggaagggt cagggaagga aaagaagtac 300
agaagatctg gctaaacaat ttctgtatgg cgaaagaaaa atttctaact gtacgccctc 360
ttcatgcatc tttaattcaa tttgaatatt ccaggcgaca tcctcactga ccgagcaaag 420
attgacattc gtatcatcac tgtgcaccat tggcttctag gcactccagt ggggtaggag 480
aaggaggtct gaaaccctcg cagagggatc ttgccctcat tctttgggtc tgaaacactg 540
gcagtcgttg gaaacaggac tcagggataa actagcgcaa tggattgggg gacgctgcac 600
actttcatcg ggggtgtcaa caaacactcc accagcatcg ggaaggtgtg gatcacagtc 660
atctttattt tccgagtcat gatcctcgtg gtggctgccc aggaagtgtg gggtgacgag 720
caagaggact tcgtctgcaa cacactgcaa ccgggatgca aaaatgtgtg ctatgaccac 780
tttttcccgg tgtcccacat ccggctgtgg gccctccagc tgatcttcgt ctccacccca 840
gcgctgctgg tggccatgca tgtggcctac tacaggcacg aaaccactcg caagttcagg 900
cgaggagaga agaggaatga tttcaaagac atagaggaca ttaaaaagca gaaggttcgg 960
atagaggggt cgctgtggtg gacgtacacc agcagcatct ttttccgaat catctttgaa 1020
gcagccttta tgtatgtgtt ttacttcctt tacaatgggt accacctgcc ctgggtgttg 1080
aaatgtggga ttgacccctg ccccaacctt gttgactgct ttatttctag gccaacagag 1140
aagaccgtgt ttaccatttt tatgatttct gcgtctgtga tttgcatgct gcttaacgtg 1200
gcagagttgt gctacctgct gctgaaagtg tgttttagga gatcaaagag agcacagacg 1260
caaaaaaatc accccaatca tgccctaaag gagagtaagc agaatgaaat gaatgagctg 1320
atttcagata gtggtcaaaa tgcaatcaca ggttcccaag ctaaacattt caaggtaaaa 1380
tgtagctgcg tcataaggag acttctgtct tctccagaag gcaataccaa cctgaaagtt 1440
ccttctgtag cctgaagagt ttgtaaatga ctttcataat aaatagacac ttgagttaac 1500
tttttgtagg atacttgctc cattcataca caacgtaatc aaatatgtgg tccatctctg 1560
aaaacaagag actgcttgac aaaggagcat tgcagtcact ttgacaggtt ccttttaagt 1620
ggactctctg acaaagtggg tactttctga aaatttatat aactgttgtt gataaggaac 1680
atttatccag gaattgatac gtttattagg aaaagatatt tttataggct tggatgtttt 1740
tagttctgac tttgaattta tataaagtat ttttataatg actggtcttc cttacctgga 1800
aaaacatgcg atgttagttt tagaattaca ccacaagtat ctaaatttgg aacttacaaa 1860
gggtctatct tgtaaatatt gttttgcatt gtctgttggc aaatttgtga actgtcatga 1920
tacgcttaag gtggaaagtg ttcattgcac aatatatttt tactgctttc tgaatgtaga 1980

cggaacagtg tggaagcaga aggctttttt aactcatccg tttgccaatc attgcaaaca 2040
actgaaatgt ggatgtgatt gcctcaataa agctcgtccc cattgcttaa aaaaaaaaa 2099

<210> 159
<211> 291
<212> PRT
<213> Homo sapien

<400> 159
Met Asp Trp Gly Thr Leu His Thr Phe Ile Gly Gly Val Asn Lys His
1               5                   10                  15
Ser Thr Ser Ile Gly Lys Val Trp Ile Thr Val Ile Phe Ile Phe Arg
            20                  25                  30
Val Met Ile Leu Val Val Ala Ala Gln Glu Val Trp Gly Asp Glu Gln
        35                  40                  45
Glu Asp Phe Val Cys Asn Thr Leu Gln Pro Gly Cys Lys Asn Val Cys
    50                  55                  60
Tyr Asp His Phe Phe Pro Val Ser His Ile Arg Leu Trp Ala Leu Gln
65                  70                  75                  80
Leu Ile Phe Val Ser Thr Pro Ala Leu Leu Val Ala Met His Val Ala
                85                  90                  95
Tyr Tyr Arg His Glu Thr Thr Arg Lys Phe Arg Arg Gly Glu Lys Arg
            100                 105                 110
Asn Asp Phe Lys Asp Ile Glu Asp Ile Lys Lys Gln Lys Val Arg Ile
        115                 120                 125
Glu Gly Ser Leu Trp Trp Thr Tyr Thr Ser Ser Ile Phe Phe Arg Ile
    130                 135                 140
Ile Phe Glu Ala Ala Phe Met Tyr Val Phe Tyr Phe Leu Tyr Asn Gly
145                 150                 155                 160
Tyr His Leu Pro Trp Val Leu Lys Cys Gly Ile Asp Pro Cys Pro Asn
                165                 170                 175
Leu Val Asp Cys Phe Ile Ser Arg Pro Thr Glu Lys Thr Val Phe Thr
            180                 185                 190
Ile Phe Met Ile Ser Ala Ser Val Ile Cys Met Leu Leu Asn Val Ala
        195                 200                 205
Glu Leu Cys Tyr Leu Leu Leu Lys Val Cys Phe Arg Arg Ser Lys Arg
    210                 215                 220
Ala Gln Thr Gln Lys Asn His Pro Asn His Ala Leu Lys Glu Ser Lys
225                 230                 235                 240
Gln Asn Glu Met Asn Glu Leu Ile Ser Asp Ser Gly Gln Asn Ala Ile
                245                 250                 255
Thr Gly Ser Gln Ala Lys His Phe Lys Val Lys Cys Ser Cys Val Ile
            260                 265                 270
Arg Arg Leu Leu Ser Ser Pro Glu Gly Asn Thr Asn Leu Lys Val Pro
        275                 280                 285
Ser Val Ala
    290

<210> 160
<211> 3951
<212> DNA
<213> Homo sapien

<400> 160
tctgcatcca tattgaaaac ctgacacaat gtatgcagca ggctcagtgt gagtgaactg 60

gaggcttctc tacaacatga cccaaaggag cattgcaggt cctatttgca acctgaagtt 120
tgtgactctc ctggttgcct taagttcaga actcccattc ctgggagctg gagtacagct 180
tcaagacaat gggtataatg gattgctcat tgcaattaat cctcaggtac ctgagaatca 240
gaacctcatc tcaaacatta aggaaatgat aactgaagct tcattttacc tatttaatgc 300
taccaagaga agagtatttt tcagaaatat aaagatttta atacctgcca catggaaagc 360
taataataac agcaaaataa aacaagaatc atatgaaaag gcaaatgtca tagtgactga 420
ctggtatggg gcacatggag atgatccata caccctacaa tacagagggt gtggaaaaga 480
gggaaaatac attcatttca cacctaattt cctactgaat gataacttaa cagctggcta 540
cggatcacga ggccgagtgt ttgtccatga atgggcccac ctccgttggg gtgtgttcga 600
tgagtataac aatgacaaac ctttctacat aaatgggcaa aatcaaatta aagtgacaag 660
gtgttcatct gacatcacag gcatttttgt gtgtgaaaaa ggtccttgcc cccaagaaaa 720
ctgtattatt agtaagcttt ttaaagaagg atgcaccttt atctacaata gcacccaaaa 780
tgcaactgca tcaataatgt tcatgcaaag tttatcttct gtggttgaat tttgtaatgc 840
aagtacccac aaccaagaag caccaaacct acagaaccag atgtgcagcc tcagaagtgc 900
atgggatgta atcacagact ctgctgactt tcaccacagc tttcccatga acgggactga 960
gcttccacct cctcccacat tctcgcttgt agaggctggt gacaaagtgg tctgtttagt 1020
gctggatgtg tccagcaaga tggcagaggc tgacagactc cttcaactac aacaagccgc 1080
agaattttat ttgatgcaga ttgttgaaat tcataccttc gtgggcattg ccagtttcga 1140
cagcaaagga gagatcagag cccagctaca ccaaattaac agcaatgatg atcgaaagtt 1200
gctggtttca tatctgccca ccactgtatc agctaaaaca gacatcagca tttgttcagg 1260
gcttaagaaa ggatttgagg tggttgaaaa actgaatgga aaagcttatg gctctgtgat 1320
gatattagtg accagcggag atgataagct tcttggcaat tgcttaccca ctgtgctcag 1380
cagtggttca acaattcact ccattgccct gggttcatct gcagccccaa atctggagga 1440
attatcacgt cttacaggag gtttaaagtt ctttgttcca gatatatcaa actccaatag 1500
catgattgat gctttcagta gaatttcctc tggaactgga gacatttttc agcaacatat 1560
tcagcttgaa agtacaggty aaaatgtcaa acctcaccat caattgaaaa acacagtgac 1620
tgtggataat actgtgggca acgacactat gtttctagtt acgtggcagg ccagtggtcc 1680
tcctgagatt atattatttg atcctgatgg acgaaaatac tacacaaata attttatcac 1740
caatctaact tttcggacag ctagtctttg gattccagga acagctaagc ctgggcactg 1800
gacttacacc ctgaacaata cccatcattc tctgcaagcc ctgaaagtga cagtgacctc 1860
tcgcgcctcc aactcagctg tgcccccagc cactgtggaa gcctttgtgg aaagagacag 1920
cctccatttt cctcatcctg tgatgattta tgccaatgtg aaacagggat tttatcccat 1980
tcttaatgcc actgtcactg ccacagttga gccagagact ggagatcctg ttacgctgag 2040
actccttgat gatggagcag gtgctgatgt tataaaaaat gatggaattt actcgaggta 2100
ttttttctcc tttgctgcaa atggtagata tagcttgaaa gtgcatgtca atcactctcc 2160
cagcataagc accccagccc actctattcc agggagtcat gctatgtatg taccaggtta 2220
cacagcaaac ggtaatattc agatgaatgc tccaaggaaa tcagtaggca gaaatgagga 2280
ggagcgaaag tggggcttta gccgagtcag ctcaggaggc tccttttcag tgctgggagt 2340
tccagctggc ccccaccctg atgtgtttcc accatgcaaa attattgacc tggaagctgt 2400
aaaagtagaa gaggaattga ccctatcttg gacagcacct ggagaagact ttgatcaggg 2460
ccaggctaca agctatgaaa taagaatgag taaaagtcta cagaatatcc aagatgactt 2520
taacaatgct attttagtaa atacatcaaa gcgaaatcct cagcaagctg gcatcaggga 2580
gatatttacg ttctcacccc aaatttccac gaatggacct gaacatcagc caaatggaga 2640
aacacatgaa agccacagaa tttatgttgc aatacgagca atggatagga actccttaca 2700
gtctgctgta tctaacattg cccaggcgcc tctgtttatt ccccccaatt ctgatcctgt 2760
acctgccaga gattatctta tattgaaagg agttttaaca gcaatgggtt tgataggaat 2820
catttgcctt attatagttg tgacacatca tactttaagc aggaaaaaga gagcagacaa 2880
gaaagagaat ggaacaaaat tattataaat aaatatccaa agtgtcttcc ttcttagata 2940
taagacccat ggccttcgac tacaaaaaca tactaacaaa gtcaaattaa catcaaaact 3000
gtattaaaat gcattgagtt tttgtacaat acagataaga tttttacatg gtagatcaac 3060
aaattctttt tgggggtaga ttagaaaacc cttacacttt ggctatgaac aaataataaa 3120
aattattctt taaagtaatg tctttaaagg caaagggaag ggtaaagtcg gaccagtgtc 3180
aaggaaagtt tgttttattg aggtggaaaa atagccccaa gcagagaaaa ggagggtagg 3240
tctgcattat aactgtctgt gtgaagcaat catttagtta ctttgattaa tttttctttt 3300
ctccttatct gtgcagaaca ggttgcttgt ttacaactga agatcatgct atatttcata 3360

tatgaagccc ctaatgcaaa gctctttacc tcttgctatt ttgttatata tattacagat 3420
gaaatctcac tgctaatgct cagagatctt ttttcactgt aagaggtaac ctttaacaat 3480
atgggtatta cctttgtctc ttcataccgg ttttatgaca aaggtctatt gaatttattt 3540
gtttgtaagt ttctactccc atcaaagcag ctttttaagt tattgccttg gttattatgg 3600
atgatagtta tagcccttat aatgccttaa ctaaggaaga aaagatgtta ttctgagttt 3660
gttttaatac atatatgaac atatagtttt attcaattaa accaaagaag aggtcagcag 3720
ggagatacta acctttggaa atgattagct ggctctgttt tttggttaaa taagagtctt 3780
taatcctttc tccatcaaga gttacttacc aagggcaggg gaagggggat atagaggtcc 3840
caaggaaata aaaatcatct ttcatcttta attttactcc ttcctcttat ttttttaaaa 3900
gattatcgaa caataaaatc atttgccttt ttaattaaaa acataaaaaa a 3951

<210> 161
<211> 943
<212> PRT
<213> Homo sapien

<400> 161

<210> 162
<211> 498
<212> DNA
<213> Homo sapien

<400> 162
tggagaacca cgtggacagc accatgaaca tgttgggcgg gggaggcagt gctggccgga 60
agcccctcaa gtcgggtatg aaggagctgg ccgtgttccg ggagaaggtc actgagcagc 120
accggcagat gggcaagggt ggcaagcatc accttggcct ggaggagccc aagaagctgc 180
gaccaccccc tgccaggact ccctgccaac aggaactgga ccaggtcctg gagcggatct 240
ccaccatgcg ccttccggat gagcggggcc ctctggagca cctctactcc ctgcacatcc 300
ccaactgtga caagcatggc ctgtacaacc tcaaacagtg gcaagatgtc tctgaacggg 360
cagcgtgggg agtgctggtg tgtgaacccc aacaccggga agctgatcca gggagccccc 420
accatccggg gggaccccga gtgtcatctc ttctacaatg agcagcagga ggctcgcggg 480
gtgcacaccc cagcggat 498

<210> 163
<211> 1128
<212> DNA
<213> Homo sapien

<400> 163
gccacctggc cctcctgatc gacgacacac gcacttgaaa cttgttctca gggtgtgtgg 60
aatcaacttt ccggaagcaa ccagcccacc agaggaggtc ccgagcgcga gcggagacga 120
tgcagcggag actggttcag cagtggagcg tcgcggtgtt cctgctgagc tacgcggtgc 180
cctcctgcgg gcgctcggtg gagggtctca gccgccgcct caaaagagct gtgtctgaac 240
atcagctcct ccatgacaag gggaagtcca tccaagattt acggcgacga ttcttccttc 300
accatctgat cgcagaaatc cacacagctg aaatcagagc tacctcggag gtgtccccta 360
actccaagcc ctctcccaac acaaagaacc accccgtccg atttgggtct gatgatgagg 420

gcagatacct aactcaggaa actaacaagg tggagacgta caaagagcag ccgctcaaga 480
cacctgggaa gaaaaagaaa ggcaagcccg ggaaacgcaa ggagcaggaa aagaaaaaac 540
ggcgaactcg ctctgcctgg ttagactctg gagtgactgg gagtgggcta gaaggggacc 600
acctgtctga cacctccaca acgtcgctgg agctcgattc acggaggcat tgaaattttc 660
agcagagacc ttccaaggac atattgcagg attctgtaat agtgaacata tggaaagtat 720
tagaaatatt tattgtctgt aaatactgta aatgcattgg aataaaactg tctcccccat 780
tgctctatga aactgcacat tggtcattgt gaatattttt ttttttgcca aggctaatcc 840
aattattatt atcacattta ccataattta ttttgtccat tgatgtattt attttgtaaa 900
tgtatcttgg tgctgctgaa tttctatatt ttttgtaaca taatgcactt tagatataca 960
tatcaagtat gttgataaat gacacaatga agtgtctcta ttttgtggtt gattttaatg 1020
aatgcctaaa tataattatc caaattgatt ttcctttgtg catgtaaaaa taacagtatt 1080
ttaaatttgt aaagaatgtc taataaaata taatctaatt acatcatg 1128

<210> 164
<211> 1310
<212> DNA
<213> Homo sapien

<400> 164
gggcctggtt cgcaaagaag ctgacttcag agggggaaac tttcttcttt taggaggcgg 60
ttagccctgt tccacgaacc caggagaact gctggccaga ttaattagac attgctatgg 120
gagacgtgta aacacactac ttatcattga tgcatatata aaaccatttt attttcgcta 180
ttatttcaga ggaagcgcct ctgatttgtt tcttttttcc cttttgctct tttctggctg 240
tgtggtttgg agaaagcaca gttggagtag ccggttgcta aataagtccc gagcgcgagc 300
ggagacgatg cagcggagac tggttcagca gtggagcgtc gcggtgttcc tgctgagcta 360
cgcggtgccc tcctgcgggc gctcggtgga gggtctcagc cgccgcctca aaagagctgt 420
gtctgaacat cagctcctcc atgacaaggg gaagtccatc caagatttac ggcgacgatt 480
cttccttcac catctgatcg cagaaatcca cacagctgaa atcagagcta cctcggaggt 540
gtcccctaac tccaagccct ctcccaacac aaagaaccac cccgtccgat ttgggtctga 600
tgatgagggc agatacctaa ctcaggaaac taacaaggtg gagacgtaca aagagcagcc 660
gctcaagaca cctgggaaga aaaagaaagg caagcccggg aaacgcaagg agcaggaaaa 720
gaaaaaacgg cgaactcgct ctgcctggtt agactctgga gtgactggga gtgggctaga 780
aggggaccac ctgtctgaca cctccacaac gtcgctggag ctcgattcac ggaggcattg 840
aaattttcag cagagacctt ccaaggacat attgcaggat tctgtaatag tgaacatatg 900
gaaagtatta gaaatattta ttgtctgtaa atactgtaaa tgcattggaa taaaactgtc 960
tcccccattg ctctatgaaa ctgcacattg gtcattgtga atattttttt ttttgccaag 1020
gctaatccaa ttattattat cacatttacc ataatttatt ttgtccattg atgtatttat 1080
tttgtaaatg tatcttggtg ctgctgaatt tctatatttt ttgtaacata atgcacttta 1140
gatatacata tcaagtatgt tgataaatga cacaatgaag tgtctctatt ttgtggttga 1200
ttttaatgaa tgcctaaata taattatcca aattgatttt cctttgtgcc cgtaaaaata 1260
acagtatttt aaatttgtaa agaatgtcta ataaaatata atctaattac 1310

<210> 165
<211> 177
<212> PRT
<213> Homo sapien

<400> 165

<210> 166
<211> 177
<212> PRT
<213> Homc sapien

<400> 166

<210> 167
<211> 3362
<212> DNA
<213> Homo sapien

<400> 167

cacaatgtat gcagcaggct cagtgtgagt gaactggagg cttctctaca acatgaccca 60
aaggagcatt gcaggtccta tttgcaacct gaagtttgtg actctcctgg ttgccttaag 120
ttcagaactc ccattcctgg gagctggagt acagcttcaa gacaatgggt ataatggatt 180
gctcattgca attaatcctc aggtacctga gaatcagaac ctcatctcaa acattaagga 240
aatgataact gaagcttcat tttacctatt taatgctacc aagagaagag tatttttcag 300
aaatataaag attttaatac ctgccacatg gaaagctaat aataacagca aaataaaaca 360
agaatcatat gaaaaggcaa atgtcatagt gactgactgg tatggggcac atggagatga 420
tccatacacc ctacaataca gagggtgtgg aaaagaggga aaatacattc atttcacacc 480
taatttccta ctgaatgata acttaacagc tggctacgga tcacgaggcc gagtgtttgt 540
ccatgaatgg gcccacctcc gttggggtgt gttcgatgag tataacaatg acaaaccttt 600
ctacataaat gggcaaaatc aaattaaagt gacaaggtgt tcatctgaca tcacaggcat 660
ttttgtgtgt gaaaaaggtc cttgccccca agaaaactgt attattagta agctttttaa 720
agaaggatgc acctttatct acaatagcac ccaaaatgca actgcatcaa taatgttcat 780
gcaaagttta tcttctgtgg ttgaattttg taatgcaagt acccacaacc aagaagcacc 840
aaacctacag aaccagatgt gcagcctcag aagtgcatgg gatgtaatca cagactctgc 900
tgactttcac cacagctttc ccatgaacgg gactgagctt ccacctcctc ccacattctc 960
gcttgtagag gctggtgaca aagtggtctg tttagtgctg gatgtgtcca gcaagatggc 1020
agaggctgac agactccttc aactacaaca agccgcagaa ttttatttga tgcagattgt 1080
tgaaattcat accttcgtgg gcattgccag tttcgacagc aaaggagaga tcagagccca 1140
gctacaccaa attaacagca atgatgatcg aaagttgctg gtttcatatc tgcccaccac 1200
tgtatcagct aaaacagaca tcagcatttg tttcagggct aagaaaggat ttgaggtggt 1260
tgaaaaactg aatggaaaag cttatggctc tgtgatgata ttagtgacca gcggagatga 1320
taagcttctt ggcaattgct tacccactgt gctcagcagt ggttcaacaa ttcactccat 1380
tgccctgggt tcatctgcag ccccaaatct ggaggaatta tcacgtctta caggaggttt 1440
aaagttcttt gttccagata tatcaaactc caatagcatg attgatgctt tcagtagaat 1500
ttcctctgga actggagaca ttttccagca acatattcag cttgaaagta caggtgaaaa 1560
tgtcaaacct caccatcaat tgaaaaacac agtgactgtg gataatactg tgggcaacga 1620
cactatgttt ctagttacgt ggcaggccag tggtcctcct gagattatat tatttgatcc 1680
tgatggacga aaatactaca caaataattt tatcaccaat ctaacttttc ggacagctag 1740
tctttggatt ccaggaacag ctaagcctgg gcactggact tacaccctga tgtgtttcca 1800
ccatgcaaaa ttattgacct ggaagctgta aaagtagaag aggaattgac cctatcttgg 1860
acagcacctg gagaagactt tgatcagggc caggctacaa gctatgaaat aagaatgagt 1920
aaaagtctac agaatatcca agatgacttt aacaatgcta ttttagtaaa tacatcaaag 1980
cgaaatcctc agcaagctgg catcagggag atatttacgt tctcacccca aatttccacg 2040
aatggacctg aacatcagcc aaatggagaa acacatgaaa gccacagaat ttatgttgca 2100
atacgagcaa tggataggaa ctccttacag tctgctgtat ctaacattgc ccaggcgcct 2160
ctgtttattc cccccaattc tgatcctgta cctgccagag attatcttat attgaaagga 2220
gttttaacag caatgggttt gataggaatc atttgcctta ttatagttgt gacacatcat 2280
actttaagca ggaaaaagag agcagacaag aaagagaatg gaacaaaatt attataaata 2340
aatatccaaa gtgtcttcct tcttagatat aagacccatg gccttcgact acaaaaacat 2400
actaacaaag tcaaattaac atcaaaactg tattaaaatg cattgagttt ttgtacaata 2460
cagataagat ttttacatgg tagatcaaca aattcttttt gggggtagat tagaaaaccc 2520
ttacactttg gctatgaaca aataataaaa attattcttt aaagtaatgt ctttaaaggc 2580
aaagggaagg gtaaagtcgg accagtgtca aggaaagttt gttttattga ggtggaaaaa 2640
tagccccaag cagagaaaag gagggtaggt ctgcattata actgtctgtg tgaagcaatc 2700
atttagttac tttgattaat ttttcttttc tccttatctg tgcagaacag gttgcttgtt 2760
tacaactgaa gatcatgcta tatttcatat atgaagcccc taatgcaaag ctctttacct 2820
cttgctattt tgttatatat attacagatg aaatctcact gctaatgctc agagatcttt 2880
tttcactgta agaggtaacc tttaacaata tgggtattac ctttgtctct tcataccggt 2940
tttatgacaa aggtctattg aatttatttg tttgtaagtt tctactccca tcaaagcagc 3000
tttctaagtt attgccttgg ttattatgga tgatagttat agcccttata atgccttaac 3060
taaggaagaa aagatgttat tctgagtttg ttttaataca tatatgaaca tatagtttta 3120
ttcaattaaa ccaaagaaga ggtcagcagg gagatactaa cctttggaaa tgattagctg 3180
gctctgtttt ttggttaaat aagagtcttt aatcctttct ccatcaagag ttacttacca 3240
agggcagggg aagggggata tagaggtcac aaggaaataa aaatcatctt tcatctttaa 3300

ttttactcct tcctcttatt tttttaaaag attatcgaac aataaaatca tttgcctttt 3360
tt 3362

<210> 168
<211> 2784
<212> DNA
<213> Homo sapien

<400> 168
tctgcatcca tattgaaaac ctgacacaat gtatgcagca ggctcagtgt gagtgaactg 60
gaggcttctc tacaacatga cccaaaggag cattgcaggt cctatttgca acctgaagtt 120
tgtgactctc ctggttgcct taagttcaga actcccattc ctgggagctg gagtacagct 180
tcaagacaat gggtataatg gattgctcat tgcaattaat cctcaggtac ctgagaatca 240
gaacctcatc tcaaacatta aggaaatgat aactgaagct tcattttacc tatttaatgc 300
taccaagaga agagtatttt tcagaaatat aaagatttta atacctgcca catggaaagc 360
taataataac agcaaaataa aacaagaatc atatgaaaag gcaaatgtca tagtgactga 420
ctggtatggg gcacatggag atgatccata caccctacaa tacagagggt gtggaaaaga 480
gggaaaatac attcatttca cacctaattt cctactgaat gataacttaa cagctggcta 540
cggatcacga ggccgagtgt ttgtccatga atgggcccac ctccgttggg gtgtgttcga 600
tgagtataac aatgacaaac ctttctacat aaatgggcaa aatcaaatta aagtgacaag 660
gtgttcatct gacatcacag gcatttttgt gtgtgaaaaa ggtccttgcc cccaagaaaa 720
ctgtattatt agtaagcttt ttaaagaagg atgcaccttt atctacaata gcacccaaaa 780
tgcaactgca tcaataatgt tcatgcaaag tttatcttct gtggttgaat tttgtaatgc 840
aagtacccac aaccaagaag caccaaacct acagaaccag atgtgcagcc tcagaagtgc 900
atgggatgta atcacagact ctgctgactt tcaccacagc tttcccatga acgggactga 960
gcttccacct cctcccacat tctcgcttgt agaggctggt gacaaagtgg tctgtttagt 1020
gctggatgtg tccagcaaga tggcagaggc tgacagactc cttcaactac aacaagccgc 1080
agaattttat ttgatgcaga ttgttgaaat tcataccttc gtgggcattg ccagtttcga 1140
cagcaaagga gagatcagag cccagctaca ccaaattaac agcaatgatg atcgaaagtt 1200
gctggtttca tatctgccca ccactgtatc agctaaaaca gacatcagca tttgttcagg 1260
gcttaagaaa ggatttgagg tggttgaaaa actgaatgga aaagcttatg gctctgtgat 1320
gatattagtg accagcggag atgataagct tcttggcaat tgcttaccca ctgtgctcag 1380
cagtggttca acaattcact ccattgccct gggttcatct gcagccccaa atctggagga 1440
attatcacgt cttacaggag gtttaaagtt ctttgttcca gatatatcaa actccaatag 1500
catgattgat gctttcagta gaatttcctc tggaactgga gacattttcc agcaacatat 1560
tcagcttgaa agtacaggtg aaaatgtcaa acctcaccat caattgaaaa acacagtgac 1620
tgtggataat actgtgggca acgacactat gtttctagtt acgtggcagg ccagtggtcc 1680
tcctgagatt atattatttg atcctgatgg acgaaaatac tacacaaata attttatcac 1740
caatctaact tttcggacag ctagtctttg gattccagga acagctaagc ctgggcactg 1800
gacttacacc ctgaacaata cccatcattc tctgcaagcc ctgaaagtga cagtgacctc 1860
tcgcgcctcc aactcagctg tgcccccagc cactgtggaa gcctttgtgg aaagagacag 1920
cctccatttt cctcatcctg tgatgattta tgccaatgtg aaacagggat tttatcccat 1980
tcttaatgcc actgtcactg ccacagttga gccagagact ggagatcctg ttacgctgag 2040
actccttgat gatggagcag gtgctgatgt tataaaaaat gatggaattt actcgaggta 2100
ttttttctcc tttgctgcaa atggtagata tagcttgaaa gtgcatgtca atcactctcc 2160
cagcataagc accccagccc actctattcc agggagtcat gctatgtatg taccaggtta 2220
cacagcaaac ggtaatattc agatgaatgc tccaaggaaa tcagtaggca gaaatgagga 2280
ggagcgaaag tggggcttta gccgagtcag ctcaggaggc tccttttcag tgctgggagt 2340
tccagctggc ccccaccctg atgtgtttcc accatgcaaa attattgacc tggaagctgt 2400
aaatagaaga ggaattgacc ctatcttgga cagcacctgg agaagacttt gatcagggcc 2460
aggctacaag ctatgaaata agaatgagta aaagtctaca gaatatccaa gatgacttta 2520
acaatgctat tttagtaaat acatcaaagc gaaatcctca gcaagctggc atcagggaga 2580
tatttacgtt ctcaccccaa atttccacga atggacctga acatcagcca aatggagaaa 2640
cacatgaaag ccacagaatt tatgttgcaa tacgagcaat ggataggaac tccttacagt 2700
ctgctgtatc taacattgcc caggcgcctc tgtttattcc ccccaattct gatcctgtac 2760

ctgccagaga ttatcttata ttga                                                2784

<210> 169
<211> 592
<212> PRT
<213> Homo sapien

<400> 169
Met Thr Gln Arg Ser Ile Ala Gly Pro Ile Cys Asn Leu Lys Phe Val
1               5                   10                  15
Thr Leu Leu Val Ala Leu Ser Ser Glu Leu Pro Phe Leu Gly Ala Gly
            20                  25                  30
Val Gln Leu Gln Asp Asn Gly Tyr Asn Gly Leu Leu Ile Ala Ile Asn
        35                  40                  45
Pro Gln Val Pro Glu Asn Gln Asn Leu Ile Ser Asn Ile Lys Glu Met
    50                  55                  60
Ile Thr Glu Ala Ser Phe Tyr Leu Phe Asn Ala Thr Lys Arg Arg Val
65                  70                  75                  80
Phe Phe Arg Asn Ile Lys Ile Leu Ile Pro Ala Thr Trp Lys Ala Asn
                85                  90                  95
Asn Asn Ser Lys Ile Lys Gln Glu Ser Tyr Glu Lys Ala Asn Val Ile
            100                 105                 110
Val Thr Asp Trp Tyr Gly Ala His Gly Asp Asp Pro Tyr Thr Leu Gln
        115                 120                 125
Tyr Arg Gly Cys Gly Lys Glu Gly Lys Tyr Ile His Phe Thr Pro Asn
    130                 135                 140
Phe Leu Leu Asn Asp Asn Leu Thr Ala Gly Tyr Gly Ser Arg Gly Arg
145                 150                 155                 160
Val Phe Val His Glu Trp Ala His Leu Arg Trp Gly Val Phe Asp Glu
                165                 170                 175
Tyr Asn Asn Asp Lys Pro Phe Tyr Ile Asn Gly Gln Asn Gln Ile Lys
            180                 185                 190
Val Thr Arg Cys Ser Ser Asp Ile Thr Gly Ile Phe Val Cys Glu Lys
        195                 200                 205
Gly Pro Cys Pro Gln Glu Asn Cys Ile Ile Ser Lys Leu Phe Lys Glu
    210                 215                 220
Gly Cys Thr Phe Ile Tyr Asn Ser Thr Gln Asn Ala Thr Ala Ser Ile
225                 230                 235                 240
Met Phe Met Gln Ser Leu Ser Ser Val Val Glu Phe Cys Asn Ala Ser
                245                 250                 255
Thr His Asn Gln Glu Ala Pro Asn Leu Gln Asn Gln Met Cys Ser Leu
            260                 265                 270
Arg Ser Ala Trp Asp Val Ile Thr Asp Ser Ala Asp Phe His His Ser
        275                 280                 285
Phe Pro Met Asn Gly Thr Glu Leu Pro Pro Pro Pro Thr Phe Ser Leu
    290                 295                 300
Val Glu Ala Gly Asp Lys Val Val Cys Leu Val Leu Asp Val Ser Ser
305                 310                 315                 320
Lys Met Ala Glu Ala Asp Arg Leu Leu Gln Leu Gln Gln Ala Ala Glu
                325                 330                 335
Phe Tyr Leu Met Gln Ile Val Glu Ile His Thr Phe Val Gly Ile Ala
            340                 345                 350
Ser Phe Asp Ser Lys Gly Glu Ile Arg Ala Gln Leu His Gln Ile Asn
        355                 360                 365
Ser Asn Asp Asp Arg Lys Leu Leu Val Ser Tyr Leu Pro Thr Thr Val

370                 375                 380
Ser Ala Lys Thr Asp Ile Ser Ile Cys Ser Gly Leu Lys Lys Gly Phe
385                 390                 395                 400
Glu Val Val Glu Lys Leu Asn Gly Lys Ala Tyr Gly Ser Val Met Ile
                405                 410                 415
Leu Val Thr Ser Gly Asp Asp Lys Leu Leu Gly Asn Cys Leu Pro Thr
            420                 425                 430
Val Leu Ser Ser Gly Ser Thr Ile His Ser Ile Ala Leu Gly Ser Ser
        435                 440                 445
Ala Ala Pro Asn Leu Glu Glu Leu Ser Arg Leu Thr Gly Gly Leu Lys
    450                 455                 460
Phe Phe Val Pro Asp Ile Ser Asn Ser Asn Ser Met Ile Asp Ala Phe
465                 470                 475                 480
Ser Arg Ile Ser Ser Gly Thr Gly Asp Ile Phe Gln Gln His Ile Gln
                485                 490                 495
Leu Glu Ser Thr Gly Glu Asn Val Lys Pro His His Gln Leu Lys Asn
            500                 505                 510
Thr Val Thr Val Asp Asn Thr Val Gly Asn Asp Thr Met Phe Leu Val
        515                 520                 525
Thr Trp Gln Ala Ser Gly Pro Pro Glu Ile Ile Leu Phe Asp Pro Asp
    530                 535                 540
Gly Arg Lys Tyr Tyr Thr Asn Asn Phe Ile Thr Asn Leu Thr Phe Arg
545                 550                 555                 560
Thr Ala Ser Leu Trp Ile Pro Gly Thr Ala Lys Pro Gly His Trp Thr
                565                 570                 575
Tyr Thr Leu Met Cys Phe His His Ala Lys Leu Leu Thr Trp Lys Leu
            580                 585                 590

<210> 170
<211> 791
<212> PRT
<213> Homo sapien

<400> 170
Met Thr Gln Arg Ser Ile Ala Gly Pro Ile Cys Asn Leu Lys Phe Val
1               5                   10                  15
Thr Leu Leu Val Ala Leu Ser Ser Glu Leu Pro Phe Leu Gly Ala Gly
            20                  25                  30
Val Gln Leu Gln Asp Asn Gly Tyr Asn Gly Leu Leu Ile Ala Ile Asn
        35                  40                  45
Pro Gln Val Pro Glu Asn Gln Asn Leu Ile Ser Asn Ile Lys Glu Met
    50                  55                  60
Ile Thr Glu Ala Ser Phe Tyr Leu Phe Asn Ala Thr Lys Arg Arg Val
65                  70                  75                  80
Phe Phe Arg Asn Ile Lys Ile Leu Ile Pro Ala Thr Trp Lys Ala Asn
                85                  90                  95
Asn Asn Ser Lys Ile Lys Gln Glu Ser Tyr Glu Lys Ala Asn Val Ile
            100                 105                 110
Val Thr Asp Trp Tyr Gly Ala His Gly Asp Asp Pro Tyr Thr Leu Gln
        115                 120                 125
Tyr Arg Gly Cys Gly Lys Glu Gly Lys Tyr Ile His Phe Thr Pro Asn
    130                 135                 140
Phe Leu Leu Asn Asp Asn Leu Thr Ala Gly Tyr Gly Ser Arg Gly Arg
145                 150                 155                 160
Val Phe Val His Glu Trp Ala His Leu Arg Trp Gly Val Phe Asp Glu

                165                 170                 175
Tyr Asn Asn Asp Lys Pro Phe Tyr Ile Asn Gly Gln Asn Gln Ile Lys
            180                 185                 190
Val Thr Arg Cys Ser Ser Asp Ile Thr Gly Ile Phe Val Cys Glu Lys
        195                 200                 205
Gly Pro Cys Pro Gln Glu Asn Cys Ile Ile Ser Lys Leu Phe Lys Glu
    210                 215                 220
Gly Cys Thr Phe Ile Tyr Asn Ser Thr Gln Asn Ala Thr Ala Ser Ile
225                 230                 235                 240
Met Phe Met Gln Ser Leu Ser Ser Val Val Glu Phe Cys Asn Ala Ser
                245                 250                 255
Thr His Asn Gln Glu Ala Pro Asn Leu Gln Asn Gln Met Cys Ser Leu
            260                 265                 270
Arg Ser Ala Trp Asp Val Ile Thr Asp Ser Ala Asp Phe His His Ser
        275                 280                 285
Phe Pro Met Asn Gly Thr Glu Leu Pro Pro Pro Pro Thr Phe Ser Leu
    290                 295                 300
Val Glu Ala Gly Asp Lys Val Val Cys Leu Val Leu Asp Val Ser Ser
305                 310                 315                 320
Lys Met Ala Glu Ala Asp Arg Leu Leu Gln Leu Gln Gln Ala Ala Glu
                325                 330                 335
Phe Tyr Leu Met Gln Ile Val Glu Ile His Thr Phe Val Gly Ile Ala
            340                 345                 350
Ser Phe Asp Ser Lys Gly Glu Ile Arg Ala Gln Leu His Gln Ile Asn
        355                 360                 365
Ser Asn Asp Asp Arg Lys Leu Leu Val Ser Tyr Leu Pro Thr Thr Val
    370                 375                 380
Ser Ala Lys Thr Asp Ile Ser Ile Cys Ser Gly Leu Lys Lys Gly Phe
385                 390                 395                 400
Glu Val Val Glu Lys Leu Asn Gly Lys Ala Tyr Gly Ser Val Met Ile
                405                 410                 415
Leu Val Thr Ser Gly Asp Asp Lys Leu Leu Gly Asn Cys Leu Pro Thr
            420                 425                 430
Val Leu Ser Ser Gly Ser Thr Ile His Ser Ile Ala Leu Gly Ser Ser
        435                 440                 445
Ala Ala Pro Asn Leu Glu Glu Leu Ser Arg Leu Thr Gly Gly Leu Lys
    450                 455                 460
Phe Phe Val Pro Asp Ile Ser Asn Ser Asn Ser Met Ile Asp Ala Phe
465                 470                 475                 480
Ser Arg Ile Ser Ser Gly Thr Gly Asp Ile Phe Gln Gln His Ile Gln
                485                 490                 495
Leu Glu Ser Thr Gly Glu Asn Val Lys Pro His His Gln Leu Lys Asn
            500                 505                 510
Thr Val Thr Val Asp Asn Thr Val Gly Asn Asp Thr Met Phe Leu Val
        515                 520                 525
Thr Trp Gln Ala Ser Gly Pro Pro Glu Ile Ile Leu Phe Asp Pro Asp
    530                 535                 540
Gly Arg Lys Tyr Tyr Thr Asn Asn Phe Ile Thr Asn Leu Thr Phe Arg
545                 550                 555                 560
Thr Ala Ser Leu Trp Ile Pro Gly Thr Ala Lys Pro Gly His Trp Thr
                565                 570                 575
Tyr Thr Leu Asn Asn Thr His His Ser Leu Gln Ala Leu Lys Val Thr
            580                 585                 590
Val Thr Ser Arg Ala Ser Asn Ser Ala Val Pro Pro Ala Thr Val Glu
        595                 600                 605

Ala Phe Val Glu Arg Asp Ser Leu His Phe Pro His Pro Val Met Ile
    610                 615                 620
Tyr Ala Asn Val Lys Gln Gly Phe Tyr Pro Ile Leu Asn Ala Thr Val
625                 630                 635                 640
Thr Ala Thr Val Glu Pro Glu Thr Gly Asp Pro Val Thr Leu Arg Leu
                645                 650                 655
Leu Asp Asp Gly Ala Gly Ala Asp Val Ile Lys Asn Asp Gly Ile Tyr
            660                 665                 670
Ser Arg Tyr Phe Phe Ser Phe Ala Ala Asn Gly Arg Tyr Ser Leu Lys
        675                 680                 685
Val His Val Asn His Ser Pro Ser Ile Ser Thr Pro Ala His Ser Ile
    690                 695                 700
Pro Gly Ser His Ala Met Tyr Val Pro Gly Tyr Thr Ala Asn Gly Asn
705                 710                 715                 720
Ile Gln Met Asn Ala Pro Arg Lys Ser Val Gly Arg Asn Glu Glu Glu
                725                 730                 735
Arg Lys Trp Gly Phe Ser Arg Val Ser Ser Gly Gly Ser Phe Ser Val
            740                 745                 750
Leu Gly Val Pro Ala Gly Pro His Pro Asp Val Phe Pro Pro Cys Lys
        755                 760                 765
Ile Ile Asp Leu Glu Ala Val Asn Arg Arg Gly Ile Asp Pro Ile Leu
    770                 775                 780
Asp Ser Thr Trp Arg Arg Leu
785                 790

<210> 171
<211> 1491
<212> DNA
<213> Homo sapien

<400> 171
cctcctgcca gccaagtgaa gacatgctta cttccccttc accttccttc atgatgtggg      60
aagagtgctg caacccagcc ctagccaacg ccgcatgaga gggagtgtgc cgagggcttc     120
tgagaaggtt tctctcacat ctagaaagaa gcgcttaaga tgtggcagcc cctcttcttc     180
aagtggctct tgtcctgttg ccctgggagt tctcaaattg ctgcagcagc ctccacccag     240
cctgaggatg acatcaatac acagaggaag aagagtcagg aaaagatgag agaagttaca     300
gactctcctg ggcgaccccg agagcttacc attcctcaga cttcttcaca tggtgctaac     360
agatttgttc ctaaaagtaa agctctagag gccgtcaaat tggcaataga agccgggttc     420
caccatattg attctgcaca tgtttacaat aatgaggagc aggttggact ggccatccga     480
agcaagattg cagatggcag tgtgaagaga gaagacatat tctacacttc aaagctttgg     540
agcaattccc atcgaccaga gttggtccga ccagccttgg aaaggtcact gaaaaatctt     600
caattggact atgttgacct ctatcttatt cattttccag tgtctgtaaa gccaggtgag     660
gaagtgatcc caaaagatga aaatggaaaa atactatttg acacagtgga tctctgtgcc     720
acatggaggg ccatggagaa gtgtaaagat gcaggattgg ccaagtccat cggggtgtcc     780
aacttcaacc acaggctgct ggagatgatc ctcaacaagc cagggctcaa gtacaagcct     840
gtctgcaacc aggtggaatg tcatccttac ttcaaccaga gaaaactgct ggatttctgc     900
aagtcaaaag acattgttct ggttgcctat agtgctctgg gatcccatcg agaagaacca     960
tgggtggacc cgaactcccc ggtgctcttg gaggacccag tcctttgtgc cttggcaaaa    1020
aagcacaagc gaaccccagc cctgattgcc ctgcgctacc agctgcagcg tggggttgtg    1080
gtcctggcca agagctacaa tgagcagcgc atcagacaga acgtgcaggt gtttgaattc    1140
cagttgactt cagaggagat gaaagccata gatggcctaa acagaaatgt gcgatatttg    1200
acccttgata tttttgctgg cccccctaat tatccatttt ctgatgaata ttaacatgga    1260
gggcattgca tgaggtctgc cagaaggccc tgcgtgtgga tggtgacaca gaggatggct    1320
ctatgctggt gactggacac atcgcctctg gttaaatctc tcctgcttgg cgacttcagt    1380
aagctacagc taagcccatc ggccggaaaa gaaagacaat aattttgttt ttcattttga    1440

aaaaattaaa tgctctctcc taaagattct tcacctaaaa aaaaaaaaaa a 1491

<210> 172
<211> 364
<212> PRT
<213> Homo sapien

<400> 172
Met Trp Gln Pro Leu Phe Phe Lys Trp Leu Leu Ser Cys Cys Pro Gly
1               5                   10                  15
Ser Ser Gln Ile Ala Ala Ala Ala Ser Thr Gln Pro Glu Asp Asp Ile
            20                  25                  30
Asn Thr Gln Arg Lys Lys Ser Gln Glu Lys Met Arg Glu Val Thr Asp
        35                  40                  45
Ser Pro Gly Arg Pro Arg Glu Leu Thr Ile Pro Gln Thr Ser Ser His
    50                  55                  60
Gly Ala Asn Arg Phe Val Pro Lys Ser Lys Ala Leu Glu Ala Val Lys
65                  70                  75                  80
Leu Ala Ile Glu Ala Gly Phe His His Ile Asp Ser Ala His Val Tyr
                85                  90                  95
Asn Asn Glu Glu Gln Val Gly Leu Ala Ile Arg Ser Lys Ile Ala Asp
            100                 105                 110
Gly Ser Val Lys Arg Glu Asp Ile Phe Tyr Thr Ser Lys Leu Trp Ser
        115                 120                 125
Asn Ser His Arg Pro Glu Leu Val Arg Pro Ala Leu Glu Arg Ser Leu
    130                 135                 140
Lys Asn Leu Gln Leu Asp Tyr Val Asp Leu Tyr Leu Ile His Phe Pro
145                 150                 155                 160
Val Ser Val Lys Pro Gly Glu Glu Val Ile Pro Lys Asp Glu Asn Gly
                165                 170                 175
Lys Ile Leu Phe Asp Thr Val Asp Leu Cys Ala Thr Trp Glu Ala Met
            180                 185                 190
Glu Lys Cys Lys Asp Ala Gly Leu Ala Lys Ser Ile Gly Val Ser Asn
        195                 200                 205
Phe Asn His Arg Leu Leu Glu Met Ile Leu Asn Lys Pro Gly Leu Lys
    210                 215                 220
Tyr Lys Pro Val Cys Asn Gln Val Glu Cys His Pro Tyr Phe Asn Gln
225                 230                 235                 240
Arg Lys Leu Leu Asp Phe Cys Lys Ser Lys Asp Ile Val Leu Val Ala
                245                 250                 255
Tyr Ser Ala Leu Gly Ser His Arg Glu Glu Pro Trp Val Asp Pro Asn
            260                 265                 270
Ser Pro Val Leu Leu Glu Asp Pro Val Leu Cys Ala Leu Ala Lys Lys
        275                 280                 285
His Lys Arg Thr Pro Ala Leu Ile Ala Leu Arg Tyr Gln Leu Gln Arg
    290                 295                 300
Gly Val Val Val Leu Ala Lys Ser Tyr Asn Glu Gln Arg Ile Arg Gln
305                 310                 315                 320
Asn Val Gln Val Phe Glu Phe Gln Leu Thr Ser Glu Glu Met Lys Ala
                325                 330                 335
Ile Asp Gly Leu Asn Arg Asn Val Arg Tyr Leu Thr Leu Asp Ile Phe
            340                 345                 350
Ala Gly Pro Pro Asn Tyr Pro Phe Ser Asp Glu Tyr
        355                 360